# EUROPEAN PATENT APPLICATION

(11) **EP 2 955 234 A1**
(43) Date of publication of application: **16.12.2015**
(21) Application number: 15163893.9
(22) Date of filing: 11.02.2010
(51) Int. Cl.: C12Q 1/68

(54) **COMBINATIONS OF POLYMORPHISMS FOR DETERMINING ALLELE-SPECIFIC EXPRESSION OF IGF2**

(30) Priority: 11.02.2009 US 207450 P
(62) Divisional of application: 10741738.8
(71) Applicant: Orion Genomics, LLC, St. Louis, MO 63108 (US)
(72) Inventor: Ordway, Jared, St. Louis, MO 63110 (US); Maloney, Rebecca, Centreville, VA 20120 (US); Lakey, Nathan, Chesterfield, MO 63005 (US); Budiman, Muhammad A., Manchester, MO 63124 (US)
(74) Representative: Campbell, Patrick John Henry

(57) **Abstract**

Combinations of SNPs and reagents for detecting such SNPs, as well as methods of detecting IGF loss-of-imprinting as provided.

## Description

### CROSS-REFERENCE TO RELATED APPLICATIONS

This application claims benefit of U.S. provisional application no. 61/207,450, filed February 11, 2009, which application is herein incorporated by reference.

### BACKGROUND OF THE INVENTION

The gene for insulin-like growth factor 2, or *IGF2,* is located in a cluster of imprinted genes on human chromosome 11p15.5. Genomic imprinting is an important mechanism of gene regulation where one copy of the gene is normally expressed and the other copy is silenced through an epigenetic mark of parental origin. *IGF2* is normally maternally imprinted in human tissues and therefore, expressed only from the paternally inherited copy of the gene (DeChiara TM, et al. Cell 64, 849-859 (1991); Rainier S, et al., Nature 362, 747-749 (1993); Ogawa, et al, Nature 362, 749-751 (1993)). Loss of imprinting of *IGF2* (referred to as loss of imprinting, or LOI) has been strongly linked to several cancer types (over 20 tumor types reviewed in Falls, et al. 1999, AJP 154, 635-647). Furthermore, mounting evidence indicates that individuals displaying LOI of *IGF2* may be at elevated risk for developing colorectal cancer (Kinochi et al., 1996, Cancer Letters 107, 105-108 (1996); Nishihara S. 2000, Int. Jour. Oncol. 17, 317-322; Cui H 1998, Nature Medicine 4-11, 1276-1280; Nakagawa H 2001, PNAS 98-2, 591-596). LOI *of IGF2* can be detected in normal tissues of cancer patients including peripheral blood and normal colonic mucosa (Kinochi et al., 1996, Cancer Letters 107, 105-108 (1996); Ogawa, et al, Nature Genetics 5, 408-412 (1993); Cui H, Science 299, 1753 (2003)) and in the normal tissues of people believed to be cancer free (Cui H, et al. Nature Medicine 4-11, 1276-1280 (1998); Cui H, Science 299,1753 (2003); Woodson K et al., JNCI 96, 407-410 (2004); Cruz-Correa Met al., Gastroenterology 126, 964-970 (2004)).

Several studies of peripheral blood of general populations report that between 7-10% of people display loss of imprinting of *IGF2* in colonic mucosa tissue. Three retrospective studies report that the odds of colorectal cancer patients displaying LOI of *IGF2* in either peripheral blood or colonic mucosa are significantly higher (between 2-21 fold) than the odds of an age matched cancer free control group displaying LOI. These studies suggest that LOI of *IGF2* may predispose otherwise healthy individuals to colorectal cancer. Therefore, a risk test based on the detection of LOI of IGF2 may have a future clinical benefit, (Cui H, et al. Nature Medicine 4-11, 1276-1280 (1998); Cui H, Science 14, 1753-1755 (2003); Woodson K 2004, JNCI 96, 407-410; Cruz-Correa M, Gastroenterology 126, 964-970 (2004)). These studies show that people with LOI *of IGF2* (also referred to as the *IGF2* biomarker) may be up to 20 times more likely to develop colorectal cancer than individuals without the *IGF2* biomarker.

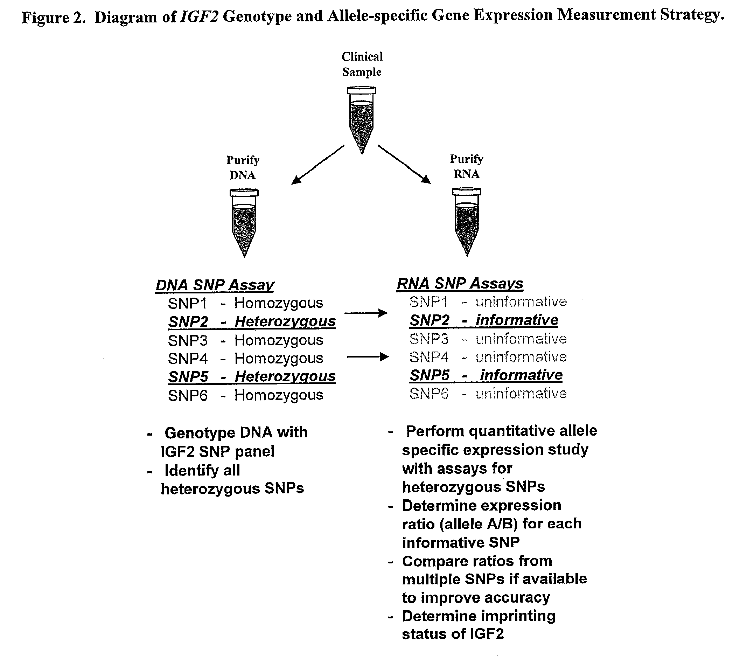

Detection of LOI of *IGF2* is based on a quantitative allele specific gene expression assay, where transcripts from both copies of the *IGF2* gene are each quantified. The quantities are then compared to one another to determine an allelic gene expression ratio, which is subsequently compared to a threshold value. If the concentration of the lesser abundant allele is "relatively similar" to the concentration of the more abundant allele, then the *IGF2* imprint is determined to be lost. If the concentration of the lesser abundant allele is "relatively dissimilar" to the concentration of the more abundant allele, then the *IGF2* imprint is determined to be present. One method of measuring the imprinting status of *IGF2* in a sample is to first determine the genotype(s) of one or more polymorphic sites in the transcribed region of the *IGF2* gene. Heterozygous markers in the transcribed region of the gene provide for convenient molecular handles by which the individual alleles of the *IGF2* gene can be distinguished from one another in a sample. RNA transcription from each of the two copies of the *IGF2* gene may be independently measured with quantitative allele specific assays. Comparison of the amount of expression of one allele to the amount of expression of the other allele can therefore be made and the imprinting status of the *IGF2* gene can be determined (see Figure 2).

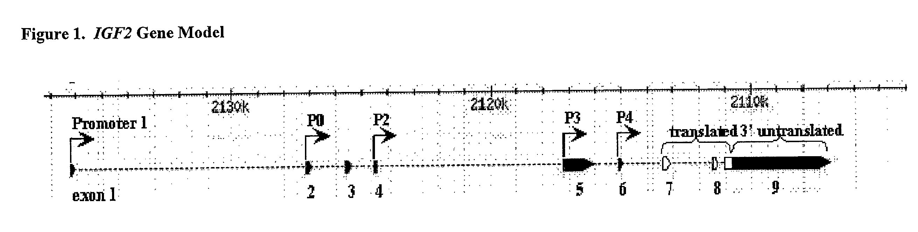

*IGF2* has four promoters, each driving expression of alternatively spliced transcripts, in a tissue specific manner (Figure 1). Exons 7, 8, and 9 are present in all transcripts, while exons 1-6 have been reported to be expressed in a promoter specific fashion. Exon 9 includes a short stretch of protein-coding sequence followed by a considerably longer 3' UTR. Polymorphic markers in exons 7, 8, and 9 are therefore useful in the determination of *IGF2* imprinting status by enabling the detection of allele specific expression of IGF2 transcription driven from any of the four *IGF2* promoters.

Four allele-specific expression assays measuring *IGF2* imprinting status are known to those skilled in the art. Woodson, *et al.* measured imprinting status of *IGF2* with a combination of two SNP based assays (rs680 and rs2230949) (Woodson K 2004, JNCI 96, 407-410). Both SNPs are in exon 9 of IGF2 but are reported by Woodson *et al.* to be in minimal linkage disequilibrium. Therefore attempts to measure LOI of an individual with such a combination of markers increases the probability that the individual will be heterozygous for at least one of the two SNPs, and thereby increase the likelihood that the LOI status of the individual can be determined. The authors demonstrated that the first SNP, the second SNP, or both SNPs were informative (*i.e.,* were heterozygous and, therefore, permitted measurement of LOI of *IGF2*) in 48 of 106 patients evaluated (or 45%). Cui *et al.* measured *IGF2* imprinting with a combination of two assays, one targeting a SNP (rs680) and a second measuring restriction fragment length polymorphisms of a simple sequence repeat within exon 9 of IGF2. The authors demonstrated that the SNP, the repeat, or both markers were informative in 191 of 421 (or 45%) patients evaluated (Cui H, et al. Nature Medicine 4-11, 1276-1280 (1998)).

Previous studies have demonstrated that use of these polymorphisms result in a low combined frequency of heterozygosity in patient populations and, therefore, a large number of individuals in these populations were "uninformative" such that their *IGF2* imprinting status could not be determined. The present application describes newly discovered SNPs in *IGF2* exon 9, and the discovery of useful combinations of SNPs, which enable successful LOI measurements in an increased proportion of the human population. The ability to measure LOI using these polymorphisms in the general population will have a profound medical benefit, serving as the basis for various molecular diagnostic and therapeutic tests.

The informativity of a given SNP for detection of LOI is based on the frequency of heterozygosity of the SNP within a population. Furthermore, the optimal informativity of a combination of different SNPs is dependent upon the linkage among the different markers. For example, if two SNPs fall within a common haplotype block, the combined use of the two SNPs provides a minimal increase in informativity relative to the use of either of the two SNPs alone. However, if two SNPs are not on the same haplotype block (i.e., are in minimal linkage disequilibrium), the combined use of the two SNPs provides an effective increase in informativity relative to the use of either of the two SNPs alone.

### BRIEF SUMMARY OF THE INVENTION

The present invention provides methods of determining loss-of-imprinting in the Insulin Growth Factor-2 (IGF2) gene of an individual. In some embodiments, the method comprises,
detecting the SNP genotype of the IGF2 gene in the individual, wherein the genotype of at least three SNPs selected from the group consisting of SEQ ID NOs: 1, 2, 3, 4, 5, 6, 7, 8, 9, 10, 11, 12, 13, 14, 15, and 16 is determined, thereby determining whether the individual is heterozygous or homozygous at each of the at least three SNPs;
quantifying in a sample from the individual the amount of RNA comprising two polymorphic options of at least one heterozygous SNP;
determining a ratio of the amount of RNA comprising two polymorphic options of at least one heterozygous SNP; and
correlating the RNA ratio to loss of imprinting of the IGF2 gene.

In some embodiments, at least one SNP in the detecting step is selected from the group consisting of SEQ ID NO: 1, 2, 3, 4, 5, and 6.

In some embodiments, the correlating step further comprises correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.

In some embodiments, at least two of the detected SNPs are heterozygous and the quantifying step comprises quantifying the amount of RNA comprising two polymorphic options at the at least two heterozygous SNPs.

In some embodiments, the sample is a blood, stool, cell scrape or tissue sample.

In some embodiments, the RNA is reverse transcribed into cDNA and the quantity of cDNA comprising each polymorphic option is used to determine the amount of RNA comprising the two polymorphic options. In some embodiments, the amount of allele-specific cDNA is quantified in a method comprising contacting the cDNA with at least one allele-specific detection polynucleotide. In some embodiments, the method comprises contacting the cDNA with a sufficient number of allele-specific detection polynucleotides such that the polymorphic option for each heterozygous SNP from the group is determined. In some embodiments, the method comprises contacting the cDNA with a number of different allele-specific detection polynucleotides, wherein the number is equal to or greater than the number of heterozygous SNPs in the group such that at least one different allele-specific detection polynucleotide is used to detect each different heterozygous SNP. In some embodiments, the at least one allele-specific detection polynucleotide is hybridized to the cDNA and extended in a template-dependent manner through the polymorphic position of the SNP in the cDNA.

In some embodiments, the at least one allele-specific detection polynucleotide comprises at least 8 (e.g., at least 10, 12, 15) contiguous nucleotides at the 3' end of the allele-specific detection polynucleotide, wherein the at least 8 contiguous nucleotides are either:
100% complementary to at least 8 (e.g., at least 10, 12, 15) nucleotides directly 3' (or is 2 or 3 nucleotides 3') of the polymorphic position of the SNP region set forth in the SEQ ID NO:; or
100% identical to at least 8 (e.g., at least 10, 12, 15) nucleotides directly 5' (or is 2 or 3 nucleotides 5') of the polymorphic position of the SNP region set forth in the SEQ ID NO: [or, e.g., 100% identical to at least 8 (e.g., at least 10, 12, 15, 20) nucleotides directly 3' (or is 2 or 3 nucleotides 3') of the polymorphic position of the complement of the SNP region].

In some embodiments, the at least one allele-specific detection polynucleotide comprises a sequence 100% identical to at least 8 (e.g., at least 10, 12, 15) contiguous nucleotides, or the complement thereof, of at least one heterozygous SNP, and the sequence consists of at least 8 (e.g., at least 10, 12, 15) contiguous nucleotides, or the complement thereof, of the SNP, wherein one of the positions of the sequence corresponds to the variable position of the SNP.

In some embodiments, the detecting step comprises detecting the SNP genotype of each polymorphic option of each of:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the detecting step comprises detecting at least 4 (e.g., 4, 5, 6, 7, 8, 9, 10, 11, 12, 13, 14, 15) SNPs selected from the group.

In some embodiments, the detecting step comprises contacting the sample with a first, second and third polynucleotides as described herein (e.g., below with regard to reaction mixtures or kits).

In some embodiments, the method further comprises providing a risk classification of cancer, diagnosis of cancer, or prognosis based on the correlating step, and/or submitting the individual to a disease screening procedure (e.g., colonoscopy) based on the correlating step, and/or submitting a sample from the individual to further diagnostic or prognostics assays based on the correlating step and/or changing a drug or other treatment regimen of the individual based on the correlating step.

The present invention also provides reaction mixtures. In some embodiments, the reaction mixture comprises,
a first polynucleotide of between 8-100 nucleotides, wherein the first polynucleotide distinguishes between one polymorphic option of a first SNP (or complement thereof) and the other polymorphic option of the first SNP (or complement thereof) in a hybridization reaction,
a second polynucleotide of between 8-100 nucleotides, wherein the second polynucleotide distinguishes between one polymorphic option of a second SNP (or complement thereof) and the other polymorphic option of the second SNP (or complement thereof) in a hybridization reaction, and
a third polynucleotide of between 8-100 nucleotides, wherein the third polynucleotide distinguishes between one polymorphic option of a third SNP (or complement thereof) and the other polymorphic option of the third SNP (or complement thereof) in a hybridization reaction, wherein the first and second and third SNP is selected from the group consisting of SEQ ID NOs:1, 2, 3, 4, 5, 6, 7, 8, 9, 10, 11, 12, 13, 14, 15, and 16, wherein at least one SNP is 1, 2, 3, 4, 5, or 6.

In some embodiments, at least one, at least two, or three of the first, second, and third polynucleotide comprise a sequence 100% identical to at least 8 (e.g., at least 10, 12, 15, 20) contiguous nucleotides, or the complement thereof, of the SEQ ID NO: corresponding to the first and second and third SNP, respectively.

In some embodiments, the sequence consists of at least 8 (e.g., at least 10, 12, 15, 20) contiguous nucleotides, or the complement thereof, of the SNP, wherein one of the positions of the sequence corresponds to the variable position of the SNP.

In some embodiments, the reaction mixture further comprises a sample comprising nucleic acids from a human.

In some embodiments, the reaction mixture further comprises a polymerase.

In some embodiments, the polymerase is a thermostable polymerase.

In some embodiments, at least one of the first and second and third polynucleotides are detectably labeled. In some embodiments, the detectable label is a fluorescent label. In some embodiments, the first and second and third polynucleotides are detectably labeled with different labels such that the different labels can be differentially detected based on different wavelength of fluorescence of the labels or different mass of the labels.

In some embodiments, the reaction mixture comprises a plurality of different allele-specific detection polynucleotide of between 8-100 nucleotides, such that at least one polynucleotide of the plurality distinguishes between one allele of a SNP (or complement thereof) and the other allele of the SNP (or complement thereof) in a hybridization reaction for each of:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the reaction mixture comprises
a first allele-specific detection polynucleotide of between 8-100 nucleotides,
a second allele-specific detection polynucleotide of between 8-100 nucleotides,
a third allele-specific detection polynucleotide of between 8-100 nucleotides,
wherein each of the first, second, and third polynucleotides comprise at least 8 (e.g., at least 10, 12, 15, 20) contiguous nucleotides at the 3' end of the allele-specific detection polynucleotide, wherein the at least 8 contiguous nucleotides are either:
100% complementary to at least 8 (e.g., at least 10, 12, 15, 20) nucleotides directly 3' (or is 2 or 3 nucleotides 3') of the polymorphic position of a SNP region; or
100% identical to at least 8 (e.g., at least 10, 12, 15,20) nucleotides directly 5' (or is 2 or 3 nucleotides 5') of the polymorphic position of a SNP region,
wherein the SNP region corresponding to each of the first, second, and third polynucleotides is different and the SNP regions are selected from the group consisting of SEQ ID NOs:1, 2, 3, 4, 5, 6, 7, 8, 9, 10, 11, 12, 13, 14, 15, and 16, wherein at least one SNP region is SEQ ID NO:1, 2, 3, 4, 5, or 6.

In some embodiments, the reaction mixture further comprises a sample comprising nucleic acids from a human.

In some embodiments, the reaction mixture further comprises a polymerase. In some embodiments, the polymerase is a thermostable polymerase.

In some embodiments, at least one of the first and second and third polynucleotides are detectably labeled. In some embodiments, the detectable label is a fluorescent label. In some embodiments, the first and second and third polynucleotides are detectably labeled with different labels such that the different labels can be differentially detected based on different wavelength of fluorescence of the labels or different mass of the labels.

In some embodiments, the reaction mixture comprises a plurality of different allele-specific detection polynucleotides, wherein each of the plurality of polynucleotides comprise at least 8 (e.g., at least 10, 12, 15, 20) contiguous nucleotides at the 3' end of the allele-specific detection polynucleotide, wherein the at least 8 contiguous nucleotides are either:
100% complementary to at least 8 (e.g., at least 10, 12, 15, 20) nucleotides directly 3' (or is 2 or 3 nucleotides 3') of the polymorphic position of a SNP region; or
100% identical to at least 8 (e.g., at least 10, 12, 15, 20) nucleotides directly 5' (or is 2 or 3 nucleotides 5') of the polymorphic position of a SNP region,
wherein the plurality includes a sufficient number of polynucleotides such at least one polynucleotide corresponds to each of the following SNP regions:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

The present invention also provides kits useful for performing the methods of the invention. In some embodiments, the kit comprises
a first polynucleotide of between 8-100 nucleotides, wherein the first polynucleotide distinguishes between one polymorphic option of a first SNP (or complement thereof) and the other polymorphic option of the first SNP (or complement thereof) in a hybridization reaction,
a second polynucleotide of between 8-100 nucleotides, wherein the second polynucleotide distinguishes between one polymorphic option of a second SNP (or complement thereof) and the other polymorphic option of the second SNP (or complement thereof) in a hybridization reaction,
a third polynucleotide of between 8-100 nucleotides, wherein the third polynucleotide distinguishes between one polymorphic option of a third SNP (or complement thereof) and the other polymorphic option of the third SNP (or complement thereof) in a hybridization reaction, wherein the first and second and third SNP is selected from the group consisting of SEQ ID NOs:1, 2, 3, 4, 5, 6, 7, 8, 9, 10, 11, 12, 13, 14, 15, and 16, wherein at least one SNP is selected from SEQ ID NO:1, 2, 3, 4, 5, or 6.

In some embodiments, at least one, at least two, or three of the first, second, and third polynucleotide comprise a sequence 100% identical to at least 8 (e.g., at least 10, 12, 15, 20) contiguous nucleotides, or the complement thereof, of the SEQ ID NO: corresponding to the first and second and third SNP, respectively.

In some embodiments, the sequence consists of at least 8 (e.g., at least 10, 12, 15, 20) contiguous nucleotides, or the complement thereof, of the SNP, wherein one of the positions of the sequence corresponds to the variable position of the SNP.

In some embodiments, the kit further comprises a polymerase. In some embodiments, the polymerase is a thermostable polymerase.

In some embodiments, at least one of the first and second and third polynucleotides are detectably labeled. In some embodiments, the detectable label is a fluorescent label. In some embodiments, the first and second and third polynucleotides are detectably labeled with different labels such that the different labels can be differentially detected based on different wavelength of fluorescence of the labels or different mass of the labels.

In some embodiments, the kit comprises a plurality of different allele-specific detection polynucleotide of between 8-100 nucleotides, such that at least one polynucleotide of the plurality distinguishes between one allele of a SNP (or complement thereof) and the other allele of the SNP (or complement thereof) in a hybridization reaction for each of:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the kit comprises:
a first allele-specific detection polynucleotide of between 8-100 nucleotides,
a second allele-specific detection polynucleotide of between 8-100 nucleotides,
a third allele-specific detection polynucleotide of between 8-100 nucleotides,
wherein each of the first, second, and third polynucleotides comprise at least 8 (e.g., at least 10, 12, 15, 20) contiguous nucleotides at the 3' end of the allele-specific detection polynucleotide, wherein the at least 8 contiguous nucleotides are either:
100% complementary to at least 8 (e.g., at least 10, 12, 15, 20) nucleotides directly 3' (or is 2 or 3 nucleotides 3') of the polymorphic position of a SNP region; or
100% identical to at least 8 (e.g., at least 10, 12, 15, 20) nucleotides directly 5' (or is 2 or 3 nucleotides 5') of the polymorphic position of a SNP region [or, e.g., 100% identical to at least 8 (e.g., at least 10, 12, 15, 20) nucleotides directly 3' (or is 2 or 3 nucleotides 3') of the polymorphic position of the complement of the SNP region],
wherein the SNP region corresponding to each of the first, second, and third polynucleotides is different and the SNP regions are selected from the group consisting of SEQ ID NOs:1, 2, 3, 4, 5, 6, 7, 8, 9, 10, 11, 12, 13, 14, 15, and 16, wherein at least one SNP region is SEQ ID NO:1, 2, 3, 4, 5, or 6.

In some embodiments, the kit further comprises a sample comprising nucleic acids from a human.

In some embodiments, the kit further comprises a polymerase. In some embodiments, the polymerase is a thermostable polymerase.

In some embodiments, at least one of the first and second and third polynucleotides are detectably labeled. In some embodiments, the detectable label is a fluorescent label. In some embodiments, the first and second and third polynucleotides are detectably labeled with different labels such that the different labels can be differentially detected based on different wavelength of fluorescence of the labels or different mass of the labels.

In some embodiments, the kit comprises a plurality of different allele-specific detection polynucleotides, wherein each of the plurality of polynucleotides comprise at least 8 (e.g., at least 10, 12, 15, 20) contiguous nucleotides at the 3' end of the allele-specific detection polynucleotide, wherein the at least 8 contiguous nucleotides are either:
100% complementary to at least 8 (e.g., at least 10, 12, 15, 20) nucleotides directly 3' (or is 2 or 3 nucleotides 3') of the polymorphic position of a SNP region; or
100% identical to at least 8 (e.g., at least 10, 12, 15, 20) nucleotides directly 5' (or is 2 or 3 nucleotides 5') of the polymorphic position of a SNP region,
wherein the plurality includes a sufficient number of polynucleotides such at least one polynucleotide corresponds to each of the following SNP regions:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

The present invention also provides computer-implemented methods for determining loss-of-imprinting in the Insulin Growth Factor-2 (IGF2) gene. In some embodiments, the method comprises:
(a) receiving, at a host computer, genotype values for at least three SNPs selected from the group consisting of SEQ ID NOs:1, 2, 3, 4, 5, 6, 7, 8, 9, 10, 11, 12, 13, 14, 15, and 16, wherein the genotype is determined from a sample from an individual; and
(b) receiving, at a host computer, a value representing the amount of RNA comprising each polymorphic option of at least one heterozygous SNP selected from the group consisting of SEQ ID NOs:1, 2, 3, 4, 5, 6, 7, 8, 9, 10, 11, 12, 13, 14, 15, and 16, wherein the RNA is from a sample from an individual; and
(c) determining, at a host computer, a ratio of the amount of RNA comprising two polymorphic options of at least one heterozygous SNP; and
(d) outputting to a human the RNA ratio or correlating in the host computer, the RNA ratio to loss of imprinting of the IGF2 gene, and optionally outputting to a human the result of the correlating step.

In some embodiments, at least one SNP is selected from SEQ ID NO: 1, 2, 3, 4, 5, or 6. In some embodiments, the correlating step further comprises correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.

In some embodiments, the receiving step (a) comprises receiving a value representing the amount of RNA having each polymorphic option of each of
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the receiving step (a) comprises receiving a value representing the amount of RNA having each polymorphic option of each of at least 4 (e.g., 4, 5, 6, 7, 8, 9, 10, 11, 12, 13, 14, 15) SNPs selected from the group.

The present invention provides a computer program product for determining loss-of-imprinting in the Insulin Growth Factor-2 (IGF2) gene. In some embodiments, the computer readable product comprises:
a computer readable medium encoded with program code, the program code including:
   program code for receiving genotype values for at least three SNPs selected from the group consisting of SEQ ID NOs:1, 2, 3, 4, 5, 6, 7, 8, 9, 10, 11, 12, 13, 14, 15, and 16, wherein the genotype is determined from a sample from an individual;
   program code for receiving a value representing the amount of RNA comprising each polymorphic option of at least one heterozygous SNP selected from the group consisting of SEQ ID NOs:1, 2, 3, 4, 5, 6, 7, 8, 9, 10, 11, 12, 13, 14, 15, and 16, wherein the RNA is from a sample from an individual;
   program code for determining a ratio of the amount of RNA comprising two polymorphic options of at least one heterozygous SNP; and
   optionally, program code for correlating in the host computer, the RNA ratio to loss of imprinting of the IGF2 gene.

In some embodiments, at least one SNP is selected from SEQ ID NO: 1, 2, 3, 4, 5, or 6.

In some embodiments, the program code for correlating the relative amount of RNA comprises the polymorphic options of the at least one heterozygous SNP to an increased risk of cancer, a diagnosis or prognosis of cancer, or a prediction of efficacy of a drug for ameliorating, treating or preventing cancer.

In some embodiments, program code includes program code for receiving information representing the presence or absence of each polymorphic option of the at least three SNPs in genomic DNA in the sample; and program code for rdetermining whether the individual is heterozygous for the at least two SNPs.

In some embodiments, program code includes program code for receiving a value representing the amount of RNA having each polymorphic option of each of
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the program code includes program code for receiving a genotype value representing for each of at least 4 (e.g., 4, 5, 6, 7, 8, 9, 10, 11, 12, 13, 14, 15) SNPs selected from the group.

In a further aspect, the invention also provide a pair of control isolated nucleic acid members, substantially free of cellular nucleic acids, wherein both members of the pair comprise at least two Insulin Growth Factor-2 (IGF2) single nucleotide polymorphism (SNP) sequences, wherein:
(a) one member of the pair comprises a polynucleotide that comprises one allele of the at least two *IGF2* SNPs, wherein the 3' nucleic acid sequence directly adjacent to the first SNP site is substantially identical to at least 15 contiguous nucleotides (e.g., at least 20, 25, or 50 contiguous nucleotides) of the corresponding region of SEQ ID NO:17, 18, 21, or 23; and the 3' nucleic acid sequence directly adjacent to the second SNP site is substantially identical to at least 15 contiguous nucleotides (e.g., at least 20, 25, or 50 contiguous nucleotides) of the corresponding region of SEQ ID NO:17, 18, 21, or 23; and
(b) the second member of the pair comprises a polynucleotide comprising an alternate allele of the at least two *IGF2* SNPs; wherein the 3' nucleic acid sequence directly adjacent to the first SNP site is substantially identical to at least 15 contiguous nucleotides (e.g., at least 20, 25, or 50 contiguous nucleotides) of the corresponding region of SEQ ID NO:17, 18, 21, or 23 and the 3' nucleic acid sequence directly adjacent to the second SNP site is substantially identical to at least 15 contiguous nucleotides (e.g., at least 20, 25, or 50 contiguous nucleotides) of the corresponding region of SEQ ID NO:17, 18, 21, or 23.

In some embodiments of the pair, at least one of the SNPs is selected from the group consisting of the SNP as shown in SEQ ID NOs: 1, 2, 3, 4, 5, 6, 7, 8, 9, 10, 11, 12, 13, 14, 15, and 16. In some embodiments, at least two of the SNPs are selected from the group consisting of the SNP as shown in SEQ ID NOs: 1, 2, 3, 4, 5, 6, 7, 8, 9, 10, 11, 12, 13, 14, 15, and 16. In some embodiments, at least one of the SNPs is selected from the group consisting of the SNP as shown in SEQ ID NOs: 1, 2, 3, 4, 5, and

In some embodiments of the pair at least two IGF2 SNPs comprise three, four, five, six, seven, eight, nine, ten, eleven, twelve, thirteen, fourteen, fifteen or sixteen IGF2 SNPs.

In some embodiments of the pair of control nucleic acids, the SNPs comprise the SNPs as shown in:
SEQ ID NOs: 1, 2, 3, 4, 5, 6, 7, 8, 9, 10, 11, 12, 13, 14, 15, and 16;
SEQ ID NOs: 1, 5, 8, 9, 10, 11, 14, 15, and 16;
SEQ ID NOs. 1, 5, 8, 9, 10, 11, 12, 14, 15, and 16; or
SEQ ID NOs. 1, 2, 4, 5, 8, 9, 10, 12, 13, 14, 15, and 16.

In some embodiment of the control pair, the first member of the pair comprises a polynucleotide that has at least 8 contiguous nucleotides (e.g., at least 10, 12. 15,20, or 25 contiguous nucleotides) of one allele of SEQ ID NO:1, 2, 3, 4, 5, 6, 7, 8, 9, 10, 11, 12, 13, 14, 15, or 16, or the complement thereof; and the second member of the pair comprises a polynucleotide that has at least 8 contiguous nucleotides (*e.g.,* at least 10, 12. 15, 20, or 25 contiguous nucleotides) of the alternative allele of SEQ ID NO:1, 2, 3, 4, 5, 6, 7, 8, 9, 10, 11, 12, 13, 14, 15, or 16, or the complement thereof.

In some embodiments, one of the members of the pair comprises at least two minor alleles of the SNPs.

In some embodiments, the pair of isolated nucleic acid members are RNA.

In some embodiments, the pair of isolated nucleic acid members are DNA.

In some embodiments, the polynucleotide of one member of the pairs is operably linked to a promoter and the polynucleotide of the second member of the pair is operably linked to a promoter.

In some embodiments, the members of the pair comprise less than 500 nucleotides of the sequence set forth in SEQ ID NO:22.

In some embodiments, the members of the pair are separated. Alternatively, the members of the pair are in a mix together.

The invention also provides a mixture comprising a pair of isolated control nucleic acid members as described herein, *e.g.,* a pair of nucleic acids described herein above. In some embodiments, the members of the pair on present in equimolar amounts.

In some embodiments, the mixture further comprises a second pair of isolated nucleic acids, substantially free of cellular nucleic acids, wherein both members of the second pair comprise at least one *IGF2* SNP sequence and wherein the at least one *IGF2* SNP sequence in the second pair is different from the *IGF2* SNPs in the first pair; and
(c) one member of the second pair comprises one allele of the at least one *IGF2* SNP, wherein the 3' nucleic acid sequence directly adjacent to the SNP site is substantially identical to at least 15 contiguous nucleotides (e.g., at least 20, 25, or 50 contiguous nucleotides) of the corresponding region of SEQ ID NO:17, 18, 21, or 23 of the corresponding region of SEQ ID NO:17, 18, 21, or 23; and
(d) the other member of the second pair comprises an alternate allele of the at least one *IGF2* SNP; wherein the 3' nucleic acid sequence directly adjace to the SNP site is substantially identical to at least 15 contiguous nucleotides (e.g., at least 20, 25, or 50 contiguous nucleotides) of the corresponding region of SEQ ID NO: 17, 18, 21, or 23.

The invention additionally comprises a kit comprising the pair of isolated nucleic acid members of any one of a control pair of nucleic acids as described herein, *e.g.,* the pairs described herein above. In some embodiments, one member of the pair is in a separate container without the second member of the pair. In some embodiments, a kit of the invention comprises a mixture comprising at least one control pair of nucleic acids.

In some embodiments, the kit further comprises a second pair of isolated nucleic acids, substantially free of cellular nucleic acids, wherein both members of the second pair comprise at least one *IGF2* SNP sequences and wherein the at least one *IGF2* SNP sequence in the second pair is different from the *IGF2* SNPs in the first pair; and (c) one member of the second pair comprises one allele of the at least one *IGF2* SNP, wherein the 3' nucleic acid sequence directly adjacent to the SNP site is substantially identical to at least 15 contiguous nucleotides (e.g., at least 20, 25, or 50 contiguous nucleotides) of the corresponding region of SEQ ID NO:17, 18, 21, or 23; and (d) the other member of the second pair comprises an alternate allele of the at least one *IGF2* SNP; wherein the 3' nucleic acid sequence directly adjacent to the SNP site is substantially identical to at least 15 contiguous nucleotides (e.g., at least 20, 25, or 50 contiguous nucleotides) of the corresponding region of SEQ ID NO:17, 18, 21, or 23. In some embodiments, the second pair of isolated nucleic acids is a separate container from the first pair of nucleic acids.

The invention also provides a method of determining loss-of-imprinting (LOI) in an *IGF2* gene from an individual, the method comprising,
(a) quantifying in a sample from the individual, the amount of maternal (first) and paternal (second) copy *of IGF2* RNA, wherein the quantifying step comprises contacting the sample RNA, or a sample cDNA thereof, with one or more oligonucleotides that distinguish between a first possible SNP allele and a second possible SNP allele in the first and second copy, respectively; and
(b) providing a control mix comprising a known ratio of a control pair of nucleic acids as described herein that comprise polynucleotides having the first possible SNP allele and the second possible SNP allele in step (a);
(c) quantifying in the control mix the amount of each member of the pair of nucleic acids;
(d) comparing the ratio of the pair of nucleic acids present in the control mix measured in step (c) to the amounts in the sample measured in step (a) of the first and second copy of *IGF2* RNA, or a sample cDNA thereof, thereby determining the presence or absence of *IGF2* LOI.

The invention additionally provides a method of providing a control standard in an *IGF2* loss-of-imprinting (LOI) assay to confirm the viability of the reaction conditions, the method comprising,
providing a control mix comprising a known ratio of a control pair of nucleic acids described herein; and
quantifying in the control mix the measured ratio of each member of the pair of nucleic acid, thereby confirming viability of the reaction conditions of the assay.

### DEFINITIONS

A "thermostable polymerase" refers to a polymerase useful for PCR applications. A thermostable polymerase can generally be heated to 75° C repeatedly (e.g., at least 20 times for a minute each time) and retain at least 80% of its original activity. Examples of such polymerases include, but are not limited to, *Taq* polymerase.

A "single nucleotide polymorphism" or "SNP" refers to a site of one nucleotide that varies between alleles.

An "allele" refers to one member of a pair or set of different forms of a gene. In a diploid organism, an individual has two copies of each autosomal gene. For a single nucleotide polymorphism, an individual has two different alleles of the polymorphic nucleotide if the genotype at the polymorphic nucleotide is different on one copy of the gene than the other copy of the gene (*i.e.* the individual is heterozygous for the polymorphic nucleotide). If an individual has the same genotype at the polymorphic nucleotide on both copies of the gene *(i. e.* the individual is homozygous for the polymorphic nucleotide), then the individual has two copies of the same allele of the polymorphic nucleotide. A given individual can be homozygous for one polymorphic nucleotide within a gene (two copies of the same allele of the polymorphic nucleotide) and heterozygous for a different polymorphic nucleotide within the same gene (two different alleles of the polymorphic nucleotide).

"Hybridization" refers to the formation of a duplex structure by two single stranded nucleic acids due to complementary base pairing. Hybridization can occur between exactly complementary nucleic acid strands or between nucleic acid strands that contain minor regions of mismatch.

"Target sequence" or "target region" refers to a region of a nucleic acid that is to be analyzed and comprises the polymorphic site of interest.

As used herein, the terms "nucleic acid," "polynucleotide" and "oligonucleotide" refer to nucleic acid regions, nucleic acid segments, primers, probes, amplicons and oligomer fragments. The terms are not limited by length and are generic to linear polymers of polydeoxyribonucleotides (containing 2-deoxy-D-ribose), polyribonucleotides (containing D-ribose), and any other N-glycoside of a purine or pyrimidine base, or modified purine or pyrimidine bases. These terms include double- and single-stranded DNA, as well as double- and single-stranded RNA.

A nucleic acid, polynucleotide or oligonucleotide can comprise, for example, phosphodiester linkages or modified linkages including, but not limited to phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages.

A nucleic acid, polynucleotide or oligonucleotide can comprise the five biologically occurring bases (adenine, guanine, thymine, cytosine and uracil) and/or bases other than the five biologically occurring bases. For example, a polynucleotide of the invention can contain one or more modified, non-standard, or derivatized base moieties, including, but not limited to, N⁶-methyl-adenine, N⁶-tert-butyl-benzyl-adenine, imidazole, substituted imidazoles, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2-thiocytosine, 5-methyl-2-thiouracil, 2-thiouracil, 4-thiouracil, uracil-5- oxyacetic acidmethylester, 3-(3-amino-3-N-2-carboxypropyl) uracil, (acp3)w, 2,6- diaminopurine, and 5-propynyl pyrimidine. Other examples of modified, non-standard, or derivatized base moieties may be found in U.S. Patent Nos. 6,001,611; 5,955,589; 5,844,106; 5,789,562; 5,750,343; 5,728,525; and 5,679,785.

Furthermore, a nucleic acid, polynucleotide or oligonucleotide can comprise one or more modified sugar moieties including, but not limited to, arabinose, 2-fluoroarabinose, xylulose, and a hexose.

"Haplotype block" refers to a region of a chromosome that contains one or more polymorphic sites (e.g., 1-10) that tend to be inherited together. In other words, combinations of polymorphic forms at the polymorphic sites within a block cosegregate in a population more frequently than combinations of polymorphic sites that occur in different haplotype blocks. Polymorphic sites within a haplotype block tend to be in linkage disequilibrium with each other. Often, the polymorphic sites that define a haplotype block are common polymorphic sites. Some haplotype blocks contain a polymorphic site that does not cosegregate with adjacent polymorphic sites in a population of individuals.

"Linkage disequilibrium" refers to the preferential segregation of a particular polymorphic form with another polymorphic form at a different chromosomal location more frequently than expected by chance. Linkage disequilibrium can also refer to a situation in which a phenotypic trait displays preferential segregation with a particular polymorphic form or another phenotypic trait more frequently than expected by chance.

### BRIEF DESCRIPTION OF THE DRAWINGS

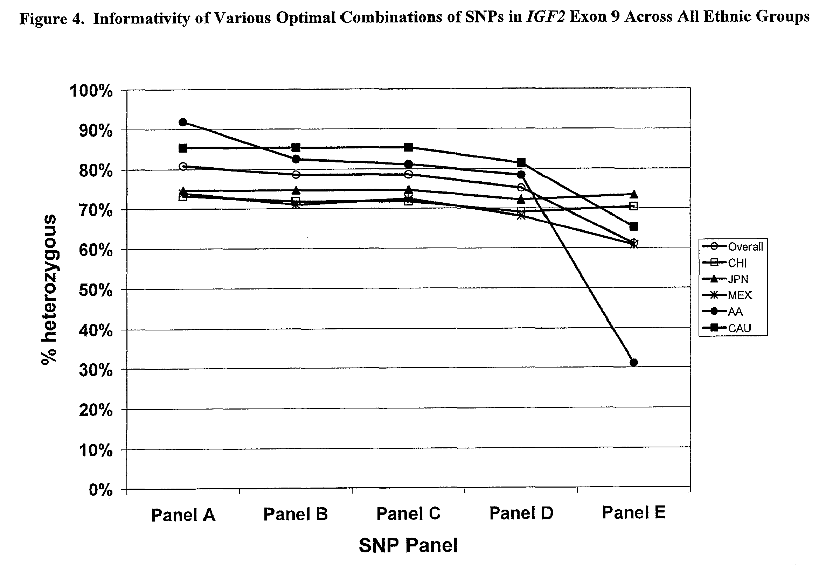

Figure 1 illustrates the structure of the *IGF2* gene. The scale bar above the gene diagram is drawn in 1 Kb increments and depicts the location of *IGF2* on chromosome 11 (NCBI build 36). The scale bar is also present in Figure 4. Arrows in Figure 1A, represent the four promoters of *IGF2.* Exons 1 to 9 are indicated below the gene diagram. Black shaded exons 1 to 6 are not protein-coding in most transcripts of *IGF2.* The white exons 7 and 8 are protein-coding, as is the small white region of exon 9. The black shaded region of exon 9 is the 3' UTR.
Figure 2 illustrates a basic strategy for determining LOI of *IGF2* in a biological sample. Genomic DNA and total or polyadenylated RNA are isolated from a biological sample (for example, peripheral blood, peripheral blood mononuclear cells, colonic biopsy or cell scrape, stool, etc.) from an individual. The genomic DNA sample is used for genotyping of one or more polymorphic markers (DNA SNP Assay). This step determines whether the individual is heterozygous for a specific SNP or combination of SNPs. Any SNP, or combination of SNPs, determined to be heterozygous may be utilized for analysis of allele-specific expression of the *IGF2* gene in the matched RNA sample (RNA SNP assay). In the example shown, an individual was determined by an allele-discriminating DNA genotyping assay to be homozygous for hypothetical SNPs 1, 3, 4 and 6 and heterozygous for hypothetical SNPs 2 and 5. cDNA is amplified from the relevant region of the *IGF2* transcript using standard reverse transcriptase and PCR methods such that PCR products including at least SNPs 2 and 5 are amplified. Expression from each of the two copies of the *IGF2* gene is independently measured using the generated cDNA with a quantitative allele specific gene expression assay, and which can sufficiently discriminate between the two alleles of the gene. A comparison between the amount of expression of one allele relative to the amount of expression of the other allele is made and the imprinting status of the *IGF2* gene is determined. Assays that discriminate SNPs 2 and 5 may be performed simultaneously, and allele specific expression ratios obtained for each assay can be compared to improve accuracy.

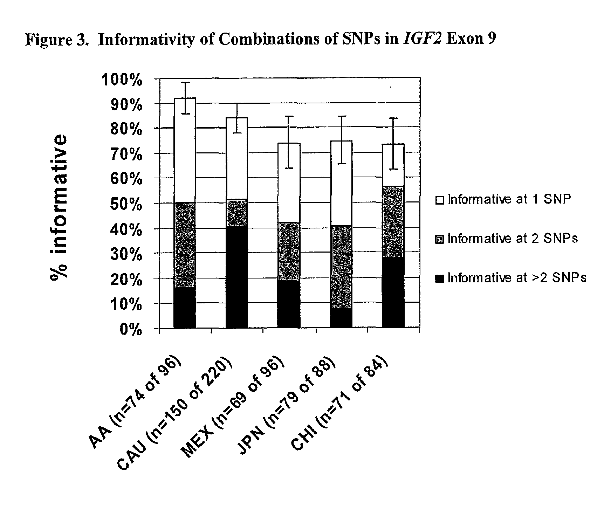

Figure 3 illustrates the informativity of combinations of SNPs in *IGF2* exon 9. The percentage of individuals that are heterozygous (informative) for one SNP (open bars), two SNPs (gray bars) or more than two SNPs (black bars) are plotted for the African American panel of samples (AA) genotyped for a first panel of SNPs, the Caucasian panel of samples (CAU) genotyped for a second panel of SNPs, the Mexican descent panel of samples (MEX) genotyped for a third panel of SNPs, the Japanese panel of samples (JPN) genotyped for a fourth panel of SNPs and the Chinese panel of samples (CHI) genotyped for a fifth panel of SNPs. A total of 96 African American panel samples, 207 Caucasian panel samples, 96 Mexican descent panel samples, 88 Japanese panel samples and 84 Chinese panel samples were genotyped. Only samples that were successfully genotyped by a single nucleotide primer extension assay for all SNPs are included in the calculation of percent informativity. The number of samples included in each ethnic group is indicated below the x-axis (n). Error bars indicate the 95% confidence interval. The combinations of SNPs for each ethnic population included SNPs represented by SEQ ID NOs: 1, 2, 3, 4, 12, 13, 15 and 16 for the first panel of SNPs genotyped in the African American panel of samples: SEQ ID NOs: 5, 8, 9, 10, 12, 15 and 16 for the second panel of SNPs genotyped in the Caucasian panel of samples; SEQ ID NOs: 1, 7, 9, 10, 11, 12, 14, 15 and 16 for the third panel of SNPs genotyped in the Mexican descent panel of samples; SEQ ID NOs: 10, 12, 14 and 16 for the fourth panel of SNPs genotyped in the Japanese panel of samples; and SEQ ID NOs: 6, 8, 12 and 16 for the fifth panel of SNPs genotyped in the Chinese panel of samples.
Figure 4 illustrates the informativity of various optimal combinations of SNPs in *IGF2* exon9. The percentage of individuals that are heterozygous (informative) for one or more SNP within each combination of SNPs are plotted for the African American sample panel (AA), the Caucasian sample panel (CAU), the Mexican descent sample panel (MEX), the Japanese sample panel (JPN), the Chinese sample panel (CHI) and all genotyped samples combined (Overall). Only samples that were successfully genotyped by a single nucleotide primer extension assay for all SNPs are included in the calculation of percent informativity. Panels of SNPs were selected based on identification of SNPs common to all maximal informativity combinations of 15, 14, 13, 12, 11 or 10 SNPs across all samples (overall). All percentages are provided in Table 4. The SEQ ID NOs for SNPs included in each panel are listed in Table 5.

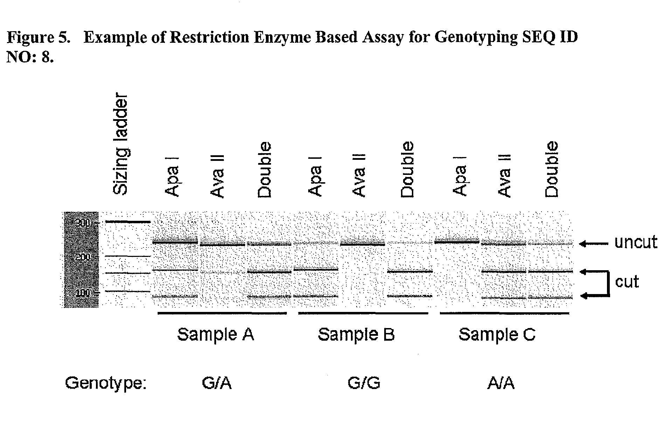

Figure 5 illustrates the use of a restriction enzyme based assay for genotyping the SNP corresponding to SEQ ID NO: 8. The polymorphic nucleotide is located within the recognition sequence of two restriction enzymes. *Apa I* recognizes and cleaves the sequence when the "G" allele is present, and *Ava II* recognizes and cleaves the sequence when the "A" allele is present. A PCR amplicon including SEQ ID NO: 8 was amplified from three independent genomic DNA samples derived from three individuals (Samples A, B and C). Amplicons were digested with *Apa I* or *Ava II* or a combination of both enzymes (Double). Digestion by *Apa I* only indicates that the individual is homozygous for the G allele (Sample B), digestion by *Ava II* only indicates that the individual is homozygous for the A allele (Sample C), and digestion by both enzymes indicates that the individual is heterozygous for the SNP (Sample A).

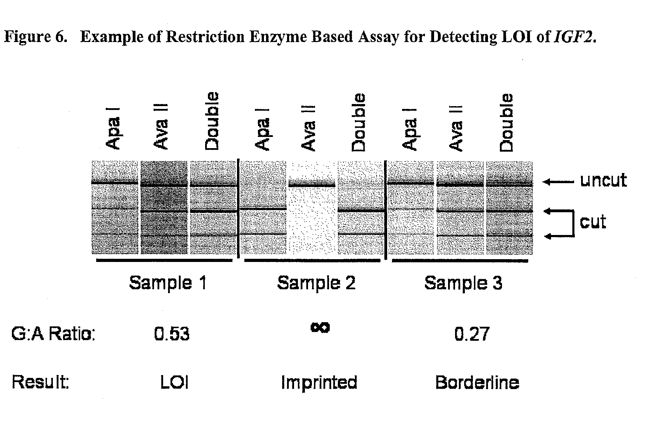

Figure 6 illustrates the use of a restriction enzyme based method for detecting LOI of *IGF2.* Total RNA was extracted from three individuals that are heterozygous for SEQ ID NO: 8. The region of the 3' UTR of exon 9 of the *IGF2* gene, which includes SEQ ID NO: 8 was RT-PCR amplified from each sample. cDNA amplicons were digested with *Apa I* or *Ava II* or a combination of both enzymes, as indicated above each lane. Digested products were resolved on an Agilent Bioanalyzer, and concentrations of cut and uncut fragments were determined. The quantity of fragments cut by *Apa I* represents the proportion of cDNA including the "G" allele. The quantity of fragments cut by *Ava II* represents the proportion of cDNA including the "A" allele. Therefore, the ratio of *Apa I* cut fragments to *Ava II* cut fragments indicates the relative ratio of expression of the two alleles in the original RNA sample. The calculated G:A ratio is shown below each triplet of lanes representing each sample. Sample 2 expresses exclusively the "A" allele and therefore the imprint of *IGF2* was present. Sample 1 expresses both copies of the *IGF2* gene, with a G:A ratio of 0.53 and therefore displays LOI of *IGF2.* Sample 3 expresses detectible amounts of both copies of the *IGF2* gene, with a G:A ratio of 0.27, which could be considered a borderline call for imprinting status (close to the threshold of 0.33 ratio for determining LOI).

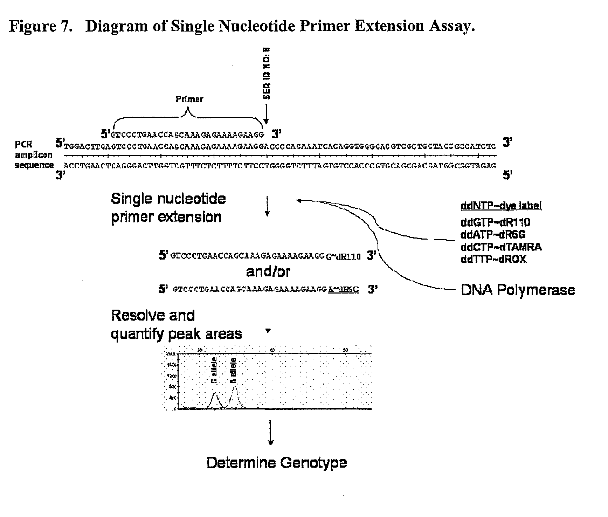

Figure 7 diagrams a method for allele-specific detection of a SNP using a single nucleotide primer extension strategy. The SNP represented by SEQ ID NO: 8 and its surrounding DNA sequence are shown as an example ("PCR amplicon sequence"). The nucleotide position of SEQ ID NO: 8 is indicated by the arrow labeled "SEQ ID NO: 8". The sequence of the polynucleotide used for single nucleotide primer extension is indicated by the bracket labeled "Primer". The PCR DNA amplicon (or, alternatively a RT-PCR cDNA amplicon) including the sequence of interest is amplified from the genomic DNA or RNA sample to be assayed. A primer is added to the purified PCR (or, alternatively RT-PCR) product that anneals with its 3' terminal nucleotide complimentary to the template nucleotide 1 base to the 3' side of the polymorphic nucleotide to be genotyped. Single nucleotide primer extension is carried out using a thermostable DNA polymerase and differentially fluorescently labeled ddNTPs. In this example, either dR110 labeled ddGTP or dR6G labeled ddATP is added to the 3' end of the primer. These labeled polynucleotides are then resolved and the peak areas representative of each possible incorporated nucleotide are calculated. Peak areas are compared to determine the genotype of the individual at that SNP position (or, alternatively to determine the allele-specific gene expression ratio).

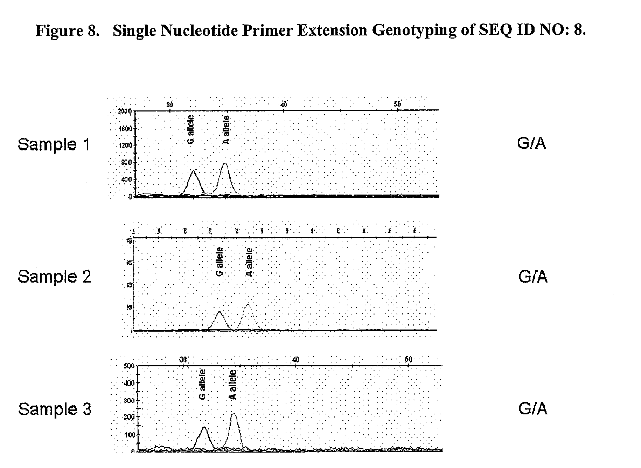

Figure 8 illustrates the use of the single nucleotide primer extension assay described in Figure 7 to genotype three individuals for SEQ ID NO: 8. The same three individuals that were assayed for LOI *of IGF2* by the restriction enzyme based assay shown in Figure 6, were genotyped. The figure shows the resulting chromatograms for each sample following electrophoretic resolution and peak detection using an ABI 3730XL Genetic Analyzer and Gene Mapper software. As expected, peaks representing both alleles of SEQ ID NO: 8 are obtained in relatively equal proportions, confirming that the three individuals are heterozygous for SEQ ID NO: 8 and demonstrating the concordance between results obtained by the restriction enzyme based method and the single nucleotide primer extension based method.

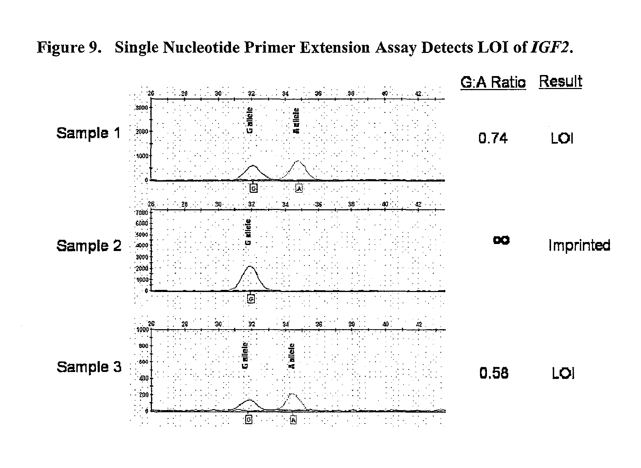

Figure 9 illustrates the application of the single nucleotide primer extension based method for detecting LOI of *IGF2.* The region including SEQ ID NO: 8 was RT-PCR amplified from a total RNA sample derived from each of the three individuals genotyped in Figure 8. The cDNA products obtained were purified and analyzed as diagrammed in Figure 7. The figure shows the resulting chromatograms for each sample following electrophoretic resolution and peak detection using an ABI 3730XL Genetic Analyzer and Gene Mapper software. For each sample, peak areas representing each of the two possible alleles were calculated and compared to each other. The calculated G:A ratios are indicated to the right of each chromatogram. Consistent with the results shown in Figure 6, Samples 1 and 3 were determined to show LOI *of IGF2,* and Sample 2 was determined to show normal imprinting *of IGF2.*

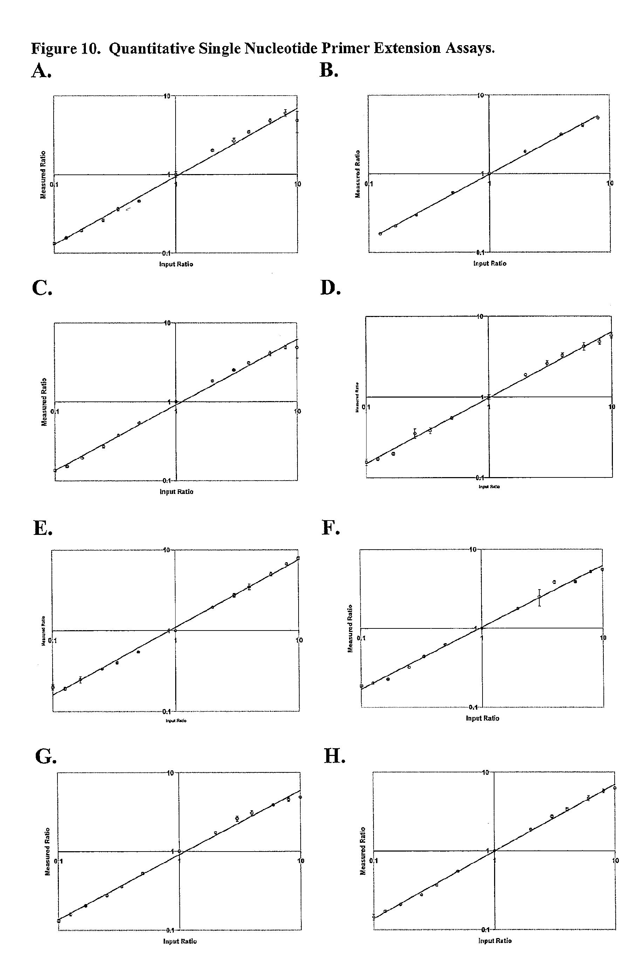

Figure 10 illustrates the quantitative analytical linearity of single nucleotide primer extension assays developed for 8 independent SNPs: SEQ ID NO: 8 (A), SEQ ID NO: 2 (B), SEQ ID NO: 9 (C), SEQ ID NO: 10 (D), SEQ ID NO:5 (E), SEQ ID NO:7 (F), SEQ ID NO:15 (G) and SEQ ID NO:16 (H). For each assay of the 8 assays, two PCR amplicons targeting the 3' UTR of exon 9 of the *IGF2* gene were separately amplified from genomic DNA samples from two individuals; one homozygous for one allele of the SNP and the other homozygous for the other allele of the SNP *(i. e.* a total of 16 amplicons representing 8 allele pairs were obtained). The 16 PCR products were purified and quantified. For each of the 8 SNPs, two PCR products (one containing one allele (allele 1) and the other containing the other allele (allele 2) of a particular SNP) were combined in the following 11 ratios (or dilution points) of allele 1 to allele 2; 1:10, 1:6, 1:4, 1:3, 1:2, 1:1, 2:1, 3:1, 4:1, 6:1, and 10:1. For each of the 8 SNPs, the single nucleotide primer extension assay was performed in triplicate on each dilution point. Ratios of peak areas representing each of the two possible alleles were calculated for each dilution point (y-axis) and compared to the known input ratio of each allele pair (x-axis). Values are plotted on a log10 scale. The R² and slope for each assay are shown in Table 6.

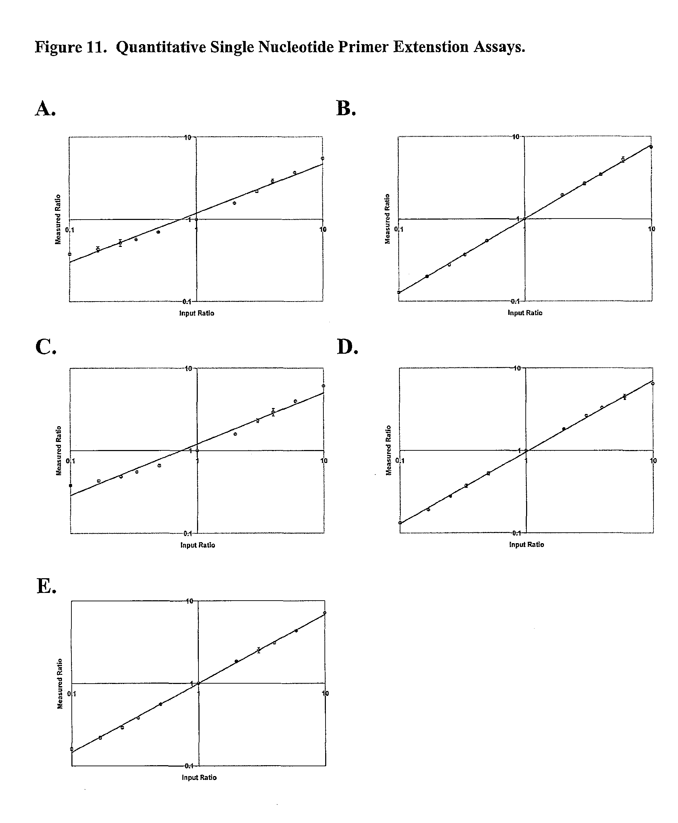

Figure 11 illustrates the quantitative analytical linearity of single nucleotide primer extension assays developed for 5 independent SNPs: SEQ ID NO: 3 (A), SEQ ID NO:4 (B), SEQ ID NO:6 (C), SEQ ID NO:11 (D) and SEQ ID NO:14 (E). For each assay, PCR products were separately amplified from genomic DNA samples derived from two individuals; one homozygous for one allele of the SNP and the other homozygous for the other allele of the SNP. The PCR products were purified and quantified. For each of the 8 SNPs, two PCR products (one amplified from the DNA sample homozygous for one allele and the other amplified from the DNA sample homozygous for the other allele) were combined in the following ratios of allele 1 to allele 2; 1:10, 1:6, 1:4, 1:3, 1:2, 1:1, 2:1, 3:1, 4:1, 6:1, and 10:1. For each of the 8 SNPs, the single nucleotide primer extension assay was performed in triplicate on each dilution point. Ratios of peak areas representing each of the two possible alleles were calculated (y-axis) and compared to the known input ratio of each allele pair (x-axis). Values are plotted on a log10 scale. The R² and slope for each assay are shown in Table 6.

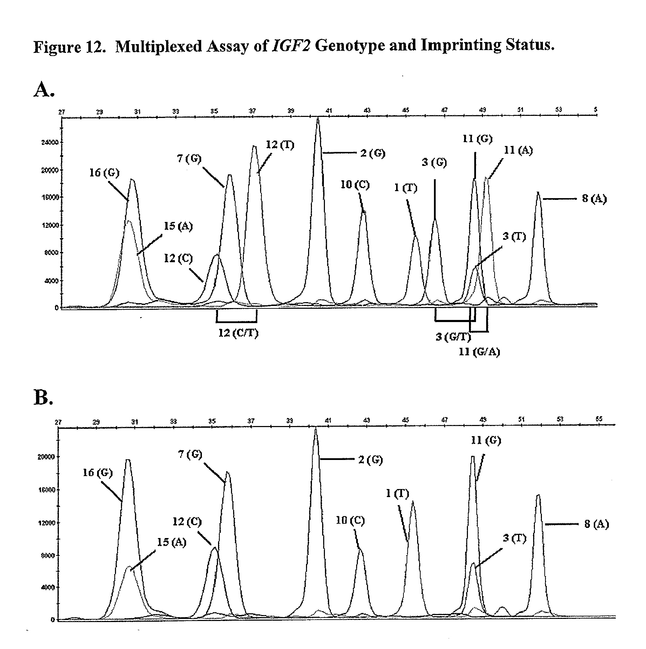

Figure 12 illustrates the use of a multiplexed assay for determining the genotype and imprinting status *of IGF2.* Figure 12A shows a chromatogram generated by GeneMapper software for a multiplexed genotyping assay of PCR products generated from genomic DNA derived from a blood sample from one individual (Sample A in Table 7). The assay determines the genotype and imprinting status at SNP positions represented by SEQ ID NOs: 1, 2, 3, 7, 8, 10, 11, 12, 15 and 16. In the figure, peaks are labeled by a corresponding SEQ ID NO, followed by the represented single nucleotide sequence in parentheses. For example, peaks labeled 12 (C) and 12 (T) indicate that the sample is heterozygous (C/T) for the SNP represented by SEQ ID NO: 12. The sample is heterozygous, and therefore is informative, at SNP positions represented by SEQ ID NO: 3, 11 and 12. Peaks representing these heterozygous positions are highlighted by brackets below the chromatogram in Figure 12A. Figure 12B shows a chromatogram generated by GeneMapper for a multiplexed imprinting assay of PCR products generated from cDNA amplified from total RNA derived from a blood sample from the same individual as in Figure 12A (Sample A in Table 7). Peaks are labeled as in Figure 12A. The sample is found to display normal imprinting (*i.e.* monoallelic expression) *of IGF2* because only a single peak is detected for the SNP positions represented by SEQ ID NOs: 3, 11 and 12. For example, the C allele of SEQ ID NO: 12 is detected, but the T allele is not detected.

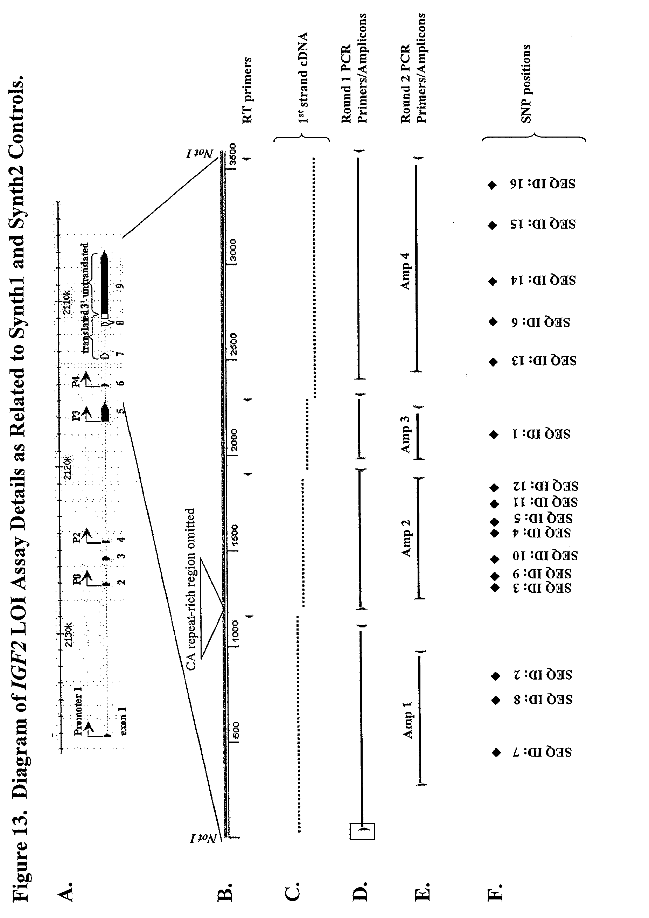

Figure 13. Diagram of *IGF2* LOI Assay Details as Related to Synth1 and Synth2 Controls. 13 A. Diagram of the *IGF2* gene. Potential promoters are indicated by arrows labeled Promoter 1, P0, P2, P3 or P4. Potential transcribed exons are indicated by block arrows connected by a hashed line that represents intronic sequence regions. Untranslated exonic sequence regions are indicated by filled block arrows, and translated exonic sequence regions are indicated by open block arrows. The scale bar at the top of diagram is shown in 1000 base pair (Kb) increments. 13 B. Expanded view of the Synth1 (or Synth2) nucleic acid region. The Synth1 (or Synth2) nucleic acid sequence includes the 3'-most 21 base pairs of exon 8 followed immediately by the sequence of exon 9. The 5' and 3' ends of Synth1 (or Synth2) include Not I restriction enzyme recognition sequences that were included to allow ligation of the sequences into a transcription vector. The CA repeat-rich sequence region present in IGF2 exon 9 was omitted from the Synth1 (or Synth2) sequence. The annealing sites of four primers within the Synth1 (or Synth2) region (or optionally, within the IGF2 mRNA region) are indicated by arrow heads (RT primers). These RT primers are used to reverse transcribe first strand cDNA from the Synth1 or Synth2 IVT RNAs (or optionally, from total RNA derived from a human biological sample). The scale bar is shown in 500 base pair increments. 13 C. The first strand cDNA molecules generated by these RT reactions are indicated by hashed lines. 13 D. Following reverse transcription, the first strand cDNA is used as template for first round PCR amplification of four independent amplicons. The annealing sites of four primer pairs relative to the generated first strand cDNA are indicated by arrow heads. Solid lines connecting the primer pairs represent the PCR amplicon produced in each first round PCR amplification reaction. The primer highlighted by the open box includes the exon 8-9 splice junction spanning sequence. 13 E. The amplicons produced in the first round PCR amplification are used as template for amplification of four independent nested amplicons. The annealing sites of the four primer pairs relative to the generated first strand cDNA are indicated by arrow heads. Solid lines connecting the primer pairs represent the PCR amplicon produced in each second round PCR amplification reaction. 13 F. Each of the four amplicons generated by the second round PCR reaction includes one or more of the SNP positions represented by SEQ ID NOs.:1-16. The position of each SNP is indicated by a diamond.

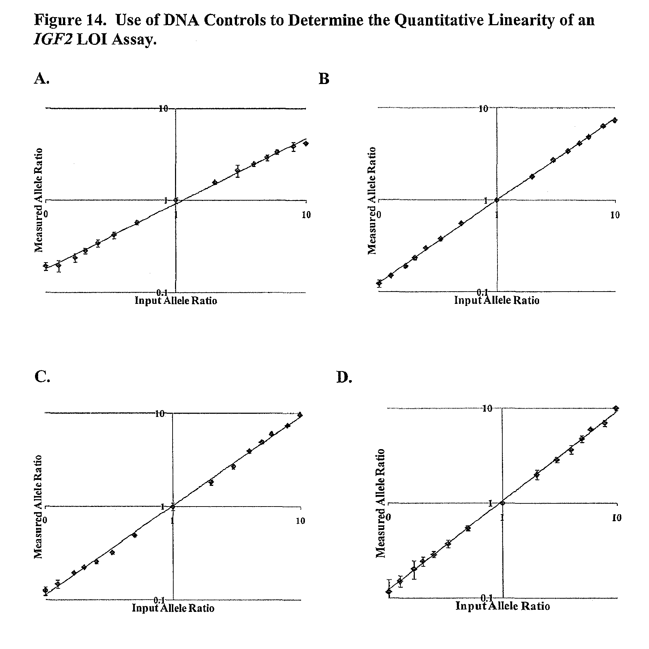

Figure 14. Use of DNA Controls to Determine the Quantitative Linearity of an *IGF2* LOI Assay. Mixtures of pEZ-Synth1 and pEZ-Synth2 were made in the following ratios: 8:1, 6:1, 4:1, 3:1, 2:1, 1:1, 1:2, 1:3, 1:4, 1:6 and 1:8 (Synth1:Synth2). Four nonoverlapping PCR amplicons were designed to amplify the transcribed regions of IGF2 exon 9 including the 16 SNP nucleotides. Amplicons derived from the Synth1/Synth2 ratio mixtures were used as template for the multiplexed single-nucleotide primer extension assay described in Example 1. Assays were performed in triplicate, and average peak areas for each peak were calculated. The known input ratio of Synth1:Synth2 template is plotted on the x-axis. The average measured ratio of peak areas representing the Synth 1 allele: the Synth2 allele is plotted on the y-axis. Error bars indicate plus and minus one standard deviation across the triplicate measurements. All average data point values were multiplied by a common normalization factor that adjusts the measured value for the known 1:1 input data point to equal exactly 1.0. Results of assays utilizing the SNPs represented by SEQ ID NO: 7 within Amplicon 1 (A), SEQ ID NO: 5 within Amplicon 2 (B), SEQ ID NO: 1 within Amplicon 3 (C) and SEQ ID NO: 13 within Amplicon 4 (D) are shown.

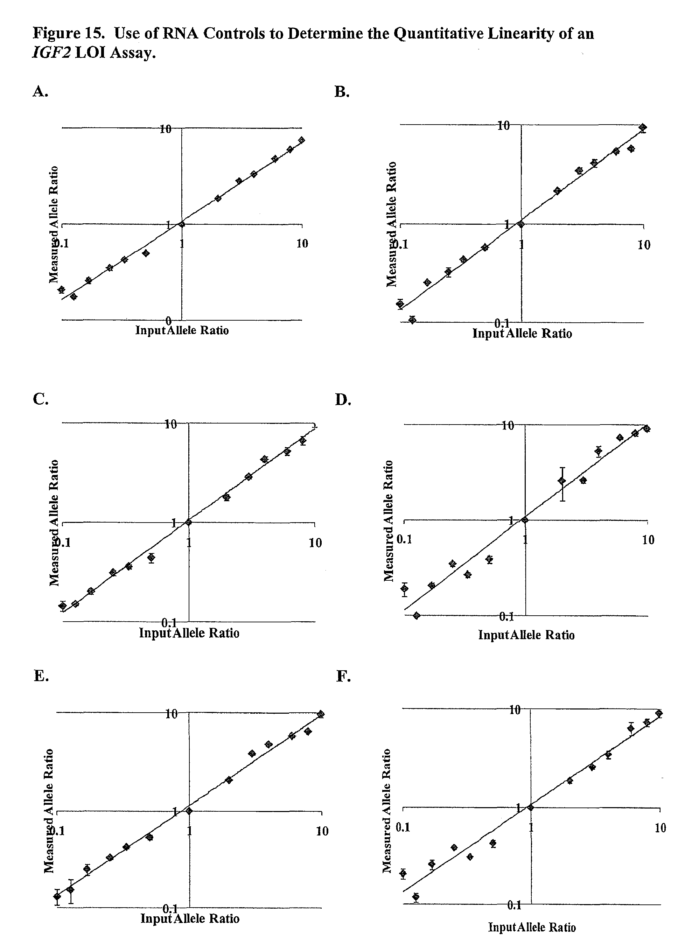

Figure 15. Use of RNA Controls to Determine the Quantitative Linearity of an *IGF2* LOI Assay. IVT RNA derived from pEZ-Synth1 was mixed with IVT RNA derived from pEZ-Synth2 in ratios including 8:1, 6:1, 4:1, 3:1, 2:1, 1:1, 1:2, 1:3, 1:4, 1:6 and 1:8 (Synth1:Synth2). A total of 120 pg of Synth1:Synth2 IVT RNA mixture (i.e. the amount of IVT control RNA derived from pEZ-Synth1 plus the amount of IVT control RNA derived from pEZ-Synth2 equaled 120 pg) was spiked into 500 ng of yeast RNA. This RNA mixture was used as template for RT-PCR amplification of the four amplicons described in Figure 13E. Amplicons derived from the Synth1/Synth2 ratio mixtures were used as template for the multiplexed single-nucleotide primer extension assay described in Example 1. Assays were performed in triplicate, and average peak areas for each peak were calculated. The known input ratio of Synth1:Synth2 IVT RNA template is plotted on the x-axis. The average measured ratio of peak area representing the Synth 1 allele: the Synth2 allele is plotted on the y-axis. Error bars indicate plus and minus one standard deviation across the triplicated measurements. All average data point values were multiplied by a common normalization factor that adjusts the measured value for the known 1:1 input data point to equal exactly 1.0. Results of assays utilizing the SNPs represented by SEQ ID NO: 7 within Amplicon 1 (A), SEQ ID NO: 5 within Amplicon 2 (B), SEQ ID NO: 1 within Amplicon 3 (C) SEQ ID NO: 13 within Amplicon 4 (D), SEQ ID NO: 4 within Amplicon 2 (E) and SEQ ID NO: 14 within Amplicon 4 (F) are shown.

### DETAILED DESCRIPTION OF THE INVENTION

### I. Introduction

The present invention provides methods of detecting LOI of *IGF2.* The inventors have found that specific combinations of IGF2 SNPs, selected from numerous possible known and newly discovered SNPs, represent optimal ways to detect for LOI of IGF2 in heterogeneous human populations. Detection of LOI relies on the ability to detect both alleles of IGF carried by an individual and therefore requires that there be at least one detectable difference between the two IGF alleles carried by the individual. Any particular IGF2 SNP therefore is a potential marker to measure LOI, but it is only useful to measure LOI in individuals that are heterozygous at that particular SNP. The inventors have now identified a very small number of IGF2 SNPs, which when used in various combinations or sub-combinations, are highly likely to have at least one, and generally two or more SNPs that are heterozygous for any particular individual assayed. Past studies typically were only successful in measuring imprinting status of *IGF2* in 40-50% of the patients tested. The present invention, for the first time, makes it possible to determine the imprinting status of *IGF2* in a high proportion of human subjects from diverse backgrounds. As shown in the examples, the combinations of SNPs described herein can be used effectively with a variety of different ethnic backgrounds and thus are also effective for use in the human population at large (being a combination of the different ethnic groups).

### II. IGF2 SNPs

The following SNPs can be detected in combinations, or subcombinations as described herein to achieve optimal detection of LOI across a variety of ethnic groups with a minimum number of markers: SEQ ID NO: 1, 2, 3, 4, 5, 6, 7, 8, 9, 10, 11, 12, 13, 14, 15, and 16. The sequence identifiers correspond to the sequence including and surrounding the SNP are provided in Table 1, with the polymorphic position (and polymorphic options) provided in brackets in the sequence. When an individual carries a copy of IGF2 having a polymorphic option, that copy is referred to as an "allele" of the SNP.

The present invention allows for detection of all of the above SNPs (e.g., as represented in SEQ ID NOs: 1-16, or subcombinations thereof). For example, in some embodiments, SNPs comprising or consisting of SEQ ID NOs: 1, 5, 8, 9, 10, 11, 14, 15 and 16 are detected. As shown in the Examples below, this particular subcombination provides a very high rate of heterozygosity across ethnic groups and thus represents a useful combination of markers for detecting LOI. In some embodiments, at least one, two three, or more (e.g., all) SNP consisting of SEQ ID NOs: 1, 5, 8, 9, 10, 11, 12, 14, 15 and 16 is detected. In some embodiments, at least one, two three, or more (e.g., all) SNP consisting of SEQ ID NOs: 1, 2, 4, 5, 8, 9, 10, 12, 13, 14, 15 and 16 is detected. In some embodiments, at least one, two three, or more (e.g., all) SNP consisting of SEQ ID NOs: 1, 2, 3, 4, 5, and 6 is detected. In some embodiments, SNPs comprising or consisting of SEQ ID NOs: 1, 5, 8, 9, 10, 11, 12, 14, 15 and 16 is detected. In some embodiments, SNPs comprising or consisting of SEQ ID NOs: 1, 2, 4, 5, 8, 9, 10, 12, 13, 14, 15 and 16 are detected. In some embodiments, SNPs comprising or consisting of SEQ ID NOs: 1-16 are detected. SNPs can be detected for genotyping, LOI determination, or both as discussed below.

In some embodiments, the individual assayed is of African descent (e.g., African or African-American) and at least one (e.g., 2, 3, 4, 5, 6, 7, or 8) SNP detected is selected from SEQ ID NOs: 1, 2, 3, 4, 12, 13, 15 and 16. In some embodiments, the individual assayed is Caucasian and at least one (e.g., 2, 3, 4, 5, 6, or 7) SNP detected is selected from SEQ ID NOs: 5, 8, 9, 10, 12, 15 and 16. In some embodiments, the individual assayed is Mexican or of Mexican descent and at least one (e.g., 2, 3, 4, 5, 6, 7, 8 or 9) SNP detected is selected from SEQ ID NOs: 1, 7, 9, 10, 11, 12, 14, 15 and 16. In some embodiments, the individual assayed is of Japanese descent (e.g., Japanese or Japanese-American) and at least one (e.g., 2, 3, or 4) SNP detected is selected from SEQ ID NOs: 10, 12, 14 and 16. In some embodiments, the individual assayed is of Chinese descent (e.g., Chinese-American) and at least one (e.g., 2, 3 or 4) SNP detected is selected from SEQ ID NOs: 6, 8, 12 and 16.

The present invention provides for combinations, and uses thereof, of polynucleotides that distinguish between two alleles of a first and second (or more) SNP as set forth in the combinations presented herein (e.g., as listed above). For example, a combination of a number of different polynucleotides can be used, where each polynucleotide is capable of distinguishing a different SNP. Thus, for example, one can use at least one polynucleotide that distinguishes between two or more alleles of each of the following SNP combination. In some embodiments, the combinations comprise 2, 3, 4, 5, 6, 7, 8, 9, 10, 11, 12, 13, 14, 15, or 16 SNPs selected from the group consisting of SEQ ID NO: 1, 2, 3, 4, 5, 6, 7, 8, 9, 10, 11, 12, 13, 14, 15, and 16.

For example, the present invention provides for combinations of polynucleotides, wherein the combination comprises different polynucleotides, wherein each of the different polynucleotides hybridize to a first allele of a particular different SNP, but does not significantly hybridize to the second allele of the SNP. "Does not significantly hybridize" means that in the presence of equal amounts of both alleles in a sample, the polynucleotide is able to detect the presence of the first allele but does not detect the presence of the second allele to such an extent so as to interfere with the interpretation of the assay. In some embodiments, in the presence of equal amounts of both alleles in a sample, the polynucleotide provides a signal for a sample having the first allele that is at least, e.g., about 10, 100; 1,000; 10,000; 100,000 times or more than the signal generated by the polynucleotide for a sample having an equal amount of the second allele. "Signal" refers to any output indicative of hybridization of the polynucleotide to a complementary sequence. In some embodiments, at least 70%, 80%, 90%, 95% of the sequence of any, a subset, or all of the polynucleotides in the group for detection of the combinations of SNPs is complementary to one (or more, depending on the number of polynucleotides) SNP selected from SEQ ID NOs: 1-16. In some embodiments, the polynucleotides are, e.g., 8-200, 8-100, 8-50,10-50, 115-100,20-200 nucleotides long. In some embodiments, the polynucleotides comprise, for example, at least 8, 10, 15, 20, 30, 40 or 50 complementary nucleotides. A plurality of allele-specific detection polynucleotides can be used to detect a plurality of different SNPs. For example, in some embodiments, the plurality of polynucleotides includes a sufficient number of polynucleotides to detect one of the following combinations:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

The plurality of polynucleotides can be designed, for example, such that there is one polynucleotide that is complementary to one allele of each of the SNPs in the combination. Accordingly, a sufficient number of polynucleotides such at least one polynucleotide corresponds to "each" of the following SNP regions means that the plurality in sum correspond to all of the SNP regions, not necessarily that one polynucleotide corresponds to all SNP regions.

Alternatively, any, a subset, or all polynucleotides in a group can distinguish between two alleles of a SNP by acting as a primer in a template-specific primer extension reaction. In these embodiments, the polynucleotides do not generally encompass the polymorphic nucleotide but instead hybridize to the genomic DNA or cDNA such that 3' extension of the polynucleotide occurs at the polymorphic nucleotide. Thus, in some embodiments, the 3' end of the polynucleotide is complementary to a nucleotide within 1000, 100, 10, 5, 3, 2, or 1 nucleotide(s) upstream from the polymorphic nucleotide. In some embodiments, the polynucleotides are complementary over at least 70%, 80%, 90%, 95%, 96%, 97%, 98%, 99%, or 100% of the polynucleotides length to an IGF2 genomic DNA or cDNA (or complement thereof). In some embodiments, the polynucleotide comprises at its 3' end, at least 2, 3, 4, 5, 6, 7, 8, 9, 10, 20, 30, 40, 50, or more contiguous nucleotides that are at least at least 70%, 80%, 90%, 95%, 96%, 97%, 98%, 99%, or 100% complementary to an IGF2 genomic DNA or cDNA (or complement thereof) (e.g., NM_000612 (SEQ ID NO: 17) or ENST00000300632 (SEQ ID NO: 18) or ENSG00000167244 (SEQ ID NO: 19)). Optionally, the 5' end of the polynucleotide will comprise a sequence tag or other sequence not complementary to an IGF2 genomic DNA or cDNA. As is well known in the art, a variety of primer extension methods can be employed to detect SNPs.

Allele-specific detection polynucleotides useful for primer extension reactions include those having at least 8 (e.g., at least 10, 12, 15, 20) contiguous nucleotides at the 3' end of the allele-specific detection polynucleotide, wherein the at least 8 contiguous nucleotides are either:
100% complementary to at least 8 (e.g., at least 10, 12, 15, 20) nucleotides directly 3' (or is 2 or 3 nucleotides 3') of the polymorphic position of a SNP region (as set forth in SEQ ID NOs: 1-16); or
100% identical to at least 8 (e.g., at least 10, 12, 15, 20) nucleotides directly 5' (or is 2 or 3 nucleotides 5') of the polymorphic position of the SNP region [or, e.g., 100% identical to at least 8 (e.g., at least 10, 12, 15, 20) nucleotides directly 3' (or is 2 or 3 nucleotides 3') of the polymorphic position of the complement of the SNP region].

This configuration allows for the 3' end of the polynucleotide to form Watson-Crick base pairing with the region just upstream of the polymorphic position (from either strand), allowing for a primer extension reaction to take place to determine which allele is in a cDNA or genomic DNA of an individual. "100% complementary" means that the designated (e.g., the "at least 8") contiguous nucleotides of the polynucleotide are completely complementary to the designated sequences in the SNP region, i.e., there are no mismatches. It will be appreciated that the polynucleotides can include non-naturally-occurring nucleotides that retain Watson-Crick base pairing. The polynucleotides can include further nucleotides at the 5' end that are either complementary to the SNP region or not as desired.

Combinations of such polynucleotides can be used to detect particular combinations of SNPs as described herein. For example, a plurality of allele-specific detection polynucleotides can be designed with the above features such that one polynucleotide can prime a separate extension reaction whereby the plurality detects one of the following combinations:
SEQ ID NOs: 1, 2, 3, 4, 5, 6, 7, 8, 9, 10, 11, 12, 13, 14, 15, and 16; or
SEQ ID NOs: 1, 5, 8, 9, 10, 11, 14, 15, and 16; or
SEQ ID NOs: 1, 5, 8, 9, 10, 11, 12, 14, 15, and 16; or
SEQ ID NOs: 1, 2, 4, 5, 8, 9, 10, 12, 13, 14, 15, and 16.

In some embodiments, the polynucleotides that distinguish between the two alleles are at least 4, 6, 8, 10, 12, 15, 20, 30, 50, or more nucleotides in length. In some embodiments, the polynucleotides are no more than 1000, 500, 200, 100, 80, 50, 40, 30, or 25 nucleotides in length. For example, the polynucleotides can be, e.g., 8-25, 8-30, 8-50, 8-100, 10-25, 10-50, 10-100, nucleotides, etc. The polynucleotides that distinguish between the two alleles will typically include a nucleotide that corresponds (i.e., aligns with) and is complementary to one of the polymorphic nucleotides of the SNP. In some embodiments, the ultimate or penultimate 3' nucleotide of the polynucleotide is complementary to a nucleotide at the polymorphic position of the SNP. Such embodiments can be particularly useful in SNP detection methods employing the polynucleotides as primers or probes, for example in amplification-based assays such as those involving the polymerase chain reaction.

The polynucleotides of the invention can be detectably labeled. Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads *(e.g.,* Dynabeads™), fluorescent dyes (*e.g.,* fluorescein, Texas red, rhodamine, green fluorescent protein, and the like, *see, e.g.,* Molecular Probes, Eugene, Oregon, USA), radiolabels (*e*.*g*., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold *(e.g.,* gold particles in the 40 -80 nm diameter size range scatter green light with high efficiency) or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241. Where two or more alleles and or multiple SNPs or polynucleotides are to be detected, it can be useful to have each polynucleotide be labeled with a different fluorescent or other label. Optionally, where primer extension is used, the free nucleotides can be detectably, and optionally differentially, labeled, thereby allowing for detecting of incorporation of a particular nucleotide.

Hybridization reaction conditions can vary depending on the assay that is used to detect the SNPs. Stringent, sequence-specific hybridization conditions, under which an oligonucleotide will hybridize only to the exactly complementary target sequence, are well known in the art. Stringent conditions are sequence dependent and will be different in different circumstances. In some embodiments, stringent conditions are selected to be about 5°C lower than the thermal melting point (Tm) for the specific sequence at a defined ionic strength and pH. The Tm is the temperature (under defined ionic strength and pH) at which 50% of the base pairs have dissociated. Relaxing the stringency of the hybridizing conditions will allow sequence mismatches to be tolerated; the degree of mismatch tolerated can be controlled by suitable adjustment of the hybridization conditions.

For Southern-type hybridization, exemplary conditions are: 50% formamide, 5X SSC, and 1% SDS, incubating at 42°C, or 5X SSC, 1% SDS, incubating at 65°C, with wash in 0.2X SSC, and 0.1% SDS at 55°C, 60°C, or 65°C. Such washes can be performed for 5, 15, 30, 60, 120, or more minutes. For PCR applications (involving hybridization and/or extension of primers and/or probes), hybridization conditions comprising annealing and extension condition are well known, e.g., as described in PCR Protocols: A Guide to Methods and Applications (Innis et al., eds., 1990).

The present invention relies on routine techniques in the field of recombinant genetics. Basic texts disclosing the general methods of use in this invention include Sambrook et al., Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

### III. Methods for Measuring Loss of Imprinting and Cancer Predisposition

Detection of LOI is based on a comparison of the amount of expression derived from each of the two copies of the *IGF2* gene within a biological sample from an individual. Thus, if an individual has two different alleles of the *IGF2* gene, then allele-specific detection can be used to quantify expression of each copy of the gene. If imprinting is functioning, then one copy of the gene (typically the maternal copy) will not be expressed in spite of the presence of a genomic copy of the gene. However, if LOI has occurred, then expression will occur from both the maternal and paternal copies of the *IGF2* gene. Accordingly, it is useful to generate a ratio of the amount of RNA having each allele or otherwise determine their relative amounts. Because expression levels are not always exactly equal when LOI has occurred, in some embodiments, a sample is determined to display LOI of *IGF2* if the quantified proportion of the lesser abundant allele is greater than or equal to 33.3% the quantified proportion of the more abundant allele in heterozygous individuals *(i. e.* a 3:1 ratio of the more abundantly expressed allele to the less abundantly expressed allele). Expression of the lesser abundant allele at a level lower than 33.3% the quantified portions of the more abundant allele may also indicate loss of imprinting of the *IGF2* gene and be associated with increased cancer risk. Therefore, in some embodiments, a sample is determined to display LOI of *IGF2* if the quantified proportion of the lesser abundant allele is greater than or equal to, for example, 10%, 15%, 20%, 25% or 30% the quantified proportion of the more abundant allele in heterozygous individuals *(i. e.* a ratio of the more abundantly expressed allele to the less abundantly expressed allele of 10.00:1, 6.67:1, 5.00:1, 4.00:1 or 3.33:1, respectively). In some embodiments, a sample is determined to display LOI *of IGF2* if the quantified proportion of the lesser abundant allele is greater than or equal to, for example, a ratio of 10:1, 9:1, 8:1, 7:1, 6:1, 5:1, or 4:1 to the more abundant allele in heterozygous individuals (*i.e.* 10%, 11.11%, 12.50%, 14.29% or 16.67%, 20.00%, or 25.00% the quantified proportion of the more abundant allele, respectively).

It is generally desirable to know whether an individual is heterozygous for a particular SNP. Thus in some embodiments, both DNA (i.e., genomic DNA) and RNA from a sample are obtained. The genomic DNA is assayed to determine whether the individual is heterozygous for a particular SNP. If the individual is heterozygous for one or more SNP of the combination of SNPs genotyped, then it is possible to measure loss of imprinting by detecting RNA having either of the two or more possible SNP alleles selected from the heterozygous SNPs and then comparing their expression. This is illustrated in Figure 2. In some embodiments, one detects the genotype of a combination of SNPs as set forth herein, and then LOI is determined for one or more of the SNPs determined to be heterozygous.

In some circumstances, however, it may be beneficial to detect only RNA without previously knowing whether the individual is a heterozygote for a particular SNP. In this circumstance, observing relatively similar quantities of expression of two alleles indicates LOI, while detecting expression of one allele is not informative because it will not be known if the negative result is due to imprinting or homozygosity of the particular SNP. However, by increasing the number of different SNPs detected, it is possible to design an assay such that the chance of an individual being homozygous for every SNP would be low. As explained herein, the combinations of SNPs provided make it unlikely that a particular individual would be homozygous for each SNP in the combination. Moreover, because imprinting is not generally fully complete, it is possible to determine the genotype of an individual by measuring RNA quantity alone if one uses a sensitive enough assay such that one can distinguish between an imprinted allele (which will still generate an extremely small amount of transcript, thus indicating a heterozygote), and a homozygote (which does not make any transcript for a second allele).

In some embodiments, one set of SNPs can be selected to allow for the greatest chance of assaying for a heterozygous SNP regardless of race. Thus, in some embodiments, a panel of SNPs selected from Table 5 is used.

### IV. Methods of SNP Detection

Detection techniques for evaluating nucleic acids for the presence of a SNP involve procedures well known in the field of molecular genetics. Further, many of the methods involve amplification of nucleic acids. Ample guidance for performing SNP detection is provided in the art. Exemplary references include manuals such as PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Current Protocols in Molecular Biology, Ausubel, 1994-1999, including supplemental updates through April 2004; Sambrook & Russell, Molecular Cloning, A Laboratory Manual (3rd Ed, 2001).

Although the methods typically employ PCR steps, other amplification or non-amplification-based protocols may also be used. Suitable amplification methods include ligase chain reaction *(see, e.g.,* Wu & Wallace, Genomics 4:560-569, 1988); strand displacement assay *(see, e.g.,* Walker et al., Proc. Natl. Acad. Sci. USA 89:392-396, 1992; U.S. Pat. No. 5,455,166); and several transcription-based amplification systems, including the methods described in U.S. Pat. Nos. 5,437,990; 5,409,818; and 5,399,491; the transcription amplification system (TAS) (Kwoh et al., Proc. Natl. Acad. Sci. USA 86:1173-1177, 1989); and self-sustained sequence replication (3SR) (Guatelli et al., Proc. Natl. Acad. Sci. USA 87:1874-1878,1990; WO 92/08800). Alternatively, methods that amplify the probe to detectable levels can be used, such as Qβ-replicase amplification (Kramer & Lizardi, Nature 339:401-402,1989; Lomeli et al., Clin. Chem. 35:1826-1831, 1989). A review of known amplification methods is provided, for example, by Abramson and Myers in Current Opinion in Biotechnology 4:41-47, 1993.

Typically, detecting SNPs in an individual is performed using oligonucleotide primers and/or probes. Oligonucleotides can be prepared by any suitable method, usually chemical synthesis. Oligonucleotides can be synthesized using commercially available reagents and instruments. Alternatively, they can be purchased through commercial sources. Methods of synthesizing oligonucleotides are well known in the art *(see, e.g,* Narang et al., Meth. Enzymol. 68:90-99, 1979; Brown et al., Meth. Enzymol. 68:109-151, 1979; Beaucage et al., Tetrahedron Lett. 22:1859-1862, 1981; and the solid support method of U.S. Pat. No. 4,458,066). In addition, modifications to the above-described methods of synthesis may be used to desirably impact enzyme behavior with respect to the synthesized oligonucleotides. For example, incorporation of modified phosphodiester linkages (e.g., phosphorothioate, methylphosphonates, phosphoamidate, or boranophosphate) or linkages other than a phosphorous acid derivative into an oligonucleotide may be used to prevent cleavage at a selected site. In addition, the use of 2'-amino modified sugars tends to favor displacement over digestion of the oligonucleotide when hybridized to a nucleic acid that is also the template for synthesis of a new nucleic acid strand.

The amount and/or presence of an allele of a SNP of the invention in a sample from an individual can be determined using many detection methods that are well known in the art. A number of SNP assay formats entail one of several general protocols: hybridization using allele-specific oligonucleotides, primer extension, allele-specific ligation, sequencing, or electrophoretic separation techniques, e.g., singled-stranded conformational polymorphism (SSCP) and heteroduplex analysis. Exemplary assays include 5' nuclease assays, template-directed dye-terminator incorporation, molecular beacon allele-specific oligonucleotide assays, single-base extension assays, and SNP scoring by real-time pyrophosphate sequences. Analysis of amplified sequences can be performed using various technologies such as microchips, fluorescence polarization assays, and matrix-assisted laser desorption ionization (MALDI) mass spectrometry. Two methods that can also be used are assays based on invasive cleavage with Flap nucleases and methodologies employing padlock probes.

Determining the presence or absence of a particular SNP allele is generally performed by analyzing a nucleic acid sample that is obtained from a biological sample from the individual to be analyzed. While the amount and/or presence of a SNP allele can be directly measured using RNA from the sample, often times the RNA in a sample will be reverse transcribed, optionally amplified, and then the SNP allele will be detected in the resulting cDNA.

Frequently used methodologies for analysis of nucleic acid samples to measure the amount and/or presence of an allele of a SNP are briefly described. However, any method known in the art can be used in the invention to measure the amount and/or presence of single nucleotide polymorphisms.

### Allele Specific Hybridization

This technique, also commonly referred to as allele specific oligonucleotide hybridization (ASO) *(e.g.,* Stoneking et al., Am. J. Hum. Genet. 48:70-382, 1991; Saiki et al., Nature 324, 163-166, 1986; EP 235,726; and WO 89/11548), relies on distinguishing between two DNA molecules differing by one base by hybridizing an oligonucleotide probe that is specific for one of the variants to an amplified product obtained from amplifying the nucleic acid sample. In some embodiments, this method employs short oligonucleotides, *e.g.,* 15-20 bases in length. The probes are designed to differentially hybridize to one variant versus another. Principles and guidance for designing such probe is available in the art, e.g., in the references cited herein. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA or cDNA such that the polymorphic site aligns with a central position (e.g., within 4 bases of the center of the oligonucleotide, for example, in a 15-base oligonucleotide at the 7 position; in a 16-based oligonucleotide at either the 8 or 9 position) of the probe (e.g., a polynucleotide of the invention distinguishes between two SNP alleles as set forth herein), but this design is not required.

The amount and/or presence of an allele is determined by measuring the amount of allele-specific oligonucleotide that is hybridized to the sample. Typically, the oligonucleotide is labeled with a label such as a fluorescent label. For example, an allele-specific oligonucleotide is applied to immobilized oligonucleotides representing potential SNP sequences. After stringent hybridization and washing conditions, fluorescence intensity is measured for each SNP oligonucleotide.

In one embodiment, the nucleotide present at the polymorphic site is identified by hybridization under sequence-specific hybridization conditions with an oligonucleotide probe exactly complementary to one of the polymorphic alleles in a region encompassing the polymorphic site. The probe hybridizing sequence and sequence-specific hybridization conditions are selected such that a single mismatch at the polymorphic site destabilizes the hybridization duplex sufficiently so that it is effectively not formed. Thus, under sequence-specific hybridization conditions, stable duplexes will form only between the probe and the exactly complementary allelic sequence. Thus, oligonucleotides from about 10 to about 35 nucleotides in length, e.g., from about 15 to about 35 nucleotides in length, which are exactly complementary to an allele sequence in a region which encompasses the polymorphic site are within the scope of the invention (e.g., one of SEQ ID NOs: 1-16).

In an alternative embodiment, the amount and/or presence of the nucleotide at the polymorphic site is identified by hybridization under sufficiently stringent hybridization conditions with an oligonucleotide substantially complementary to one of the SNP alleles in a region encompassing the polymorphic site, and exactly complementary to the allele at the polymorphic site. Because mismatches that occur at non-polymorphic sites are mismatches with both allele sequences, the difference in the number of mismatches in a duplex formed with the target allele sequence and in a duplex formed with the corresponding non-target allele sequence is the same as when an oligonucleotide exactly complementary to the target allele sequence is used. In this embodiment, the hybridization conditions are relaxed sufficiently to allow the formation of stable duplexes with the target sequence, while maintaining sufficient stringency to preclude the formation of stable duplexes with non-target sequences. Under such sufficiently stringent hybridization conditions, stable duplexes will form only between the probe and the target allele. Thus, oligonucleotides from about 10 to about 35 nucleotides in length, preferably from about 15 to about 35 nucleotides in length, which are substantially complementary to an allele sequence in a region which encompasses the polymorphic site, and are exactly complementary to the allele sequence at the polymorphic site, are within the scope of the invention.

The use of substantially, rather than exactly, complementary oligonucleotides may be desirable in assay formats in which optimization of hybridization conditions is limited. For example, in a typical multi-target immobilized-probe assay format, probes for each target are immobilized on a single solid support. Hybridizations are carried out simultaneously by contacting the solid support with a solution containing target DNA or cDNA. As all hybridizations are carried out under identical conditions, the hybridization conditions cannot be separately optimized for each probe. The incorporation of mismatches into a probe can be used to adjust duplex stability when the assay format precludes adjusting the hybridization conditions. The effect of a particular introduced mismatch on duplex stability is well known, and the duplex stability can be routinely both estimated and empirically determined, as described above. Suitable hybridization conditions, which depend on the exact size and sequence of the probe, can be selected empirically using the guidance provided herein and well known in the art. The use of oligonucleotide probes to detect single base pair differences in sequence is described in, for example, Conner et al., 1983, Proc. Natl. Acad. Sci. USA 80:278-282, and U.S. Pat. Nos. 5,468,613 and 5,604,099, each incorporated herein by reference.

The proportional change in stability between a perfectly matched and a single-base mismatched hybridization duplex depends on the length of the hybridized oligonucleotides. Duplexes formed with shorter probe sequences are destabilized proportionally more by the presence of a mismatch. In practice, oligonucleotides between about 15 and about 35 nucleotides in length are preferred for sequence-specific detection. Furthermore, because the ends of a hybridized oligonucleotide undergo continuous random dissociation and re-annealing due to thermal energy, a mismatch at either end destabilizes the hybridization duplex less than a mismatch occurring internally. Preferably, for discrimination of a single base pair change in target sequence, the probe sequence is selected which hybridizes to the target sequence such that the polymorphic site occurs in the interior region of the probe.

The above criteria for selecting a probe sequence that hybridizes to a particular SNP apply to the hybridizing region of the probe, i.e., that part of the probe which is involved in hybridization with the target sequence. A probe may be bound to an additional nucleic acid sequence, such as a poly-T tail used to immobilize the probe, without significantly altering the hybridization characteristics of the probe. One of skill in the art will recognize that for use in the present methods, a probe bound to an additional nucleic acid sequence which is not complementary to the target sequence and, thus, is not involved in the hybridization, is essentially equivalent to the unbound probe.

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Pat. Nos. 5,310,893; 5,451,512; 5,468,613; and 5,604,099; each incorporated herein by reference.

In a dot-blot format, amplified target DNA or cDNA is immobilized on a solid support, such as a nylon membrane. The membrane-target complex is incubated with labeled probe under suitable hybridization conditions, unhybridized probe is removed by washing under suitably stringent conditions, and the membrane is monitored for the presence of bound probe.

In the reverse dot-blot (or line-blot) format, the probes are immobilized on a solid support, such as a nylon membrane or a microtiter plate. The target DNA or cDNA is labeled, typically during amplification by the incorporation of labeled primers. One or both of the primers can be labeled. The membrane-probe complex is incubated with the labeled amplified target DNA or cDNA under suitable hybridization conditions, unhybridized target DNA or cDNA is removed by washing under suitably stringent conditions, and the membrane is monitored for the presence of bound target DNA or cDNA.

An allele-specific probe that is specific for one of the polymorphism variants is often used in conjunction with the allele-specific probe for the other polymorphism variant. In some embodiments, the probes are immobilized on a solid support and the target sequence in an individual is analyzed using both probes simultaneously. Examples of nucleic acid arrays are described by WO 95/11995. The same array or a different array can be used for analysis of characterized polymorphisms. WO 95/11995 also describes subarrays that are optimized for detection of variant forms of a pre-characterized polymorphism.

### Allele-Specific Primers

The amount and/or presence of an allele is also commonly detected using allele-specific amplification or primer extension methods. These reactions typically involve use of primers that are designed to specifically target a polymorphism via a mismatch at the 3' end of a primer. The presence of a mismatch affects the ability of a polymerase to extend a primer when the polymerase lacks error-correcting activity. For example, to detect an allele sequence using an allele-specific amplification- or extension-based method, a primer complementary to the polymorphic nucleotide of a SNP is designed such that the 3' terminal nucleotide hybridizes at the polymorphic position. The presence of the particular allele can be determined by the ability of the primer to initiate extension. If the 3' terminus is mismatched, the extension is impeded. If a primer matches the polymorphic nucleotide at the 3' end, the primer will be efficiently extended.

Typically, the primer is used in conjunction with a second primer in an amplification reaction. The second primer hybridizes at a site unrelated to the polymorphic position. Amplification proceeds from the two primers leading to a detectable product signifying the particular allelic form is present. Allele-specific amplification- or extension-based methods are described in, for example, WO 93/22456; U.S. Pat. Nos. 5,137,806; 5,595,890; 5,639,611; and U.S. Pat. No. 4,851,331.

Using allele-specific amplification-based methods, identification and/or quantification of the alleles require detection of the presence or absence of amplified target sequences. Methods for the detection of amplified target sequences are well known in the art. For example, gel electrophoresis and probe hybridization assays described are often used to detect the presence of nucleic acids.

In an alternative probe-less method, the amplified nucleic acid is detected by monitoring the increase in the total amount of double-stranded DNA in the reaction mixture, is described, e.g., in U.S. Pat. No. 5,994,056; and European Patent Publication Nos. 487,218 and 512,334. The detection of double-stranded target DNA or cDNA relies on the increased fluorescence various DNA-binding dyes, e.g., SYBR Green, exhibit when bound to double-stranded DNA.

As appreciated by one in the art, allele-specific amplification methods can be performed in reactions that employ multiple allele-specific primers to target particular alleles. Primers for such multiplex applications are generally labeled with distinguishable labels or are selected such that the amplification products produced from the alleles are distinguishable by size. Thus, for example, both alleles in a single sample can be identified and/or quantified using a single amplification by various methods.

As in the case of allele-specific probes, an allele-specific oligonucleotide primer may be exactly complementary to one of the polymorphic alleles in the hybridizing region or may have some mismatches at positions other than the 3' terminus of the oligonucleotide, which mismatches occur at non-polymorphic sites in both allele sequences.

### 5'-nuclease assay

The amount and/or presence of an allele can also be determined using a "TaqMan®" or "5'-nuclease assay", as described in U.S. Pat. Nos. 5,210,015; 5,487,972; and 5,804,375; and Holland et al., 1988, Proc. Natl. Acad. Sci. USA 88:7276-7280. In the TaqMan® assay, labeled detection probes that hybridize within the amplified region are added during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

The hybridization probe can be an allele-specific probe that discriminates between the SNP alleles. Alternatively, the method can be performed using an allele-specific primer and a labeled probe that binds to amplified product.

Any method suitable for detecting degradation product can be used in a 5' nuclease assay. Often, the detection probe is labeled with two fluorescent dyes, one of which is capable of quenching the fluorescence of the other dye. The dyes are attached to the probe, preferably one attached to the 5' terminus and the other is attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Pat. Nos. 5,491,063 and 5,571,673, both incorporated herein by reference, describe alternative methods for detecting the degradation of probe which occurs concomitant with amplification.

### DNA Sequencing and single base or other primer extensions

The amount and/or presence of an allele can also be determined by direct sequencing. Methods include e.g., dideoxy sequencing-based methods and other methods such as Maxam and Gilbert sequence *(see, e.g.,* Sambrook and Russell, *supra*).

Other detection methods include Pyrosequencing™ of oligonucleotide-length products. Such methods often employ amplification techniques such as PCR. For example, in pyrosequencing, a sequencing primer is hybridized to a single stranded, PCR-amplified, DNA or cDNA template; and incubated with the enzymes, DNA polymerase, ATP sulfurylase, luciferase and apyrase, and the substrates, adenosine 5' phosphosulfate (APS) and luciferin. The first of four deoxynucleotide triphosphates (dNTP) is added to the reaction. DNA polymerase catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide. ATP sulfurylase quantitatively converts PPi to ATP in the presence of adenosine 5' phosphosulfate. This ATP drives the luciferase-mediated conversion of luciferin to oxyluciferin that generates visible light in amounts that are proportional to the amount of ATP. The light produced in the luciferase-catalyzed reaction is detected by a charge coupled device (CCD) camera and seen as a peak in a pyrogram™. Each light signal is proportional to the number of nucleotides incorporated. Apyrase, a nucleotide degrading enzyme, continuously degrades unincorporated dNTPs and excess ATP. When degradation is complete, another dNTP is added.

Another similar method for characterizing SNPs does not require use of a complete PCR, but typically uses only the extension of a primer by a single nucleotide, which is complementary to any of the allelic options of the SNP, and in some cases which is modified so as to be easily detected. Modifications of ddNTPs can include, but are not limited to, fluorescent labeling or mass modification. The incorporated nucleotide at the polymorphic site can then be identified via detection of a primer that has been extended by one base and is fluorescently labeled or mass modified (*e.g.,* Kobayashi et al, Mol. Cell. Probes, 9:175-182, 1995). Of course extension products can also be detected based on other types of labels, or by mass-spectrometry, as desired.

In a similar method, PCR amplified target DNA or RT-PCR amplified target cDNA may be used as template for a single nucleotide primer extension reaction whereby a single fluorescently labeled ddNTP complementary to the polymorphic nucleotide is incorporated on the 3' end of a single primer. Each specific ddNTP can be labeled with a different fluorescent dye (*e.g.* ddATP labeled with dR6G, ddCTP labeled with dTAMRA™, ddGTP labeled with dR110 and ddTTP or ddUTP labeled with dROX™). Therefore, single nucleotide extension of the initially unlabeled primer tags the primer with a specific fluorescent dye that identifies the base that was added to the 3' end of the unlabeled primer. Extended primers can be resolved and analyzed to determine the presence and relative quantity of each specific dye-tagged primer, representing the relative quantities of each allele in the target DNA or target cDNA template.

### Restriction Fragment Length Polymorphism Analysis

In other embodiments, the amount and/or presence of an allele of a SNP can be determined by differential digestion of amplified target DNA or cDNA when the polymorphic nucleotide of interest lies within the recognition sequence of a restriction enzyme. In one case, one allele of the SNP (the first allele) maintains the recognition sequence of the restriction enzyme and the other allele (the second allele) does not. In this case, the restriction enzyme will cleave the target DNA or cDNA including the first allele, but not the target DNA or cDNA including the second allele. In another case, one allele (the first allele) of the SNP maintains the recognition sequence of a restriction enzyme (the first restriction enzyme) and the other allele (the second allele) maintains the recognition sequence of a different restriction enzyme (the second restriction enzyme). In this case, the first restriction enzyme will cleave the target DNA or cDNA including the first allele, but not the target DNA or cDNA including the second allele. The second restriction enzyme will cleave the target DNA or cDNA including the second allele, but not the target DNA or cDNA including the first allele. The amount and/or presence of alleles can be determined by various methods including, but not limited to, Southern blot hybridization to immobilized restricted fragments and quantification of band intensities, resolution and visualization of restriction fragments by gel electrophoresis, resolution and quantification of restriction fragments by capillary electrophoresis (such as performed using an Agilent BioAnalyzer), or differential quantitative PCR amplification of cleaved versus uncleaved template DNA or cDNA.

### Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution *(see, e.g.,* Erlich, ed., PCR Technology, Principles and Applications for DNA Amplification, W. H. Freeman and Co, New York, 1992, Chapter 7).

### Single-Strand Conformation Polymorphism Analysis

Alleles of target sequences can be differentiated using single-strand conformation polymorphism analysis, which identifies base differences by alteration in electrophoretic migration of single stranded PCR products, as described, *e.g,* in Orita et al., Proc. Nat. Acad. Sci. 86, 2766-2770 (1989). Amplified PCR or RT-PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of target

SNP detection methods often employ labeled oligonucleotides. Oligonucleotides can be labeled by incorporating a label detectable by spectroscopic, photochemical, biochemical, immunochemical, or chemical means. Useful labels include fluorescent dyes, radioactive labels, e.g., ³²P, electron-dense reagents, enzyme, such as peroxidase or alkaline phosphatase, biotin, or haptens and proteins for which antisera or monoclonal antibodies are available. Labeling techniques are well known in the art *(see, e.g.,* Current Protocols in Molecular Biology, supra; Sambrook & Russell, supra).

### V. Methods for Quantifying RNA

The presence and quantity of RNA corresponding to a particular SNP can be readily determined according to any method for quantifying RNA. Various methods involving linkage of RNA to a solid support and probing the RNA (e.g., northern blots, dot blots, etc.) can be used.

In some embodiments, the target RNA is first reverse transcribed (e.g., with reverse transcriptase) and then the resulting cDNA is quantified by any methods known in the art (blot hybridization, RT-PCR, etc.) as a surrogate for RNA quantity. Various methods of reverse transcription are known and described, e.g., in Sambrook et al., Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)), and can involve reverse transcription using either specific or non -specific primers.

In some embodiments, RT-PCR or other quantitative amplification techniques are used to quantify the target RNA. Amplification of cDNA using reactions is well known (see U.S. Patents 4,683,195 and 4,683,202; PCR PROTOCOLS: A GUIDE TO METHODS AND APPLICATIONS (Innis et al., eds, 1990)).

Sequences amplified by the methods of the invention can be further evaluated, detected, cloned, sequenced, and the like, either in solution or after binding to a solid support, by any method usually applied to the detection of a specific DNA sequence such as PCR, oligomer restriction (Saiki, et al., Bio/Technology 3:1008-1012 (1985)), allele-specific oligonucleotide (ASO) probe analysis (Conner, et al., PNAS USA 80:278 (1983)), oligonucleotide ligation assays (OLAs) (Landegren, et al., Science 241:1077, (1988)), and the like. Molecular techniques for DNA analysis have been reviewed (Landegren, et al., Science 242:229-237 (1988)).

Methods of quantitative amplification are disclosed in, *e.g.,* U.S. Patent Nos. 6,180,349; 6,033,854; and 5,972,602, as well as in, *e.g.,* Gibson et al., Genome Research 6:995-1001 (1996); DeGraves, et al., Biotechniques 34(1):106-10,112-5 (2003); Deiman B, et al., Mol Biotechnol. 20(2):163-79 (2002). Amplifications may be monitored in "real time."

In general, quantitative amplification is based on the monitoring of the signal (e.g., fluorescence of a probe) representing copies of the template in cycles of an amplification *(e.g.,* PCR) reaction. In the initial cycles of the PCR, a very low signal is observed because the quantity of the amplicon formed does not support a measurable signal output from the assay. After the initial cycles, as the amount of formed amplicon increases, the signal intensity increases to a measurable level and reaches a plateau in later cycles when the PCR enters into a non-logarithmic phase. Through a plot of the signal intensity versus the cycle number, the specific cycle at which a measurable signal is obtained from the PCR reaction can be deduced and used to back-calculate the quantity of the target before the start of the PCR. The number of the specific cycles that is determined by this method is typically referred to as the cycle threshold (Ct). Exemplary methods are described in, *e.g.,* Heid et al. Genome Methods 6:986-94 (1996) with reference to hydrolysis probes.

One method for detection of amplification products is the 5'-3' exonuclease "hydrolysis" PCR assay (also referred to as the TaqMan™ assay) (U.S. Pat. Nos. 5,210,015 and 5,487,972; Holland et al., PNAS USA 88: 7276-7280 (1991); Lee et al., Nucleic Acids Res. 21: 3761-3766 (1993)). This assay detects the accumulation of a specific PCR product by hybridization and cleavage of a doubly labeled fluorogenic probe (the "TaqMan™" probe) during the amplification reaction. The fluorogenic probe consists of an oligonucleotide labeled with both a fluorescent reporter dye and a quencher dye. During PCR, this probe is cleaved by the 5'-exonuclease activity of DNA polymerase if, and only if, it hybridizes to the segment being amplified. Cleavage of the probe generates an increase in the fluorescence intensity of the reporter dye.

Another method of detecting amplification products that relies on the use of energy transfer is the "beacon probe" method described by Tyagi and Kramer, Nature Biotech. 14:303-309 (1996), which is also the subject of U.S. Patent Nos. 5,119,801 and 5,312,728. This method employs oligonucleotide hybridization probes that can form hairpin structures. On one end of the hybridization probe (either the 5' or 3' end), there is a donor fluorophore, and on the other end, an acceptor moiety. In the case of the Tyagi and Kramer method, this acceptor moiety is a quencher, that is, the acceptor absorbs energy released by the donor, but then does not itself fluoresce. Thus, when the beacon is in the open conformation, the fluorescence of the donor fluorophore is detectable, whereas when the beacon is in hairpin (closed) conformation, the fluorescence of the donor fluorophore is quenched. When employed in PCR, the molecular beacon probe, which hybridizes to one of the strands of the PCR product, is in "open conformation," and fluorescence is detected, while those that remain unhybridized will not fluoresce (Tyagi and Kramer, Nature Biotechnol. 14: 303-306 (1996)). As a result, the amount of fluorescence will increase as the amount of PCR product increases, and thus may be used as a measure of the progress of the PCR. Those of skill in the art will recognize that other methods of quantitative amplification are also available.

Various other techniques for performing quantitative amplification of nucleic acids are also known. For example, some methodologies employ one or more probe oligonucleotides that are structured such that a change in fluorescence is generated when the oligonucleotide(s) is hybridized to a target nucleic acid. For example, one such method involves is a dual fluorophore approach that exploits fluorescence resonance energy transfer (FRET), *e.g.,* LightCycler™ hybridization probes, where two oligo probes anneal to the amplicon. The oligonucleotides are designed to hybridize in a head-to-tail orientation with the fluorophores separated at a distance that is compatible with efficient energy transfer. Other examples of labeled oligonucleotides that are structured to emit a signal when bound to a nucleic acid or incorporated into an extension product include: Scorpions™ probes (e.g., Whitcombe et al., Nature Biotechnology 17:804-807, 1999, and U.S. Pat. No. 6,326,145), Sunrise™ (or Amplifluor™) probes (*e.g.,* Nazarenko et al., Nuc. Acids Res. 25:2516-2521, 1997, and U.S. Pat. No. 6,117,635), and probes that form a secondary structure that results in reduced signal without a quencher and that emits increased signal when hybridized to a target (e.g., Lux probes™).

In other embodiments, intercalating agents that produce a signal when intercalated in double stranded DNA may be used. Exemplary agents include SYBR GREEN™ and SYBR GOLD™. Since these agents are not template-specific, it is assumed that the signal is generated based on template-specific amplification. This can be confirmed by monitoring signal as a function of temperature because melting point of template sequences will generally be much higher than, for example, primer-dimers, *etc.*

### VI. Kits

The invention also provides kits comprising useful components for practicing the methods. In some embodiments, the kit may comprise one or both allele-specific detection polynucleotides (e.g., primers or probes) for any of the combinations of SNPs described herein. Such polynucleotides can optionally be fixed to an appropriate support membrane. In some embodiments, the kits comprise a sufficient number of polynucleotides to detection a combination of SNPs as described herein. In some embodiments, the first and second SNP is selected from the group consisting of SEQ ID NOs:1, 2, 3, 4, 5, 6, 7, 8, 9, 10, 11, 12, 13, 14, 15, and 16, optionally wherein at least one SNP is 1, 2, 3, 4, 5, or 6.

In some embodiments, the kit comprises one or both allele-specific detection polynucleotides (e.g., primers or probes) for each of SEQ ID NOs: 1, 5, 8, 9, 10, 11, 14, 15 and 16. In some embodiments, the kit comprises one or both allele-specific detection polynucleotides (e.g., primers or probes) for each of SEQ ID NOs: 1, 5, 8, 9, 10, 11, 12, 14, 15 or 16. In some embodiments, the kit comprises one or both allele-specific detection polynucleotides (e.g., primers or probes) for each of SEQ ID NOs: 1, 2, 4, 5, 8, 9, 10, 12, 13, 14, 15 or 16. In some embodiments, the kit comprises one or both allele-specific detection polynucleotides (e.g., primers or probes) for each of SEQ ID NOs: 1-16.

In some embodiments, one, some or all of the polynucleotides for detection of the combination of SNPs comprise a sequence 100% identical to at least 8 (e.g., at least 10, 12, 15) contiguous nucleotides, or the complement thereof, of the SNPs to be detected. For example, if three SNPs are to be detected (arbitrarily designated A, B, and C), a kit can comprise at least three poynucelotides wherein:
the first polynucleotide detects (and optionally comprises a sequence 100% identical to at least 8 (e.g., at least 10, 12, 15) contiguous nucleotides, or the complement thereof, of SNP A;
the second polynucleotide detects (and optionally comprises a sequence 100% identical to at least 8 (e.g., at least 10, 12, 15) contiguous nucleotides, or the complement thereof, of SNP B;
the third polynucleotide detects (and optionally comprises a sequence 100% identical to at least 8 (e.g., at least 10, 12, 15) contiguous nucleotides, or the complement thereof, of SNP C.

In some embodiments, the sequence of the allele-specific detection polynucleotide comprises or consists of at least 8 (e.g., at least 10, 12, 15) contiguous nucleotides directly 5' (or is 2 or 3 nucleotides 5') from the variable position of the target SNP and the sequence is at the 3' end of the polynucleotide. Such options are conveniently used for, e.g., primer extension reactions. In some embodiments, the sequence comprises or consists of the complement of at least 8 (e.g., at least 10, 12, 15) contiguous nucleotides directly 3' (or is 2 or 3 nucleotides 3') from the variable position of the SNP and the sequence is at the 3' end of the polynucleotide. Such options are also conveniently used for, e.g., primer extension reactions.

In some embodiments, the sequence consists of at least 8 (e.g., at least 10, 12, 15) contiguous nucleotides, or the complement thereof, of the SNP, wherein one of the positions of the sequence corresponds to the variable position of the SNP. Such embodiments are useful, for example, for assays where differential hybridization to SNP alleles is involved.

Such a kit can also contain amplification primers for amplifying a region of the *IGF2* locus encompassing the polymorphic site. Alternatively, useful kits can contain a set of primers comprising an allele-specific primer for the specific amplification of the polymorphic alleles. Such a kit may also comprise probes for the detection of amplification products. Alternatively, useful kits can contain a set of primers complementary to sequences 5' to but not including the SNP positions of interest (or complements thereof) for use in primer extension methods as described above.

Other optional components of the kits include additional reagents used for genotyping patients and/or quantifying the relative amount of specific alleles present. For example, a kit can contain a polymerase, labeled or unlabeled substrate nucleoside triphosphates (optionally, where some or each different nucleotides are labeled with a different, distinguishable label, means for labeling and/or detecting nucleic acid, appropriate buffers for amplification or hybridization reactions, and/or instructions for carrying out the present method.

Optionally kit swill comprise control nucleic acid samples, e.g., a sample comprising only one possible allele and optionally a control having two (or, where appropriate, all three) possible alleles of a particular SNP. Controls can include at least one control nucleic acid or mixture of nucleic acids for each SNP (e.g., having one or both alleles). Negative and other controls can also be included.

### VII. Reaction Mixtures

The present invention provides for reaction mixtures for carrying out the invention. In some embodiments, the reaction mixtures may comprise one or both allele-specific detection polynucleotides (e.g., primers or probes) for any of the combinations of SNPs described herein. Such polynucleotides can optionally be fixed to an appropriate support membrane. In some embodiments, the reaction mixtures comprise a sufficient number of polynucleotides to detection a combination of SNPs as described herein. For example, in some embodiments, the reaction mixtures can comprise a first polynucleotide of between 8-100 nucleotides, wherein the first polynucleotide distinguishes between one allele of a first SNP (or complement thereof) and the other allele of the first SNP (or complement thereof) in a hybridization reaction, a second polynucleotide of between 8-100 nucleotides, wherein the second polynucleotide distinguishes between one allele of a second SNP (or complement thereof) and the other allele of the second SNP (or complement thereof) in a hybridization reaction, wherein the first and second SNP is selected from the group consisting of SEQ ID NOs:1, 2, 3, 4, 5, 6, 7, 8, 9, 10, 11, 12, 13, 14, 15, and 16, optionally wherein at least one SNP is selected from SEQ ID NO:s 1, 2, 3, 4, 5, or 6. Exemplary combinations include, but are not limited to:
SEQ ID NOs: 1,2, 3,4, 5, 6, 7, 8, 9,10,11,12,13,14,15, and 16; or
SEQ ID NOs: 1, 5, 8, 9,10,11,14,15, and 16; or
SEQ ID NOs: 1,5, 8, 9,10,11,12,14,15, and 16; or
SEQ ID NOs: 1, 2, 4, 5, 8, 9, 10, 12, 13, 14, 15, and 16.

In some embodiments, the reaction mixture comprises one or both allele-specific detection polynucleotides (e.g., primers or probes) for each of SEQ ID NOs: 1, 5, 8, 9, 10, 11, 14, 15 and 16. In some embodiments, the reaction mixture comprises one or both allele-specific detection polynucleotides (e.g., primers or probes) for each of SEQ ID NOs: 1, 5, 8, 9, 10, 11, 12, 14, 15 or 16. In some embodiments, the reaction mixture comprises one or both allele-specific detection polynucleotides (e.g., primers or probes) for each of SEQ ID NOs: 1, 2, 4, 5, 8, 9, 10, 12, 13, 14, 15 or 16. In some embodiments, the reaction mixture comprises one or both allele-specific detection polynucleotides (e.g., primers or probes) for each of SEQ ID NOs: 1-16.

In some embodiments, one, some or all of the polynucleotides for detection of the combination of SNPs comprise a sequence 100% identical to at least 8 (e.g., at least 10, 12, 15) contiguous nucleotides, or the complement thereof, of the SNPs to be detected. For example, if three SNPs are to be detected (arbitrarily designated A, B, and C), a kit can comprise at least three polynucleotides wherein:
the first polynucleotide detects (and optionally comprises a sequence 100% identical to at least 8 (e.g., at least 10, 12, 15) contiguous nucleotides, or the complement thereof, of SNP A;
the second polynucleotide detects (and optionally comprises a sequence 100% identical to at least 8 (e.g., at least 10, 12, 15) contiguous nucleotides, or the complement thereof, of SNP B;
the third polynucleotide detects (and optionally comprises a sequence 100% identical to at least 8 (e.g., at least 10, 12, 15) contiguous nucleotides, or the complement thereof, of SNP C.

In some embodiments, the sequence comprises or consists of at least 8 (e.g., at least 10, 12, 15) contiguous nucleotides directly 5' (or is 2 or 3 nucleotides 5') from the variable position of the target SNP and the sequence is at the 3' end of the polynucleotide. Such options are conveniently used for, e.g., primer extension reactions. In some embodiments, the sequence comprises or consists of the complement of at least 8 (e.g., at least 10, 12, 15) contiguous nucleotides directly 3' (or is 2 or 3 nucleotides 3') from the variable position of the SNP and the sequence is at the 3' end of the polynucleotide. Such options are also conveniently used for, e.g., primer extension reactions.

In some embodiments, the sequence consists of at least 8 (e.g., at least 10, 12, 15) contiguous nucleotides, or the complement thereof, of the SNP, wherein one of the positions of the sequence corresponds to the variable position of the SNP. Such embodiments are useful, for example, for assays where differential hybridization to SNP alleles is involved.

Such reaction mixtures can also contain amplification primers for amplifying a region of the *IGF2* locus encompassing the polymorphic site. Alternatively, reaction mixtures can contain a set of primers comprising an allele-specific primer for the specific amplification of the polymorphic alleles. Such a reaction mixtures may also comprises probes for the detection of amplification products. Alternatively, useful reaction mixtures can contain a set of primers complementary to sequences 5' to but not including the SNP positions of interest (or complements thereof) for use in primer extension methods as described above.

Other optional components of the reaction mixtures include additional reagents used for genotyping patients and/or quantifying the relative amount of specific alleles present. For example, a kit can contain a polymerase, labeled or unlabeled substrate nucleoside triphosphates (optionally, where some or each different nucleotides are labeled with a different, distinguishable label, means for labeling and/or detecting nucleic acid, appropriate buffers for amplification or hybridization reactions, and/or instructions for carrying out the present method. Optionally, the reaction mixtures include a nucleic cid sample from a human.

### VIII. CONTROLS FOR VALIDATING LOI MEASUREMENTS

An assay that determines the imprinting status of IGF2 in a clinical sample involves a determination of the ratio of the amount of expression from one allele of the gene and the amount of expression of the other allele, and a comparison of the ratio to a cutoff value associated with loss of imprinting of the gene, and or associated with a clinical factor such as the presence of cancer, the risk of cancer or the selection of a treatment regime for the patient.

*IGF2* imprinting status measurements are subjected to many process factors that have the potential to introduce either random or systematic error in the assay measurement and therefore have the potential to lower the accuracy of measurement results. For example, imprinting status assays typically require multiple steps. These steps can include the isolation of RNA from the sample, the reverse transcription of RNA into 1st strand cDNA, the second strand synthesis of 1st strand cDNA into 2nd strand cDNA, the amplification of cDNA into amplified cDNA, and the SNP-based quantification of the amplified cDNA copies arising from each transcribed allele of the gene in the sample.

In the clinical testing environment, from time to time, different reagent lots, different equipment, different laboratory personnel, and even different standard operating procedures might be used to measure the same clinical sample multiple times, or optionally to measure different clinical samples. An assay which measures more than one heterozygous SNP may contain components that differ for each measured SNP. For example, different RT primers may be used to reverse transcribe different sequence regions of the transcript, or different PCR primers may be used to amplify cDNA of different sequence regions, or different detection probes used to detect the polymorphic options of the different SNPs of the panel.

If any step or any assay component in a given imprinting status measurement leads to an allele-specific amplification bias, or optionally, to an allele-specific detection bias, then such step or component will effect the measured ratio of alleles and introduce error in the resulting imprinting status ratio. Therefore is it useful to develop controls that can be used to measure the variation of the steps and components of any given imprinting status measurement, and that allow for the correction of these measurements resulting in improved measurement results.

A preferred source of controls for the assays measuring the optimized panels of SNPs described herein would be DNA or RNA isolated from a single pair of individuals, where one individual is homozygous for one allele of every SNP in a given panel, and the another individual is homozygous for the other allele of every SNP in the panel. However, due to the combined heterozygosity frequencies of the SNPs of the optimized panels in the general population, the likelihood of finding such a pair of individuals is extremely low.

An optional source of controls for the assays measuring the optimized panels of SNPs described herein is DNA and/or RNA derived from a set of multiple pairs of individuals; where in each pair of individuals, one individual is homozygous for one allele of a given SNP (e.g., is homozygous for the G allele at a G/A SNP nucleotide position) and the another individual is homozygous for the other allele of the same SNP *(e.g.,* is homozygous for the A allele at the same G/A SNP nucleotide position); and where the set of multiple pairs of individuals represent the different allelic options of all of the SNPs in an optimized panel. By identifying such a set of pairs of individuals, representative sources of DNA or RNA useful for controlling each SNP could be established. However, the use of multiple pairs of controls, where each pair represents one or a few SNPs, would necessitate the generation and use of multiple control curves, and this in turn would greatly increase assay complexity, assay cost, and the cumulative error of the controls thereby eroding the power of a inter assay comparison of ASE ratios determined between multiple heterozygous SNPs within the same clinical sample.

To address these problems, one aspect of the invention provides synthetic control nucleic acid sequences for *IGF2* LOI assays and methods of using the controls. The control nucleic acids of the invention (also referred to herein as "LOI control pairs") are pairs of polynucleotides that represent alleles and the alternative alleles of least two *IGF2* SNPs. Thus, one member of the pair comprises at least two *IGF2* SNPs, *e.g.,* at least two of the exon 9 IGF2 SNPs set forth in SEQ ID NOs. 1-16, or the complement thereof. The nucleotide complementary to one of the SNP alleles (a first SNP) is present on one of the members of the pair; a nucleotide complementary to an alternative allele of the first SNP is present on the second member of the pair. Similarly, for the second *IGF2* SNP for the control, one member of the pair comprises a nucleotide complementary to one of the alleles of the second SNP and the other member of the pair comprises a nucleotide complementary to an alternative allele of the second SNP. In the following description of the control pair of nucleic acids, references to reference SEQ ID NOs also refer to the complementary sequence of the SEQ ID NOs, even though it may not be explicitly stated.

A "LOI control pair" of the invention is "substantially free" of cellular nucleic acids; thus, the nucleic acids are substantially free of genomic DNA, RNA, and the like from a human or a host cell, *e.g.,* a bacterial host cell. For example, the control pair may be obtained using an *in vitro* transcription reaction. By being substantially free of cellular nucleic acids, the LOI control pair can be stored in mixtures at high concentrations and in the presence of nucleic acid stabilizers, so as to remain un-degraded for long periods of time, and can be diluted to working volumes at the time imprinting assays are run on clinical samples to serve as a control.

The control polynucleotide pair may represent any number of SNPs, e.g., the control pair may have 1, 2, 3, 4, 5, 6, 7, 8, 9, 10, 11, 12, 13, 14, 15, or 16, or more, *IGF2* SNPs, where one member of the pair has a nucleotide for one of the SNP alleles for each of the different SNPs and the second member of the pair has a nucleotide for an alternative allele for each of those SNPs. In some embodiments, one member of the pair may have minor allele nucleotides for 2 or more, e.g., 3, 4, 5, 6, 7, 8, 9, 10, 11, 12, 13, 14, 15, or 16, of the SNPs. For example, for a LOI control nucleic acid pair for ten of the SNPs shown in SEQ ID NOs 1-16, one member of the pair may have the nucleotides for a minor allele for all ten SNPs and the other member of the pair may have nucleotides for all of the major alleles of the ten SNPs. Alternatively, for example, one member of the pair may have the nucleotide for a minor allele for two of the ten SNPs with the other eight SNP nucleotides being for the major allele; and the other member of the pair has the nucleotides for the major alleles of the two SNPs and the minor alleles for the other eight SNPs.

In some embodiments, the polynucleotides that make up the control LOI pair are anywhere from 40 nucleotides to about 3600 or 4000 nucleotides, or 4500, 5000, or 6,000 nucleotides, in length. In some embodiments, the control LOI pair is from 40, 50, 60, 70, or 80 nucleotides in length to 100, 200, 300, 400, 600, 800, 1000, 1500, 2000, 2500, or 3000 nucleotides in length. The members of the pair may be of the same length or different lengths.

The control pair polynucleotides can comprise any SNP present in an *IGF2* transcript, including SNPs present in exon 9, exon 8, and exon 7. In some embodiments, the LOI pair comprises exon 9 SNPs.

In some embodiments, an LOI control polynucleotide member of the nucleic acid pair has a 3' nucleic acid sequence adj acent to the first SNP (referred to herein as "3' adjacent sequence") that is substantially identical to at least 15 contiguous nucleotides, typically at least 20, 25, or 50 contiguous nucleotides, to the corresponding region of an *IGF2* transcript, *e.g.,* SEQ ID NO 17 or SEQ ID NO:18; and has a 3' adjacent sequence for the second SNP that is substantially identical to at least 15 contiguous nucleotides, typically at least 20, 25, or 50, contiguous nucleotides, of the corresponding region of an *IGF2* transcript, *e.g*., SEQ ID NO 17 or SEQ ID NO:18. In some embodiments, at least 8 contiguous nucleotides, or at least 10, 12, 15, 20, or 25 contiguous nucleotides, of the 3' nucleic acid sequence adjacent to the first SNP is identical to the corresponding region of an *IGF2* transcript, *e.g.,* SEQ ID NO: 17 or SEQ ID NO:18; and at least at least 8 contiguous nucleotides, or at least 10, 12,15,20, or 25 contiguous nucleotides, of the 3' nucleic acid sequence adjacent to the second SNP is identical to the corresponding region of SEQ ID NO: 17 or SEQ ID NO:18. In the context of this invention as it relates to the description of the control pair of synthetic nucleic acids, "3' nucleic acid sequence adjacent to a SNP" refers to the nucleic acid sequence immediately 3' of the SNP position, including the nucleotide 3' to the SNP site.

In some embodiments, an LOI control polynucleotide member of the nucleic acid pair has a 3' nucleic acid sequence adjacent to the first SNP that is substantially identical to at least 15 contiguous nucleotides, typically at least 20, 25, or 50 contiguous nucleotides, to the corresponding region of an *IGF2* nucleid acid sequence that comprises exon 9 polymorphic sites, *e.g.,* SEQ ID NO:21 or SEQ ID NO:23; and has a 3' adjacent sequence for the second SNP that is substantially identical to at least 15 contiguous nucleotides, typically at least 20, 25, or 50, contiguous nucleotides, of the corresponding region of an *IGF2* nucleic acid sequence *e.g.,* SEQ ID NO:21 or SEQ ID NO:23. In some embodiments, at least 8 contiguous nucleotides, or at least 10, 12, 15, 20, or 25 contiguous nucleotides, of the 3' nucleic acid sequence adjacent to the first SNP is identical to the corresponding region of an *IGF2* sequence *e.g.,* SEQ ID NO:21 or SEQ ID NO:23; and at least at least 8 contiguous nucleotides, or at least 10, 12, 15, 20, or 25 contiguous nucleotides, of the 3' nucleic acid sequence adjacent to the second SNP is identical to the corresponding region of SEQ ID NO:21 or SEQ ID NO:23.

The terms "identical" or "100% identity," in the context of two or more nucleic acids refer to two or more sequences or subsequences that are the same sequences. Two sequences are "substantially identical" or a certain percent identity if two sequences have a specified percentage of amino acid residues or nucleotides that are the same (i.e., 70% identity, optionally 75%, 80%, 85%, 90%, or 95% identity, over a specified region, or, when not specified, over the entire sequence), when compared and aligned for maximum correspondence over a comparison window, or designated region as measured using known sequence comparison algorithms, e.g., BLAST using the default parameters, or by manual alignment and visual inspection.

In some embodiments, at least one of the SNPs included in the control pair is selected from the group consisting of the SNPs as shown in SEQ ID NOs: 1, 2, 3, 4, 5, 6, 7, 8, 9, 10, 11, 12, 13, 14, 15, and 16. In typical embodiments, at least two of the SNPs shown in SEQ ID NOs. 1-16 are include in the control nucleic acid pair. Often, at least 2, 3, 4, 5, 6, 7, 8, 9,10,11,12,13,14,15, or all 16 of the SNPs shown in SEQ ID NOs. 1-16 are included in the control LOI nucleic acid pair. In some embodiments, at least one of the SNPs in the control is selected from SEQ ID NO:1, 2, 3, 4, 5, or 6. In some embodiments, the control LOI nucleic acid pair comprises the SNPs shown in SEQ ID NOs: 1, 5, 8, 9, 10, 11, 14, 15, and 16; SEQ ID NOs. 1, 5, 8, 9, 10, 11, 12, 14, 15, and 16; or SEQ ID NOs. 1, 2, 4, 5, 8, 9, 10, 12, 13, 14, 15, and 16.

In some embodiments, the first member of a control pair of nucleic acids of the invention that has at least two *IGF2* SNPs comprises a polynucleotide having at least 8 contiguous nucleotides, or at least 10, 12, 15, 20, 25, or 30 contiguous nucleotides, including the SNP site, of one allele of a SNP shown in SEQ ID NO:1, 2, 3, 4, 5, 6, 7, 8, 9, 10,11, 12, 13, 14, 15, or 16, or the complement thereof; and the second *IGF2* SNP site present on the first member of the control polynucleotide has at least 8 contiguous nucleotides, or at least 10, 12, 15, 20, 25, or 30 contiguous nucleotides, including the SNP site, of one allele of a different SNP shown in SEQ ID NO:1, 2, 3, 4, 5, 6, 7, 8, 9, 10, 11, 12, 13, 14, 15, or 16, or the complement thereof. The second member of the control pair has the same polynucleotide sequence as the first member of the pair, however, the nucleotides present at the polymorphic positions correspond to alternative alleles of the two SNPS.

In some embodiments, the one member of a control pair of nucleic acids of the invention has at least 70% identity, typically at least 80% identity, at least 90% identity, or at least 95%, 96%, 97%, 98%, 99%, or 100% identity, to SEQ ID NO: 21 or SEQ ID NO:23 and includes the SNPs shown in SEQ ID NOs. 1-16. In some embodiments, the second member of a control pair of nucleic acids of the invention has at least 70% identity, typically at least 80% identity, at least 90% identity, or at least 95%, 96%, 97%, 98%, 99%, or 100% identity, to SEQ ID NO 21 or SEQ ID NO:23 and includes alternative alleles of the SNPs shown in SEQ ID NOs. 1-16.

In some embodiments, the first member of a control pair of nucleic acids of the invention that has at least two *IGF2* SNPs comprises a polynucleotide that has at least 8 contiguous nucleotides, or at least 10, 12, 15, 20, 25, 30, 35, 40, 50, or 100 contiguous nucleotides, including at least one SNP site, of SEQ ID NO:21 or SEQ ID NO:23.

In some embodiments, the polynucleotides comprised by the LOI control pair have substantially the same sequence as SEQ ID NO:20, or the complement thereof, however, comprises less than 500 nucleotides of the CA-rich polymorphic region. The CA-rich polymorphic region of SEQ ID NO:20 occurs between positions 1115 and 1692 of SEQ ID NO:20 and is immediately flanked by positions 1168 and 1169 of the nucleotide sequence set forth in SEQ ID NO:21. The CA-rich region of SEQ ID NO:20 is provided in SEQ ID NO:22. Thus, in some embodiments, the first and second polynucleotides of the control pair comprise substantially the same sequence as SEQ ID NO:20, but comprises less than 500 nucleotides of SEQ ID NO:22. In some embodiments, the LOI control polynculetoides may contain additional sequences to facilitate specific amplification. For example, where the control comprises *IGF2* exon 9 SNPs, additional sequences from the 3' end of *IGF2* exon 8 may be incorporated into the 5' sequence of a control polynucleotide.

The control nucleic acid may be RNA or DNA or other base paring nucleic acid. In some embodiments, the polynucleotides corresponding to the members of the pair are operably linked to an RNA polymerase promoter, e.g., a T7 promoter, so that RNA transcripts can be made from the polynucleotide to provide an RNA control. In some embodiments, the polynucleotides may be linked to promoters on separate vectors such that one vector encodes one member of the control pair and a separate vector encodes the other member of the pair. Control nucleic acid pairs may be obtained using methods well known in the art. For example, in some embodiments, e.g., for pairs where each member is less than 100 nucleotides in length, the polynucleotides may be synthesized chemically. In typical embodiments, the control polynucletoides may be obtained using amplification reactions.

In some embodiments, the members of the LOI control pair are separated. In the context of this invention, "separated" means that the two members of a pair are not present in the same mixture or solution. In some embodiments, the members of the pair are present in a mix together.

In a further aspect, the invention provides a mixture comprising an LOI control pair. In some embodiments, the members of the pair are present in equimolar amounts. In some embodiments, the members of the pair are present in a molar ratio, *e.g.,* of 1:2, 1:4, 1:8, and the like. In some embodiments, the invention provides a plurality of mixtures, where each mixture contains a different predetermined amount of pair members. For example, the invention also provides a plurality of mixtures where the mixtures have the individual members of the pair present at 10:1, 8:1, 6:1, 4:1, 3:1, 2:1, 1:1, 1:2, 1:3, 1:4, 1:6, 1:8 and 1:10.

In some embodiments, the mixture may comprise a second LOI control pair of isolated nucleic acids, substantially free of cellular nucleic acids, wherein both members of the second pair comprise at least one *IGF2* SNP sequence where the *IGF2* SNP sequence(s) in the second pair is different from the *IGF2* SNPs in the first pair. Thus, LOI at various *IGF2* SNPs, e.g., the SNPs shown in SEQ ID NO:1, 2, 3, 4, 5, 6, 7, 8, 9, 10, 11, 12, 13, 14, 15, or 16, can be evaluated using one control pair of polynucleotides where each polynucleotide has all 16 SNPs, or can be evaluated using multiple control pairs of nucleic acids, *e.g.,* 2 pairs, 3 pairs, 4 pairs, 5 pairs, 6 pairs, 7 pairs, or 8 pairs, or more.

The invention additionally provides a kit comprising LOI control pairs of nucleic acids as described herein. The control nucleic acids may be provided in the kit in a mixture. Alternatively, the kit may be provided with the two members of the control pair in separate containers. In some embodiments, a kit of the invention comprises one or more transcription vectors that comprise polynucleotides encoding the members of the pair.

In some embodiments, a kit of the invention also comprises a second pair *of IGF2* LOI control nucleic acids wherein both members of the second pair comprise at least one *IGF2* SNP sequence and wherein the at least one *IGF2* SNP sequence in the second pair is different from the *IGF2* SNPs in the first pair. In some embodiments the second pair of control nucleic acids is in a separate container from the first pair of nucleic acids.

A kit of the invention that comprises a control pair of *IGF2* nucleic acids may further comprise any of the reagents, including primers and probes, described in the KIT section.

The invention also provides methods of using the control pair of nucleic acids in an IGF2 LOI assay as a control to validate assay conditions. A method of the invention typically comprises quantifying the maternal and paternal copies of *IGF2* genomic DNA or cellular RNA as described herein using oligonucleotides that distinguish between one SNP allele and a second SNP allele; quantifying the SNP; using a control pair of oligonucleotides where the members of the pair are present in a known ratio and quantifying the ratio of the two members of the control pair that is actually detected in the assay and comparing this ratio to the ratio of maternal and paternal copies quantified in the patient sample. This provides a means of validating the genotyping or imprinting status assay conditions and further quantifying the actual amounts of the maternal vs. paternal IGF2 genomic DNA or RNA in the patient sample

### IX. MANAGEMENT OF CANCER

LOI *of IGF2* is associated with, for example, the presence of cancer and may be associated with a predisposition of cancer as well as predicting the efficacy of treatment of cancer using various drugs. *See*, *e.g.,* WO2004/003003; Kaneda et al. Proc. Natl. Acad. Sci. USA 104(52):20926-20931 (2007). Accordingly, detection of LOI in IGF2 as described herein can be used in the diagnosis, prognosis, classification, prediction of cancer risk, detection of recurrence of cancer, and the selection of a treatment for a number of types of cancers. A cancer at any stage of progression can be detected, such as primary, metastatic, and recurrent cancers. Information regarding numerous types of cancer can be found, *e.g*., from the American Cancer Society (available on the worldwide web at cancer.org), or from, *e.g.,* Harrison's Principles of Internal Medicine, Kaspar, et al., eds., 16th Edition, 2005, McGraw-Hill, Inc. Exemplary cancers that can be detected include bladder, breast, cervical, choriocarcinoma, colorectal neoplasia (colorectal adenoma or colorectal cancer), esophageal, gastric adenocarcinoma, glioma, hepatocellular, acute myeloid leukemia, chronic myelogenous leukemia, lung, medulloblastoma, prostate, mesothelioma, ovarian, renal cell carcinoma, testicular germ cell, and uterine cancer.

The present invention provides methods for determining whether or not a mammal (e.g., a human) has cancer, whether or not a biological sample taken from a mammal contains cancerous cells, estimating the risk or likelihood of a mammal developing cancer, classifying cancer types and stages, monitoring the efficacy of anti-cancer treatment, or selecting the appropriate anti-cancer treatment in a mammal with cancer.

In some embodiments, the biological sample comprises a tissue sample from a tissue suspected of containing cancerous cells. For example, in an individual suspected of having cancer, breast tissue, lymph tissue, lung tissue, brain tissue, or blood can be evaluated. Alternatively, lung, renal, liver, ovarian, head and neck, thyroid, bladder, cervical, colon, endometrial, esophageal, prostate, or skin tissue can be evaluated. The tissue or cells can be obtained by any method known in the art including, *e.g*., by surgery, biopsy, phlebotomy, swab, nipple discharge, stool, etc. In other embodiments, a tissue sample known to contain cancerous cells, *e.g*., from a tumor, will be analyzed for LOI status to determine information about the cancer, *e.g.*, the efficacy of certain treatments, the survival expectancy of the individual, *etc.* In some embodiments, the methods will be used in conjunction with additional diagnostic methods, *e.g*., detection of other cancer biomarkers, etc.

The methods of the invention can be used to evaluate individuals known or suspected to have cancer or as a routine clinical test, *i.e.,* in an individual not necessarily suspected to have cancer. Further diagnostic assays can be performed to confirm the status of cancer in the individual.

Further, the present methods may be used to assess the efficacy of a course of treatment. For example, the efficacy of an anti-cancer treatment can be assessed by monitoring LOI over time in a mammal having cancer. For example, a reduction or absence of LOI in *IGF2* in a biological sample taken from a mammal following a treatment, compared to a level in a sample taken from the mammal before, or earlier in, the treatment, may indicate efficacious treatment. Further, an individual can be screened for LOI of *IGF2* prior to selection of an appropriate drug for either cancer prevention or cancer treatment. For example, once LOI is detected, the patient is likely a good candidate for an IGF1R inhibitor, which can either be taken as a preventative therapy if the patient is found to be cancer free and is believed to be at an increased risk of developing cancer in the future, or can be taken as a chemotherapy if the patient is found to have cancer in order to treat existing disease. *See, e.g.,* Kaneda *et al., supra*.

In some embodiments, the methods comprise recording a diagnosis, prognosis, risk assessment or classification, based on the LOI status determined from an individual. Any type of recordation is contemplated, including electronic recordation, e.g., by a computer.

### X. COMPUTER-BASED METHODS

The calculations for the methods described herein can involve computer-based calculations and tools. For example, LOI can be determined using the data generated as described herein. For example, inputs relating to the presence, absence or quantity of each allele of a SNP, optionally also including genotype information about an individual, can be used in isolation, or in comparison to similar measurements made of a control pair of nucleic acids to generate a predicted LOI. The tools are advantageously provided in the form of computer programs that are executable by a general purpose computer system (referred to herein as a "host computer") of conventional design. The host computer may be configured with many different hardware components and can be made in many dimensions and styles (e.g., desktop PC, laptop, tablet PC, handheld computer, server, workstation, mainframe). Standard components, such as monitors, keyboards, disk drives, CD and/or DVD drives, and the like, may be included. Where the host computer is attached to a network, the connections may be provided via any suitable transport media (e.g., wired, optical, and/or wireless media) and any suitable communication protocol (e.g., TCP/IP); the host computer may include suitable networking hardware (e.g., modem, Ethernet card, WiFi card). The host computer may implement any of a variety of operating systems, including UNIX, Linux, Microsoft Windows, MacOS, or any other operating system.

Computer code for implementing aspects of the present invention may be written in a variety of languages, including PERL, C, C++, Java, JavaScript, VBScript, AWK, or any other scripting or programming language that can be executed on the host computer or that can be compiled to execute on the host computer. Code may also be written or distributed in low level languages such as assembler languages or machine languages.

The host computer system advantageously provides an interface via which the user controls operation of the tools. In the examples described herein, software tools are implemented as scripts (e.g., using PERL), execution of which can be initiated by a user from a standard command line interface of an operating system such as Linux or UNIX. Those skilled in the art will appreciate that commands can be adapted to the operating system as appropriate. In other embodiments, a graphical user interface may be provided, allowing the user to control operations using a pointing device. Thus, the present invention is not limited to any particular user interface.

Scripts or programs incorporating various features of the present invention may be encoded on various computer readable media for storage and/or transmission. Examples of suitable media include magnetic disk or tape, optical storage media such as compact disk (CD) or DVD (digital versatile disk), flash memory, and carrier signals adapted for transmission via wired, optical, and/or wireless networks conforming to a variety of protocols, including the Internet.

### EXAMPLES

The following examples are offered to illustrate, but not to limit the claimed invention.

### Example 1. Discovery and independent validation of SNPs within exon 9 of the IGF2 gene.

A collection of a limited number of SNPs within exon 9 of the *IGF2* gene has previously been reported. To identify previously uncharacterized SNPs, 12 PCR amplicons were designed to tile the entirety *of IGF2* exon 9. Each was used to amplify a PCR amplicon from genomic DNA derived from a panel of 571 individuals, including 433 samples that are part of the International HapMap Project collection. The 571 individual panel included a subpanel of 207 Caucasian individuals (96 from Coriell Human Variation Panel including unrelated healthy Caucasian individuals who were Utah residents with ancestry from northern and western Europe, and 111 individuals from whom blood samples were commercially obtained). This subpanel is hereafter referred to as the Caucasian panel and is denoted as (CAU). The 571 individual panel also included a subpanel 96 unrelated healthy individuals with African ancestry in the southwestern United States (from Coriell Human Variation Panel HD100A). This subpanel is hereafter referred to as the African American panel and is denoted as (AA). The 571 individual panel also included a subpanel of 96 unrelated individuals of Mexican descent (from Coriell Human Variation Panel HD100MEX). Each individual in this subpanel was from the Mexican-American Community of Los Angeles, each having either three or four grandparents born in Mexico). This subpanel is hereafter referred to as the Mexican descent panel and is denoted as (MEX). The 571 individual panel also included a subpanel of 88 unrelated Japanese individuals from Tokyo, Japan (International Hapmap Project collection HAPMAPPT02 and HAPMAPPT05). This subpanel is hereafter referred to as the Japanese panel and is denoted as (JPN). The 571 individual panel also included a subpanel of 84 unrelated Han Chinese individuals from Beijing, China (International Hapmap Project collection HAPMAPPT02 and HAPMAPPT05). This subpanel is hereafter referred to as the Chinese panel and is denoted as (CHI). Amplicons generated from each of the 571 individuals were sequenced in both directions. Sequences were assembled and aligned, and SNPs were identified by an automated polyphred and polyscan analysis pipeline, and were confirmed by human review. We previously reported the confirmation of 65 previously identified SNPs and identification of 47 previously unpublished putative SNPs in *IGF2* exon 9 (PCT/US2008/072356).

To further confirm SNPs and obtain precise frequencies of heterozygosity for selected SNPs, primer extension-based genotyping assays were designed for SNPs that were determined by DNA sequencing to be heterozygous in greater than 1% of individuals within any ethnic population. Various PCR amplicons were designed to include one or more SNP nucleotides to be analyzed. These were used to amplify PCR products from the appropriate subpanels of individual genomic DNA samples. For single nucleotide primer extension assays, a primer was designed so that the 3' terminal nucleotide of the primer anneals to the PCR template strand immediately 3' to each SNP nucleotide to be analyzed. Single nucleotide primer extension reactions were performed using differentially fluorescently labeled dideoxynucleotide triphosphates (ddNTPs). Extended primers were resolved on an ABI 3730XL instrument, raw fluorescent intensity data was transferred to a receiving computer, and genotypes were determined on the receiving computer by GeneMapper software.

As described in detail in Example 3, the observed genotypes within each human subpanel of samples were analyzed in combination to identify a panel of SNPs that together provided the maximum informativity across each of five individual populations and across all five human populations considered as a single population. For example, if the combination of two or SNPs provides an increase in informativity relative to a smaller combination of SNPs, these SNPs are not in complete linkage disequilibrium with each other and can therefore be effectively combined to allow for an improved informativity for determining the imprinting status *of IGF2.* This analysis revealed a panel of 16 SNPs that provided maximal informativity across all populations. This panel includes 6 previously unpublished SNPs. Table 1 lists the genomic coordinates (NCBI build 36), single nucleotide sequence variants, and surrounding nucleotide sequences of these previously unpublished SNPs. In addition, the panel includes 10 previously published SNPs. Table 2 lists the genomic coordinates (NCBI build 36), single nucleotide sequence variants, NCBI dbSNP reference identifier and surrounding nucleotide sequences of these previously identified SNPs. As described in the Examples below, combinations of previously published and previously unpublished SNPs allow for an increase in overall informativity, which increases the number of patients who can successfully be monitored for loss of imprinting *of IGF2* relative to combinations of previously published SNPs alone, especially where the patient population includes African-Americans or others of black African descent.

The observed frequencies of heterozygosity for each SNP (including both previously unpublished and previously identified SNPs) in the panel of all individuals genotyped for that SNP (overall), as well as in the African American (AA), Caucasian (CAU), Chinese (CHI), Japanese (JPN) and Mexican descent (MEX) panels separately are listed in Table 3.

### Example 2. Use of any one or more SNPs for improved detection of LOI of the IGF2 gene

As described above, the detection of LOI of the *IGF2* gene is based on the independent comparison of the amount of expression derived from each of the two copies of the *IGF2* gene isolated from a biological sample from a given individual. The *IGF2* gene is normally maternally imprinted, (*i.e.* the copy inherited from an individual's mother is normally transcriptionally repressed), while the paternally inherited copy of the gene is normally expressed. LOI occurs when the *IGF2* maternal imprint is relaxed, resulting in similar expression levels of both the paternally and maternally inherited copies of the gene. One method of measuring the imprinting status of *IGF2* in a sample is to first isolate genomic DNA from a biological sample and then determine the genotype(s) of one or more polymorphic sites in the transcribed region of the *IGF2* gene. Second, allele-specific expression *of IGF2* is then measured by utilizing one or more heterozygous nucleotides in RNA that is extracted from the same biological sample, or from a different biological sample from the same individual. Expression from each of the two copies of the *IGF2* gene may be independently measured with an assay(s) that is quantitative, and that can sufficiently discriminate between the two alleles of one or more heterozygous SNPs within the sample. Third, a ratio of the amount of expression from one allele to the amount of expression of the other allele is computed, is optionally compared to a control value and optionally adjusted, and compared to a threshold value, thereby determining the imprinting status of the *IGF2* gene in the sample.

One specific intended approach is described here as an example of the utility of using any one or more of the previously unpublished SNPs reported in the present application for determining the imprinting status *of IGF2* in a patient sample. It is apparent to those skilled in the art that multiple approaches for detection and quantification of SNPs exist, and any of these may be utilized for both the genotyping of genomic DNA from a biological sample for a particular SNP and the quantification of relative levels of each sequence variant present in expressed mRNA of a biological sample. A basic strategy is outlined in Figure 2. This strategy involves isolating both genomic DNA and total RNA (or polyadenylated RNA) from the same biological sample or matched pairs of biological samples taken from the same individual (for example, peripheral blood, peripheral blood mononuclear cells, colonic mucosa sample, stool sample, etc.). The genomic DNA sample is then genotyped with assays detecting the alleles of one or more SNPs. This step determines what SNPs, if any, may be utilized for analysis of allele-specific expression of the *IGF2* gene in the matched RNA sample. If the individual is homozygous for all SNPs evaluated by an assay, the individual is not informative for those SNPs and can not be measured for LOI *of IGF2* using SNP assays. If the individual is heterozygous (informative) for one or more SNPs, cDNA is amplified from the relevant region of the *IGF2* transcript using standard reverse transcriptase/PCR (RT-PCR) methods. Expression from each of the two copies of the *IGF2* gene is independently measured using the generated cDNA with an assay that is quantitative, and that can sufficiently discriminate between the two alleles. Computation of the ratio of the amount of expression of one allele relative to the amount of expression of the other allele, and comparison of this ratio to a threshold value determines the imprinting status of the IGF2 gene. If multiple heterozygous SNPs exist for a given sample, assays that discriminate each of the SNPs may be used simultaneously. This allows redundant measurements of allele-specific expression within a sample, and comparison of these measurements may be used to determine a new value representing the average or mode or median value, and to determine an intra-assay coefficient of variance. While a range of threshold values can be used (such as the quantified proportion of the lesser abundant allele being greater than or equal 10%, 15%, 20%, 25% or 30% the quantified proportion of the more abundant allele), typically, a sample is classically determined to display LOI of *IGF2* if the quantified proportion of the lesser abundant allele is greater than or equal to 33.3% the quantified proportion of the more abundant allele.

One method for genotyping an individual for a given SNP is accomplished by designing an oligonucleotide primer that is complementary to the sequence of the *IGF2* gene and that has a 3' terminal nucleotide that is complementary to the *IGF2* template nucleotide one base 3' to the template polymorphic nucleotide. Assays may be designed to genotype any one or more of the SNPs listed in Tables 1 and 2. The oligonucleotide primer is combined with and hybridized to the PCR amplified DNA product from the genomic DNA sample in a mixture including all ddNTPs (ddATP, ddCTP, ddGTP, ddTTP (or ddUTP)), each tagged with a different fluorescent moiety, is added. For example, if a G/A polymorphism is to be genotyped (and the G/A nucleotide is on the template strand of the genomic DNA sample), the oligonucleotide primer is designed to hybridize to the complementary template with its 3' terminal nucleotide hybridized to the complementary template nucleotide one base 3' to the template G/A position. Single nucleotide primer extension is catalyzed by a DNA polymerase in the presence of the differentially fluorescently labeled ddNTPs such that oligonucleotides that extend by incorporation of ddCTP (representing the G allele) or by incorporation of ddTTP (representing the A allele) are differentially fluorescently labeled at their 3' termini. Extended oligonucleotides are then resolved by capillary electrophoresis and analyzed in the presence of a fifth-fluorescent dye-labeled size standard. Peaks representing specific single nucleotide primer extension products are detected and quantified to determine the genotype for the given DNA sample. Multiple SNPs may be genotyped in one reaction by multiplexing with oligonucleotides of different lengths designed to terminate just 3' to different polymorphic sites. Different genotypes are obtained based on i) resolution of different length extended oligonucleotides and ii) the specific fluorescent tagged ddNTP incorporated during single nucleotide extension.

One method for determining the imprinting status of *IGF2* involves an analogous single nucleotide primer extension approach that is designed to discriminate different alleles of a particular SNP. Assays may be designed to utilize any one or more of the SNPs listed in Tables 1 and 2. If a given SNP is determined to be heterozygous in a genomic DNA sample, first strand cDNA is amplified from the matched RNA sample by a reverse transcriptase (RT) using random hexamer or decamer primers, oligodT primers complementary to polyA tails of mRNA or a primer complementary to a specific region of the *IGF2* transcript. Oligonucleotide primers complementary to sequences flanking the SNP site are subsequently used to PCR amplify a cDNA product including the polymorphic site. Alternatively, nested PCR approaches may be used to generate cDNA products. Alternatively, approaches including generation of antisense RNA from cDNA by linear *in vitro* transcription, followed by a second reverse transcription reaction using random hexamer or decamer primers or *IGF2* transcript-specific primer and subsequent PCR amplification may be used to generate cDNA products. These RT-PCR products are then assayed for the specific sequence variants of the polymorphic site using the same single nucleotide primer extension assay(s) described above. Peaks representing specific single nucleotide primer extension products are detected and quantified. The ratio of the quantified amount of one allele to the other allele is determined. This ratio can be compared to, and potentially adjusted by, similar ratios determined by a control curve. LOI is detected if the quantified proportion of the PCR product representing the lesser abundant allele is greater than or equal to 33.3% the quantified proportion of the PCR product representing the more abundant allele. As described above, multiple heterozygous SNPs may be used to measure LOI in a common reaction by multiplexing with oligonucleotides of different lengths designed to terminate just 3' to different polymorphic sites or with oligonucleotides that incorporate different labeled or mass modified ddNTPs into the extended primer.

It is possible for an individual that is determined to display normal imprinting of *IGF2* to express both copies of the *IGF2* gene, provided that expression of the lesser abundantly expressed copy of the *IGF2* gene is below a particular threshold level relative to the more abundantly expressed copy of the *IGF2* gene. In this case, a quantitative assay that is capable of detecting the low level of *IGF2* gene expression from the imprinted copy of the gene can be used to determine both the genotype of an individual for any of the SNPs and the *IGF2* imprinting status of the individual by performing the assay only on RNA or cDNA amplified from RNA (*i.e.* excluding the step of determining the genotype of the individual by performing the assay on PCR products amplified from genomic DNA). For example, consider that normal expression from the imprinted maternal copy of the *IGF2* gene is 1/1000 the level of expression of the paternal copy of the *IGF2* gene. A quantitative assay capable of detection of, for example, 1 maternally expressed copy in the presence of 10,000 paternally expressed copies of the *IGF2* gene could be used to determine both the genotype of an individual at any of the SNPs and the imprinting status of *IGF2,* provided that the individual is heterozygous for at least one of the assayed SNPs. Detection of expression of the *IGF2* gene including different alleles of a particular SNP would indicate that the individual is heterozygous for that particular SNP. The quantified relative level of expression of the two alleles would then be compared to determine the imprinting status of *IGF2.* The individual would be determined to display LOI *of IGF2* if the quantified proportion of the lesser abundant allele is greater than or equal to a threshold percentage of the quantified proportion of the more abundant allele.

### Example 3. Demonstration of use of SNP combinations for improved detection of LOI of the IGF2 gene

The application of one particular SNP to detection of LOI of *IGF2* allows only those individuals who are heterozygous *(i.e.* informative) for that one SNP to be analyzed. For example, Table 3 indicates that the most informative SNP across the entire cohort of individuals is the SNP represented by SEQ ID NO: 8 (45.6% of individuals were informative for this SNP). However, the application of combinations of individual SNPs which are not in linkage disequilibrium would result in a higher rate of informativity of the combination relative to a single SNP alone. To identify combinations of SNPs that improve informativity for detection of LOI of *IGF2,* the combination of SNPs that together provides the maximal informativity within each ethnic population was identified. Only samples that were successfully genotyped for all 16 SNPs listed in Tables 1 and 2 were included in the analysis. 74 of 96 African American panel samples, 150 of 207 Caucasian panel samples, 69 of 96 Mexican descent panel samples, 79 of 88 Japanese panel samples and 71 of 84 Chinese panel samples were successfully genotyped for al 16 SNPs. As shown in Fig. 3, combinations of SNPs dramatically increase informativity relative to that of any one particular SNP, demonstrating the lack of complete linkage disequilibrium among SNPs listed in Tables 1 and 2. This finding is surprising given the close proximity of each of the 16 SNPs to one another, and multiple published reports suggest that the region is comprised of a few linkage blocks and generally displays linkage disequilibrium. Furthermore, a large percentage of individuals analyzed were informative for 2 or more SNPs among the SNP combination panels (indicated by black and gray bars in Fig. 3. The combinations of SNPs for each ethnic population included SNPs represented by SEQ ID NOs: 1, 2, 3, 4, 12, 13, 15 and 16 for the African American sample panel: SEQ ID NOs: 5, 8, 9, 10, 12, 15 and 16 for the Caucasian sample panel; SEQ ID NOs: 1, 7, 9, 10, 11, 12, 14, 15 and 16 for the Mexican descent sample panel; SEQ ID NOs: 10, 12, 14 and 16 for the Japanese sample panel; and SEQ ID NOs: 6, 8, 12 and 16 for the Chinese sample panel.

To identify combinations of SNPs that provide optimal informativity across general populations, the overall informativity for all possible combinations of SNPs including at least one SNP listed in Table 1 across all samples genotyped for each of the 16 SNPs listed in Tables 1 and 2 was calculated. For each panel of a particular number of SNPs, multiple combinations are capable of providing similar informativity. For example, 4 different combinations of 15 SNPs provide 80.59% informativity across all samples; 5 different combinations of 14 SNPs provide 80.36% informativity, 10 different combinations of 13 SNPs provide 79.91 % to 80.14% informativity, and so on. For each panel of a particular number of SNPs, the specific SNPs that were present in each possible optimal panel were identified, and the informativity for each of these combinations across all samples was calculated. The combined informativity for panels of 16, 12, 10, 9 and 4 SNPs common to optimal combination panels (Panels A, B, C, D and E, respectively) is listed in Table 4 and graphed in Figure 4. Although the entire panel of 16 SNPs (Panel A) provides the highest informativity overall and for each ethnic group specifically, sub-panels provide similar observed informativity. The SEQ ID NOs representing SNPs included in each of these panels are provided in Table 5. As shown in Figure 4, the observed informativity of a panel of 10 SNPs (Panel C) is only slightly lower than the combination of 16 SNPs (Panel A). The optimal informativity across all samples for a panel including the 10 previously known SNPs (Table 2) is 76.98%, indicating that the previously unpublished SNPs reported in the present application offer improvements in informativity.

### Example 4. Demonstration of use of SNPs to determine LOI status of IGF2.

The SNP corresponding to SEQ ID NO: 8 (rs680) falls within the target recognition sequences of two restriction enzymes, *Apa I* and *Ava II.* These two enzymes cleave in an allele-specific manner. *Apa I* recognizes and cleaves the sequence when the "G" allele is present, and *Ava II* recognizes and cleaves the sequence when the "A" allele is present. To independently assess genotypes within a selected panel of individuals, a PCR amplicon including the position of SEQ ID NO: 8 was amplified from a genomic DNA sample derived from each individual. Amplicons were digested with *Apa I* or *Ava II* or a combination of both enzymes. Digestion by *Apa I* only indicates that the individual is homozygous for the G allele, digestion by *Ava II* only indicates that the individual is homozygous for the A allele, and digestion by both enzymes indicates that the individual is heterozygous for the SNP. An example of the data output for each possible genotype of SEQ ID NO: 8 is shown in Figure 5.

The same basic assay strategy can be utilized to detect LOI *of IGF2,* provided the individual being tested is heterozygous for SEQ ID NO: 8. An example is shown in Figure 6. Total RNA was extracted from whole blood from three individuals heterozygous for SEQ ID NO: 8. Two individuals were previously shown to be LOI for *IGF2* and the third was previously shown to display normal imprinting of *IGF2.* The region including SEQ ID NO: 8 was RT-PCR amplified from each sample. Reactions lacking reverse transcriptase were performed in parallel to confirm that there was no amplification from genomic DNA. RT-PCR amplicons were then digested with *Apa I* or *Ava II* or a combination of both enzymes, as described above. Digested products were resolved on an Agilent Bioanalyzer, and concentrations of cut and uncut fragments were determined. The quantity of fragments cut by *Apa I* represents the proportion of cDNA amplified from the "G" allele. The quantity of fragments cut by *Ava II* represents the proportion of cDNA amplified from the "A" allele. Therefore, the ratio of *Apa I* cut fragments to *Ava II* cut fragments indicates the relative ratio of expression of the two alleles in the original RNA sample. As shown in Figure 6, Sample 2 expresses exclusively the "A" allele and therefore displays normal imprinting of *IGF2.* Sample 1 expresses both alleles (*i.e.* display LOI *IGF2*), with a G:A ratio of 0.53 and therefore displays loss of imprinting of *IGF2.* Sample 3 expresses detectable levels of both alleles, with a G:A ratio of 0.27. As described above, previous studies have used a threshold of 33.3% expression from the lesser abundant allele relative to the more abundant allele as the definition for LOI of *IGF2.* Therefore, using a restriction enzyme based method for determining LOI of *IGF2* status, Sample 3 reports a ratio of alleles close to the typical 33.3% threshold for determining LOI of *IGF2* and could be considered to represent a borderline call for LOI status. One limitation of a restriction enzyme-based approach for determining LOI of *IGF2* status is that, during PCR amplification of cDNA, amplified strands including one allele of a heterozygous SNP can hybridize with amplified complimentary strands including the compliment of the second allele. This results in a heteroduplexed molecule with mis-matched bases at the SNP position. These molecules are not recognized and digested by either of the two restriction enzymes, resulting in uncut DNA. As a result, the calculated ratio of alleles may not be absolutely precise. As describe below, the use of methods that do not involve restriction enzyme digestion can overcome the issues introduced by PCR product heteroduplex formation.

Other SNPs that can be useful for detecting LOI *of IGF2* do not fall within restriction enzyme recognition sequences. Therefore, the ability to monitor LOI in a given individual is improved by developing allele-specific gene expression assays that do not require restriction enzyme digestion. As a demonstration, we developed a primer extension based assay for SEQ ID NO: 8. Figure 7 diagrams the use of a primer extension assay for genotyping SEQ ID NO: 8. The region (SEQ ID NO:25) including the SNP of interest is PCR amplified using genomic DNA obtained from the individual to be genotyped. A primer (SEQ ID NO:24) is added to the purified PCR product that anneals with its 3' terminal nucleotide complimentary to the template nucleotide 1 base to the 3' side of the polymorphic nucleotide to be genotyped. Single nucleotide primer extension is carried out using a thermostable DNA polymerase and differentially fluorescently labeled ddNTPs. In the example diagrammed in Figure 7, either dR110 labeled ddGTP or dR6G labeled ddATP is added to the 3' end of the primer (SEQ ID NOs: 25 and 26). These labeled polynucleotides are then resolved and quantified by capillary gel electrophoresis and fluorescent imaging. The peak areas representative of each possible incorporated nucleotide are calculated *(i.e.* using an ABI 3730XL Gene Analyzer with Gene Mapper software). Peak areas are compared to determine the genotype of the individual at that SNP position.

The three individuals that were assayed for LOI *of IGF2* by the restriction enzyme based assay (Figure 6) were genotyped for SEQ ID NO: 8 using the primer extension assay (Figure 8). As expected, peaks representing both alleles of the SNP were detected, confirming that the three individuals are heterozygous for SEQ ID NO: 8.

To measure allele-specific expression *of IGF2* in the same three individuals, the region including SEQ ID NO: 8 was RT-PCR amplified from a total RNA sample derived from each individual. Reactions lacking reverse transcriptase were performed in parallel to confirm that there was no amplification from genomic DNA. The cDNA products obtained were purified and analyzed with the single nucleotide primer extension assay as diagrammed in Figure 7. Peak areas representing each of the two possible alleles were calculated. To correct for differences in dye intensities, these values were normalized based on comparisons of peak areas calculated using predetermined 1:1 ratios of each allele (*i.e.* 1:1 ratio of DNA amplicons derived from individuals that are homozygous for each of the two alleles). The resulting chromatograms and calculated allele ratios are shown in Figure 9. In agreement with the results shown in Figure 6, Samples 1 and 3 were determined to show LOI of *IGF2,* and Sample 2 was determined to show normal imprinting of *IGF2.* It is obvious that the same type of single nucleotide primer extension assay that utilizes any SNP within the transcribed region *of IGF2* could be used to monitor allele-specific expression of *IGF2* (see Figures 10 and 11).

To demonstrate the use of additional SNPs for measuring allele-specific expression of *IGF2,* single nucleotide primer extension assays were designed based on 12 additional SNPs (SEQ ID NOs: 2, 3, 4, 5, 6, 7, 9, 10, 11, 14, 15 and 16). For each of the 13 SNPs (including SEQ ID NO: 8), PCR products were separately amplified from genomic DNA samples derived from two individuals; one homozygous for one allele of the SNP and the other homozygous for the other allele of the SNP. The PCR products were purified and quantified. For each of the 13 SNPs, two PCR products (one amplified from the DNA sample homozygous for one allele and the other amplified from the DNA sample homozygous for the other allele) were combined in the following ratios of allele 1 to allele 2; 1:10, 1:6, 1:4, 1:3, 1:2, 1:1, 2:1, 3:1, 4:1, 6:1, and 10:1. For each of the nine SNPs, the single nucleotide primer extension assay was performed in triplicate on each dilution point. Peak areas representing each of the two possible alleles were calculated. To correct for differences in dye intensities, these values were normalized based on comparisons of peak areas calculated using predetermined 1:1 ratios of each allele. The analytical quantitative linearity of each assay is shown in Figure 10 and Figure 11. The R² and slope for each assay is shown in Table 6.

Single nucleotide primer extension assays may be used in a multiplexed format so that a combination of SNPs is genotyped (or alternatively, so that a combination of SNPs is used to measure allelic expression in RNA or cDNA), in a single assay. Each SNP that is found to be informative in a given individual can then be used to determine imprinting status of *IGF2* of that individual. Assays can be combined into a multiplexed assay panel by using primers that migrate differentially during capillary electrophoresis or that have unique masses and can be differentially quantified by mass spectrometry, for example. Figure 12A shows the result of a multiplexed genotyping assay performed on genomic DNA which was extracted from a blood sample from one individual. The assay determines the genotypes and determines imprinting status at SNP positions represented by SEQ ID NOs: 1, 2, 3, 7, 8, 10, 11, 12, 15 and 16. Single nucleotide primer extension assays may be designed to determine the single nucleotide sequence on either of the two strands (or of the two or more allelic options) at the SNP position. It is noted that the nucleotides indicated in Figure 12A and 12B and Table 7 are complimentary to the sequences indicated in Table 1 and Table 2 for the SNPs represented by SEQ ID NOs: 2, 3, 7 and 11 because the primer extension assay monitors the opposite strand as that represented in Table 1 and Table 2. As shown in Figure 12A and in Table 7, Sample A is homozygous at the SNPs represented by SEQ ID NOs: 1, 2, 7, 8, 10, 15 and 16. However, the same sample is heterozygous (and therefore, informative) at the SNPs represented by SEQ ID NOs: 3, 11 and 12. It should be noted that while all three heterozygous SNPs were used in the present example, any one of these three heterozygous SNPs, or any combination of two of these SNPs, can now be used to determine the imprinting status of *IGF2* in this individual. Figure 12B shows the result of the same multiplexed assay panel when used to detect allele-specific expression *of IGF2* in cDNA generated from total RNA derived from the same blood sample. As shown in Figure 12A and in Table 7, Sample A displays normally imprinted expression of *IGF2.* That is, expression is derived from a single copy of the *IGF2* gene. The expressed allele includes a T at the SNP position represented by SEQ ID NO: 3, a G at the SNP position represented by SEQ ID NO: 11 and a C at the SNP position represented by SEQ ID NO: 12. The copy of the *IGF2* gene containing a G at the SNP position represented by SEQ ID NO: 3, an A at the SNP position represented by SEQ ID NO: 11 and a T at the SNP position represented by SEQ ID NO: 12 is not detected. Table 7 displays the results of the same multiplexed assay performed on a second sample (Sample B). This sample is heterozygous (and therefore, informative) at the SNP represented by SEQ ID NO: 16. Sample B displays normally imprinted expression *of IGF2.* The expressed allele includes a G at the SNP position represented by SEQ ID NO: 16. The copy of the *IGF2* gene containing the A allele is not detected.

The same two samples shown in Figure 12 (Sample A and Sample B) were analyzed using single-plex assays for SNPs represented by SEQ ID NOs: 4, 5, 6, 9, 13 and 14 to determine the genotypes at these SNP positions and to determine imprinting status based on these SNPs (Table 7). Sample A is homozygous at the SNP positions represented by SEQ ID NOs: 4, 5, 6, 9,13 and 14. Sample B is homozygous at the SNP positions represented by SEQ ID NOs: 4, 6, 9, 13 and 14. However, Sample B is heterozygous (informative) at the SNP position represented by SEQ ID NO: 5. Analysis of allele-specific expression of *IGF2* detected both the G and A alleles of SEQ ID NO:5 (Table 7).

Table 8 lists the values obtained for each peak detected in the experiment described above (Sample A and Sample B, analyzed for all 16 SNPs). Following capillary electrophoresis of single nucleotide primer extension reactions and measurement of fluorescent intensities using an ABI 3730XL Genetic Analyzer, peak areas were calculated by a computer program (GeneMapper). Values were received at host computer. Peak area values represented the presence or absence of each polymorphic option at each of the 16 SNP positions derived from the assay of PCR products amplified from genomic DNA (Genomic DNA Peak Area 1 and Genomic DNA Peak Area 2 in Table 8). A computer program correlated the presence or absence of detected peak areas to a determination of genotype at each SNP position. For example, Sample A is homozygous (T/T) at the SNP position represented by SEQ ID NO: 1 because a peak was detected for the T allele (Peak Area 1), but not the G allele (Peak Area 2). Furthermore, Sample A is heterozygous (C/T) at the SNP position represented by SEQ ID NO: 12 because a peak was detected for the C allele (Peak Area 1 = 54813) and for the T allele (Peak Area 2 = 163646). Genotypes at each SNP position and in both samples were similarly determined. A computer implemented method was used to output these results, as shown in Table 7.

To calculate ratios representing the abundance of detected alleles, it is necessary to normalize for differences in fluorescent tag intensities among different labeled ddNTPs used for the single nucleotide primer extension reaction. For a given SNP position, the ratio of the presence of two alleles in genomic DNA extracted from an diploid individual that is heterozygous for that SNP is 1:1. Therefore, a computer implemented method was used to normalize for differences in fluorescent intensities. For example, the calculated peak area for the G allele of the SNP represented by SEQ ID NO: 5 in Sample B (16043) was divided by the calculated peak area for the A allele of the SNP represented by SEQ ID NO: 5 in Sample B (12107) to obtain Allele 1:Allele 2 ratio of 1.3251. A Normalization Factor (Table 8) was calculated so that the ratio of the two alleles is readjusted to account for dye effects to 1.0 (1/1.3251 = 0.7547). Normalization factors may also be determined using standard mixtures.

To determine a value representing the imprinting status of *IGF2,* peak area values represented the presence or absence of each polymorphic option at each of the 16 SNP positions derived from the assay of PCR products amplified from cDNA (RNA Peak Area 1 and RNA Peak Area 2 in Table 8). Values were received at host computer, and a computer implemented method was used to compare peak areas detected for each possible allele. For example, Sample A is determined to be informative for the SNPs represented by SEQ ID NOs: 3, 11 and 12. The individual is determined to display normal imprinting *of IGF2* because, at each of the three SNP positions, a peak area for only one of the two possible alleles was detected. Sample B is determined to be informative for the SNPs represented by SEQ ID NOs: 5 and 16. A peak area for only one of the two possible alleles was detected for the SNP represented by SEQ ID NO: 12. However, a peak area for both of the two possible alleles was detected for the SNP represented by SEQ ID NO: 5 (RNA Peak Area 1 = 6283 and RNA Peak Area 2 = 14115 in Table 8). A computer implemented method was used to calculate a normalized ratio of Allele 1 to Allele 2 ((RNA Peak Area 1 (6283)/RNA Peak Area 2 (14115)) * Normalization Factor (0.7541) = Normalized Ratio (0.3359)). The individual is determined to display borderline imprinting status *of IGF2* because one informative SNP reported detection of only one of two possible alleles (SEQ ID NO: 12) and a different SNP reported detection of both alleles at a ratio very close to the threshold for determining imprinting vs. loss of imprinting. A computer implemented method was used to output these results, as shown in Table 8.

As described above, the analysis of more than one heterozygous SNP in a given individual (compound heterozygotes) provides a method for determining multiple independent measurements of imprinting status for an individual, thereby allowing calculations of additional values including, but not limited to, i) the average value of calculated peak areas, genotype peak area ratios, normalization factors, allele-specific expression peak area ratios and normalized allele-specific expression ratios, ii) the standard deviation between calculated peak areas, genotype peak area ratios, normalization factors, allele-specific expression peak area ratios and normalized allele-specific expression ratios, iii) the mode of calculated peak areas, genotype peak area ratios, normalization factors, allele-specific expression peak area ratios and normalized allele-specific expression ratios, and iv) coefficients of variation for calculated peak areas, genotype peak area ratios, normalization factors, allele-specific expression peak area ratios and normalized allele-specific expression ratios. Applying these methods can allow for a more precise single determination of imprinting status for a given individual. For example, consider an individual that is heterozygous for three SNPs (SNP1, SNP2, SNP3) in a panel, and analysis of imprinting status resulted in the detection of peaks representing both alleles of each SNP. If the calculated Peak Area 1 (allele 1) was 153330 (SNP1), 136263 (SNP2) and 147544 (SNP3), and the calculated Peak Area 2 (allele 2) was 124589 (SNP1), 132581 (SNP2) and 126598 (SNP3). If the normalization factor for these Peak Areas is 0.778 (SNP1), 0.897 (SNP2) and 1.258 (SNP3), then the normalized ratios are 0.9575 (SNP2), 0.9219 (SNP2) and 1.4661 (SNP3). The average allele-specific expression ratio is 1.115, the median allele-specific expression ratio is 0.9574, the standard deviation among the allele-specific expression ratios is 0.3045, and the coefficient of variation between the allele-specific expression ratios is 0.273. It is noted that multiple different algorithms of comparing values obtained from assaying compound heterozygous SNPs may be used to calculate a precise value correlating with the imprinting status of *IGF2.*

### Example 5. Development of a pair of synthetic nucleic acids, useful for controlling genotype and allele specific expression assays measuring SNPs within exon 9 of the IGF2 gene.

A pair of control synthetic nucleic acids were designed, which were engineered to provide a vastly improved control for genotyping and imprinting assays for *IGF2.* Four objectives influenced the design.

First, the two nucleic acid members of the pair ("Synth1"; SEQ ID NO:21), and ("Synth 2"; SEQ ID NO:23) were designed to contain a portion of the 3' end of the sequence *of IGF2* exon 8 and a majority of the sequence of IGF2 exon 9 (a CA repeat-rich region which occurs naturally in exon 9 of human *IGF2* was omitted from the control sequence). The 3' end of exon 8 and a majority of the sequence of exon 9 of *IGF2* were included in the design of the controls to allow polynucleotides used in genotyping and imprinting assays for *IGF2* (including reverse transcriptase primers, PCR primers, and SNP primers or probes that are designed to anneal specifically to the transcribed portion of IGF2 in clinical samples) to anneal to the nucleic acids members of the control and to *IGF2* transcripts from clinical samples with equal efficiency. Additionally, the CA repeat-rich region was omitted from the design of the control nucleic acid pair due to the fact that such a semi-repetitive region can be difficult to synthesize *in vitro,* may be unstable when cloned into bacteria, and may introduce difficulties for PCR amplification in the subsequent use of the control.

Second, the nucleic acid members of the pair (Synth1 and Synth2) were designed to be identical in their sequence, with the exception of the polymorphic option designed in each control member at each of the 16 SNP positions. Synth1 was designed to contain one polymorphic option of each of the 16 SNPs, while Synth2 was designed to contain the other polymorphic option of each of the 16 SNPs. For purposes of this example in developing the control, the nucleic acid members of the pair were designed to be identical in all base positions except for the 16 SNP positions to avoid potential inadvertent introduction of a sequence specific amplification or detection bias of one member of the control pair relative to the other, or between a member of the control pair and an assay measurement of a clinical sample.

Third, the sequence of the 3' end of *IGF2* exon 8 followed by the sequence at the 5' end of *IGF2* exon 9, (i.e., containing the exon 8-9 splice junction), was included in the design of the 5' end of each of SYNTH1 and SYNTH2 (Figure 13 A). The use of polynucleotides that overlap exon splice junctions in imprinting assays measuring clinical samples may significantly reduce the possibility that the imprinting assay will inadvertently amplify contaminating genomic DNA in the RNA extracted from patient samples during the RT-PCR amplification step. By including the 3' region of exon 8, by omitting the intron between exon 8 and 9, and by including the 5'region of exon 9 in the design of the 5' end of the sequence of Synth1 and Synth2, any exon 8-9 spanning polynucleotides used in an *IGF2* imprinting assay will likely anneal as efficiently to the pair of nucleic acids in the control as they will to *IGF2* transcripts in from clinical samples.

Fourth, a T7 promoter sequence was included 5' to the beginning exon 8 sequence to allow for in vitro transcription of RNA including the engineered exon8-9 sequences, and sequences including Not I restriction enzyme sites for cloning were included on each end of the Synth1 and Synth2 sequences (Figure 13 B) to allow for convenient insertion into plasmid vectors.

Once the synthesis of the nucleic acid pair was complete, the design of Synth1 and Synth2 was confirmed by DNA sequence analysis. The DNA sequence of Synth1 is listed as SEQ ID NO:21. The sequence of and Synth2 is listed as SEQ ID 20. The location of the 16 SNP positions and the polymorphic option present in each of Synth1 and Synth2 is provided in Table 9, and the SNP positions for each of the 16 SNPs designed into Synth1 and Synth2 are listed as SEQ ID NOs:1-16. The complete sequence of *IGF2* exon 9 is provided as SEQ ID NO:20, a sequence which corresponds exactly to Ensemble exon entry ENSE00001488587 (Human GRCh37, February 2009). The CA-rich semi-repetitive and length-polymorphic region, which was omitted from the synthesized control nucleic acids as described above, is provided in SEQ ID NO:22, and is located between positions 1115 and 1692 of SEQ ID NO:20, the native sequence of *IGF2* exon 9. The omitted CA repeat-rich region is immediately flanked by positions 1168 and 1169 of SEQ ID NO:21 and SEQ ID NO:23. This omitted sequence region does not include any of the 16 SNP positions listed in SEQ ID 1-16. The exon 8 derived sequence is located between positions 34 and 54 of SEQ IDs NOs. 21 and 23. The T7 promoter sequences is located between positions 11 and 30 of SEQ IDs 21 and 23.

Once Synth1 and Synth2 were confirmed by DNA sequencing, the fragments were separately digested with Not I and ligated into the Not I cloning site of the pEZ vector, producing two independent plasmids; pEZ-Synth1 and pEZ-Synth2. These plasmids were individually transformed into the DH5α *E. coli* strain.

One embodiment of the invention uses the nucleic acid members of the control in mixtures of Synth1 and Synth2 in known quantitative ratios and using these mixtures as template in an assay designed to monitor allele-specific expression *of IGF2.* Therefore, we designed a strategy to confirm that the quantification of the control molecules is accurate so that assay results from clinical samples of unknown imprinting status can be compared to accurate standard control results. The strategy for the assay designed to monitor allele-specific expression *of IGF2,* and the use of the control nucleic acids in this assay, is diagrammed in Figure 13. Figure 13A shows a diagram of the *IGF2* gene. Various potential promoters for *IGF2* are indicated by arrows labeled Promoter 1, P0, P2, P3 or P4. Potential transcribed exons are indicated by block arrows connected by a hashed line that represents intronic sequence regions. Untranslated exonic sequence regions are indicated by filled block arrows, and translated exonic sequence regions are indicated by open block arrows. The scale bar at the top of Figure 13A is shown in 1000 base pair (Kb) increments. An expanded view of the Synth1 (or Synth2) nucleic acid region is shown in Figure 13B. The scale bar is shown in 500 base pair increments. The Synth1 (or Synth2) nucleic acid sequence includes the 3'-most 21 base pairs of exon 8 followed immediately by the sequence of exon 9 (i.e. the intronic sequence between exons 8 and 9 are omitted, as diagrammed in Figure 13 A). The 5' and 3' ends of Synth1 (or Synth2) include Not I restriction enzyme recognition sequences that were included to allow ligation of the sequences into a transcription vector. The CA repeat-rich sequence region present in *IGF2* exon 9 was omitted from the Synth1 (or Synth2) sequence, as indicated in Figure 13B.

To generate cDNA from endogenous *IGF2* mRNA or from *in vitro* transcribed RNA derived from pEZ-Synth1 or pEZ-Synth2 plasmids, four primers are used to generate first strand cDNA by reverse transcriptase. These reactions may be performed as four separate reactions for each primer, or optionally, as one multiplexed reaction including all four primers. The annealing sites of these four primers within the Synth1 (or Synth2) region (or optionally, within the *IGF2* mRNA region) are indicated by arrow heads in Figure 13B (RT primers). The first strand cDNA molecules generated by this RT reaction are indicated by hashed lines in Figure 13C.

Following reverse transcription, the first strand cDNAs are used as template for first round PCR amplification of four independent amplicons. The annealing sites of the four primer pairs relative to the generated first strand cDNA are indicated by arrow heads in Figure 13D. Solid lines connecting the primer pairs represent the PCR amplicon produced in each first round PCR amplification reaction. These reactions may be performed as four separate reactions for each primer pair, or optionally, as one multiplexed reaction including all four primer pairs. Note that the primer indicated by the open box in Figure 13 D includes the exon 8-9 splice junction spanning sequence.

Next, the amplicons produced in the first round PCR amplification are used as template for amplification of four independent nested amplicons. The annealing sites of the four primer pairs relative to the generated first strand cDNA are indicated by arrow heads in Figure 13E. Solid lines connecting the primer pairs represent the PCR amplicon produced in each second round PCR amplification reaction. These reactions may be performed as four separate reactions for each primer pair, or optionally, as one multiplexed reaction including all four primer pairs.

Each of the four amplicons generated by the second round PCR reaction includes one or more of the SNP positions represented by SEQ ID NOs: 1-16. The position of each SNP is indicated by a diamond in Figure 13F. The amplicons generated by this PCR reaction can be used to quantify the amount of each polymorphic option of each SNP position present. For an RNA sample derived from an individual that is heterozygous for one or more SNPs, quantifying the amount of a generated amplicon including one polymorphic option of the SNP relative to the amount of a generated amplicon including the other polymorphic option of the SNP allows the determination of the amount of expression from one allele of *IGF2* relative to the amount of expression from the other allele of *IGF2* within the sample derived from that individual.

To confirm the precision of the quantification achieved with the controls, the nucleotide at each SNP position represented by SEQ ID NOs: 1-16 in pEZ-Synth1 and pEZ-Synth2 was measured using a multiplex single-nucleotide primer extension assay. Sixteen primers were designed that hybridized such that the 3' end of each primer annealed one base position to the 5' side of each SNP position. Single nucleotide primer extension reactions were performed in the presence of differentially fluorescently labeled ddNTPs (for example, see https://products.appliedbiosystems.com/ab/en/US/adirect/ab?cmd=catNavigate2&catID=60 0762&tab=DetailInfo; or Biotechniques. 2001 Dec;31(6):1374-80). Resolution of single nucleotide extended primers was performed by capillary electrophoresis (ABI 3730XL), and specific peak areas were calculated using ABI GeneMapper software. Results are shown in Table 10.

These results confirmed that the SNP-based single nucleotide primer extension assay recapitulated the genotype calls generated by DNA sequencing for each SNP position in each control nucleic acid. Furthermore, these results demonstrated that the control nucleic acids may be used as positive controls for any type of genotyping assay that determines the nucleotide present at these 16 SNP positions. For example, pEZ-Synth1 (or molecules derived from pEZ-Synth1, such as by restriction enzyme digestion or by PCR amplification) can serve as a positive control for genotyping assay results that are to be expected for an individual homozygous for any of the alleles included in the Synth1 construct. Likewise, pEZ-Synth2 (or molecules derived from pEZ-Synth2, such as by restriction enzyme digestion or by PCR amplification) can serve as a positive control for genotyping assay results that are to be expected for an individual homozygous for any of the alleles included in the Synth2 construct. Finally, a mixture containing equal amounts of pEZ-Synth1 and pEZ-Synth2 (or equal amounts of molecules derived from pEZ-Synth1 and pEZ-Synth2, can serve as a positive control for genotyping assay results for an individual heterozygous for any of the 16 SNPs.

### Example 6. Use of DNA controls to determine the quantitative linearity of an assay monitoring relative abundance of different alleles.

The plasmids pEZ-Synth1 and pEZ-Synth2 were quantified by Nanodrop, and the concentration of each purified plasmid was normalized to 1 ng/µL. Mixtures of pEZ-Synth1 and pEZ-Synth2 were made in the following ratios: 8:1, 6:1, 4:1, 3:1, 2:1, 1:1, 1:2, 1:3, 1:4, 1:6 and 1:8 (Synth1:Synth2). Four non-overlapping PCR amplicons were designed to amplify the transcribed regions of *IGF2* exon 9 including the 16 SNP nucleotides, as shown in Figure 13E. For each ratio point, 1 ng of the plasmid mixture was used as template for PCR amplification of the four amplicons. Amplicons derived from the Synth1/Synth2 ratio mixtures were individually purified on PCR purification spin columns (Qiagen), and used as template for the multiplexed single-nucleotide primer extension assay described in Example 5. Assays were performed in triplicate, and average peak areas for each peak were calculated. Results are shown in Figure 14. The known input ratio of Synth1:Synth2 template was plotted on the x-axis. For each SNP-based single nucleotide primer extension assay within the multiplexed assay, the average measured ratio of peak areas representing the Synth 1 allele: the Synth2 allele was plotted on the y-axis. Error bars indicate plus and minus one standard deviation across the triplicated measurements. All average data point values were multiplied by a common normalization factor that adjusts the measured value for the known 1:1 input data point to equal exactly 1.0. Data were plotted in log scale to evenly visualize all data points across the ratio series. Data derived from one SNP-based assay utilizing one SNP position within each of the four amplicons are shown. These include the assays utilizing the SNPs represented by SEQ ID NO: 7 within Amplicon 1 (Figure 14 A), SEQ ID NO: 5 within Amplicon 2 (Figure 14 B), SEQ ID NO: 1 within Amplicon 3 (Figure 14 C) and SEQ ID NO: 13 within Amplicon 4 (Figure 14 D).

These data demonstrate that the assay was quantitative and reproducible in this range, and that the pair of control nucleic acid mixtures provided in a single dilution series, a standard curve dataset sufficient to control for all of the assay components required to detect all 16 SNPs. The present example thus demonstrates how the assay measurements of the standard curve of mixtures can be run along with clinical samples such that assay measurements of samples of unknown allele ratios may be extrapolated against this curve to determine the relative abundance of molecules containing the two alleles.

### Example7. Use of RNA controls to determine the quantitative linearity of an assay monitoring the relative abundance of different alleles.

Because both control nucleic acids include a T7 promoter upstream of the IGF2 exon 8-9 junction sequence region, RNA molecules analogous to *IGF2* mRNA may be generated by *in vitro* transcription (IVT). Each of the control nucleic acids were excised from pEZ-Synth1 or pEZ-Synth2 by Not I digestion and used as templates in IVT reactions using T7 RNA polymerase. The resulting RNA was then treated with DNase to remove residual plasmid DNA template. IVT RNAs were quantified by Nanodrop.

Next, RNA control mixtures were constructed comprising background RNA, which mimicked the RNA complexity of clinical samples, added to known mixtures of IVT RNA derived from Synth1 and Synth2. In principle, a series of RNA control mixtures for an imprinting assay will generally have the following properties: i) resemble the transriptome complexity of a typical test sample, ii) contain a known ratio of the quantities of the two nucleic acids of the control, and iii) will be constructed such that the ratio of the quantity of IVT control RNA (i.e., the sum of all Synth1 and Synth2 IVT RNA molecules in this example) relative to the quantity of total RNA of the control (i.e., the total quantity of the background RNA added to the Synth1 and Synth2 IVT RNA mixtures) is similar to the ratio of the quantity of native *IGF2* mRNA typically present in a clinical sample relative to the total quantity of RNA that is typically present in a clinical sample.

To accurately model the potential sources of variability in an assay designed to monitor LOI in a biological sample using known quantities of input control RNA, the amount of *IGF2* mRNA that would be expected to be present in a given biological sample was first determined. To accomplish this, a series of RNA template mixtures were generated. In order for the RNA mixture to reflect the transcriptome complexity of a biological sample, a series of known quantities of IVT RNA generated from Synth2 was spiked into aliquots of 500ng of yeast total RNA. This amount of yeast total RNA was used because the typical total RNA input amount for the LOI assay is 500 ng. Yeast total RNA was used as an RNA source lacking *IGF2* mRNA to mimic the complexity of RNAs present in the blood RNA sample, yet ensure that RT-PCR amplification would be derived from the spiked-in control RNA rather than from the background RNA source. The series of RNA template mixtures included spiked-in IVT RNA in the quantity of 120 ng, 12 ng, 1.2 ng, 120 pg, 12 pg, 1.2 pg, 120 fg, 12 fg or 1.2 fg *(i.e.,* nine independent mixtures were made, each with one of these amounts of input IVT RNA).

Alternatively, Synth1 and/or Synth2 IVT RNA can be added to other complex RNA sources that are similar in complexity to the RNA source in the clinical samples with unknown imprinting status, and that substantially lack *IGF2* mRNA. Examples of alternative background RNA sources, which are useful in combination with Synth1 and/or Synth2 IVT RNA as a control for imprinting assays of human samples include i) RNA derived from eukaryotic cells or cell lines lacking *IGF2* expression (*e.g., IGF2*-knock out mouse cells), ii) RNA derived from eukaryotic cells or cell lines that are lacking an *IGF2* homologous gene or have sufficient sequence divergence from the human *IGF2* sequence such that the polynucleotides of the *IGF2* genotyping and imprinting assays do not anneal to the transcripts of the eukaryotic cell *IGF2* homolog, iii) RNA derived from human cells lacking *IGF2* expression (*e.g.,* ovarian teratoma cells in which both copies of *IGF2* are maternally imprinted and are confirmed to be transcriptionally silent), iv) RNA derived from genetically modified human cells lacking *IGF2* expression, or v) RNA extractions of human cells where once-present *IGF2* mRNA has been removed.

Next, four qRT-PCR assays were designed that utilized the four PCR primer pairs indicated in Figure 13E. Each of the nine RNA mixtures (each including 500ng of yeast total RNA and one of the nine quantities of spiked-in IVT control RNA) was used as template for RT-PCR as diagrammed in Figure 13. A control lacking reverse transcriptase was performed for each template mixture to ensure that downstream amplification was derived from cDNA rather than potential contaminating DNA. The second-round PCR reactions were performed in reaction buffer including SYBR Green, and Cₜ values were determined. Reactions were performed in duplicate and Cₜ values were averaged. These data were used to construct a standard curve of Cₜ values across a wide range of known input control IVT RNA quantities.

Next, the same qRT-PCR assays were performed on total RNA extracted from four blood samples derived from four different individuals. In each reaction, 500ng of total RNA was used as template for RT-PCR as diagrammed in Figure 13. A control lacking reverse transcriptase was performed for each template mixture to ensure that downstream amplification was derived from cDNA rather than potential contaminating genomic DNA. The second round PCR reactions were performed in reaction buffer including SYBR Green, and Cₜ values were determined. Reactions were performed in duplicate and Cₜ values were averaged.

The Cₜ values obtained for each of the four PCR amplicons derived from blood RNA were extrapolated relative to the standard curve of Cₜ values derived from the series of IVT RNA/yeast RNA mixtures. The average Cₜ value of the four blood samples was similar to the Cₜ value that was obtained from 84 pg of Synth1, Synth2, or a mixture of Synth1 and Synth2. Therefore, the amount of total IVT control RNA (*i.e.,* the amount of IVT control RNA derived from Synth1 plus the amount of IVT control RNA derived from Synth2) to be used in control curve experients, was set at 120 pg, an amount ∼50% greater than the average amount in 500ng of blood RNA. This amount *(i.e.,* 120 pg) of control IVT RNA was selected so that i) it would slightly exceed the typical amount of *IGF2* mRNA that is expected to be present in a 500 ng sample of total RNA isolated from a whole blood sample, and ii) it would provide consistent RT-PCR amplification success when used in multiple control RNA mixture experiments.

To determine the quantitative linearity of all steps of the assay to monitor allele-specific expression of IGF2, IVT RNA derived from Synth1 was mixed with IVT RNA derived from Synth2 in ratios including 10:1,8:1,6:1,4:1,3:1,2:1, 1:1, 1:2, 1:3, 1:4, 1:6, 1:8, and 1:10 (Synth1:Synth2). A total of 120 pg of each Synth1:Synth2 IVT RNA mixture (*i.e.,* the amount of IVT control RNA derived from Synth1 plus the amount of IVT control RNA derived from Synth2 equaled 120 pg) was spiked into 500 ng of yeast RNA (*i.e.,* thirteen mixtures were produced, each containing 500 ng of yeast RNA plus 120 ng of one of the thirteen Synth1:Synth2 ratios described above). This RNA mixture was used as template for the RT-PCR reactions described in Figure 13. For each ratio point, a control reaction lacking RT was performed to ensure that amplification was derived from the IVT RNA rather than any residual contaminating DNA. Following first-strand cDNA synthesis, 1 µL of each RT+ or RT- reaction was used as template for nested PCR amplification of the four amplicons diagrammed in Figure 13E. An aliquot of each PCR reaction was analyzed on an agarose gel to confirm that no amplification was present in reactions derived from the RT- control reaction. Amplicons derived from the Synth1:Synth2 ratio mixtures were individually purified on PCR purification spin columns (Qiagen), and used as template for the multiplexed single-nucleotide primer extension assay described in Example 5. Assays were performed in triplicate (specifically, each reaction triplicate included one RT reaction, three independent first round PCR reactions per amplicon, three independent second round PCR reactions per amplicon and three independent multiplexed primer extension assay reactions), and average peak area ratios for each primer extension assay were calculated. The resulting data are plotted in Figure 15.

In Figure 15, the known input ratio of Synth1:Synth2 IVT RNA template was plotted on the x-axis. For each SNP-based single nucleotide primer extension assay within the multiplexed assay, the average measured ratio of peak area representing the Synth 1 allele and the Synth2 allele was plotted on the y-axis. In the plot, error bars indicate plus and minus one standard deviation across the triplicated measurements. All average data point values were multiplied by a common normalization factor that adjusts the measured value for the known 1:1 input data point to equal exactly 1.0. Data were plotted in log scale to evenly visualize all data points across the ratio series. Data derived from one SNP-based assay utilizing one SNP position within each of the four amplicons are shown. These include the assays utilizing the SNPs represented by SEQ ID NO: 7 within Amplicon 1 (Figure 15 A), SEQ ID NO: 5 within Amplicon 2 (Figure 15 B), SEQ ID NO: 1 within Amplicon 3 (Figure 15 C) and SEQ ID NO: 13 within Amplicon 4 (Figure 15 D).

These data demonstrated that the imprinting assay is quantitative and reproducible in this range, and that the pair of control nucleic acid mixtures in the form of IVT RNA derived from Synth1 and Synth2 provided, in a single dilution series, a standard curve dataset for all 16 SNPs. The allele-specific expression measurements of this series of mixtures can be run along with imprinting assay measurements of clinical samples such that measurements of samples of unknown allele ratios may be extrapolated against a standard curve generated from the series of control mixtures to determine the relative abundance of molecules containing the two alleles.

Additionally, these data demonstrate the use of control RNA mixtures to indicate deviations of assay results from expected results, as well as to point out what components of the assay are responsible for this variation. For example, quantified allele ratios obtained from one SNP located on three of the four first round and second round amplicons (Amp1, Amp2 and Amp3 - see amplicon locations in Figure 13 E) demonstrated a tight correlation with the expected input allele ratios (Figure 15A, B and C). Furthermore, allele ratios obtained from a second SNP located on the same amplicon reported a very similar tight correlation between measured and input allele ratios (compare Figure 15B depicting the curve of a first SNP on Amp1 and Figure 15E depicting the curve of a second SNP also on Amp1). These data confirmed that the control RNA ratio mixtures were accurate and that all steps and all assay components in the assays involving amplicons 1, 2 and 3 (including RT, PCR and single nucleotide primer extension reactions) reported accurate quantitative measurements. However, the quantified allele ratios obtained from one SNP located on the first round and second round amplicon Amp4 (see location in Figure 13 E) demonstrated a significant deviation between input and measured allele ratios (note the deviations from the line for the data points in the lower left quadrants of Figure 15D and F). Figure 15D plots the allelic ratios obtained from a first SNP within amplicon 4 represented by SEQ ID NO: 13, while Figure 15F plots the allelic ratios obtained from a second SNP within Amp4, represented by SEQ ID NO: 14. For a given datapoint in each plot, the standard deviations across triplicated measurements are small, indicating that the deviations were not primarily due to variation in the first round PCR, second round PCR and the primer extension reaction component of the assay, since the triplicate measurements were independent replicates of these three steps. Furthermore, the direction and magnitude that each data point for each input ratio deviated from the expected line in the two plots (D and F) is remarkably similar between data derived from the two independent SNP positions within the same amplicon. Based on a comparison to the control curve, it can be determined that these results indicate that the variability occurred upstream of the triplicated primer extension reactions, the triplicated 2nd round PCR amplification, and the triplicated 1st round PCR amplification. Furthermore, since three of four amplicons reported expected results, the variability did not occur in the IVT reaction or in the preparation of IVT RNA mixtures. The results are instead consistent with the variability being introduced at the non-replicated RT step, specifically the reverse transcription of first strand cDNA using the RT primer required to RT-PCR amplify Amp4 (the right-most RT primer diagrammed in Figure 13 B). In contrast to this source of variability, consider the data point for the 2:1 Synth1:Synth2 mixture in Figure 15D. In this case, substantial variability is seen in the triplicated allele ratio measurements (reflected by the large standard deviation). In this case, the source of variability likely occurred at one or more of the triplicated reactions (1st round PCR, 2nd round PCR, primer extension reaction or peak detection and area calculation).

### Example 8. Use of RNA controls to determine relative abundance of allele-specific gene expression in blood samples.

Genomic DNAs extracted from two whole blood samples (A and B) were genotyped using the single-nucleotide primer extension assay described in Example 5. Sample A was heterozygous (and, therefore, informative) for SNPs represented by SEQ ID NOs: 3, 11 and 12. Sample B was heterozygous (and, therefore, informative) for SNPs represented by SEQ IDs: 5 and 16 (Table 11).

Total RNA was extracted from the same two blood samples (A and B). For each, 500 ng of RNA was used as template for first-strand cDNA synthesis as described in Example 7. A control reaction lacking reverse transcriptase was performed to ensure that downstream amplification was derived from cDNA rather than potential contaminating genomic DNA. Following first-strand cDNA synthesis, 1 µL of each RT+ or RT- reaction was used as template for nested PCR amplification of the four amplicons, as described in Example 7 and diagrammed in Figure 13. An aliquot of each PCR reaction was analyzed on an agarose gel to confirm that no amplification was present in reactions derived from the RT- control reaction. Amplicons were individually purified on PCR purification spin columns (Qiagen), and used as template for the multiplexed single-nucleotide primer extension assay described in Example 5. The resulting raw peak areas and raw peak area ratios are provided in Table 11.

Because Sample A was heterozygous at the SNP positions represented by SEQ ID NOs: 3, 11 and 12, these three specific SNP-based assays were informative for measuring LOI of *IGF2* (Table 11). In the cDNAs tested for Sample A, the single nucleotide primer extension assay for SEQ ID NO: 3 detected a peak only for the C allele (no peak was detected for the A allele). In the cDNAs tested for Sample A, the single nucleotide primer extension assay for SEQ ID NO: 11 detected a peak only for the G allele (no peak was detected for the A allele). In the cDNAs tested for Sample A, the single nucleotide primer extension assay for SEQ ID NO: 12 detected a peak only for the C allele (no peak was detected for the T allele). Therefore, this sample was determined to exhibit normal imprinting of *IGF2.* That is, only one of the two alleles of the IGF2 gene was expressed. This allele includes the nucleotides C, G and C at the SNP positions represented by SEQ ID NO: 3, 11 and 12, respectively. The other allele (including the nucleotides A, A and T at the SNP positions represented by SEQ ID NO: 3, 11 and 12, respectively) was imprinted and no expression was detected.

Sample B was heterozygous at the SNP positions represented by SEQ ID NOs: 5 and 16 (Table 11). For the cDNAs tested for Sample B, the single nucleotide primer extension assay for SEQ ID NO: 16 detected a peak only for the A allele (no peak was detected for the G allele). However, the single nucleotide primer extension assay for SEQ ID NO: 5 detected peaks above background for both alleles (G and A, respectively). Note that these nucleotides represent the complementary strand relative to the sequence indicated in SEQ ID NO: 5. The ratio of Peak Area 1:Peak Area 2 was 0.445. In some reports, loss of imprinting *of IGF2* is considered to be present if the ratio of the two detected alleles is between 1:3 and 3:1 (*i.e.,* ratios between 0.333 and 3.0). This raw result for Sample B would seem to indicate that SNP assays for SEQ ID NO: 5 and SEQ ID NO: 16 reported different LOI results (i.e. SEQ ID NO: 16 reported normal imprinting and SEQ ID NO: 5 reported LOI). However, the ratio of 0.445 was not corrected by extrapolating against the standard control series of known input RNA allele ratios shown in Figure 15B. The linear equation for this control data series is y = 0.8717x + 0.2032. The measure ratio, y, is 0.445. Therefore, by using this value to solve for the input ratio, x, the corrected value was determined to be 0.278. Therefore, by the definition of LOI as described above (0.333-3.0), this assay indicated that Sample B does not display LOI *of IGF2.*

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

**Table 1. Previously Unpublished Single Nucleotide Polymorphisms in IGF2 exon 9.**

| SEQ ID NO: | Genomic Position* | Alleles | Sequence |
|---|---|---|---|
| 1 | 2108417 | [T/G] | TTCCCCCTCTTTGTTTCTTGGGGCA[T/G]TTTTCCTTTTTTTTTTTTTTTTGTT |
| 2 | 2110187 | [C/T/G/A] | GGACCCCAGAAATCACAGGTGGGCA[C/T/G/A]GTCGCTGCTACCGCCATCTCCCTTC |
| 3 | 2109220 | [A/C] | GCGCACACACACGCACACCCCCACA[A/C]AATTGGATGAAAACAATAAGCATAT |
| 4 | 2108843 | [T/C] | CATTCCCGATACACCTTACTTACTG[T/C]GTGTTGGCCCAGCCAGAGTGAGGAA |
| 5 | 2108835 | [C/T] | ATACACCTTACTTACTGTGTGTTGG[C/T]CCAGCCAGAGTGAGGAAGGAGTTTG |
| 6 | 2107668 | [G/A] | ATGCCATAGCAGCCACCACCGCGGC[G/A]CCTAGGGCTGCGGCAGGGACTCGGC |

| | | | |
|---|---|---|---|
| * Coordinates relative to NCBI Build 36, Chr:11 | | | |

**Table 2. Previously Identified Single Nucleotide Polymorphisms in IGF2 exon 9.**

| SEQ ID NO: | Genomic Position* | dbSNP ID | Alleles | Sequence |
|---|---|---|---|---|
| 7 | 2110764 | rs2230949 | [C/T] | AAACTGCCGCAAGTCTGCAGCCCGG[C/T]GCCACCATCCTGCAGCCTCCTCCTG |
| 8 | 2110210 | rs680 | [G/A] | CTGAACCAGCAAAGAGAAAAGAAGG[G/A]CCCCAGAAATCACAGGTGGGCACGT |
| 9 | 2109215 | rs1065687 | [G/A] | CACACACGCACACCCCCACAAAATT[G/A]GATGAAAACAATAAGCATATCTAAG |
| 10 | 2109117 | rs3168310 | [C/G] | CCAATGTTTTCATGGTCTGAGCCCC[C/G]CTCCTGTTCCCATCTCCACTGCCCC |
| 11 | 2108682 | rs57156844 | [C/T] | ACATTTCTTGGGGGGTCCCCAGGAGA[C/T]GGGCAAAGATGATCCCTAGGTGTGC |
| 12 | 2108628 | rs3802971 | [C/T] | AGTCCTCGGGGGCCGTGCACTGATG[C/T]GGGGAGTGTGGGAAGTCTGGCGGTT |
| 13 | 2107971 | rs11825733 | [G/A] | AGGCTGGCCGGAGGGGAAGGGGCTA[G/A]CAGGTGTGTA.AACAGAGGGTTCCAT |
| 14 | 2107471 | rs11564732 | [G/A] | AGTCGCAGAGGGTCCCTCGGCAAGC[G/A]CCCTGTGAGTGGGCCATTCGGAACA |
| 15 | 2107273 | rs7873 | [A/G] | GTGTTCCCGGGGGCACTTGCCGACC[A/G]GCCCCTTGCGTCCCCAGGTTTGCAG |
| 16 | 2107020 | rs2585 | [G/A] | TGCGGCCCGTGTTTGACTCAACTCA[G/A]CTCCTTTAACGCTAATATTTCCGGC |

| | | | | |
|---|---|---|---|---|
| * Coordinates relative to NCBI Build 36, Chr:11 | | | | |

**Table 3. Observed Heterozygosity of Transcribed IGF2 SNPs in Human Populations**

| | | Overall | | AA | | CAU | | MEX | | JPN | | CHI | |
|---|---|---|---|---|---|---|---|---|---|---|---|---|---|
| SEQ ID NO: | Genomic Position* | % het | het of total | %het | het of total | %het | het of total | %het | het of total | %het | het of total | %het | het of total |
| 1 | 2108417 | 16.1% | 36 of 223 | 16.2% | 12 of 74 | 18.8% | 15 of 80 | 13.0% | 9 of 69 | --- | --- | --- | --- |
| 2 | 2110187 | 31.1% | 23 of 74 | 31.1% | 23 of 74 | --- | --- | --- | --- | --- | --- | --- | --- |
| 3 | 2109220 | 24.3% | 18 of 74 | 24.3% | 18 of 74 | --- | --- | --- | --- | --- | --- | --- | --- |
| 4 | 2108843 | 6.8% | 5 of 74 | 6.8% | 5 of 74 | --- | --- | --- | --- | --- | --- | --- | --- |
| 5 | 2108835 | 14.0% | 21 of 150 | --- | --- | 14.0% | 21 of 150 | --- | --- | --- | --- | --- | --- |
| 6 | 2107668 | 6.7% | 10 of 150 | --- | --- | --- | --- | --- | --- | 2.5% | 2 of 79 | 11.3% | 8 of 71 |
| 7 | 2110764 | 11.9% | 35 of 293 | 14.9% | 11 of 74 | 10.7% | 16 of 150 | 11.6% | 8 of 69 | --- | --- | --- | --- |
| 8 | 2110210 | 45.6% | 202 of 443 | 18.9% | 14 of 74 | 54.7% | 82 of 150 | 31.9% | 22 of 69 | 57.0% | 45 of 79 | 54.9% | 39 of 71 |
| 9 | 2109215 | 9.1% | 20 of 219 | --- | --- | 12.7% | 19 of 150 | 1.4% | 1 of 69 | --- | --- | --- | --- |
| 10 | 2109117 | 35.0% | 155 of 443 | 28.4% | 21 of 74 | 36.7% | 55 of 150 | 33.3% | 23 of 69 | 30.4% | 24 of 79 | 45.1% | 32 of 71 |
| 11 | 2108682 | 20.3% | 29 of 143 | 37.8% | 28 of 74 | --- | --- | 1.4% | 1 of 69 | --- | --- | --- | --- |
| 12 | 2108628 | 15.3% | 68 of 443 | 9.5% | 7 of 74 | 10.7% | 16 of 150 | 13.0% | 9 of 69 | 21.5% | 17 of 79 | 26.8% | 19 of 71 |
| 13 | 2107971 | 14.9% | 11 of 74 | 14.9% | 11 of 74 | --- | --- | --- | --- | --- | --- | --- | --- |
| 14 | 2107471 | 7.0% | 21 of 298 | --- | --- | 0.7% | 1 of 150 | 20.3% | 14 of 69 | 7.6% | 6 of 79 | --- | --- |
| 15 | 2107273 | 20.5% | 60 of 293 | 35.1% | 26 of 74 | 16.0% | 24 of 150 | 14.5% | 10 of 69 | --- | --- | --- | --- |
| 16 | 2107020 | 45.1% | 200 of 443 | 21.6% | 16 of 74 | 41.3% | 62 of 150 | 37.7% | 26 of 69 | 63.3% | 50 of 79 | 64.8% | 46 of 71 |

**Table 4. Observed Heterozygosity for Combinations of Transcribed IGF2 SNPs in Human Populations**

| Human Group | # Het | # Total | Panel A | Panel B | Panel C | Panel D | Panel E |
|---|---|---|---|---|---|---|---|
| Overall | 358 | 443 | 80.81% | 78.56% | 78.56% | 75.17% | 61.17% |
| Chinese | 52 | 71 | 73.24% | 71.83% | 71.83% | 69.01% | 70.42% |
| Japanese | 59 | 79 | 74.68% | 74.68% | 74.68% | 72.15% | 73.42% |
| Mexican | 51 | 69 | 73.91% | 71.01% | 72.46% | 68.12% | 60.87% |
| African American | 68 | 74 | 91.89% | 82.43% | 81.08% | 78.38% | 31.08% |
| Caucasian | 128 | 150 | 85.33% | 85.33% | 85.33% | 81.33% | 65.33% |

**Table 5. Seq ID NOs for SNP combinations described in Table 4.**

| Panel: | Panel A | Panel B | Panel C | Panel D | Panel E |
|---|---|---|---|---|---|
| | 1 | 1 | 1 | 1 | 8 |
| | 2 | 2 | 5 | 5 | 11 |
| | 3 | 4 | 8 | 8 | 14 |
| | 4 | 5 | 9 | 9 | 16 |
| | 5 | 8 | 10 | 10 | |
| | 6 | 9 | 11 | 11 | |
| | 7 | 10 | 12 | 14 | |
| | 8 | 12 | 14 | 15 | |
| | 9 | 13 | 15 | 16 | |
| | 10 | 14 | 16 | | |
| | 11 | 15 | | | |
| | 12 | 16 | | | |
| | 13 | | | | |
| | 14 | | | | |
| | 15 | | | | |
| | 16 | | | | |
| Total # SNPs: | 16 | 12 | 10 | 9 | 4 |

**Table 6. Linearity of assays measuring allele-specific rations of 13 independent SNPs**

| **SEQ ID** | **R²** | **slope** |
|---|---|---|
| 2 | 0.9986 | 0.8256 |
| 3 | 0.9817 | 0.5946 |
| 4 | 0.9995 | 0.8973 |
| 5 | 0.9963 | 0.8351 |
| 6 | 0.9757 | 0.6219 |
| 7 | 0.9949 | 0.7825 |
| 8 | 0.9915 | 0.8494 |
| 9 | 0.9949 | 0.8297 |
| 10 | 0.9964 | 0.814 |
| 11 | 0.9988 | 0.8681 |
| 14 | 0.9987 | 0.835 |
| 15 | 0.9953 | 0.818 |
| 16 | 0.9984 | 0.8531 |

**Table 7. Results of Multiplexed Assay for IGF2 Genotype and Imprinting Status.**

| | | Genomic DNA* | | Total PNA^{†} | | | |
|---|---|---|---|---|---|---|---|
| Sample | SEQ ID NO: | Allele 1 | Allele 2 | Allele 1 | Allele 2 | Genotype^{‡} | Expression^{a} |
| A | 1 | T | | T | | hom | n/a |
| A | 2 | G | | G | | hom | n/a |
| A | 3 | G | T | | T | informative | imprinted |
| A | 4 | T | | T | | hom | n/a |
| A | 5 | G | | G | | hom | n/a |
| A | 6 | G | | G | | hom | n/a |
| A | 7 | G | | G | | hom | n/a |
| A | 8 | G | | G | | hom | n/a |
| A | 9 | G | | G | | hom | n/a |
| A | 10 | C | | C | | hom | n/a |
| A | 11 | G | A | G | | informative | imprinted |
| A | 12 | C | T | C | | informative | imprinted |
| A | 13 | G | | G | | hom | n/a |
| A | 14 | G | | G | | hom | n/a |
| A | 15 | A | | A | | hom | n/a |
| A | 16 | G | | G | | hom | n/a |
| B | 1 | T | | T | | hom | n/a |
| B | 2 | | T | | T | hom | n/a |
| B | 3 | G | | G | | hom | n/a |
| B | 4 | T | | T | | hom | n/a |
| B | 5 | G | A | G | A | informative | borderline |
| B | 6 | G | | G | | hom | n/a |
| B | 7 | G | | G | | hom | n/a |
| B | 8 | G | | G | | hom | n/a |
| B | 9 | G | | G | | hom | n/a |
| B | 10 | C | | C | | hom | n/a |
| B | 11 | G | | G | | hom | n/a |
| B | 12 | C | | C | | hom | n/a |
| B | 13 | G | | G | | hom | n/a |
| B | 14 | G | | G | | hom | n/a |
| B | 15 | A | | A | | hom | n/a |
| B | 16 | G | A | G | | informative | imprinted |

| | | | | | | | |
|---|---|---|---|---|---|---|---|
| *Genotypes at each of the ten assayed SNPs. † Alleles detected in expressed *IGF2* transcripts. ‡ Indicates homozygous (hom) or heterozygous and informative (informative) at each SNP. ^{a}Imprinted: monoallelic expression of *IGF2*; n/a: result not applicable due to homozygosity at the particular SNP. | | | | | | | |

**Table 8. Comparisons of Peak Areas for IGF2 Genotype and Imprinting Status Analysis.**

| | | Genomic DNA* | | | | Total RNA^{†} | | |
|---|---|---|---|---|---|---|---|---|
| Sample | SEQ ID NO: | Peak area 1 | Peak Area 2 | Allele 1: Allele 2^{a} | Normalization Factor^{b} | Peak Area 1 | Peak Area 2 | Allele 1: Allele 2^{c} |
| A | 1 | 58328 | 0 | - | | 78730 | 0 | - |
| A | 2 | 153330 | 0 | - | | 126343 | 0 | - |
| A | 3 | 67871 | 30193 | 2.2479 | 0.4449 | 0 | 36005 | - |
| A | 4 | 5030 | 0 | - | | 3669 | 0 | - |
| A | 5 | 40432 | 0 | - | | 28478 | 0 | - |
| A | 6 | 25890 | 0 | - | | 9038 | 0 | - |
| A | 7 | 134730 | 0 | - | | 120747 | 0 | - |
| A | 8 | 88657 | 0 | - | | 77231 | 0 | - |
| A | 9 | 22926 | 0 | - | | 39965 | 0 | - |
| A | 10 | 79426 | 0 | - | | 48415 | 0 | - |
| A | 11 | 97000 | 106229 | 0.9131 | 1.0951 | 103671 | 0 | - |
| A | 12 | 54813 | 163646 | 0.3349 | 2.9855 | 59419 | 0 | - |
| A | 13 | 40100 | 0 | - | | 33286 | 0 | - |
| A | 14 | 10205 | 0 | - | | 6230 | 0 | - |
| A | 15 | 96528 | 0 | - | | 49735 | 0 | - |
| A | 16 | 136263 | 0 | - | | 138675 | 0 | - |
| B | 1 | 60360 | 0 | - | | 71841 | 0 | - |
| B | 2 | 0 | 124496 | - | | 0 | 79158 | - |
| B | 3 | 37814 | 0 | - | | 35854 | 0 | - |
| B | 4 | 3850 | 0 | - | | 4377 | 0 | - |
| B | 5 | 16043 | 12107 | 1.3251 | 0.7547 | 6283 | 14115 | 0.3359 |
| B | 6 | 19750 | 0 | - | | 8647 | 0 | - |
| B | 7 | 95420 | 0 | - | | 96206 | 0 | - |
| B | 8 | 61002 | 0 | - | | 38661 | 0 | - |
| B | 9 | 29844 | 0 | - | | 54430 | 0 | - |
| B | 10 | 50863 | 0 | - | | 48856 | 0 | - |
| B | 11 | 109546 | 0 | - | | 112126 | 0 | - |
| B | 12 | 60364 | 0 | - | | 61683 | 0 | - |
| B | 13 | 39767 | 0 | - | | 38418 | 0 | - |
| B | 14 | 14754 | 0 | - | | 9525 | 0 | - |
| B | 15 | 110227 | 0 | - | | 55892 | 0 | - |
| B | 16 | 63791 | 58086 | 1.0982 | 0.9106 | 108307 | 0 | - |

| | | | | | | | | |
|---|---|---|---|---|---|---|---|---|
| *Data for Genotype assays. ^{†}Data for Allele-Specific Expression Analysis. ^{a}Calculated ratio of genotyping Peak Area 1/Genotyping Peak Area 2 ^{b}Normalization to account for dye intensity differences (1/(Genotyping Peak Area 1/Genotyping Peak Area 2)) ^{c}Normalized calculated ratio for expression Peak Area 1/expression Peak Area 2 ((Expression Peak Area 1/Expression Peak Area 2)*Normalization Factor) | | | | | | | | |

**Table 9. SNP positions and genotypes in Synth1 and Synth2**

| SEQ ID NO: | Genomic Position^{a} | Synth1 or Synth2 Position^{b} | Possible Alleles | Synth1 Allele | Synth2 Allele | Sequence |
|---|---|---|---|---|---|---|
| 1 | 2108417 | 2089 | [T/G] | T | G | TTCCCCCTCTTTGTTTCTTGGGGCA[T/G]TTTTCCTTTTTTTTTTTTTTTTGTT |
| 2 | 2110187 | 897 | [C/T] | C | T | GGACCCCAGAAATCACAGGTGGGCA[C/T/G/A]GTCGCTGCTACCGCCATCTCCCTTC |
| 3 | 2109220 | 1286 | [A/C] | A | C | GCGCACACACACGCACACCCCCACA[A/C]AATTGGATGAAAACAATAAGCATAT |
| 4 | 2108843 | 1663 | [T/C] | T | C | CATTCCCGATACACCTTACTTACTG[T/C]GTGTTGGCCCAGCCAGAGTGAGGAA |
| 5 | 2108835 | 1671 | [C/T] | C | T | ATACACCTTACTTACTGTGTGTTGG[C/T]CCAGCCAGAGTGAGGAAGGAGTTTG |
| 6 | 2107668 | 2838 | [G/A] | G | A | ATGCCATAGCAGCCACCACCGCGGC[G/A]CCTAGGGCTGCGGCAGGGACTCGGC |
| 7 | 2110764 | 320 | [C/T] | C | T | AAACTGCCGCAAGTCTGCAGCCCGG[C/T]GCCACCATCCTGCAGCCTCCTCCTG |
| 8 | 2110210 | 874 | [G/A] | A | G | CTGAACCAGCAAAGAGAAAAGAAGG[G/A]CCCCAGAAATCACAGGTGGGCACGT |
| 9 | 2109215 | 1291 | [G/A] | G | A | CACACACGCACACCCCCACAAAATT[G/A]GATGAAAACAATAAGCATATCTAAG |
| 10 | 2109117 | 1389 | [C/G] | G | C | CCAATGTTTTCATGGTCTGAGCCCC[C/G]CTCCTGTTCCCATCTCCACTGCCCC |
| 11 | 2108682 | 1824 | [C/T] | G^{c} | A^{c} | ACATTTCTTGGGGGGTCCCCAGGAGA[C/T]GGGCAAAGATGATCCCTAGGTGTGC |
| 12 | 2108628 | 1878 | [C/T] | C | T | AGTCCTCGGGGGCCGTGCACTGATG[C/T]GGGGAGTGTGGGAAGTCTGGCGGTT |
| 13 | 2107971 | 2535 | [G/A] | G | A | AGGCTGGCCGGAGGGGAAGGGGCTA[G/A]CAGGTGTGTAAACAGAGGGTTCCAT |
| 14 | 2107471 | 3035 | [G/A] | G | A | AGTCGCAGAGGGTCCCTCGGCAAGC[G/A]CCCTGTGAGTGGGCCATTCGGAACA |
| 15 | 2107273 | 3233 | [A/G] | A | G | GTGTTCCCGGGGGCACTTGCCGACC[A/G]GCCCCTTGCGTCCCCAGGTTTGCAG |
| 16 | 2107020 | 3486 | [G/A] | A | G | TGCGGCCCGTGTTTGACTCAACTCA[G/A]CTCCTTTAACGCTAATATTTCCGGC |

| | | | | | | |
|---|---|---|---|---|---|---|
| ^{a} Coordinates relative to NCBI Build 36, Chr:11 ^{b} Coordinates relative to SEQ ID: 18 and SEQ ID: 20 ^{c} Sequence provided in "Sequence" and "Possible Alleles" columns correspond to the opposite strand relative to that shown in "Synth1 Allele" and "Synth2 Allele" columns. | | | | | | |

**Table 10. SNP-based primer extension assay confirmation of SNP alleles of Synth1 and Synth2**

| Synth Control^{a} | SEQ ID of SNP | Genomic Position^{b} | Designed Allele | Detected Allele 1 | Detected Allele 2 | Peak Area Allele 1 | Peak Area Allele 2 | Genotype Call |
|---|---|---|---|---|---|---|---|---|
| 1 | 1 | 2108417 | T | T | - | 67148 | 0 | T |
| 1 | 2 | 2110187 | C | G* | - | 63747 | 0 | G* |
| 1 | 3 | 2109220 | A | A | - | 150505 | 0 | A |
| 1 | 4 | 2108843 | T | T | - | 57166 | 0 | T |
| 1 | 5 | 2108835 | C | G* | - | 34768 | 0 | G* |
| 1 | 6 | 2107668 | G | G | - | 17764 | 0 | G |
| 1 | 7 | 2110764 | C | G* | - | 57660 | 0 | G* |
| 1 | 8 | 2110210 | A | A | - | 165497 | 0 | A |
| 1 | 9 | 2109215 | G | C* | - | 102012 | 0 | C* |
| 1 | 10 | 2109117 | G | G | - | 174460 | 0 | G |
| 1 | 11 | 2108682 | Gc | G c | - | 54188 | 0 | G |
| 1 | 12 | 2108628 | C | C | - | 101444 | 0 | C |
| 1 | 13 | 2107971 | G | G | - | 31305 | 0 | G |
| 1 | 14 | 2107471 | G | C* | - | 42605 | 0 | C* |
| 1 | 15 | 2107273 | A | A | - | 68516 | 0 | A |
| 1 | 16 | 2107020 | A | A | - | 76774 | 0 | A |
| 2 | 1 | 2108417 | G | - | G | 0 | 86007 | G |
| 2 | 2 | 2110187 | T | - | A* | 0 | 87563 | A* |
| 2 | 3 | 2109220 | C | - | C | 0 | 80903 | C |
| 2 | 4 | 2108843 | C | - | C | 0 | 45169 | C |
| 2 | 5 | 2108835 | T | - | A* | 0 | 46015 | A* |
| 2 | 6 | 2107668 | A | - | A | 0 | 33683 | A |
| 2 | 7 | 2110764 | T | - | A* | 0 | 72463 | A* |
| 2 | 8 | 2110210 | G | - | G | 0 | 93147 | G |
| 2 | 9 | 2109215 | A | - | T* | 0 | 92444 | T* |
| 2 | 10 | 2109117 | C | - | C | 0 | 98591 | C |
| 2 | 11 | 2108682 | A c | - | A c | 0 | 115660 | A* |
| 2 | 12 | 2108628 | T | - | T | 0 | 98328 | T |
| 2 | 13 | 2107971 | A | - | A | 0 | 50002 | A |
| 2 | 14 | 2107471 | A | - | T* | 0 | 63342 | T* |
| 2 | 15 | 2107273 | G | A | G | 10393 | 27474 | G |
| 2 | 16 | 2107020 | G | - | G | 0 | 45753 | G |

| | | | | | | | | |
|---|---|---|---|---|---|---|---|---|
| ^{a} Indicates genotyping reaction for either Synth 1 (1) or Synth 2 (2) ^{b}Genomic position of SNP relative to NCBI Build 36, Chr:11 ^{c} Synth1 and Synth2 allele designation reflects the opposite strand relative to the sequence shown in SEQ ID NO: 11 * Genotyping assay reports the opposite strand relative to the sequence shown in the SEQ ID sequence | | | | | | | | |

**Table 11. Comparisons of Peak Areas for IGF2 Genotype and Imprinting Status Analysis.**

| | | Genomic DNA ^{a} | | | Total RNA ^{b} | | | |
|---|---|---|---|---|---|---|---|---|
| Sample | SEQ ID NO: | Peak area 1 | Peak Area 2 | Genotype | Peak Area 1 | Peak Area 2 | Allele 1: Allele 2 | Corrected Ratio ^{c} |
| A | 1 | 58328 | 0 | - | 78730 | 0 | - | - |
| A | 2 | 153330 | 0 | - | 126343 | 0 | - | - |
| A | 3 | 67871 | 30193 | het | 0 | 36005 | - | - |
| A | 4 | 5030 | 0 | - | 3669 | 0 | - | - |
| A | 5 | 40432 | 0 | - | 28478 | 0 | - | - |
| A | 6 | 25890 | 0 | - | 9038 | 0 | - | - |
| A | 7 | 134730 | 0 | - | 120747 | 0 | - | - |
| A | 8 | 88657 | 0 | - | 77231 | 0 | - | - |
| A | 9 | 22926 | 0 | - | 39965 | 0 | - | - |
| A | 10 | 79426 | 0 | - | 48415 | 0 | - | - |
| A | 11 | 97000 | 106229 | het | 103671 | 0 | - | - |
| A | 12 | 54813 | 163646 | het | 59419 | 0 | - | - |
| A | 13 | 40100 | 0 | - | 33286 | 0 | - | - |
| A | 14 | 10205 | 0 | - | 6230 | 0 | - | - |
| A | 15 | 96528 | 0 | - | 49735 | 0 | - | - |
| A | 16 | 136263 | 0 | - | 138675 | 0 | - | - |
| B | 1 | 60360 | 0 | - | 71841 | 0 | - | - |
| B | 2 | 0 | 124496 | - | 0 | 79158 | - | - |
| B | 3 | 37814 | 0 | - | 35854 | 0 | - | - |
| B | 4 | 3850 | 0 | - | 4377 | 0 | - | - |
| B | 5 | 16043 | 12107 | het | 6283 | 14115 | 0.445 | 0.000 |
| B | 6 | 19750 | 0 | - | 8647 | 0 | - | - |
| B | 7 | 95420 | 0 | - | 96206 | 0 | - | - |
| B | 8 | 61002 | 0 | - | 38661 | 0 | - | - |
| B | 9 | 29844 | 0 | - | 54430 | 0 | - | - |
| B | 10 | 50863 | 0 | - | 48856 | 0 | - | - |
| B | 11 | 109546 | 0 | - | 112126 | 0 | - | - |
| B | 12 | 60364 | 0 | - | 61683 | 0 | - | - |
| B | 13 | 39767 | 0 | - | 38418 | 0 | - | - |
| B | 14 | 14754 | 0 | - | 9525 | 0 | - | - |
| B | 15 | 110227 | 0 | - | 55892 | 0 | - | - |
| B | 16 | 63791 | 58086 | het | 108307 | 0 | - | - |

| | | | | | | | | |
|---|---|---|---|---|---|---|---|---|
| ^{a}Data for Genotype assays. ^{b} Data for Allele-Specific Expression Analysis. ^{c} Ratio corrected by extrapolating ratio value from the RNA control standard curve shown in Figure X+3 | | | | | | | | |

### SEQUENCE TABLE

SEQ ID NO:1
   TTCCCCCTCTTTGTTTCTTGGGGCA[T/G]TTTTCCTTTTTTTTTTTTTTTTGTT

SEQ ID NO:2
GGACCCCAGAAATCACAGGTGGGCA[C/T/G/A]GTCGCTGCTACCGCCATCTCCCT
TC

SEQ ID NO:3
   GCGCACACACACGCACACCCCCACA[A/C]AATTGGATGAAAACAATAAGCATAT
SEQ ID NO:4
   CATTCCCGATACACCTTACTTACTG[T/C]GTGTTGGCCCAGCCAGAGTGAGGAA
SEQ ID NO:5
   ATACACCTTACTTACTGTGTGTTGG[C/T]CCAGCCAGAGTGAGGAAGGAGTTTG
SEQ ID NO:6
   ATGCCATAGCAGCCACCACCGCGGC[G/A]CCTAGGGCTGCGGCAGGGACTCGGC
SEQ ID NO:7
   AAACTGCCGCAAGTCTGCAGCCCGG[C/T]GCCACCATCCTGCAGCCTCCTCCTG

SEQ ID NO:8
CTGAACCAGCAAAGAGAAAAGAAGG[G/A]CCCCAGAAATCACAGGTGGGCACG
T

SEQ ID NO:9
   CACACACGCACACCCCCACAAAATT[G/A]GATGAAAACAATAAGCATATCTAAG
SEQ ID NO: 10
   CCAATGTTTTCATGGTCTGAGCCCC[C/G]CTCCTGTTCCCATCTCCACTGCCCC

SEQ ID NO:11
ACATTTCTTGGGGGGTCCCCAGGAGA[C/T]GGGCAAAGATGATCCCTAGGTGTG
C

SEQ ID NO:12
   AGTCCTCGGGGGCCGTGCACTGATG[C/T]GGGGAGTGTGGGAAGTCTGGCGGTT

SEQ ID NO:13
AGGCTGGCCGGAGGGGAAGGGGCTA[G/A]CAGGTGTGTAAACAGAGGGTTCCA
T

SEQ ID NO:14
   AGTCGCAGAGGGTCCCTCGGCAAGC[G/A]CCCTGTGAGTGGGCCATTCGGAACA
SEQ ID NO:15
   GTGTTCCCGGGGGCACTTGCCGACC[A/G]GCCCCTTGCGTCCCCAGGTTTGCAG
SEQ ID NO:16
   TGCGGCCCGTGTTTGACTCAACTCA[G/A]CTCCTTTAACGCTAATATTTCCGGC

SEQ ID NO:17
NM_000612
1 cgcctgtccc cctcccgagg cccgggctcg cgacggcaga gggctccgtc
ggcccaaacc
61 gagctgggcg cccgcggtcc gggtgcagcc tccactccgc cccccagtca
ccgcctcccc
121 cggcccctcg acgtggcgcc cttccctccg cttctctgtg ctccccgcgc
ccctcttggc
181 gtctggcccc ggcccccgct ctttctcccg caaccttccc ttcgctccct
cccgtccccc
241 ccagctccta gcctccgact ccctcccccc ctcacgcccg ccctctcgcc
ttcgccgaac
301 caaagtggat taattacacg ctttctgttt ctctccgtgc tgttctctcc
cgctgtgcgc
361 ctgcccgcct ctcgctgtcc tctctccccc tcgccctctc ttcggccccc
ccctttcacg
421 ttcactctgt ctctcccact atctctgccc ccctctatcc ttgatacaac
agctgacctc
481 atttcccgat accttttccc ccccgaaaag tacaacatct ggcccgcccc
agcccgaaga
541 cagcccgtcc tccctggaca atcagacgaa ttctcccccc cccccaaaa
aaaagccatc
601 cccccgctct gccccgtcgc acattcggcc cccgcgactc ggccagagcg
gcgctggcag
661 aggagtgtcc ggcaggaggg ccaacgcccg ctgttcggtt tgcgacacgc
agcagggagg
721 tgggcggcag cgtcgccggc ttccagacac caatgggaat cccaatgggg
aagtcgatgc
781 tggtgcttct caccttcttg gccttcgcct cgtgctgcat tgctgcttac
cgccccagtg
841 agaccctgtg cggcggggag ctggtggaca ccctccagtt cgtctgtggg
gaccgcggct
901 tctacttcag caggcccgca agccgtgtga gccgtcgcag ccgtggcatc
gttgaggagt
961 gctgtttccg cagctgtgac ctggccctcc tggagacgta ctgtgctacc
cccgccaagt
1021 ccgagaggga cgtgtcgacc cctccgaccg tgcttccgga caacttcccc
agataccccg
1081 tgggcaagtt cttccaatat gacacctgga agcagtccac ccagcgcctg
cgcaggggcc
1141 tgcctgccct cctgcgtgcc cgccggggtc acgtgctcgc caaggagctc
gaggcgttca
1201 gggaggccaa acgtcaccgt cccctgattg ctctacccac ccaagacccc
gcccacgggg
1261 gcgccccccc agagatggcc agcaatcgga agtgagcaaa actgccgcaa
gtctgcagcc
1321 cggcgccacc atcctgcagc ctcctcctga ccacggacgt ttccatcagg
ttccatcccg
1381 aaaatctctc ggttccacgt ccccctgggg cttctcctga cccagtcccc
gtgccccgcc
1441 tccccgaaac aggctactct cctcggcccc ctccatcggg ctgaggaagc
acagcagcat
1501 cttcaaacat gtacaaaatc gattggcttt aaacaccctt cacataccct
ccccccaaat
1561 tatccccaat tatccccaca cataaaaaat caaaacatta aactaacccc
cttccccccc

1621 ccccacaaca accctcttaa aactaattgg ctttttagaa acaccccaca
aaagctcaga
1681 aattggcttt aaaaaaaaca accaccaaaa aaaatcaatt ggctaaaaaa
aaaaagtatt
1741 aaaaacgaat tggctgagaa acaattggca aaataaagga atttggcact
ccccaccccc
1801 ctctttctct tctcccttgg actttgagtc aaattggcct ggacttgagt
ccctgaacca
1861 gcaaagagaa aagaaggacc ccagaaatca caggtgggca cgtcgctgct
accgccatct
1921 cccttctcac gggaattttc agggtaaact ggccatccga aaatagcaac
aacccagact
1981 ggctcctcac tcccttttcc atcactaaaa atcacagagc agtcagaggg
acccagtaag
2041 accaaaggag gggaggacag agcatgaaaa ccaaaatcca tgcaaatgaa
atgtaattgg
2101 cacgaccctc acccccaaat cttacatctc aattcccatc ctaaaaagca
ctcatacttt
2161 atgcatcccc gcagctacac acacacaaca cacagcacac gcatgaacac
agcacacaca
2221 cgagcacagc acacacacaa acgcacagca cacacagcac acagatgagc
acacagcaca
2281 cacacaaacg cacagcacac acacgcacac acatgcacac acagcacaca
aacgcacggc
2341 acacacacgc acacacatgc acacacagca cacacacaaa cgcacagcac
acacaaacgc
2401 acagcacaca cgcacacaca gcacacacac gagcacacag cacacaaacg
cacagcacac
2461 gcacacacat gcacacacag cacacacact agcacacagc acacacacaa
agacacagca
2521 cacacatgca cacacagcac acacacgcga acacagcaca cacgaacaca
gcacacacag
2581 cacacacaca aacacagcac acacatgcac acagcacacg cacacacagc
acacacatga
2641 acacagcaca cagcacacac atgcacacac agcacacacg catgcacagc
acacatgaac
2701 acagcacaca cacaaacaca cagcacacac atgcacacac agcacacaca
ctcatgcgca
2761 gcacatacat gaacacagct cacagcacac aaacacgcag cacacacgtt
gcacacgcaa
2821 gcacccacct gcacacacac atgcgcacac acacgcacac ccccacaaaa
ttggatgaaa
2881 acaataagca tatctaagca actacgatat ctgtatggat caggccaaag
tcccgctaag
2941 attctccaat gttttcatgg tctgagcccc gctcctgttc ccatctccac
tgcccctcgg
3001 ccctgtctgt gccctgcctc tcagaggagg gggctcagat ggtgcggcct
gagtgtgcgg
3061 ccggcggcat ttgggataca cccgtagggt gggcggggtg tgtcccaggc
ctaattccat
3121 ctttccacca tgacagagat gcccttgtga ggctggcctc cttggcgcct
gtccccacgg
3181 cccccgcagc gtgagccacg atgctcccca taccccaccc attcccgata
caccttactt
3241 actgtgtgtt ggcccagcca gagtgaggaa ggagtttggc cacattggag
atggcggtag
3301 ctgagcagac atgcccccac gagtagcctg actccctggt gtgctcctgg
aaggaagatc
3361 ttggggaccc ccccaccgga gcacacctag ggatcatctt tgcccgtctc
ctggggaccc

3421 cccaagaaat gtggagtcct cgggggccgt gcactgatgc ggggagtgtg
ggaagtctgg
3481 cggttggagg ggtgggtggg gggcagtggg ggctgggcgg ggggagttct
ggggtaggaa
3541 gtggtcccgg gagattttgg atggaaaagt caggaggatt gacagcagac
ttgcagaatt
3601 acatagagaa attaggaacc cccaaatttc atgtcaattg atctattccc
cctctttgtt
3661 tcttggggca tttttccttt tttttttttt tttgtttttt ttttacccct
ccttagcttt
3721 atgcgctcag aaaccaaatt aaaccccccc cccatgtaac aggggggcag
tgacaaaagc
3781 aagaacgcac gaagccagcc tggagaccac cacgtcctgc ccccgccat
ttatcgccct
3841 gattggattt tgtttttcat ctgtccctgt tgcttgggtt gagttgaggg
tggagcctcc
3901 tggggggcac tggccactga gcccccttgg agaagtcaga ggggagtgga
gaaggccact
3961 gtccggcctg gcttctgggg acagtggctg gtccccagaa gtcctgaggg
cggagggggg
4021 ggttgggcag ggtctcctca ggtgtcagga gggtgctcgg aggccacagg
agggggctcc
4081 tggctggcct gaggctggcc ggaggggaag gggctagcag gtgtgtaaac
agagggttcc
4141 atcaggctgg ggcagggtgg ccgccttccg cacacttgag gaaccctccc
ctctccctcg
4201 gtgacatctt gcccgcccct cagcaccctg ccttgtctcc aggaggtccg
aagctctgtg
4261 ggacctcttg ggggcaaggt ggggtgaggc cggggagtag ggaggtcagg
cgggtctgag
4321 cccacagagc aggagagctg ccaggtctgc ccatcgacca ggttgcttgg
gccccggagc
4381 ccacgggtct ggtgatgcca tagcagccac caccgcggcg cctagggctg
cggcagggac
4441 tcggcctctg ggaggtttac ctcgccccca cttgtgcccc cagctcagcc
cccctgcacg
4501 cagcccgact agcagtctag aggcctgagg cttctgggtc ctggtgacgg
ggctggcatg
4561 accccggggg tcgtccatgc cagtccgcct cagtcgcaga gggtccctcg
gcaagcgccc
4621 tgtgagtggg ccattcggaa cattggacag aagcccaaag agccaaattg
tcacaattgt
4681 ggaacccaca ttggcctgag atccaaaacg cttcgaggca ccccaaatta
cctgcccatt
4741 cgtcaggaca cccacccacc cagtgttata ttctgcctcg ccggagtggg
tgttcccggg
4801 ggcacttgcc gaccagcccc ttgcgtcccc aggtttgcag ctctcccctg
ggccactaac
4861 catcctggcc cgggctgcct gtctgacctc cgtgcctagt cgtggctctc
catcttgtct
4921 cctccccgtg tccccaatgt cttcagtggg gggccccctc ttgggtcccc
tcctctgcca
4981 tcacctgaag acccccacgc caaacactga atgtcacctg tgcctgccgc
ctcggtccac
5041 cttgcggccc gtgtttgact caactcaact cctttaacgc taatatttcc
ggcaaaatcc
5101 catgcttggg ttttgtcttt aaccttgtaa cgcttgcaat cccaataaag
cattaaaagt
5161 catgaaaaaa aaaaaaaaaa aa

SEQ ID NO:18

ENST00000300632
CCGCTAATGTACCATGCCCTGGTGCTGGAAAGTGCCTGAGCCAGCTGCCCCAGCGGCCTC
AGCACTACCAAGTTGGCACAAAGCTCCCCAAATTCGGAGGGGCTCAGGGAAACGAGTGGA
GGGGATGAGGAGGTGAGGGGTAAACCCATCATTTCAGTTGGCATTTGAGCAGGTGCCATG
CTCAGCGGAGATGAGGCTCTCCCATCTGTAGGGGCCGTATTAACATGCACACTCTAAAAG
TGCCCTTCGTTTCTCCAGCCTCAGCTTTGTCCCTCTCCTCCTCCACGTCAACCTGGCCAG
AGGGTCTGGACGCCACAGCCAGGGCACCCCCTGCTTTGGTGGTGACTGCTAATATTGGCC
AGGCCGGCGGATCATCGTCCAGGCAGTTTCGGCAGAGAGCCTTGGGCACCAGTGACTCCC
CGGTCCTCTTTATCCACTGTCCAGGAGCTGCGGGACTGCGCAGGGACTAGAGTACAGGG
GCCGAAGAGTCACCACCGAGCTTGTGTGGAGGAGGTGGATTCCAGCCCCCAGCCCCAGG
GCTCTGAATCGCTGCCAGCTCAGCCCCCTGCCCAGCCTGCCCCACAGCCTGAGCCCCAGC
AGGCCAGAGAGCCCAGTCCTGAGGTGAGCTGCTGTGGCCTGTGGCCCAGGCGACCCCAGC
GCTCCCAGAACTGAGGCTGGCAGCCAGCCCCAGCCTCAGCCCCAACTGCGAGGCAGAGAG
ACACCAATGGGAATCCCAATGGGAAGTCGATGCTGGTGCTTCTCACCTTCTTGGCCTTC
GCCTCGTGCTGCATTGCTGCTTACCGCCCCAGTGAGACCCTGTGCGGCGGGGAGCTGGTG
GACACCCTCCAGTTCGTCTGTGGGGACCGCGGCTTCTACTTCAGCAGGCCCGCAAGCCGT
GTGAGCCGTCGCAGCCGTGGCATCGTTGAGGAGTGCTGTTTCCGCAGCTGTGACCTGGCC
CTCCTGGAGACGTACTGTGCTACCCCCGCCAAGTCCGAGAGGGACGTGTCGACCCCTCCG
ACCGTGCTTCCGGACAACTTCCCCAGATACCCCGTGGGCAAGTTCTTCCAATATGACACC
TGGAAGCAGTCCACCCAGCGCCTGCGCAGGGGCCTGCCTGCCCTCCTGCGTGCCCGCCGG
GGTCACGTGCTCGCCAAGGAGCTCGAGGCGTTCAGGGAGGCCAAACGTCACCGTCCCCTG
ATTGCTCTACCCACCCAAGACCCCGCCCACGGGGGCGCCCCCCCAGAGATGGCCAGCAAT
CGGAAGTGAGCAAAACTGCCGCAAGTCTGCAGCCCGGCGCCACCATCCTGCAGCCTCCTC
CTGACCACGGACGTTTCCATCAGGTTCCATCCCGAAAATCTCTCGGTTCCACGTCCCCCT
GGGGCTTCTCCTGACCCAGTCCCCGTGCCCCGCCTCCCCGAAACAGGCTACTCTCCTCGG
CCCCCTCCATCGGGCTGAGGAAGCACAGCAGCATCTTCAAACATGTACAAAATCGATTGG
CTTTAAACACCCTTCACATACCCTCCCCCAAATTATCCCCAATTATCCCCACACATAAA
AAATCAAAACATTAAACTAACCCCCTTCCCCCCCCCCACAACAACCCTCTTAAAACTAA
TTGGCTTTTTAGAAACACCCCACAAAAGCTCAGAAATTGGCTTTAAAAAAAACAACCACC
AAAAAAAATCAATTGGCTAAAAAAAAAAAGTATTAAAAACGAATTGGCTGAGAAACAATT
GGCAAAATAAAGGAATTTGGCACTCCCCACCCCCCTCTTTCTCTTCTCCCTTGGACTTTG
AGTCAAATTGGCCTGGACTTGAGTCCCTGAACCAGCAAAGAGAAAAGAAGGACCCCAGAA
ATCACAGGTGGGCACGTCGCTGCTACCGCCATCTCCCTTCTCACGGGAATTTTCAGGGTA
AACTGGCCATCCGAAAATAGCAACAACCCAGACTGGCTCCTCACTCCCTTTTCCATCACT
AAAAATCACAGAGCAGTCAGAGGGACCCAGTAAGACCAAAGGAGGGGAGGACAGAGCATG
AAAACCAAAATCCATGCAAATGAAATGTAATTGGCACGACCCTCACCCCCAAATCTTACA
TCTCAATTCCCATCCTAAAAAGCACTCATACTTTATGCATCCCCGCAGCTACACACACAC
AACACACAGCACACGCATGAACACAGCACACACACGAGCACAGCACACACACAAACGCAC
AGCACACACAGCACACAGATGAGCACACAGCACACACACAAACGCACAGCACACACACGC
ACACACATGCACACACAGCACACAAACGCACGGCACACACACGCACACACATGCACACAC
AGCACACACACAAACGCACAGCACACACAAACGCACAGCACACACGCACACACAGCACAC
ACACGAGCACACAGCACACAAACGCACAGCACACGCACACACATGCACACACAGCACACA
CACTAGCACACAGCACACACACAAAGACACAGCACACACATGCACACACAGCACACACAC
GCGAACACAGCACACACGAACACAGCACACACAGCACACACACAAACACAGCACACACAT
GCACACAGCACACGCACACACAGCACACACATGAACACAGCACACAGCACACACATGCAC
ACACAGCACACACGCATGCACAGCACACATGAACACAGCACACACACAAACACACAGCAC
ACACATGCACACACAGCACACACACTCATGCGCAGCACATACATGAACACAGCTCACAGC
ACACAAACACGCAGCACACACGTTGCACACGCAAGCACCCACCTGCACACACACATGCGC
ACACACACGCACACCCCCACAAAATTGGATGAAAACAATAAGCATATCTAAGCAACTACG
ATATCTGTATGGATCAGGCCAAAGTCCCGCTAAGATTCTCCAATGTTTTCATGGTCTGAG
CCCCGCTCCTGTTCCCATCTCCACTGCCCCTCGGCCCTGTCTGTGCCCTGCCTCTCAGAG
GAGGGGGCTCAGATGGTGCGGCCTGAGTGTGCGGCCGGCGGCATTTGGGATACACCCGTA
GGGTGGGCGGGGTGTGTCCCAGGCCTAATTCCATCTTTCCACCATGACAGAGATGCCCTT
GTGAGGCTGGCCTCCTTGGCGCCTGTCCCCACGGCCCCCGCAGCGTGAGCCACGATGCTC
CCCATACCCCACCCATTCCCGATACACCTTACTTACTGTGTGTTGGCCCAGCCAGAGTGA
GGAAGGAGTTTGGCCACATTGGAGATGGCGGTAGCTGAGCAGACATGCCCCCACGAGTAG
CCTGACTCCCTGGTGTGCTCCTGGAAGGAAGATCTTGGGGACCCCCCCACCGGAGCACAC
CTAGGGATCATCTTTGCCCGTCTCCTGGGGACCCCCCAAGAAATGTGGAGTCCTCGGGGG
CCGTGCACTGATGCGGGGAGTGTGGGAAGTCTGGCGGTTGGAGGGGTGGGTGGGGGGCAG
TGGGGGCTGGGCGGGGGGAGTTCTGGGGTAGGAAGTGGTCCCGGGAGATTTTGGATGGAA
AAGTCAGGAGGATTGACAGCAGACTTGCAGAATTACATAGAGAAATTAGGAACCCCCAAA

TTTCATGTCAATTGATCTATTCCCCCTCTTTGTTTCTTGGGCATTTTTCCTTTTTTTTT
TTTTTTTGTTTTTTTTTTACCCCTCCTTAGCTTTATGCGCTCAGAAACCAAATTAAACCC
CCCCCCCATGTAACAGGGGGGCAGTGACAAAAGCAAGAACGCACGAAGCCAGCCTGGAGA
CCACCACGTCCTGCCCCCCGCCATTTATCGCCCTGATTGGATTTTGTTTTTCATCTGTCC
CTGTTGCTTGGGTTGAGTTGAGGGTGGAGCCTCCTGGGGGGCACTGGCCACTGAGCCCCC
TTGGAGAAGTCAGAGGGGAGTGGAGAAGGCCACTGTCCGGCCTGGCTTCTGGGGACAGTG
GCTGGTCCCCAGAAGTCCTGAGGGCGGAGGGGGGGTTGGGCAGGGTCTCCTCAGGTGTC
AGGAGGGTGCTCGGAGGCCACAGGAGGGGGCTCCTGGCTGGCCTGAGGCTGGCCGGAGGG
GAAGGGGCTAGCAGGTGTGTAAACAGAGGGTTCCATCAGGCTGGGGCAGGGTGGCCGCCT
TCCGCACACTTGAGGAACCCTCCCCTCTCCCTCGGTGACATCTTGCCCGCCCCTCAGCAC
CCTGCCTTGTCTCCAGGAGGTCCGAAGCTCTGTGGGACCTCTTGGGGGCAAGGTGGGGTG
AGGCCGGGGAGTAGGGAGGTCAGGCGGGTCTGAGCCCACAGAGCAGGAGAGCTGCCAGGT
CTGCCCATCGACCAGGTTGCTTGGGCCCCGGAGCCCACGGGTCTGGTGATGCCATAGCAG
CCACCACCGCGGCGCCTAGGGCTGCGGCAGGGACTCGGCCTCTGGGAGGTTTACCTCGCC
CCCACTTGTGCCCCCAGCTCAGCCCCCCTGCACGCAGCCCGACTAGCAGTCTAGAGGCCT
GAGGCTTCTGGGTCCTGGTGACGGGGCTGGCATGACCCCGGGGGTCGTCCATGCCAGTCC
GCCTCAGTCGCAGAGGGTCCCTCGGCAAGCGCCCTGTGAGTGGGCCATTCGGAACATTGG
ACAGAAGCCCAAAGAGCCAAATTGTCACAATTGTGGAACCCACATTGGCCTGAGATCCAA
AACGCTTCGAGGCACCCCAAATTACCTGCCCATTCGTCAGGACACCCACCCACCCAGTGT
TATATTCTGCCTCGCCGGAGTGGGTGTTCCCGGGGGCACTTGCCGACCAGCCCCTTGCGT
CCCCAGGTTTGCAGCTCTCCCCTGGCCACTAACCATCCTGGCCCGGGCTGCCTGTCTGA
CCTCCGTGCCTAGTCGTGGCTCTCCATCTTGTCTCCTCCCCGTGTCCCCAATGTCTTCAG
TGGGGGGCCCCCTCTTGGGTCCCCTCCTCTGCCATCACCTGAAGACCCCCACGCCAAACA
CTGAATGTCACCTGTGCCTGCCGCCTCGGTCCACCTTGCGGCCCGTGTTTGACTCAACTC
AACTCCTTTAACGCTAATATTTCCGGCAAAATCCCATGCTTGGGTTTTGTCTTTAACCTT
GTAACGCTTGCAATCCCAATAAAGCATTAAAAGTCATGA

SEQ ID NO:19
ENSG00000167244

TCTTTGCCTCTTCCTGAGTCTTTGCTAGCTTGGGGAAGAGGTTAGAAATTTCCAGTG
TGATTTAGTCCAATCTGGTGGATGCTTCTATATGAACCCTCACCCAGCTGCTGTCAG
CTTTGAGTCTGTGCTTTAATTAGAAAGGCTGCGAACCTGTCCAATCGCCCAATCCAG
AAATGCCTGCAAGTGCCCCCTGCAGCAGTGTCATTAGAGCTTAGTACCACACAGGGA
GTGTCTGAGGTCAGATCAGACGTGACCAGTGAAGTGAAAAACCCCCCAGCTGAGTAC
CTTGCTCATGGAAGATGGAAGGACATAACTGCACAGTACATAACTGTACCGGGAGCA
CCTAAAATCCAATTTTTCTGCTGATTCTTGAACCCTGCTTTGTCCCCCTTATTCCCC
CCCCCCCCGCCATTTTACCAGTGCCACGTCCACCAACATTCCAGGGTGTCAAGTAAC
TGCCAAGTGTCACTCTAAGTAAAGCTACACCCACTCCCCACCACCTCCACATAGCCC
CCACCTCCTAGCTGGCAGGGAGCTTCTGGCTTATGCCCACGCCCACAGGCGCCTTTC
TGCCAGGTCAGGGGTGGGCCAAACCTCCACCCGCTAATGTACCATGCCCTGGTGCTG
GAAAGTGCCTGAGCCAGCTGCCCCAGCGGCCTCAGCACTACCAAGTTGGCACAAAGC
TCCCCAAATTCGGAGGGGCTCAGGGAAACGAGTGGAGGGGATGAGGAGGTGAGGGGT
AAACCCATCATTTCAGTTGGCATTTGAGCAGGTGCCATGCTCAGCGGAGATGAGGCT
CTCCCATCTGTAGGGGCCGTATTAACATGCACACTCTAAAAG
TGCCCTTCGTTTCTCCAGCCTCAGCTTTGTCCCTCTCCTCCTCCACGTCAACCTGGCCAG
AGGGTCTGGACGCCACAGCCAGGGCACCCCCTGCTTTGGTGGTGACTGCTAATATTGGCC
AGGCCGGCGGATCATCGTCCAGGCAGTTTCGGCAGAGAGCCTTGGGCACCAGTGACTCCC
CGGTCCTCTTTATCCACTGTCCAGGAGCTGCGGGGACTGCGCAGGGACTAGAGTACAGGT
AACTGGGCTCCCATCCCTTGAGTGTGAGAGAAAAGCTGCACTTTAAGTGTTCAGGCGTGG
GTGGGCCGGGAGCTTGGGGCAGCTGCCAGGATCCTGGTTTCTGAAGGAGGGGAAGAACTT
CTGCTGCTGGAGGGTGCAGGGAAGCCTCCTGAGAGCAGCCTCAACTTCAGGGATGGGGTG
TGCAGGAAAGGCCATTGTGGAGAGGGTTCTCCTTTAGGGCTGCACAAAGCCACTGAGGCT
TTTGCAAGGAAAATAGGTTTTCCTTGACTAATTCACCAAGCAAAATGGGAGGGGTAGGGG
AGGAGGGCTAGGCCGCTCTTCCCAGCGGGAACACACAGCTGTCTTCACAAGTGTGAAAGG
AAGAGTCTTTCTGTGTGAAAAGTTTCCTCCCGTTGCATCCCCCATCCCATTCCCAGAGAC
AAACAGGAGACTTTGCAGAGGAGCCAGGGGCCCGAGATTCTGGCGCAGAGATTTTATTTA
TACATATATACACCATTTTACAGGTAAAGCTTCCTTCCCTCCTGCCTCCCTATGCCTCCT

GACCACCAGCAAGAAATTGGACAGGAGACTGAGGAGAAATGCCGGGAGAGGCAACAACCG
CCCTCCATGTCCCCCCTAGGTTTAGCTTCTCTCCTCCTGATGGCGCACCTGGTCCCCCTT
GCTGCTCTCCCAGCCTCCCTGGCACAGAGAGGCGCCCTGGGGCCAAGGCAGTTTCCCTGG
GAATGCTCATTCATGCATGAAGTTTTTCTCTGTTGCACCCTGGACCCAGACTCCTCGATC
CACCCAGGGTGGTGTCTGTGGGGAGGGGGTTCATTTCCCCAGGAAGCACAGCCACGCCGT
CCCTCACTGGCCTCGTCAAGCAGAGCTGTGTGTCCAGTGGCTTTTGCTGGGGCCCCCTCC
TTATCTCCTTCCAAGGTGGGGGTGTTTGGAGGTGGAGGAGGCTTTCATATTCCGTGCCAT
GACCCCTCAAGGCGGGCCATTCGTGTGCACCCTCCACCCCCAGTGCCAGGCAGAAGCCCA
TCCTCACCCAGGAACAGGGCAGCCTGTCCAACAGAAGGGTCTCGGCCTCTCCATCAGCAC
CGGGAAGCCCTTTCTAGGCAAACTTCTCACCACTTCTTCCCTCCCTTATACTTTGAAAGA
GGGAGCTCTAGGCAGGGGAGGGGCTAGAGGGGGAAGCCGCTGCCCAGATCCTGACAAGGT
GACCTGAAGGAACCCGGGGAGGGGGATGGGACAGGGCTCAGGCTTGGGGTGTATGGGGAG
GGGGGCTTTGCTTTTAAAAGAGGTCATCTCAGCAATATCTTTTTGTTTTTCCCCAGGGGC
CGAAGAGTCACCACCGAGCTTGTGTGGGAGGAGGTGGATTCCAGCCCCCAGCCCCAGGGC
TCTGAATCGCTGCCAGCTCAGCCCCCTGCCCAGCCTGCCCCACAGCCTGAGCCCCAGCAG
GCCAGAGAGCCCAGTCCTGAGGTGAGCTGCTGTGGCCTGTGGCCCAGGCGACCCCAGCGC
TCCCAGAACTGAGGCTGGCAGCCAGCCCCAGCCTCAGCCCCAACTGCGAGGCAGAGAGGT
GAGTGTCTCAGGCACCCTGAGGCCTGGCAGAGAGGGCCACAGGCTCTGCGCGGGAGTCTT
CGAACTGGGATCTCCCCCTTCTGCAAGCAGCTTTGGCTCAGAGAGGCTGGCGTGGATTCA
GTCACACAGCTGGGATCTGGAGTTCCGTGGTTGGCTCCAGGTGCTTCCGTCTAGGGGCCA
GAGCAGGTGTGGGCAGAGCAGGTTCCCCGCAGTCTCCACGGCACCGAGGTCCTGGCAGGG
GAGCTCCTGGGAGACGAAAGAGGGCAAAGAAGGGGAGAGGGGCAGGGAGAGAGCGGGCAG
CCAAAGGGGAGAAGATGGGGGGCAGAAAGTGGGTAGAGAGGGAAAAAGGAAAATATCAT
TGGGGAAGAACCTAAAAACCCAAGGAAAGCTGGGCTCTGCTGGGGGCTGTGAGACCCCCG
GGTTCTCCCCGCCCCAGGCTGCTGGCCATGGGGTCTTGCACCAATGGCCTGACCTTTCTG
TCGGTCTGTATTTATCAAAGTGGGTGACAGTCTCAGGCCTCCTGGCTGTTCAGAATTGAG
GTAATAACCAGAGGCCTTCTGAGCAAAGGGCCTAAGGGCTCCGGCGTCAGGATCCCATT
GTGGTCAGGAGCCTGCGGGCTTCCCGTGTGCAAGAGGGGTGAAAGGTGGCTAGAAAGGC
CCAGCCAGTGGCCTCTGCCTCAGCCAGAGGGAGCTCTGTAGTGGGGGCAGCACCCATTCA
CTGGTCAGGCACTGGGGTGACAGGGGAGGCTCCAGGACTTGGGGAGCGTTGGAGCTGGAG
GCACATGGATTGGAGTCCCTGTACCTGCCCCACGACAGGGCCTGCAGGGAGGGATCCAGC
AGGTGACTCTTCAGGCTGATTTGCCCATCCCAGATAGAAGCCGGGAGTGTTCTTTCAAAG
GTGTCTTTACCTTAGACACTCAATAAAATGGTAACACAGTGGCGCCGCCTCAGTCCTTTG
GAGTGTGCACCGTCTGAACCCCTCTCCCAGGGCCCTCTCCCAAGCACCCCAACCTGGACC
CATATCCCCCACGTACTTTTGGCTTTGGCAGATTGAGCAGCCTTGGGGTGGTCTGTGCT
GTCTGGTGTGGAGGGTTGCAGTTCGGGTCCTTAGTCCTACTTCCCAGGCCGGCCGGGCTG
ACGCCAGCGAGTGTGTCCTTCCCCAGCGAGGGGAGTGAGCGCAAGGTCAGCGCCTCGTCT
GCGGCGCCCTGCAGGGGGTGACGGAGGGGCGCTCTGAGGACCCTTGGAGAAAGGAGCTGG
GTTTGTAAAATGCTGGGCTTGGTCCCACGGACGGCGGAGCGGTGAGCTCAGAGCCAGAGC
TGGGGAGGAAATGGAATGAGAAAGGCCCACTTCAGGGCTGGTGAGCGAGGGGATGGGGA
GCAGCCACAGGCCGAGGCTGGGGCATGGGCCAGGCTCCATGGGGTGAGTCTGAGTCCTTG
AGGGGATGTTCATCCTCTGTGGAATGTGGGTTTGCCAGTGGAGAGGAGACCAGCGTTGCC
CTGGTGAGGTGCTGGTTCAGGGCTGGGGGGCGGACGCTGCTTGGGGCTAAAGTTCCTGCC
GGCCAAGCTCTGGGTGGAGGAGACCCTGGCCCCCTCCCAACACCCTTGGACTGCTGGCG
GGACCCTTCCTACCTCCGGGGGCTGGAAGTAGTGGGGAGGAGCCAGTCTTGAGGAAGAA
CCCCGATGCTGGTCTTGACTAGAGGGGAGCCGGTGTGCTTTTCGAGCCTCAGGGTGACCC
GCGTCTGCCCCAGCCTCCAGCCTGCCCTGGTCACTTCTGACTAAATAAGGAGAGCACTCA
GCAGGCAGCCCCACGAGGGAGGGGGAACATGTGTGCACCCCCACTCCCCCACCTGCTCCT
CCCTCCCTACAGGGCCACTACACCCTGCTGTGGGCACCCCAAGGTGACCCTCAGCCTTCT
TCCTACCTTAAAAAGTCCAGGCATGCATTTTCAAGCATGAGCGGTGGCCCCCTGGGGGAA
GGCACCTCGGCAGGGCAGAACAAAGGGAAGGGACCCCCAAACAGGTCACTGGTGTAATTG
TCCCCAGCACCCCCAAAGAGGAGGAGAACCCACAACTCGGAACTGGGGCTCACCCCCGAT
GCCCAACCTGTCCCCAGCCTGGGAAGCAGGCGTGGAGGAGAAGGTGGGGGGAGCCTAGAG
CTGGCCCTGGGGGCCCTGGTTTTGTCCATGACGGAGCCTCGGCAACCTAGTCCGCTCTC
CCGGGGACCAGGTTTGCAGACAGGCACCTTTCAAATGCTCCTCACCCCCAAATTTACAAG
TCACCCTGCAGAGGAAAACATCAACACAGCCAGGGGTTCTCTGCTGGAGGCTCCCCCTTC
TATAGGCACAGCCGGAGAGGCCAGAGAGCTGGGGACACGGGGAGGCTGCAGAAGGCTGGT
GGGAAGGGGGGCAGTGATGGGTGGGGAGAGATGGCCAGATGTTCTTGGAATGGACATG
GGGGTGATTGATGCAGACAGAAATTTGAAGGGACATTCCCACGTGTCTTGTTCTGTGGG
TGGAAAATGGGCTGTTTTTCATGGTGGGGCGGGTTCTCCCTGTCTTGCCAAGCTAATGT
GAAAGAGATGCCTCATCCTGCCCAGCTCCCCACACCTGTCCAAGGCCATTAACTTCTGCC

TCCCCAGTGTCAGGCTTTGAGATGCCCCCCTTCTAGCCGGGGTCCTCCTATGGGGTGACA
ATGGGGACAAGCAATGCCCACTGTAGTTGCCCCAGGATCCCCCACCATTCTGCTGGTCCC
CAGCGGTGCCCCCTCTCTGGCAGTACCCCCACCCACCCCACAGGTCCCCTTAGGGCCACT
GCCCCATCGCCCGACATTGCCCAACGCCAAGGGGTGACCTTGTTCCTGCCGACAGGGCCG
TTGGGCGCCTGCATGCGGTTTAATATTTGCCTATAAGGAACTGGGCTTTCCCCAGCCGG
AGTGGACAGACTTTCCCTGAAAATTCGCTTGGAGAGAACGAAAAGAGACCCCTGGCACCC
CAGCGGCGTGCAGCCCTGCACCCCCCTCCTCCCGGGCCCCGTGTTTCTCATTTTCCTCCC
CACTTCCTCTGCTCTTCAGTGTTACCCAAACAAAACTGGTTTCACCCTTGTTTGGTGCTG
GCGAAGGCCCGAACGGCGCGCGCAAAGCTCCGGGGCAGGCCGGAGGTGGCCACCGGGGGT
GCTCCGGGCCCCCAAGCCAAGCCGGGGACTAGCCTGCCCCCGGTGGCGGCTCGGCCGCGG
CTTCGCCTAGGCTCGCAGCGCGGAGGCGAGTGGGGCGCAGTGGCGAGGGGGAGCCTGCGG
ACCTCCCACGCGGGGACCGAGCAGGTATCTGGGAGTCCCGGGAGCGCCCGGGAAGCAGCG
TCCTGGTCGCTCCCTCGCGGCCCTTGGGTTTCTTCCTTACACCCGGACGCCCGCTAAGCT
CGGGCTGCCGCCACAAACGCGCTCTCCGTGTGGAGAAGGCAAAGAAAAAAAAAAATAAAA
GCAAAAGGAAGAAAAACCCCAAAGAACGAAAAGCAGAATTTCAGCCGGCCGTGCGCGCCA
GGGCGCTCCGCGCTACCTGCCCGCGCCGCCCGCGCTCGGGTTCCCGGGGAGGGCGCCAGT
GCTCCGCGCGCGCCCCAGCCAAGGTGAATCCCCGGCAGCGCCTTCCTTCCGCTGCCCGGG
AAGCTTGAGCTCAACAATTAGCCCTTGATCCTCGGGGGATTCCAATCCACGGAACAACTT
CCCTGCTTTCCCCGAACTCGGACATTTTACTTTTTCTGGGATCCTCTAAATTTAAGCATT
GCTTCCCAAGTCTTCTAAATATACTCACCATTTCGACGGGTCACAATAATTTTCTTGGAC
GTTAATTTCCGGGGACGTCAAAACACATCAGTCCCGGCGGGCTTTTCCAGACTTACACTA
TGTGGCCTGGGGCCCCAGATGTGCTTTCTCCCAGGCTCTGGACAGTTTTATACACCCCCT
CCAGGTCCCACAGATTTACAGGCCACTAACCCGGCTTCCCTAATTTTAAAGACGAAGCCC
TTGGTCCGTGGTGGTCGCGCTGACCAATTTGCCTGGCTCCCCAGGATGTGGACAGTGCCT
TTTCCACATTTAGGCATTGTTTCCAAAACAAGTGGAACTTTCCCGCATAATTTTGAATAT
TAACTCCAGGGTCTCCTAAGCTTACTGTTTCCGGCACACGTCCGCCCCATTCCGCGCCCC
CCCACCCCACCCCCGCGCCCCTTCCCGTTCACCTCAGCATGGGACATTTGCTGTTGGGTC
CCGCAAATCTATTCACACTAACCTGGGTTCCCTAAACTTTACACGTTGAATCCCAAGTCC
CTCATGACACTCAGCAGGGCTGGAAGGTTGGAACCCTCAGAGTATGAAAATTGCTTCCCA
TACCTTCCCCCAAATTCGGTCATTACACCCAGAGCATGTTGAATCCTTTTCTGAATCATA
AACGACCTGCCCTCTGATTGCCTGAGTTTCATAAAATGGAGGGATTTCCCCAGTGATTCC
CCAAAGCTATTGAGAATGTTGTGGGTGTGTGAGTCACAAAGCTTTGGGGCATTAACGTCT
ATGGCTCTATATGCTGCCTGGCCACGAATCAGGTCCCTTAAGATGTAGACAGTGCCACCC
AGGTCCCTGGAGCACACTGAGCAGTTACCAGGAGGTGCTCAAGTGTGACCCAGGATTCTC
CAGGTCCCCCCAAATCACACAGGGTCTCCCGGGTCCCTCTGGGCTACACCAAGCACAAAA
GGACCCCTTGGGCAGAGCCTACTTTTATTTCTGTTATGCCAGGTGTTGCTAACGGCCCCA
GTTCCCAAACATTCCGAGCACTTTCTCTGCATCACAACATATTGACTATTAAACAATTCT
CTGGGTCCCAGGAGCTTCATAACAAGAATCTTGCTTTTCTAAAATTCAGGCATTGGTCTG
AAACCCCAACGGCCAGGATCACACTGGACCCTTTTCCTGGTCCCCCATACTTGGATGTTC
TGGACGCTGCCCTCCAGGCCCTCTAGGAACATTCAGCATTGCCCTCGGATCACAGGACAG
CACTGATTTCTTGGGCTCCAAACAACCCACTGAGTCATCTCAAAGTTAAGCAATATTTCC
TTCAAGCACACTGCACACTCCCTATCACAAAATCTGAAAATTCCTAAGTCCTAAGACCTA
GGAATTCTGAATCCCCTTTCTTTAAAATGTACATATGGACCCCCAAGTCCTCCAAGGACT
CTGAGCAACTTCCCTAGATCTTTAGATTCAAAAACGATTTCCTGGAGCCCCCAAATTGC
GGTATTGTCTCCCAGCCTTCCAAAGCAAATTGAGATTTTTTTCCCTTCACAAAACAATTG
AGGTTTTTTTTTTTTTAATACTGATTTATGAGTCTCCTGACTTTATGGTCCCTGCCCTGG
GTCCCCCTACATTTAGAAAATGTTCCATGGACCCCCAAAGCACACTAAAAAATGTCCCTG
GGTCCCAAGAAATCCCAGGCATGGAAAAACCTGCGACCTATAAGTTTCCTAGCTACTAAC
TAGGTTTCCAGAAATTTAGATATCAAATCTCCATTGGGTAATTTCCATGTGTCCCAAAAA
CTTGAAATGTGTTTCACTGGGGCTCCCCCAAATGCAGACGACATCCAGGAAAATATATAG
TCTTTTTCTTATTTACCAAAAATAAGCTAATGGAAATCATTTAAAAATTAGCATAGAAAA
ATAATACTGATTTTTTATTTTTTTATTTTTTATTTTGCTTTCCCCAAATGTACTGATCAC
ACTCCAGGCTCCCCCAAAATCTAGACAGTGCTTTCTTCCATCTCTGAAGGGTGTTAAAAC
CTTTCCCTGAAGCCACAGTAATTATGAAGGTTATTTTTTCCCGGCTGCTGCCAGCGTCC
AGGCCACTAACTTATATTCTTAAGATGTGAAAATTAATCTCAGCTTCCCCCTAACACACC
AAGAATGTGTTTGGATCCCCAAAATGTGTTCCTTGCTTTCATCTGCCAATTTTACGTAAT
ATGGCTCTACGGCAAAATTCCCAATTTCATATGGAGAATTTTCTTTAACTACCCCTCCTC
ACAAATTGGTCCCCCAAGCTAGCTGGCCCCTATTTGAGACCTCTTTCTCTATGTTCCCAA
TTGCATGGAGCAACTTCTCTCATCCCCCAAACCTGTAATCTATTTTTCTGGAGTCTCGAG
TTTAGTCATTAATCACGGTTCCCACATTAACGGAGTCCCCGGGGTCCCCTCCTCCAGGAC
ACCCATTCGCTAAGCCCGCAAGGCAGAAAGAACTCTGCCTTGCGTTCCCCAAAATTTGGG

CATTGTTCCCGGCTCGCCGGCCACCCACTGCAGCTTCCCCAACCCCGCGCACAGCGGGCA
CTGGTTTCGGGCCTCTCTGTCTCCTACGAAGTCCCCAGAGCAACTCGGATTTGGGAAATT
TCTCTCTAGCGTTGCCCAAACACACTTGGGTCGGCCGCGCGCCCTCAGGACGTGGACAGG
GAGGGCTTCCCCGTGTCCAGGAAAGCGACCGGGCATTGCCCCCAGTCTCCCCCAAATTTG
GGCATTGTCCCCGGGTCTTCCAACGGACTGGGCGTTGCTCCCGGACACTGAGGACTGGCC
CCGGGGTCTCGCTCACCTTCAGCAGCGTCCACCGCCTGCCACAGAGCGTTCGATCGCTCG
CTGCCTGAGCTCCTGGTGCGCCCGCGGACGCAGCCTCCAGCTTCGCGGTGAGCTCCCCGC
CGCGCCGATCCCCTCCGCCTCTGCGCCCCTGACCGGCTCTCGGCCCGCATCTGCTGCTGT
CCCGCCGGTGCTGGCGCTCGTCTCCGGCTGCCGCCGGGGAGGCCGGCGTGGGCGCGGGA
CACGGCTGCGGACTTGCGGCTGGCGGCTGCGCTCGCTCCTGCTGGGCGCCCCGAAATCCG
CGCCACTTTCGTTTGCTCATTGCAAAGATCTCATTTGTGGGAAAGCGGCTGGAGGGTCC
CAAAGTGGGGCGGGCAGGGGGCTGGGGCGAGGGACGCGGAGGAGAGGCGCTCCCGCCGGG
CGGTAAAGTGCCTCTAGCCCGCGGGCCTAGGACTCCGCCGGGAGGCGCGCGCGGAGCGCG
GGCGAAGTGATTGATGGCGGAGCGAGGGGGGCGAGGGGGGCCAGGGGGGCGCGAGATTCC
GCCGGCGGCCCCTTCCCCTTGGCTAGGCTTAGGCGGCGGGGGGCTGGCGGGGTGCGGGAT
TTTGTGCGTGGTTTTTGACTTGGTAAAAATCACAGTGCTTTCTTACATCGTTCAAACTCT
CCAGGAGATGGTTTCCCCAGACCCCCAAATTATCGTGGTGGCCCCCGAGACCGAACTCGC
GTCTATGCAAGTCCAACGCACTGAGGACGGGGTAACCATTATCCAGATATTTTGGGTGGG
CCGCAAAGGCGAGCTACTTAGACGCACCCCGGTGAGCTCGGCCATGCAGGTAGGATTTGA
GCTGTGTTTCCCGCCCTGATCCTCTCTCCTCTGGCGGCCGGAGCCTCCGTAGGCTCCAAG
CCTGGCCCAGATTCGGCCCGGCGCAGCCGGCCTTCCGCGCGTCCCGCACCTGGCGGGGGC
TCCGGGGCTCCGGCGCGGCACCGGGGGGCGCTCGGGATCTGGCTGAGGCTCCAAGCGCCG
CGTGGCCGGCTCCTCCTGCTGGGGCAGGTGGCGGCTGCGCGCCCCGCCCGAGCCCAGGGG
CCCCCTCAGCCGCAACAACCAGCAAGGACCCCCCGACTCAGCCCCAAGCCACCTGCATCT
GCACTCAGACGGGGCGCACCCGCAGTGCAGCCTCCTGGTGGGGCGCTGGAGCCCGCCTG
CCCCTGCCTGCCCGGAGACCCCAGCTCACGAGCACAGGCCGCCCGGGCACCCCAGAAACC
CGGGATGGGGCCCCTGAATTCTCTAGAACGGGCATTCAGCATGGCCTTGGCGCTCTGCGG
CTCCCTGCCCCCCACCCAGCCTCGCCCCCGCGCACCCCCCAGCCCCTGCGACCGCCGCCC
CCCCCCCGGGGCCCCAGGGCCCCCAGCCCGCACCCCCCGCCCCGCTCTTGGCTCGGGTTG
CGGGGGCGGGCCGGGGGCGGGGCGAGGGCTCCGCGGGCGCCCATTGGCGCGGCGCGAGG
CCAGCGAGGCCCGCGCGGGCCCTGGCCGCGGGCTGGCGCGACTATAAGAGCCGGGCGTG
GGCGCCCGCAGTTCGCCTGCTCTCCGGCGGAGCTGCGTGAGGCCCGGCCGGCCCCGGCCC
CCCCCTTCCGGCCGCCCCCGCCTCCTGGCCCACGCCTGCCCGCGCTCTGCCCACCAGCGC
CTCCATCGGGCAAGGCGGCCCCGCGTCGACGCCGCCCGCTGCCTCGCTGCTGACTCCCGT
CCCGGGCGCCGTCCGCGGGGTCGCGCTCCGCCGGGCCTGCGGATTCCCCGCCGCCTCCTC
TTCATCTACCTCAACTCCCCCCATCCCCGCTTCGCCCGAGGAGGCGGTTCCCCCCGCAGG
CAGTCCGGCTCGCAGGCCGCCGGCGTTGTCACCCCCCCCCGCGCTCCCCCTCCAGCCCTC
CCCCCGGCGCGCAGCCTCGGGCCGCTCCCCTTTCCGCGCTGCGTCCCGGAGCGGCCCCGG
TGCCGCCACCGCCTGTCCCCCTCCCGAGGCCCGGGCTCGCGACGGCAGAGGGCTCCGTCG
GCCCAAACCGAGCTGGGCGCCCGCGGTCCGGGTGCAGCCTCCACTCCGCCCCCCAGTCAC
CGCCTCCCCCGGCCCCTCGACGTGGCGCCCTTCCCTCCGCTTCTCTGTGCTCCCCGCGCC
CCTCTTGGCGTCTGGCCCCGGCCCCCGCTCTTTCTCCCGCAACCTTCCCTTCGCTCCCTC
CCGTCCCCCCCAGCTCCTAGCCTCCGACTCCCTCCCCCCCCTCACGCCCGCCCTCTCGCCT
TCGCCGAACCAAAGTGGATTAATTACACGCTTTCTGTTTCTCTCCGTGCTGTTCTCTCCC
GCTGTGCGCCTGCCCGCCTCTCGCTGTCCTCTCTCCCCCTCGCCCTCTCTTCGGCCCCCC
CCTTTCACGTTCACTCTGTCTCTCCCACTATCTCTGCCCCCCTCTATCCTTGATACAACA
GCTGACCTCATTTCCCGATACCTTTTCCCCCCCGAAAAGTACAACATCTGGCCCGCCCCA
GCCCGAAGACAGCCCGTCCTCCCTGGACAATCAGACGAATTCTCCCCCCCCCCCCAAAAA
AAAGCCATCCCCCCGCTCTGCCCCGTCGCACATTCGGCCCCCGCGACTCGGCCAGAGCGG
CGCTGGCAGAGGAGTGTCCGGCAGGAGGGCCAACGCCCGCTGTTCGGTTTGCGACACGCA
GCAGGGAGGTGGGCGGCAGCGTCGCCGGCTTCCAGGTAAGCGGCGTGTGCGGGCCGGGCC
GGGGCCGGGGCTGGGGCGGCGCGGGCTTGCGCCGGACGCCCGGCCCTTCCTCCGCCCGCT
CCCGGCCCGGGGCCTGCGGGGCTCGGCGGGGCGGCTGAGCCCGGGGGGGAGGAGGAGGAG
GAGGAGGAGGACGGACGGCTGCGGGTCCCGTTCCCTGCGCGGAGCCCCGCGCTCACCCTG
GCGGCGGAGCTGGGGGTGGGGTGGGGGCGTCGGGAAGGGCCGAGGGAGGTGTGAGGTGTC
TGCAGGGGCGACTTCCCGGTCGGTCTGTGGGTGCAGGGGGTGCCGCCTCACATGTGTGAT
TCGTGCCTTGCGGGCCCTGGCCTCCGGGGTGCTGGGTAACGAGGAGGGGCGCGGAGCCGC
AGAAGCCCACCCTGGTATGTTGACGCGGTGCCAGCGAGACCGCGAGAGGAAGACGGGGGT
GGGCGGGGCCAGGATGGAGAGGGGCCGAGTTGGCAGGAGTCATGGCAGACGCCACATTCG
CGACATCTCCCCCACACCCCCTCTGGCTCTGTCCGCAACATTTCCAAACAGGAGTCCCGG
GAGAGGGGGAGAGGGGCTGCTGGTCTGAGGCTAAGAAGGGCAGAGCCTTCGACCCGGAGA

GAGGCCGCGGCCCCTGCCCAGTGGGCAGCGTGGAAGTTTCCATACAAGGAGGTGGGAAGG
AGACCCCCCCCCCCTTCACTGCCCTGTGCAGAGATGAGCCGGGGGTGCAGGATGGGAGCC
CATGGCACTTCGCTACGGATGGTCCAGGGCTCCCGGTTGGGGGTGCAGGAGAGAAGAGA
CTGGCTGGGAGGAGGGAGAGGGCGGGAGCAAAGGCGCGGGGGAGTGGTCAGCAGGGAGAG
GGGTGGGGGGTAGGGTGGAGCCCGGGCTGGGAGGAGTCGGCTCACACATAAAAGCTGAGG
CACTGACCAGCCTGCAAACTGGACATTAGCTTCTCCTGTGAAAGAGACTTCCAGCTTCCT
CCTCCTCCTCTTCCTCCTCCTCCTCCTGCCCCAGCGAGCCTTCTGCTGAGCTGTAGGTAA
CCAGGGCTGTGGAGTGAGGACCCCCGCTGCCATCCCACTCCAGCCTGAGGCAGGGCAGCA
GGGGGCACGGCCCACGCCTGGGCCTCGGGCCCTGCAGCCGCCAGCCCGCTGCCTCTCGGA
CAGCACCCCCCTCCCCTCTTTTCCTCTGCCCCTGCCCCCACCTGGTCTCTGCTCCCTCAC
CTGCTCCTTCCCTTTCTGTTCCTTCCCTTCGGCCCCCTCCTTGCCCAGCTCAGGACTTTT
CCTGGGCCCTCACCTGCTCCGCACCGCTGCATGCTTCCTGTCCTGCTTTCTGCCGGTCCC
CTGACCCGGACCTCCAAGTTCAGAGTGGTGGGGCTTGTTGCGGAAGCGCGGCGAGGGCTA
GAGTGGCCAGCTGGCGGAGTGTGCTCTTAGAATTTGGAAGGGGGTGGCAGAGGGGGCGGT
GAGAGGACTGGCCAGGGTCCAGTCAAGGAGATGACCAAGGAGGCTTTCAGATCCTCGGCG
CAGCTGCCCACTAGTCTTTAGAGAGGGCATGCAAAGTTGTGCTTCTGTCCCACTGCCTGC
TCAGTCGCTCACATAATTTATTGCATCAAAAACTCCCCTGGGTCTGCGGAGCGAAGGCTG
GGGCTGCCCGCCTGGAGGGTTCCACCTTCTGCAGGGGCAGGGCCAACTTGCTGTGGTGGC
TCCCGGCCTCCCACCCCCGAGTGGGTTAACCCGGCCCTGTGGCCCTGCAGCCTGTGGAGG
GGGTGTGTCCTAAGACTGGCCTCCCCTTCCAGATTGTAGTCTGGGAACCTGGTGTCGGA
CTTCCCAGGTGGCCTGAGCTGGTCTCTCCAGCTCCCACGGGGAGAGTTTGGTAGCGCAAA
TAGGGAGATGTTCTGGAGGCCCCTGGCCTTACTGGTTCGATTTGAGGCCTGGAAAGGAGG
CTCTGGGCGTGTGTGTGTGTGTTTGGGGGTACCCAAGGCAGACTGGAGTTGGAGAACTGG
GTGACTGGGAAAACAAGGTTTCTAGAGCATGGGTGGCGTGGTTGTGTTAACCATTGGAGT
CCTTGACCCAGGCCTGGCTCAGCTGCAGACTGGAAAGGTGGAAAAGCCAGGGGGAGGGGC
GGGGCTGGCCCAGCAGGACTGGCCTGCTGCTTTGAGGGCGATGGTCCTCCTGGACCCCCC
CTGCTCAGCTGGGGGTTGTGGGGAGGAAGGGACTGGTCCTCCTGGATGCACATGCTCTGT
AGGGGTGGGGCTGTCTGCCATCTTGGCTGGCGCTGGAGGCCTGAGAAGTGGCGATGTGAC
GCTGGGCTGGCCCTGCCCCCATGGTGTCATAGGACGGAGGCCAGGTCGGGTGTCCAGCCT
GGGCCCCTGCAGCTGTGGATGCCGCTGAGCTCCTGCAATAATGACCGTGGAGATGGTCAC
CCCTCGTGTAAAATTACTAGTGCTTCTTGCAAATGGAAGGAACTGGGCCTTTTCTGTGTG
CTTCTGGACGCTTCATTCTGCACATGGCCCTGCGCCCTCACCTCGGCATTATGACCTGTG
TGTTACTTTTGTAATAAAAATAATGTTTATAGGAAAGCCGTGCTTTCAATTTTCAACTGA
ATTTGTAGGTTGGCAAATTTGGTTTGGAGGGGCACCTCTGGCCTGGGGCTTGGCCTGGC
TGCCCCGCTCACGCCACTTCTCTCCCGCCCCCAGACACCAATGGGAATCCCAATGGGGAA
GTCGATGCTGGTGCTTCTCACCTTCTTGGCCTTCGCCTCGTGCTGCATTGCTGCTTACCG
CCCCAGTGAGACCCTGTGCGGCGGGGAGCTGGTGGACACCCTCCAGTTCGTCTGTGGGGA
CCGCGGCTTCTACTTCAGTAAGTAGCTGGGAGGGGCTTCCTCAGACCTGGTCAGGCCCCT
AGAGTGACCGGTGAGGACGCCCAACCTCAAGCCAGGGGAGCACACTCCTAGGTCAGCAGC
CCAGCCGCTTGCTCTGAGACTTTGACCTTCCCGCCGCGTTTCTGAGCACGTGCGGTGTCC
CAGGGCATCCACACCAGCTGCCTTTCCCATCACACGCCTCCTTCGAAGGGTGGGCCAGAG
GTGCCCCCTAGACGTCAGGGGCACTCACAGGGGTCTCCCTGGGCATCAGAATTTCTGTTG
GGGGCCGTGAGGCTCCTGCTCCTGAGGCACCGCACGCCTAGTGCAGGGCTTCAGGCTCTG
GAGGAAGAGCCTGCCTTTCTTCCTGCACCTTTTGGACATTCTGACAAGGGACGTGCGTTC
GGTGAATGATCAGAATTAAAATCAATAAAGTGATTTATATAATTAAAATCAATAAGACAA
GTGCAGTTGGTGGGTGGCAGGGGTGAGCGGTGCATGCGCCTCCTTGGCCCCAAGGCTGC
CGTGGGGGGTGCCCACCTGCTGACCTCAAGGACGCTTCAGCCTTTCCTCATGTTTCTCTC
TTGGTTCTCCAGCCTGGGGGCTGGCAGGTGGGTGCATGGCCCATTGTCCTTGAGACCCCA
CCCCCAGATAGGGGGGCTGGGTGGATGCAGAGGCAGGCATGGTGCCTGGGCATGCCTGAT
GGGGCAGGGGAGGGGCCGCTCCTTACTGGCAGAGGCCGCACGGCTTATTCCACCTGACAC
TCACCACGTGACATCTTTACCACCACTGCTTACTCACGCTGTGAAATGGGCTCACAGGAT
GCAAATGCACTTCAAAGCTTCTCTCTGAAAAGTTCCTGCTGCTTGACTCTGGAAGCCCCT
GCCCGCCCTGGCCTCTCCTGTGCCCTCTCTCTTGCCTGCCCCATTTGGGGGTAGGAAGTG
GCACTGCAGGGCCTGGTGCCAGCCAGTCCTTGCCCAGGGAGAAGCTTCCCTGCACCAGGC
TTTCCTGAGAGGAGGGGAGGGCCAAGCCCCCACTTGGGGGACCCCCGTGATGGGGCTCCT
GCTCCCTCCTCCGGCTGATGGCACCTGCCCTTTGGCACCCCAAGGTGGAGCCCCCAGCGA
CCTTCCCCTTCCAGCTGAGCATTGCTGTGGGGGAGAGGGGGAAGACGGGAGGAAAGAAGG
GAGTGGTTCCATCACGCCTCCTCACTCCTCTCCTCCCGTCTTCTCCTCTCCTGCCCTTGT
CTCCCTGTCTCAGCAGCTCCAGGGGTGGTGTGGGCCCCTCCAGCCTCCTAGGTGGTGCCA
GGCCAGAGTCCAAGCTCAGGGACAGCAGTCCCTCCTGTGGGGGCCCCTGAACTGGGCTCA
CATCCCACACATTTTCCAAACCACTCCCATTGTGAGCCTTTGGTCCTGGTGGTGTCCCTC

TGGTTGTGGACCAAGAGCTTGTGCCCATTTTTCATCTGAGGAAGGAGGCAGCAGAGGCC
ACGGGCTGGTCTGGGTCCCACTCACCTCCCCTCTCACCTCTCTTCTTCCTGGGACGCCTC
TGCCTGCCAGCTCTCACTTCCCTCCCCTGACCCGCAGGGTGGCTGCGTCCTTCCAGGGCC
TGGCCTGAGGGCAGGGGTGGTTTGCTCCCCCTTCAGCCTCCGGGGGCTGGGGTCAGTGCG
GTGCTAACACGGCTCTCTCTGTGCTGTGGGACTTCCAGGCAGGCCCGCAAGCCGTGTGAG
CCGTCGCAGCCGTGGCATCGTTGAGGAGTGCTGTTTCCGCAGCTGTGACCTGGCCCTCCT
GGAGACGTACTGTGCTACCCCCGCCAAGTCCGAGAGGGACGTGTCGACCCCTCCGACCGT
GCTTCCGGTGAGGGTCCTGGGCCCCTTTCCCACTCTCTAGAGACAGAGAAATAGGGCTTC
GGGCGCCCAGCGTTTCCTGTGGCCTCTGGGACCTCTTGGCCAGGGACAAGGACCCGTGAC
TTCCTTGCTTGCTGTGTGGCCCGGGAGCAGCTCAGACGCTGGCTCCTTCTGTCCCTCTGC
CCGTGGACATTAGCTCAAGTCACTGATCAGTCACAGGGGTGGCCTGTCAGGTCAGGCGGG
CGGCTCAGGCGGAAGAGCGTGGAGAGCAGGCACCTGCTGACCAGCCCCTTCCCCTCCCAG
GACAACTTCCCCAGATACCCCGTGGGCAAGTTCTTCCAATATGACACCTGGAAGCAGTCC
ACCCAGCGCCTGCGCAGGGGCCTGCCTGCCCTCCTGCGTGCCCGCCGGGGTCACGTGCTC
GCCAAGGAGCTCGAGGCGTTCAGGGAGGCCAAACGTCACCGTCCCCTGATTGCTCTACCC
ACCCAAGACCCCGCCCACGGGGGCGCCCCCCCAGAGATGGCCAGCAATCGGAAGTGAGCA
AAACTGCCGCAAGTCTGCAGCCCGGCGCCACCATCCTGCAGCCTCCTCCTGACCACGGAC
GTTTCCATCAGGTTCCATCCCGAAAATCTCTCGGTTCCACGTCCCCCTGGGGCTTCTCCT
GACCCAGTCCCCGTGCCCCGCCTCCCCGAAACAGGCTACTCTCCTCGGCCCCCTCCATCG
GGCTGAGGAAGCACAGCAGCATCTTCAAACATGTACAAAATCGATTGGCTTTAAACACCC
TTCACATACCCTCCCCCCAAATTATCCCCAATTATCCCCACACATAAAAAATCAAAACAT
TAAACTAACCCCCTTCCCCCCCCCCACAACAACCCTCTTAAAACTAATTGGCTTTTTAG
AAACACCCCACAAAAGCTCAGAAATTGGCTTTAAAAAAACAACCACCAAAAAAAATCAA
TTGGCTAAAAAAAAAAAGTATTAAAAACGAATTGGCTGAGAAACAATTGGCAAAATAAAG
GAATTTGGCACTCCCCACCCCCCTCTTTCTCTTCTCCCTTGGACTTTGAGTCAAATTGGC
CTGGACTTGAGTCCCTGAACCAGCAAAGAGAAAAGAAGGACCCCAGAAATCACAGGTGGG
CACGTCGCTGCTACCGCCATCTCCCTTCTCACGGGAATTTTCAGGGTAAACTGGCCATCC
GAAAATAGCAACAACCCAGACTGGCTCCTCACTCCCTTTTCCATCACTAAAAATCACAGA
GCAGTCAGAGGGACCCAGTAAGACCAAAGGAGGGGAGGACAGAGCATGAAAACCAAAATC
CATGCAAATGAAATGTAATTGGCACGACCCTCACCCCCAAATCTTACATCTCAATTCCCA
TCCTAAAAAGCACTCATACTTTATGCATCCCCGCAGCTACACACACACAACACACAGCAC
ACGCATGAACACAGCACACACACGAGCACAGCACACACACAAACGCACAGCACACACAGC
ACACAGATGAGCACACAGCACACACACAAACGCACAGCACACACACGCACACACATGCAC
ACACAGCACACAAACGCACGGCACACACACGCACACACATGCACACACAGCACACACACA
AACGCACAGCACACACAAACGCACAGCACACACGCACACACAGCACACACACGAGCACAC
AGCACACAAACGCACAGCACACGCACACACATGCACACACAGCACACACACTAGCACACA
GCACACACACAAAGACACAGCACACACATGCACACACAGCACACACACGCGAACACAGCA
CACACGAACACAGCACACACAGCACACACACAAACACAGCACACACATGCACACAGCACA
CGCACACACAGCACACACATGAACACAGCACACAGCACACACATGCACACACAGCACACA
CGCATGCACAGCACACATGAACACAGCACACACACAAACACACAGCACACACATGCACAC
ACAGCACACACACTCATGCGCAGCACATACATGAACACAGCTCACAGCACACAAACACGC
AGCACACACGTTGCACACGCAAGCACCCACCTGCACACACACATGCGCACACACACGCAC
ACCCCCACAAAATTGGATGAAAACAATAAGCATATCTAAGCAACTACGATATCTGTATGG
ATCAGGCCAAAGTCCCGCTAAGATTCTCCAATGTTTTCATGGTCTGAGCCCCGCTCCTGT
TCCCATCTCCACTGCCCCTCGGCCCTGTCTGTGCCCTGCCTCTCAGAGGAGGGGGCTCAG
ATGGTGCGGCCTGAGTGTGCGGCCGGCGGCATTTGGGATACACCCGTAGGGTGGGCGGGG
TGTGTCCCAGGCCTAATTCCATCTTTCCACCATGACAGAGATGCCCTTGTGAGGCTGGCC
TCCTTGGCGCCTGTCCCCACGGCCCCCGCAGCGTGAGCCACGATGCTCCCCATACCCCAC
CCATTCCCGATACACCTTACTTACTGTGTGTTGGCCCAGCCAGAGTGAGGAAGGAGTTTG
GCCACATTGGAGATGGCGGTAGCTGAGCAGACATGCCCCCACGAGTAGCCTGACTCCCTG
GTGTGCTCCTGGAAGGAAGATCTTGGGGACCCCCCCACCGGAGCACACCTAGGGATCATC
TTTGCCCGTCTCCTGGGGACCCCCCAAGAAATGTGGAGTCCTCGGGGGCCGTGCACTGAT
GCGGGGAGTGTGGGAAGTCTGGCGGTTGGAGGGGTGGGTGGGGGGCAGTGGGGGCTGGGC
GGGGGGAGTTCTGGGGTAGGAAGTGGTCCCGGGAGATTTTGGATGGAAAAGTCAGGAGGA
TTGACAGCAGACTTGCAGAATTACATAGAGAAATTAGGAACCCCCAAATTTCATGTCAAT
TGATCTATTCCCCCTCTTTGTTTCTTGGGGCATTTTTCCTTTTTTTTTTTTTTTTGTTTT
TTTTTTACCCCTCCTTAGCTTTATGCGCTCAGAAACCAAATTAAACCCCCCCCCATGTA
ACAGGGGGGCAGTGACAAAAGCAAGAACGCACGAAGCCAGCCTGGAGACCACCACGTCCT
GCCCCCCGCCATTTATCGCCCTGATTGGATTTTGTTTTTCATCTGTCCCTGTTGCTTGGG
TTGAGTTGAGGGTGGAGCCTCCTGGGGGGCACTGGCCACTGAGCCCCCTTGGAGAAGTCA
GAGGGGAGTGGAGAAGGCCACTGTCCGGCCTGGCTTCTGGGGACAGTGGCTGGTCCCCAG

AAGTCCTGAGGGCGGAGGGGGGGTTGGCAGGGTCTCCTCAGGTGTCAGGAGGGTGCTC
GGAGGCCACAGGAGGGGGCTCCTGGCTGGCCTGAGGCTGGCCGGAGGGGAAGGGGCTAGC
AGGTGTGTAAACAGAGGGTTCCATCAGGCTGGGGCAGGGTGGCCGCCTTCCGCACACTTG
AGGAACCCTCCCCTCTCCCTCGGTGACATCTTGCCCGCCCCTCAGCACCCTGCCTTGTCT
CCAGGAGGTCCGAAGCTCTGTGGGACCTCTTGGGGGCAAGGTGGGGTGAGGCCGGGGAGT
AGGGAGGTCAGGCGGGTCTGAGCCCACAGAGCAGGAGAGCTGCCAGGTCTGCCCATCGAC
CAGGTTGCTTGGGCCCCGGAGCCCACGGGTCTGGTGATGCCATAGCAGCCACCACCGCGG
CGCCTAGGGCTGCGGCAGGGACTCGGCCTCTGGGAGGTTTACCTCGCCCCCACTTGTGCC
CCCAGCTCAGCCCCCCTGCACGCAGCCCGACTAGCAGTCTAGAGGCCTGAGGCTTCTGGG
TCCTGGTGACGGGGCTGGCATGACCCCGGGGGTCGTCCATGCCAGTCCGCCTCAGTCGCA
GAGGGTCCCTCGGCAAGCGCCCTGTGAGTGGGCCATTCGGAACATTGGACAGAAGCCCAA
AGAGCCAAATTGTCACAATTGTGGAACCCACATTGGCCTGAGATCCAAAACGCTTCGAGG
CACCCCAAATTACCTGCCCATTCGTCAGGACACCCACCCACCCAGTGTTATATTCTGCCT
CGCCGGAGTGGGTGTTCCCGGGGGCACTTGCCGACCAGCCCCTTGCGTCCCCAGGTTTGC
AGCTCTCCCCTGGGCCACTAACCATCCTGGCCCGGGCTGCCTGTCTGACCTCCGTGCCTA
GTCGTGGCTCTCCATCTTGTCTCCTCCCCGTGTCCCCAATGTCTTCAGTGGGGGGCCCCC
TCTTGGGTCCCCTCCTCTGCCATCACCTGAAGACCCCCACGCCAAACACTGAATGTCACC
TGTGCCTGCCGCCTCGGTCCACCTTGCGGCCCGTGTTTGACTCAACTCAACTCCTTTAAC
GCTAATATTTCCGGCAAAATCCCATGCTTGGGTTTTGTCTTTAACCTTGTAACGCTTGCA
ATCCCAATAAAGCATTAAAAGTCATGATCTTCTGAGTGTTCCACTCTCTGACTTGGGTAC
TGGACTGCCAGAGGGAGGGAAGGGGCTGAGCACCTGGAAGCAGGCAGAGGGGGATAGAAG
AGGGAAGGGGAAGGAAGGCCTTAGGGGTGTGGACACCTCTCTCCGTCCCCTGATCACATA
CATGGAGAAATGAGAGAGCTGGAAGCCAGACTCTCAGACTCACTGTCGTGCACCTGAAGC
CAGGGGGTCTGGGACAGTGTCAGGCACCAAGTTCTCAAAGATGGGGGTGCCACGAAGGGT
AGGAGCCTGGGGGGCTTTTTCAGAGAAAAAGCAAAGTACCATCAGTACCAACTCCAGGGA
ATGCCTCCCCCACCAACAGCCTTAGAGGACTGGGGCTGGCAACCTCTAAAAGGTTCTGG
AAACCGTATCTGTGGCTGGAGATGGGGGCTCAGGGCCACTCTCACCCAGGGACTCAGCCC
CCTTCCAAGGTTGAGGCTGCCCAGATGTCATTAGTACCACTGCCTCCTGCCAGGGCTCAG
CCTGTACCCACCTGTCCCAAGGGTGCTGGCCTCTAGCTTTCAAGGGTCCCTGAGCATGGG
AGGAGGGGTAGGCTGGGCTGCTGGGAGCCTGG

SEQ ID NO:20
IGF2 exon 9 sequence, Ensemble exon entry ENSE00001488587 (Human GRCh37,
February 2009)
GACAACTTCCCCAGATACCCCGTGGGCAAGTTCTTCCAATATGACACCTGGAAGCAGTCCACCCAGCGCCTGCG
CAGGGGCCTGCCTGCCCTCCTGCGTGCCCGCCGGGGTCACGTGCTCGCCAAGGAGCTCGAGGCGTTCAGGGAGG
CCAAACGTCACCGTCCCCTGATTGCTCTACCCACCCAAGACCCCGCCCACGGGGGCGCCCCCCCAGAGATGGCC
AGCAATCGGAAGTGAGCAAAACTGCCGCAAGTCTGCAGCCCGGCGCCACCATCCTGCAGCCTCCTCCTGACCAC
GGACGTTTCCATCAGGTTCCATCCCGAAAATCTCTCGGTTCCACGTCCCCCTGGGGCTTCTCCTGACCCAGTCC
CCGTGCCCCGCCTCCCCGAAACAGGCTACTCTCCTCGGCCCCCTCCATCGGGCTGAGGAAGCACAGCAGCATCT
TCAAACATGTACAAAATCGATTGGCTTTAAACACCCTTCACATACCCTCCCCCCAAATTATCCCCAATTATCCC
CACACATAAAAAATCAAAACATTAAACTAACCCCCTTCCCCCCCCCCCACAACAACCCTCTTAAAACTAATTGG
CTTTTTAGAAACACCCCACAAAAGCTCAGAAATTGGCTTTAAAAAAAACAACCACCAAAAAAAATCAATTGGCT
AAAAAAAAAAAGTATTAAAAACGAATTGGCTGAGAAACAATTGGCAAAATAAAGGAATTTGGCACTCCCCACCC
CCCTCTTTCTCTTCTCCCTTGGACTTTGAGTCAAATTGGCCTGGACTTGAGTCCCTGAACCAGCAAAGAGAAAA
GAAGGACCCCAGAAATCACAGGTGGGCACGTCGCTGCTACCGCCATCTCCCTTCTCACGGAATTTTCAGGGTA
AACTGGCCATCCGAAAATAGCAACAACCCAGACTGGCTCCTCACTCCCTTTTCCATCACTAAAAATCACAGAGC
AGTCAGAGGGACCCAGTAAGACCAAAGGAGGGGAGGACAGAGCATGAAAACCAAAATCCATGCAAATGAAATGT
AATTGGCACGACCCTCACCCCCAAATCTTACATCTCAATTCCCATCCTAAAAAGCACTCATACTTTATGCATCC
CCGCAGCTACACACACACAACACACAGCACACGCATGAACACAGCACACACACGAGCACAGCACACACACAAAC
GCACAGCACACACAGCACACAGATGAGCACACAGCACACACACAAACGCACAGCACACACACGCACACACATGC
ACACACAGCACACAAACGCACGGCACACACACGCACACACATGCACACACAGCACACACACAAACGCACAGCAC
ACACAAACGCACAGCACACACGCACACACAGCACACACACGAGCACACAGCACACAAACGCACAGCACACGCAC
ACACATGCACACACAGCACACACACTAGCACACAGCACACACACAAAGACACAGCACACACATGCACACACAGC
ACACACACGCGAACACAGCACACACGAACACAGCACACACAGCACACACACAAACACAGCACACACATGCACAC
AGCACACGCACACACAGCACACACATGAACACAGCACACAGCACACACATGCACACACAGCACACACGCATGCA
CAGCACACATGAACACAGCACACACACAAACACACAGCACACACATGCACACACAGCACACACACTCATGCGCA
GCACATACATGAACACAGCTCACAGCACACAAACACGCAGCACACACGTTGCACACGCAAGCACCCACCTGCAC
ACACACATGCGCACACACACGCACACCCCCACAAAATTGGATGAAAACAATAAGCATATCTAAGCAACTACGAT

ATCTGTATGGATCAGGCCAAAGTCCCGCTAAGATTCTCCAATGTTTTCATGGTCTGAGCCCCGCTCCTGTTCCC
ATCTCCACTGCCCCTCGGCCCTGTCTGTGCCCTGCCTCTCAGAGGAGGGGCTCAGATGGTGCGGCCTGAGTGT
GCGGCCGGCGGCATTTGGATACACCCGTAGGGTGGCGGGGTGTGTCCCAGGCCTAATTCCATCTTTCCACCA
TGACAGAGATGCCCTTGTGAGGCTGGCCTCCTTGGCGCCTGTCCCCACGGCCCCCGCAGCGTGAGCCACGATGC
TCCCCATACCCCACCCATTCCCGATACACCTTACTTACTGTGTGTTGGCCCAGCCAGAGTGAGGAAGGAGTTTG
GCCACATTGGAGATGGCGGTAGCTGAGCAGACATGCCCCCACGAGTAGCCTGACTCCCTGGTGTGCTCCTGGAA
GGAAGATCTTGGGGACCCCCCCACCGGAGCACACCTAGGGATCATCTTTGCCCGTCTCCTGGGGACCCCCCAAG
AAATGTGGAGTCCTCGGGGGCCGTGCACTGATGCGGGGAGTGTGGGAAGTCTGGCGGTTGGAGGGGTGGGTGGG
GGGCAGTGGGGGCTGGGCGGGGGGAGTTCTGGGGTAGGAAGTGGTCCCGGGAGATTTTGGATGGAAAAGTCAGG
AGGATTGACAGCAGACTTGCAGAATTACATAGAGAAATTAGGAACCCCCAAATTTCATGTCAATTGATCTATTC
CCCCTCTTTGTTTCTTGGGGCATTTTTCCTTTTTTTTTTTTTTTGTTTTTTTTTACCCCTCCTTAGCTTTAT
GCGCTCAGAAACCAAATTAAACCCCCCCCCCATGTAACAGGGGGGCAGTGACAAAAGCAAGAACGCACGAAGCC
AGCCTGGAGACCACCACGTCCTGCCCCCCGCCATTTATCGCCCTGATTGGATTTTGTTTTTCATCTGTCCCTGT
TGCTTGGGTTGAGTTGAGGGTGGAGCCTCCTGGGGGGCACTGGCCACTGAGCCCCCTTGGAGAAGTCAGAGGGG
AGTGGAGAAGGCCACTGTCCGGCCTGGCTTCTGGGGACAGTGGCTGGTCCCCAGAAGTCCTGAGGGCGGAGGGG
GGGGTTGGGCAGGGTCTCCTCAGGTGTCAGGAGGGTGCTCGGAGGCCACAGGAGGGGGCTCCTGGCTGGCCTGA
GGCTGGCCGGAGGGGAAGGGGCTAGCAGGTGTGTAAACAGAGGGTTCCATCAGGCTGGGGCAGGGTGGCCGCCT
TCCGCACACTTGAGGAACCCTCCCCTCTCCCTCGGTGACATCTTGCCCGCCCCTCAGCACCCTGCCTTGTCTCC
AGGAGGTCCGAAGCTCTGTGGGACCTCTTGGGGGCAAGGTGGGGTGAGGCCGGGGAGTAGGGAGGTCAGGCGGG
TCTGAGCCCACAGAGCAGGAGAGCTGCCAGGTCTGCCCATCGACCAGGTTGCTTGGCCCCGGAGCCCACGGGT
CTGGTGATGCCATAGCAGCCACCACCGCGGCGCCTAGGGCTGCGGCAGGGACTCGGCCTCTGGGAGGTTTACCT
CGCCCCCACTTGTGCCCCCAGCTCAGCCCCCCTGCACGCAGCCCGACTAGCAGTCTAGAGGCCTGAGGCTTCTG
GGTCCTGGTGACGGGGCTGGCATGACCCCGGGGGTCGTCCATGCCAGTCCGCCTCAGTCGCAGAGGGTCCCTCG
GCAAGCGCCCTGTGAGTGGGCCATTCGGAACATTGGACAGAAGCCCAAAGAGCCAAATTGTCACAATTGTGGAA
CCCACATTGGCCTGAGATCCAAAACGCTTCGAGGCACCCCAAATTACCTGCCCATTCGTCAGGACACCCACCCA
CCCAGTGTTATATTCTGCCTCGCCGGAGTGGGTGTTCCCGGGGGCACTTGCCGACCAGCCCCTTGCGTCCCCAG
GTTTGCAGCTCTCCCCTGGGCCACTAACCATCCTGGCCCGGGCTGCCTGTCTGACCTCCGTGCCTAGTCGTGGC
TCTCCATCTTGTCTCCTCCCCGTGTCCCCAATGTCTTCAGTGGGGGCCCCCTCTTGGGTCCCCTCCTCTGCCA
TCACCTGAAGACCCCCACGCCAAACACTGAATGTCACCTGTGCCTGCCGCCTCGGTCCACCTTGCGGCCCGTGT
TTGACTCAACTCAACTCCTTTAACGCTAATATTTCCGGCAAAATCCCATGCTTGGGTTTTGTCTTTAACCTTGT
AACGCTTGCAATCCCAATAAAGCATTAAAAGTCATGATCTTC

SEQ ID NO:21
Synth 1

GGCGGCCGCTTAATACGACTCACTATAGGGAGAACCCCTCCGACCGTGCTTCCGGACAACTTCCCCAGATACCC
CGTGGGCAAGTTCTTCCAATATGACACCTGGAAGCAGTCCACCCAGCGCCTGCGCAGGGGCCTGCCTGCCCTCC
TGCGTGCCCGCCGGGGTCACGTGCTCGCCAAGGAGCTCGAGGCGTTCAGGGAGGCCAAACGTCACCGTCCCCTG
ATTGCTCTACCCACCCAAGACCCCGCCCACGGGGGCGCCCCCCCAGAGATGGCCAGCAATCGGAAGTGAGCAAA
ACTGCCGCAAGTCTGCAGCCCGGCGCCACCATCCTGCAGCCTCCTCCTGACCACGGACGTTTCCATCAGGTTCC
ATCCCGAAAATCTCTCGGTTCCACGTCCCCCTGGGGCTTCTCCTGACCCAGTCCCCGTGCCCCGCCTCCCCGAA
ACAGGCTACTCTCCTCGGCCCCCTCCATCGGGCTGAGGAAGCACAGCAGCATCTTCAAACATGTACAAAATCGA
TTGGCTTTAAACACCCTTCACATACCCTCCCCCAAATTATCCCAATTATCCCCACACATAAAAAATCAAAAC
ATTAAACTAACCCCCTTCCCCCCCCCCACAACAACCCTCTTAAAACTAATTGGCTTTTTAGAAACACCCCACA
AAAGCTCAGAAATTGGCTTTAAAAAAAACAACCACCAAAAAAAATCAATTGGCTAAAAAAAAAAAGTATTAAAA
ACGAATTGGCTGAGAAACAATTGGCAAAATAAAGGAATTTGGCACTCCCCACCCCCCTCTTTCTCTTCTCCCTT
GGACTTTGAGTCAAATTGGCCTGGACTTGAGTCCCTGAACCAGCAAAGAGAAAAGAAGGACCCCAGAAATCACA
GGTGGGCACGTCGCTGCTACCGCCATCTCCCTTCTCACGGAATTTTCAGGGTAAACTGGCCATCCGAAAATAG
CAACAACCCAGACTGGCTCCTCACTCCCTTTTCCATCACTAAAAATCACAGAGCAGTCAGAGGGACCCAGTAAG
ACCAAAGGAGGGGAGGACAGAGCATGAAAACCAAAATCCATGCAAATGAAATGTAATTGGCACGACCCTCACCC
CCAAATCTTACATCTCAATTCCCATCCTAAAAAGCACTCATACTTTATGCATCCCCGCCTCATGCGCAGCACAT
ACATGAACACAGCTCACAGCACACAAACACGCAGCACACACGTTGCACACGCAAGCACCCACCTGCACACACAC
ATGCGCACACACACGCACACCCCCACAAAATTGGATGAAAACAATAAGCATATCTAAGCAACTACGATATCTGT
ATGGATCAGGCCAAAGTCCCGCTAAGATTCTCCAATGTTTTCATGGTCTGAGCCCCGCTCCTGTTCCCATCTCC
ACTGCCCCTCGGCCCTGTCTGTGCCCTGCCTCTCAGAGGAGGGGGCTCAGATGGTGCGGCCTGAGTGTGCGGCC
GGCGGCATTTGGGATACACCCGTAGGGTGGGCGGGGTGTGTCCCAGGCCTAATTCCATCTTTCCACCATGACAG
AGATGCCCTTGTGAGGCTGGCCTCCTTGGCGCCTGTCCCCACGGCCCCCGCAGCGTGAGCCACGATGCTCCCCA
TACCCCACCCATTCCCGATACACCTTACTTACTGTGTGTTGGCCCAGCCAGAGTGAGGAAGGAGTTTGGCCACA
TTGGAGATGGCGGTAGCTGAGCAGACATGCCCCCACGAGTAGCCTGACTCCCTGGTGTGCTCCTGGAAGGAAGA
TCTTGGGGACCCCCCCACCGGAGCACACCTAGGGATCATCTTTGCCCGTCTCCTGGGGACCCCCCAAGAAATGT

GGAGTCCTCGGGGGCCGTGCACTGATGCGGGGAGTGTGGGAAGTCTGGCGGTTGGAGGGGTGGTGGGGGGCAG
TGGGGGCTGGGCGGGGGGAGTTCTGGGGTAGGAAGTGGTCCCGGGAGATTTTGGATGGAAAAGTCAGGAGGATT
GACAGCAGACTTGCAGAATTACATAGAGAAATTAGGAACCCCCAAATTTCATGTCAATTGATCTATTCCCCCTC
TTTGTTTCTTGGGGCATTTTTCCTTTTTTTTTTTTTTTGTTTTTTTTTACCCCTCCTTAGCTTTATGCGCTC
AGAAACCAAATTAAACCCCCCCCCCATGTAACAGGGGGGCAGTGACAAAAGCAAGAACGCACGAAGCCAGCCTG
GAGACCACCACGTCCTGCCCCCCGCCATTTATCGCCCTGATTGGATTTTGTTTTTCATCTGTCCCTGTTGCTTG
GGTTGAGTTGAGGGTGGAGCCTCCTGGGGGGCACTGGCCACTGAGCCCCCTTGGAGAAGTCAGAGGGGAGTGGA
GAAGGCCACTGTCCGGCCTGGCTTCTGGGGACAGTGGCTGGTCCCCAGAAGTCCTGAGGGCGGAGGGGGGGGTT
GGGCAGGGTCTCCTCAGGTGTCAGGAGGGTGCTCGGAGGCCACAGGAGGGGGCTCCTGGCTGGCCTGAGGCTGG
CCGGAGGGGAAGGGGCTAGCAGGTGTGTAAACAGAGGGTTCCATCAGGCTGGGGCAGGGTGGCCGCCTTCCGCA
CACTTGAGGAACCCTCCCCTCTCCCTCGGTGACATCTTGCCCGCCCCTCAGCACCCTGCCTTGTCTCCAGGAGG
TCCGAAGCTCTGTGGGACCTCTTGGGGGCAAGGTGGGGTGAGGCCGGGGAGTAGGGAGGTCAGGCGGGTCTGAG
CCCACAGAGCAGGAGAGCTGCCAGGTCTGCCCATCGACCAGGTTGCTTGGCCCCGGAGCCCACGGGTCTGGTG
ATGCCATAGCAGCCACCACCGCGGCGCCTAGGGCTGCGGCAGGGACTCGGCCTCTGGGAGGTTTACCTCGCCCC
CACTTGTGCCCCCAGCTCAGCCCCCCTGCACGCAGCCCGACTAGCAGTCTAGAGGCCTGAGGCTTCTGGGTCCT
GGTGACGGGGCTGGCATGACCCCGGGGGTCGTCCATGCCAGTCCGCCTCAGTCGCAGAGGGTCCCTCGGCAAGC
GCCCTGTGAGTGGGCCATTCGGAACATTGGACAGAAGCCCAAAGAGCCAAATTGTCACAATTGTGGAACCCACA
TTGGCCTGAGATCCAAAACGCTTCGAGGCACCCCAAATTACCTGCCCATTCGTCAGGACACCCACCCACCCAGT
GTTATATTCTGCCTCGCCGGAGTGGGTGTTCCCGGGGGCACTTGCCGACCAGCCCCTTGCGTCCCCAGGTTTGC
AGCTCTCCCCTGGGCCACTAACCATCCTGGCCCGGGCTGCCTGTCTGACCTCCGTGCCTAGTCGTGGCTCTCCA
TCTTGTCTCCTCCCCGTGTCCCCAATGTCTTCAGTGGGGGGCCCCCTCTTGGGTCCCCTCCTCTGCCATCACCT
GAAGACCCCCACGCCAAACACTGAATGTCACCTGTGCCTGCCGCCTCGGTCCACCTTGCGGCCCGTGTTTGACT
CAACTCAACTCCTTTAACGCTAATATTTCCGGCAAAATCCCATGCTTGGGTTTTGTCTTTAACCTTGTAACGCT
TGCAATCCCAATAAAGCATTAAAAGTCATGATCTTCGGCGGCCGCT

SEQ ID NO:22
CA repeat-rich region from SEQ ID NO:20

AGCTACACACACACAACACACAGCACACGCATGAACACAGCACACACACGAGCACAGCACACACACAAACGCAC
AGCACACACAGCACACAGATGAGCACACAGCACACACACAAACGCACAGCACACACACGCACACACATGCACAC
ACAGCACACAAACGCACGGCACACACACGCACACACATGCACACACAGCACACACACAAACGCACAGCACACAC
AAACGCACAGCACACACGCACACACAGCACACACACGAGCACACAGCACACAAACGCACAGCACACGCACACAC
ATGCACACACAGCACACACACTAGCACACAGCACACACACAAAGACACAGCACACACATGCACACACAGCACAC
ACACGCGAACACAGCACACACGAACACAGCACACACAGCACACACACAAACACAGCACACACATGCACACAGCA
CACGCACACACAGCACACACATGAACACAGCACACAGCACACACATGCACACACAGCACACACGCATGCACAGC
ACACATGAACACAGCACACACACAAACACACAGCACACACATGCACACACAGCACACACA

SEQ ID NO:23
Synth 2 sequence

GGCGGCCGCTTAATACGACTCACTATAGGGAGAACCCCTCCGACCGTGCTTCCGGACAACTTCCCCAGATACCC
CGTGGGCAAGTTCTTCCAATATGACACCTGGAAGCAGTCCACCCAGCGCCTGCGCAGGGGCCTGCCTGCCCTCC
TGCGTGCCCGCCGGGGTCACGTGCTCGCCAAGGAGCTCGAGGCGTTCAGGGAGGCCAAACGTCACCGTCCCCTG
ATTGCTCTACCCACCCAAGACCCCGCCCACGGGGGCGCCCCCCCAGAGATGGCCAGCAATCGGAAGTGAGCAAA
ACTGCCGCAAGTCTGCAGCCCGGTGCCACCATCCTGCAGCCTCCTCCTGACCACGGACGTTTCCATCAGGTTCC
ATCCCGAAAATCTCTCGGTTCCACGTCCCCCTGGGGCTTCTCCTGACCCAGTCCCCGTGCCCCGCCTCCCCGAA
ACAGGCTACTCTCCTCGGCCCCCTCCATCGGGCTGAGGAAGCACAGCAGCATCTTCAAACATGTACAAAATCGA
TTGGCTTTAAACACCCTTCACATACCCTCCCCCCAAATTATCCCCAATTATCCCACACATAAAAAATCAAAAC
ATTAAACTAACCCCCTTCCCCCCCCCCCACAACAACCCTCTTAAAACTAATTGGCTTTTTAGAAACACCCCACA
AAAGCTCAGAAATTGGCTTTAAAAAAAACAACCACCAAAAAAAATCAATTGGCTAAAAAAAAAAAGTATTAAAA
ACGAATTGGCTGAGAAACAATTGGCAAAATAAAGGAATTTGGCACTCCCCACCCCCCTCTTTCTCTTCTCCCTT
GGACTTTGAGTCAAATTGGCCTGGACTTGAGTCCCTGAACCAGCAAAGAGAAAAGAAGGGCCCCAGAAATCACA
GGTGGGCATGTCGCTGCTACCGCCATCTCCCTTCTCACGGGAATTTTCAGGGTAAACTGGCCATCCGAAAATAG
CAACAACCCAGACTGGCTCCTCACTCCCTTTTCCATCACTAAAAATCACAGAGCAGTCAGAGGGACCCAGTAAG
ACCAAAGGAGGGGAGGACAGAGCATGAAAACCAAAATCCATGCAAATGAAATGTAATTGGCACGACCCTCACCC
CCAAATCTTACATCTCAATTCCCATCCTAAAAAGCACTCATACTTTATGCATCCCCGCCTCATGCGCAGCACAT
ACATGAACACAGCTCACAGCACACAAACACGCAGCACACACGTTGCACACGCAAGCACCCACCTGCACACACAC
ATGCGCACACACACGCACACCCCCACACAATTAGATGAAAACAATAAGCATATCTAAGCAACTACGATATCTGT
ATGGATCAGGCCAAAGTCCCGCTAAGATTCTCCAATGTTTTCATGGTCTGAGCCCCCCTCCTGTTCCCATCTCC

ACTGCCCCTCGGCCCTGTCTGTGCCCTGCCTCTCAGAGGAGGGGCTCAGATGGTGCGGCCTGAGTGTGCGGCC
GGCGGCATTTGGGATACACCCGTAGGGTGGGCGGGGTGTGTCCCAGGCCTAATTCCATCTTTCCACCATGACAG
AGATGCCCTTGTGAGGCTGGCCTCCTTGGCGCCTGTCCCCACGGCCCCCGCAGCGTGAGCCACGATGCTCCCCA
TACCCCACCCATTCCCGATACACCTTACTTACTGCGTGTTGGTCCAGCCAGAGTGAGGAAGGAGTTTGGCCACA
TTGGAGATGGCGGTAGCTGAGCAGACATGCCCCCACGAGTAGCCTGACTCCCTGGTGTGCTCCTGGAAGGAAGA
TCTTGGGGACCCCCCCACCGGAGCACACCTAGGGATCATCTTTGCCCATCTCCTGGGGACCCCCCAAGAAATGT
GGAGTCCTCGGGGGCCGTGCACTGATGTGGGGAGTGTGGAAGTCTGGCGGTTGGAGGGGTGGGTGGGGGGCAG
TGGGGGCTGGCGGGGGGAGTTCTGGGGTAGGAAGTGGTCCCGGGAGATTTTGGATGGAAAAGTCAGGAGGATT
GACAGCAGACTTGCAGAATTACATAGAGAAATTAGGAACCCCCAAATTTCATGTCAATTGATCTATTCCCCCTC
TTTGTTTCTTGGGGCAGTTTTCCTTTTTTTTTTTTTTGTTTTTTTTTACCCCTCCTTAGCTTTATGCGCTC
AGAAACCAAATTAAACCCCCCCCCCATGTAACAGGGGGGCAGTGACAAAAGCAAGAACGCACGAAGCCAGCCTG
GAGACCACCACGTCCTGCCCCCCGCCATTTATCGCCCTGATTGGATTTTGTTTTTCATCTGTCCCTGTTGCTTG
GGTTGAGTTGAGGGTGGAGCCTCCTGGGGGGCACTGGCCACTGAGCCCCCTTGGAGAAGTCAGAGGGGAGTGGA
GAAGGCCACTGTCCGGCCTGGCTTCTGGGGACAGTGGCTGGTCCCCAGAAGTCCTGAGGGCGGAGGGGGGGGTT
GGGCAGGGTCTCCTCAGGTGTCAGGAGGGTGCTCGGAGGCCACAGGAGGGGGCTCCTGGCTGGCCTGAGGCTGG
CCGGAGGGGAAGGGGCTAACAGGTGTGTAAACAGAGGGTTCCATCAGGCTGGGGCAGGGTGGCCGCCTTCCGCA
CACTTGAGGAACCCTCCCCTCTCCCTCGGTGACATCTTGCCCGCCCCTCAGCACCCTGCCTTGTCTCCAGGAGG
TCCGAAGCTCTGTGGGACCTCTTGGGGGCAAGGTGGGGTGAGGCCGGGGAGTAGGGAGGTCAGGCGGTCTGAG
CCCACAGAGCAGGAGAGCTGCCAGGTCTGCCCATCGACCAGGTTGCTTGGGCCCCGGAGCCCACGGGTCTGGTG
ATGCCATAGCAGCCACCACCGCGGCACCTAGGGCTGCGGCAGGGACTCGGCCTCTGGGAGGTTTACCTCGCCCC
CACTTGTGCCCCCAGCTCAGCCCCCCTGCACGCAGCCCGACTAGCAGTCTAGAGGCCTGAGGCTTCTGGGTCCT
GGTGACGGGGCTGGCATGACCCCGGGGGTCGTCCATGCCAGTCCGCCTCAGTCGCAGAGGGTCCCTCGGCAAGC
ACCCTGTGAGTGGGCCATTCGGAACATTGGACAGAAGCCCAAAGAGCCAAATTGTCACAATTGTGGAACCCACA
TTGGCCTGAGATCCAAAACGCTTCGAGGCACCCCAAATTACCTGCCCATTCGTCAGGACACCCACCCACCCAGT
GTTATATTCTGCCTCGCCGGAGTGGGTGTTCCCGGGGGCACTTGCCGACCGGCCCCTTGCGTCCCCAGGTTTGC
AGCTCTCCCCTGGGCCACTAACCATCCTGGCCCGGGCTGCCTGTCTGACCTCCGTGCCTAGTCGTGGCTCTCCA
TCTTGTCTCCTCCCCGTGTCCCCAATGTCTTCAGTGGGGGGCCCCCTCTTGGGTCCCCTCCTCTGCCATCACCT
GAAGACCCCCACGCCAAACACTGAATGTCACCTGTGCCTGCCGCCTCGGTCCACCTTGCGGCCCGTGTTTGACT
CAACTCAGCTCCTTTAACGCTAATATTTCCGGCAAAATCCCATGCTTGGGTTTTGTCTTTAACCTTGTAACGCT
TGCAATCCCAATAAAGCATTAAAAGTCATGATCTTCGGCGGCCGCT

### FURTHER ASPECTS OF THE INVENTION

The following represent further numbered aspects of the invention:
(1) A method of determining loss-of-imprinting in the Insulin Growth Factor-2 (IGF2) gene of an individual, the method comprising,
   detecting the SNP genotype of the IGF2 gene in the individual, wherein the genotype of at least three SNPs selected from the group consisting of SEQ ID NOs: 1, 2, 3, 4, 5, 6, 7, 8, 9, 10, 11, 12, 13, 14, 15, and 16 is determined, thereby determining whether the individual is heterozygous or homozygous at each of the at least three SNPs;
   quantifying in a sample from the individual the amount of RNA comprising two polymorphic options of at least one heterozygous SNP;
   determining a ratio of the amount of RNA comprising two polymorphic options of at least one heterozygous SNP; and
   correlating the RNA ratio to loss of imprinting of the IGF2 gene.
(2) The method of (1), wherein at least one SNP in the detecting step is selected from the group consisting of SEQ ID NO: 1, 2, 3, 4, 5, and 6.
(3) The method of (1), wherein the correlating step further comprises correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.
(4) The method of (1), wherein at least two of the detected SNPs are heterozygous and the quantifying step comprises quantifying the amount of RNA comprising two polymorphic options at the at least two heterozygous SNPs.
(5) The method of (1), wherein the sample is a blood, stool, cell scrape or tissue sample.
(6) The method of (1), wherein the RNA is reverse transcribed into cDNA and the quantity of cDNA comprising each polymorphic option is used to determine the amount of RNA comprising the two polymorphic options.
(7) The method of (6), wherein the amount of allele-specific cDNA is quantified in a method comprising contacting the cDNA with at least one allele-specific detection polynucleotide.
(8) The method of (6), comprising contacting the cDNA with a sufficient number of allele-specific detection polynucleotides such that the polymorphic option for each heterozygous SNP from the group is determined.
(9) The method of (8), comprising contacting the cDNA with a number of different allele-specific detection polynucleotides, wherein the number is equal to or greater than the number of heterozygous SNPs in the group such that at least one different allele-specific detection polynucleotide is used to detect each different heterozygous SNP.
(10) The method of (7), wherein the at least one allele-specific detection polynucleotide is hybridized to the cDNA and extended in a template-dependent manner through the polymorphic position of the SNP in the cDNA.
(11) The method of (10), wherein the at least one allele-specific detection polynucleotide comprises at least 8 (e.g., at least 10, 12, 15) contiguous nucleotides at the 3' end of the allele-specific detection polynucleotide, wherein the at least 8 contiguous nucleotides are either:
   100% complementary to at least 8 (e.g., at least 10, 12, 15) nucleotides directly 3' (or is 2 or 3 nucleotides 3') of the polymorphic position of the SNP region set forth in the SEQ ID NO:; or
   100% identical to at least 8 (e.g., at least 10, 12, 15) nucleotides directly 5' (or is 2 or 3 nucleotides 5') of the polymorphic position of the SNP region set forth in the SEQ ID NO:.
(12) The method of (7), wherein the at least one allele-specific detection polynucleotide comprises a sequence 100% identical to at least 8 (e.g., at least 10, 12, 15) contiguous nucleotides, or the complement thereof, of at least one heterozygous SNP, and the sequence consists of at least 8 (e.g., at least 10, 12, 15) contiguous nucleotides, or the complement thereof, of the SNP, wherein one of the positions of the sequence corresponds to the variable position of the SNP.
(13) The method of (1), wherein the detecting step comprises detecting the SNP genotype of each polymorphic option of each of:
   SEQ ID NOs: 1, 2, 3, 4, 5, 6, 7, 8, 9, 10, 11, 12, 13, 14, 15, and 16; or
   SEQ ID NOs: 1, 5, 8, 9, 10, 11, 14, 15, and 16; or
   SEQ ID NOs: 1, 5, 8, 9, 10, 11, 12, 14, 15, and 16; or
   SEQ ID NOs: 1, 2, 4, 5, 8, 9, 10, 12, 13, 14, 15, and 16.
(14) The method of (1), wherein the detecting step comprises detecting at least 4 (e.g., 4, 5, 6, 7, 8, 9, 10, 11, 12, 13, 14, 15) SNPs selected from the group.
(15) The method of (1), wherein the detecting step comprises contacting the sample with a first, second and third polynucleotides of any of (17)-(19) or (27).
(16) The method of (3), further comprising providing a risk classification of cancer, diagnosis of cancer, or prognosis based on the correlating step, and/or submitting the individual to a disease screening procedure (e.g., colonoscopy) based on the correlating step, and/or submitting a sample from the individual to further diagnostic or prognostics assays based on the correlating step and/or changing a drug or other treatment regimen of the individual based on the correlating step.
(17) A reaction mixture comprising,
   a first polynucleotide of between 8-100 nucleotides, wherein the first polynucleotide distinguishes between one polymorphic option of a first SNP (or complement thereof) and the other polymorphic option of the first SNP (or complement thereof) in a hybridization reaction,
   a second polynucleotide of between 8-100 nucleotides, wherein the second polynucleotide distinguishes between one polymorphic option of a second SNP (or complement thereof) and the other polymorphic option of the second SNP (or complement thereof) in a hybridization reaction, and
   a third polynucleotide of between 8-100 nucleotides, wherein the third polynucleotide distinguishes between one polymorphic option of a third SNP (or complement thereof) and the other polymorphic option of the third SNP (or complement thereof) in a hybridization reaction, wherein the first and second and third SNP is selected from the group consisting of SEQ ID NOs: 1, 2, 3, 4, 5, 6, 7, 8, 9, 10, 11, 12, 13, 14, 15, and 16, wherein at least one SNP is 1, 2, 3, 4, 5, or 6.
(18) The reaction mixture of (17), wherein at least one, at least two, or three of the first, second, and third polynucleotide comprise a sequence 100% identical to at least 8 (e.g., at least 10, 12, 15, 20) contiguous nucleotides, or the complement thereof, of the SEQ ID NO: corresponding to the first and second and third SNP, respectively.
(19) The reaction mixture of (18), wherein the sequence consists of at least 8 (e.g., at least 10, 12, 15, 20) contiguous nucleotides, or the complement thereof, of the SNP, wherein one of the positions of the sequence corresponds to the variable position of the SNP.
(20) The reaction mixture of (17), further comprising a sample comprising nucleic acids from a human.
(21) The reaction mixture of (17), further comprising a polymerase.
(22) The reaction mixture of (20), wherein the polymerase is a thermostable polymerase.
(23) The reaction mixture of (17), wherein at least one of the first and second and third polynucleotides are detectably labeled.
(24) The reaction mixture of (20), wherein the detectable label is a fluorescent label.
(25) The reaction mixture of (20), wherein the first and second polynucleotides are detectably labeled with different labels such that the different labels can be differentially detected based on different wavelength of fluorescence of the labels or different mass of the labels.
(26) The reaction mixture of (17), comprising a plurality of different allele-specific detection polynucleotide of between 8-100 nucleotides, such that at least one polynucleotide of the plurality distinguishes between one allele of a SNP (or complement thereof) and the other allele of the SNP (or complement thereof) in a hybridization reaction for each of:
   SEQ ID NOs: 1, 2, 3, 4, 5, 6, 7, 8, 9, 10, 11, 12, 13, 14, 15, and 16; or
   SEQ ID NOs: 1, 5, 8, 9, 10, 11, 14, 15, and 16; or
   SEQ ID NOs: 1, 5, 8, 9, 10, 11, 12, 14, 15, and 16; or
   SEQ ID NOs: 1, 2, 4, 5, 8, 9, 10, 12, 13, 14, 15, and 16.
(27) A reaction mixture comprising,
   a first allele-specific detection polynucleotide of between 8-100 nucleotides,
   a second allele-specific detection polynucleotide of between 8-100 nucleotides,
   a third allele-specific detection polynucleotide of between 8-100 nucleotides, wherein each of the first, second, and third polynucleotides comprise at least 8 (e.g., at least 10, 12, 15, 20) contiguous nucleotides at the 3' end of the allele-specific detection polynucleotide, wherein the at least 8 contiguous nucleotides are either:
   100% complementary to at least 8 (e.g., at least 10, 12, 15, 20) nucleotides directly 3' (or is 2 or 3 nucleotides 3') of the polymorphic position of a SNP region; or
   100% identical to at least 8 (e.g., at least 10, 12, 15, 20) nucleotides directly 5' (or is 2 or 3 nucleotides 5') of the polymorphic position of a SNP region [or, e.g., 100% identical to at least 8 (e.g., at least 10, 12, 15, 20) nucleotides directly 3' (or is 2 or 3 nucleotides 3') of the polymorphic position of the complement of the SNP region],
   wherein the SNP region corresponding to each of the first, second, and third polynucleotides is different and the SNP regions are selected from the group consisting of SEQ ID NOs: 1, 2, 3, 4, 5, 6, 7, 8, 9, 10, 11, 12, 13, 14, 15, and 16, wherein at least one SNP region is SEQ ID NO:1, 2, 3, 4, 5, or 6.
(28) The reaction mixture of (27), further comprising a sample comprising nucleic acids from a human.
(29) The reaction mixture of (27), further comprising a polymerase.
(30) The reaction mixture of (29), wherein the polymerase is a thermostable polymerase.
(31) The reaction mixture of (27), wherein at least one of the first and second and third polynucleotides are detectably labeled.
(32) The reaction mixture of (31), wherein the detectable label is a fluorescent label.
(33) The reaction mixture of (27), wherein the first and second and third polynucleotides are detectably labeled with different labels such that the different labels can be differentially detected based on different wavelength of fluorescence of the labels or different mass of the labels.
(34) The reaction mixture of (27), comprising a plurality of different allele-specific detection polynucleotides, wherein each of the plurality of polynucleotides comprise at least 8 (e.g., at least 10, 12, 15, 20) contiguous nucleotides at the 3' end of the allele-specific detection polynucleotide, wherein the at least 8 contiguous nucleotides are either:
   100% complementary to at least 8 (e.g., at least 10, 12, 15, 20) nucleotides directly 3' (or is 2 or 3 nucleotides 3') of the polymorphic position of a SNP region; or
   100% identical to at least 8 (e.g., at least 10, 12, 15, 20) nucleotides directly 5' (or is 2 or 3 nucleotides 5') of the polymorphic position of a SNP region,
      wherein the plurality includes a sufficient number of polynucleotides such at least one polynucleotide corresponds to each of the following SNP regions:
      SEQ ID NOs: 1, 2, 3, 4, 5, 6, 7, 8, 9, 10, 11, 12, 13, 14, 15, and 16; or
      SEQ ID NOs: 1, 5, 8, 9, 10, 11, 14, 15, and 16; or
      SEQ ID NOs: 1, 5, 8, 9, 10, 11, 12, 14, 15, and 16; or
      SEQ ID NOs: 1,2,4, 5, 8, 9, 10, 12, 13, 14, 15, and 16.
(35) A kit comprising,
   a first polynucleotide of between 8-100 nucleotides, wherein the first polynucleotide distinguishes between one polymorphic option of a first SNP (or complement thereof) and the other polymorphic option of the first SNP (or complement thereof) in a hybridization reaction,
   a second polynucleotide of between 8-100 nucleotides, wherein the second polynucleotide distinguishes between one polymorphic option of a second SNP (or complement thereof) and the other polymorphic option of the second SNP (or complement thereof) in a hybridization reaction,
   a third polynucleotide of between 8- 100 nucleotides, wherein the third polynucleotide distinguishes between one polymorphic option of a third SNP (or complement thereof) and the other polymorphic option of the third SNP (or complement thereof) in a hybridization reaction, wherein the first and second and third SNP is selected from the group consisting of SEQ ID NOs: 1, 2, 3, 4, 5, 6, 7, 8, 9, 10, 11, 12, 13, 14, 15, and 16, wherein at least one SNP is selected from SEQ ID NO: 1, 2, 3, 4, 5, or 6.
(36) The kit of (35), wherein at least one, at least two, or three of the first, second, and third polynucleotide comprise a sequence 100% identical to at least 8 (e.g., at least 10, 12, 15, 20) contiguous nucleotides, or the complement thereof, of the SEQ ID NO: corresponding to the first and second and third SNP, respectively.
(37) The kit of (36), wherein the sequence consists of at least 8 (e.g., at least 10, 12, 15, 20) contiguous nucleotides, or the complement thereof, of the SNP, wherein one of the positions of the sequence corresponds to the variable position of the SNP.
(38) The kit of claim, further comprising a polymerase.
(39) The kit of (38), wherein the polymerase is a thermostable polymerase.
(40) The kit of (35), wherein at least one of the first and second and third polynucleotides are detectably labeled.
(41) The kit of (40), wherein the detectable label is a fluorescent label.
(42) The kit of (40), wherein the first and second and third polynucleotides are detectably labeled with different labels such that the different labels can be differentially detected based on different wavelength of fluorescence of the labels or different mass of the labels.
(43) The kit of (35), comprising a plurality of different allele-specific detection polynucleotide of between 8-100 nucleotides, such that at least one polynucleotide of the plurality distinguishes between one allele of a SNP (or complement thereof) and the other allele of the SNP (or complement thereof) in a hybridization reaction for each of:
   SEQ ID NOs: 1, 2, 3, 4, 5, 6, 7, 8, 9, 10, 11, 12, 13, 14, 15, and 16; or
   SEQ ID NOs: 1, 5, 8, 9, 10, 11, 14, 15, and 16; or
   SEQ ID NOs: 1, 5, 8, 9, 10, 11, 12, 14, 15, and 16; or
   SEQ ID NOs: 1, 2, 4, 5, 8, 9, 10, 12, 13, 14, 15, and 16.
(44) A kit comprising,
   a first allele-specific detection polynucleotide of between 8-100 nucleotides,
   a second allele-specific detection polynucleotide of between 8-100 nucleotides,
   a third allele-specific detection polynucleotide of between 8-100 nucleotides, wherein each of the first, second, and third polynucleotides comprise at least 8 (e.g., at least 10, 12, 15, 20) contiguous nucleotides at the 3' end of the allele-specific detection polynucleotide, wherein the at least 8 contiguous nucleotides are either:
   100% complementary to at least 8 (e.g., at least 10, 12, 15, 20) nucleotides directly 3' (or is 2 or 3 nucleotides 3') of the polymorphic position of a SNP region; or
   100% identical to at least 8 (e.g., at least 10, 12, 15, 20) nucleotides directly 5' (or is 2 or 3 nucleotides 5') of the polymorphic position of a SNP region [or, e.g., 100% identical to at least 8 (e.g., at least 10, 12, 15, 20) nucleotides directly 3' (or is 2 or 3 nucleotides 3') of the polymorphic position of the complement of the SNP region],
   wherein the SNP region corresponding to each of the first, second, and third polynucleotides is different and the SNP regions are selected from the group consisting of SEQ ID NOs: 1, 2, 3, 4, 5, 6, 7, 8, 9, 10, 11, 12, 13, 14, 15, and 16, wherein at least one SNP region is SEQ ID NO:1, 2, 3, 4, 5, or 6.
(45) The kit of (44), further comprising a sample comprising nucleic acids from a human.
(46) The kit of (44), further comprising a polymerase.
(47) The kit of (46), wherein the polymerase is a thermostable polymerase.
(48) The kit of (44), wherein at least one of the first and second and third polynucleotides are detectably labeled.
(49) The kit of (48), wherein the detectable label is a fluorescent label.
(50) The kit of (44), wherein the first and second and third polynucleotides are detectably labeled with different labels such that the different labels can be differentially detected based on different wavelength of fluorescence of the labels or different mass of the labels.
(51) The kit of (44), comprising a plurality of different allele-specific detection polynucleotides, wherein each of the plurality of polynucleotides comprise at least 8 (e.g., at least 10, 12, 15, 20) contiguous nucleotides at the 3' end of the allele-specific detection polynucleotide, wherein the at least 8 contiguous nucleotides are either:
   100% complementary to at least 8 (e.g., at least 10, 12, 15, 20) nucleotides directly 3' (or is 2 or 3 nucleotides 3') of the polymorphic position of a SNP region; or
   100% identical to at least 8 (e.g., at least 10, 12, 15, 20) nucleotides directly 5' (or is 2 or 3 nucleotides 5') of the polymorphic position of a SNP region [or, e.g., 100% identical to at least 8 (e.g., at least 10, 12, 15, 20) nucleotides directly 3' (or is 2 or 3 nucleotides 3 ') of the polymorphic position of the complement of the SNP region],
      wherein the plurality includes a sufficient number of polynucleotides such at least one polynucleotide corresponds to each of the following SNP regions:
      SEQ ID NOs: 1, 2, 3, 4, 5, 6, 7, 8, 9, 10, 11, 12, 13, 14, 15, and 16; or
      SEQ ID NOs: 1, 5, 8, 9, 10, 11, 14, 15, and 16; or
      SEQ ID NOs: 1, 5, 8, 9, 10, 11, 12, 14, 15, and 16; or
      SEQ ID NOs: 1, 2, 4, 5, 8, 9, 10, 12, 13, 14, 15, and 16.
(52) A computer-implemented method for determining loss-of-imprinting in the Insulin Growth Factor-2 (IGF2) gene, the method comprising:
   (a) receiving, at a host computer, genotype values for at least three SNPs selected from the group consisting of SEQ ID NOs:1, 2, 3, 4, 5, 6, 7, 8, 9, 10, 11, 12, 13, 14, 15, and 16, wherein the genotype is determined from a sample from an individual; and
   (b) receiving, at a host computer, a value representing the amount of RNA comprising each polymorphic option of at least one heterozygous SNP selected from the group consisting of SEQ ID NOs: 1, 2, 3, 4, 5, 6, 7, 8, 9, 10, 11, 12, 13, 14, 15, and 16, wherein the RNA is from a sample from an individual; and
   (c) determining, at a host computer, a ratio of the amount of RNA comprising two polymorphic options of at least one heterozygous SNP; and
   (d) outputting to a human the RNA ratio or correlating in the host computer, the RNA ratio to loss of imprinting of the IGF2 gene, and optionally outputting to a human the result of the correlating step.
(53) The computer-implemented method of (52), wherein at least one SNP is selected from SEQ ID NO: 1, 2, 3, 4, 5, or 6.
(54) The computer-implemented method of (52), wherein the correlating step further comprises correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.
(55) The computer- implemented method of (52), wherein the receiving step (a) comprises receiving a value representing the amount of RNA having each polymorphic option of each of
   SEQ ID NOs: 1, 2, 3, 4, 5, 6, 7, 8, 9, 10, 11, 12, 13, 14, 15, and 16; or
   SEQ ID NOs: 1, 5, 8, 9, 10, 11, 14, 15, and 16; or
   SEQ ID NOs: 1, 5, 8, 9, 10, 11, 12, 14, 15, and 16; or
   SEQ ID NOs: 1, 2, 4, 5, 8, 9, 10, 12, 13, 14, 15, and 16.
(56) The computer-implemented method of (52), wherein the receiving step (a) comprises receiving a value representing the amount of RNA having each polymorphic option of each of at least 4 (e.g., 4, 5, 6, 7, 8, 9, 10, 11, 12, 13, 14, 15) SNPs selected from the group.
(57) A computer program product for determining loss-of-imprinting in the Insulin Growth Factor-2 (IGF2) gene, the computer readable product comprising:
   a computer readable medium encoded with program code, the program code including:
      program code for receiving genotype values for at least three SNPs selected from the group consisting of SEQ ID NOs:1, 2, 3, 4, 5, 6, 7, 8, 9, 10, 11, 12, 13, 14, 15, and 16, wherein the genotype is determined from a sample from an individual;
      program code for receiving a value representing the amount of RNA comprising each polymorphic option of at least one heterozygous SNP selected from the group consisting of SEQ ID NOs:1, 2, 3, 4, 5, 6, 7, 8, 9, 10, 11, 12, 13, 14, 15, and 16, wherein the RNA is from a sample from an individual;
      program code for determining a ratio of the amount of RNA comprising two polymorphic options of at least one heterozygous SNP; and
      optionally, program code for correlating in the host computer, the RNA ratio to loss of imprinting of the IGF2 gene.
(58) The computer program product of (57), at least one SNP is selected from SEQ ID NO: 1, 2, 3, 4, 5, or 6.
(59) The computer program product of (57), comprising program code for correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.
(60) The computer program product of (57), comprising program code for receiving information representing the presence or absence of each polymorphic option of the at least three SNPs in genomic DNA in the sample; and program code for rdetermining whether the individual is heterozygous for the at least two SNPs.
(61) The computer program product of (57), comprising program code for receiving a value representing the amount of RNA having each polymorphic option of each of
   SEQ ID NOs: 1, 2, 3, 4, 5, 6, 7, 8, 9, 10, 11, 12, 13, 14, 15, and 16; or
   SEQ ID NOs: 1, 5, 8, 9, 10, 11, 14, 15, and 16; or
   SEQ ID NOs: 1, 5, 8, 9, 10, 11, 12, 14, 15, and 16; or
   SEQ ID NOs: 1, 2, 4, 5, 8, 9, 10, 12, 13, 14, 15, and 16.
(62) The computer program product of (57), comprising program code for receiving a genotype value representing for each of at least 4 (e.g., 4, 5, 6, 7, 8, 9, 10, 11, 12, 13, 14, 15) SNPs selected from the group.
(63) A pair of control isolated nucleic acid members, substantially free of cellular nucleic acids, wherein both members of the pair comprise at least two Insulin Growth Factor-2 (IGF2) single nucleotide polymorphism (SNP) sequences, wherein:
   (a) one member of the pair comprises a polynucleotide that comprises one allele of the at least two *IGF2* SNPs, wherein the nucleic acid sequence adjacent to the first SNP is substantially identical to at least 15 contiguous nucleotides of the corresponding region of SEQ ID NO:17, 18, 21, or 23, or the complement therof, and the nucleic acid sequence adjacent to the second SNP is substantially identical to at least 15 contiguous nucleotides of the corresponding region of SEQ ID NO:17, 18, 21, or 23, or the complement thereof; and
   (b) the second member of the pair comprises a polynucleotide comprising an alternate allele of the at least two *IGF2* SNPs; wherein the nucleic acid sequence adjacent to the first SNP is substantially identical to at least 15 contiguous nucleotides of the corresponding region of SEQ ID NO: 17, 18, 21, or 23, or the complement thereof; and the nucleic acid sequence adjacent to the second SNP is substantially identical to at least 15 contiguous nucleotides of the corresponding region of SEQ ID NO:17, 18, 21, or 23, or the complement thereof.
(64) The pair of (63), wherein at least one of the SNPs is selected from the group consisting of the SNP as shown in SEQ ID NOs: 1, 2, 3, 4, 5, 6, 7, 8, 9, 10, 11, 12, 13, 14, 15, and 16.
(65) The pair of (63), wherein at least two of the SNPs are selected from the group consisting of the SNP as shown in SEQ ID NOs: 1, 2, 3, 4, 5, 6, 7, 8, 9, 10, 11, 12, 13, 14, 15, and 16.
(66) The pair of (63), wherein at least one of the SNPs is selected from the group consisting of the SNP as shown in SEQ ID NOs: 1, 2, 3, 4, 5, and
(67) The pair of (63), wherein the at least two IGF2 SNPs comprise three, four, five, six, seven, eight, nine, ten, eleven, twelve, thirteen, fourteen, fifteen or sixteen IGF2 SNPs.
(68) The pair of (67), wherein the SNPs comprise the SNPs as shown in:
   SEQ ID NOs: 1, 2, 3, 4, 5, 6, 7, 8, 9, 10, 11, 12, 13, 14, 15, and 16;
   SEQ ID NOs: 1, 5, 8, 9, 10, 11, 14, 15, and 16;
   SEQ ID NOs. 1, 5, 8, 9, 10, 11, 12, 14, 15, and 16; or
   SEQ ID NOs. 1, 2, 4, 5, 8, 9, 10, 12, 13, 14, 15, and 16.
(69) The pair of (63), wherein the first member of the pair comprises a polynucleotide that has at least 15 contiguous nucleotides of one allele of SEQ ID NO: 1, 2, 3, 4, 5, 6, 7, 8, 9, 10, 11, 12, 13, 14, 15, or 16, or the complement thereof; and the second member of the pair comprises a polynucleotide that has at least 15 contiguous nucleotides of the alternative allele of SEQ ID NO:1, 2, 3, 4, 5, 6, 7, 8, 9, 10, 11, 12, 13, 14, 15, or 16, or the complement thereof.
(70) The pair of (63), wherein one of the members of the pair comprises at least two minor alleles of the SNPs.
(71) The pair of (63), wherein the pair of isolated nucleic acid members are RNA.
(72) The pair of (63), wherein the pair of isolated nucleic acid members are DNA.
(73) The pair of (63), wherein the polynucleotide of (a) is operably linked to a promoter and the polynucleotide of (b) is operably linked to a promoter.
(74) The pair of (63), wherein the members of the pair comprise less than 500 nucleotides of the sequence set forth in SEQ ID NO:22.
(75) The pair of (63), wherein the members of the pair are separated.
(76) The pair of (63), wherein the members of the pair are in a mix together.
(77) A mixture comprising the pair of isolated nucleic acid members of any one of the preceding aspects.
(78) The mixture of (77), wherein the members of the pair on present in equimolar amounts.
(79) The mixture of (77), further comprising a second pair of isolated nucleic acids, substantially free of cellular nucleic acids, wherein both members of the second pair comprise at least one *IGF2* SNP sequence and wherein the at least one *IGF2* SNP sequence in the second pair is different from the *IGF2* SNPs in the first pair; and
   (c) one member of the second pair comprises one allele of the at least one *IGF2* SNP, wherein the nucleic acid sequence adjacent to the at least one SNP is substantially identical to at least 15 contiguous nucleotides of the corresponding region of SEQ ID NO: 17, 18, 21, or 23, or the complement thereof; and
   (d) the other member of the second pair comprises an alternate allele of the at least one *IG2F* SNP; wherein the nucleic acid sequence adjacent to the at least one SNP is substantially identical to at least 15 contiguous nucleotides of the corresponding region of SEQ ID NO:17, 18, 21, or 23, or the complement thereof
(80) A kit comprising the pair of isolated nucleic acid members of any one of (63) to (76).
(81) The kit of (80), wherein one member of the pair is in a separate container without the second member of the pair.
(82) A kit comprising a mixture of any one of (77) to (79).
(83) The kit of (80), further comprising a second pair of isolated nucleic acids, substantially free of cellular nucleic acids, wherein both members of the second pair comprise at least one *IGF2* SNP sequences and wherein the at least one *IGF2* SNP sequence in the second pair is different from the *IGF2* SNPs in the first pair; and
   (c) one member of the second pair comprises one allele of the at least one *IGF2* SNP, wherein the nucleic acid sequence adjacent to the at least one SNP is substantially identical to at least 15 contiguous nucleotides of the corresponding region of SEQ ID NO: 17, 18, 21 , or 23 , or the complement thereof and
   (d) the other member of the second pair comprises an alternate allele of the at least one *IGF2* SNP; wherein the nucleic acid sequence adjacent to the at least one SNP is substantially identical to at least 15 contiguous nucleotides of the corresponding region of SEQ ID NO: 17, 18, 21, or 23, or the complement thereof.
(84) The kit of (83), wherein the second pair of isolated nucleic acids is a separate container from the first pair of nucleic acids.
(85) A method of determining loss-of-imprinting (LOI) in an *IGF2* gene from an individual, the method comprising,
   (a) quantifying in a sample from the individual, the amount of maternal (first) and paternal (second) copy of *IGF2* RNA, wherein the quantifying step comprises contacting the sample RNA, or a sample cDNA thereof, with one or more oligonucleotides that distinguish between a first possible SNP allele and a second possible SNP allele in the first and second copy, respectively; and
   (b) providing a control mix comprising a known ratio of the pair of nucleic acids of any one of (63) to (76) that comprise polynucleotides having the first possible SNP allele and the second possible SNP allele in step (a);
   (c) quantifying in the control mix the amount of each member of the pair of nucleic acids;
   (d) comparing the ratio of the pair of nucleic acids present in the control mix measured in step (c) to the amounts of the first and second copy of *IGF2* RNA, or a sample cDNA thereof, measured in step (a), thereby determining the presence or absence of *IGF2* LOI.
(86) A method of providing a control standard in an *IGF2* loss-of- imprinting (LOI) assay to confirm the viability of the reaction conditions, the method comprising, providing a control mix comprising a known ratio of the pair of nucleic acids of any one of (63) to (76); and
   quantifying in the control mix the measured ratio of each member of the pair of nucleic acid, thereby confirming viability of the reaction conditions of the assay.

## Claims

1. A method of determining loss-of-imprinting in the Insulin Growth Factor-2 (IGF2) gene of an individual, the method comprising,
detecting the SNP genotype of the IGF2 gene in the individual, wherein the genotype of the SNP represented by SEQ ID NO:1 and at least two additional SNPs selected from the group consisting of SEQ ID NOs: 2, 3, 4, 5, 6, 7, 8, 9, 10, 11, 12, 13, 14, 15, and 16 is determined, thereby determining whether the individual is heterozygous or homozygous at each of the at least three SNPs;
quantifying in a sample from the individual the amount of RNA comprising two polymorphic options of at least one heterozygous SNP;
determining a ratio of the amount of RNA comprising two polymorphic options of at least one heterozygous SNP; and
correlating the RNA ratio to loss of imprinting of the IGF2 gene.

2. The method of claim 1, wherein at least one of said at least two additional SNPs is selected from the group consisting of SEQ ID NO: 2, 3, 4, 5, and 6.

3. The method of claim 1, wherein the correlating step further comprises correlating the relative amount of RNA comprising the polymorphic options of the at least one heterozygous SNP to an increased risk of cancer, a diagnosis or prognosis of cancer, or a prediction of efficacy of a drug for ameliorating, treating or preventing cancer.

4. The method of claim 1, wherein at least two of the detected SNPs are heterozygous and the quantifying step comprises quantifying the amount of RNA comprising two polymorphic options at the at least two heterozygous SNPs.

5. The method of claim 1, wherein the sample is a blood, stool, cell scrape or tissue sample.

6. The method of claim 1, wherein the RNA is reverse transcribed into cDNA and the quantity of cDNA comprising each polymorphic option is used to determine the amount of RNA comprising the two polymorphic options.

7. The method of claim 6, wherein the amount of allele-specific cDNA is quantified in a method comprising contacting the cDNA with at least one allele-specific detection polynucleotide.

8. The method of claim 6, comprising contacting the cDNA with a sufficient number of allele-specific detection polynucleotides such that the polymorphic option for each heterozygous SNP from the group is determined.

9. The method of claim 8, comprising contacting the cDNA with a number of different allele-specific detection polynucleotides, wherein the number is equal to or greater than the number of heterozygous SNPs in the group such that at least one different allele-specific detection polynucleotide is used to detect each different heterozygous SNP.

10. The method of claim 7, wherein the at least one allele-specific detection polynucleotide is hybridized to the cDNA and extended in a template-dependent manner through the polymorphic position of the SNP in the cDNA.

11. The method of claim 10, wherein the at least one allele-specific detection polynucleotide comprises at least 8 contiguous nucleotides at the 3' end of the allele-specific detection polynucleotide, wherein the at least 8 contiguous nucleotides are either:
100% complementary to at least 8 nucleotides directly 3' (or is 2 or 3 nucleotides 3') of the polymorphic position of the SNP region set forth in the SEQ ID NO:; or
100% identical to at least 8 nucleotides directly 5' or is 2 or 3 nucleotides 5' of the polymorphic position of the SNP region set forth in the SEQ ID NO:.

12. The method of claim 7, wherein the at least one allele-specific detection polynucleotide comprises a sequence 100% identical to at least 8 contiguous nucleotides, or the complement thereof, of at least one heterozygous SNP, and the sequence consists of at least 8 contiguous nucleotides, or the complement thereof, of the SNP, wherein one of the positions of the sequence corresponds to the variable position of the SNP.

13. The method of claim 1, wherein the detecting step comprises detecting at least 4 SNPs selected from the group.

14. The method of claim 3, further comprising providing a risk classification of cancer, diagnosis of cancer, or prognosis based on the correlating step, and/or submitting the individual to a disease screening procedure (e.g., colonoscopy) based on the correlating step, and/or submitting a sample from the individual to further diagnostic or prognostics assays based on the correlating step and/or changing a drug or other treatment regimen of the individual based on the correlating step.


SEQUENCE LISTING

<110>  Orion Genomics, LLC

<120>  COMBINATIONS OF POLYMORPHISMS FOR DETERMINING ALLELE-SPECIFIC
       EXPRESSION OF IFG2

<130>  N114657A-EP

<150>  US 61/207,450
<151>  2009-02-11

<160>  27

<170>  PatentIn version 3.5

<210>  1
<211>  51
<212>  DNA
<213>  Artificial Sequence

<220>
<223>  IFG2 SNP polynucleotide sequence

<400>  1
ttccccctct ttgtttcttg gggcaktttt cctttttttt ttttttttgt t                51


<210>  2
<211>  51
<212>  DNA
<213>  Artificial Sequence

<220>
<223>  IFG2 SNP polynucleotide sequence


<220>
<221>  misc_feature
<222>  (26)..(26)
<223>  n = A,T,C or G

<400>  2
ggaccccaga aatcacaggt gggcangtcg ctgctaccgc catctccctt c                51


<210>  3
<211>  51
<212>  DNA
<213>  Artificial Sequence

<220>
<223>  IFG2 SNP polynucleotide sequence

<400>  3
gcgcacacac acgcacaccc ccacamaatt ggatgaaaac aataagcata t                51


<210>  4
<211>  51
<212>  DNA
<213>  Artificial Sequence

<220>
<223>  IFG2 SNP polynucleotide sequence

<400>  4
cattcccgat acaccttact tactgygtgt tggcccagcc agagtgagga a          51


<210>  5
<211>  51
<212>  DNA
<213>  Artificial Sequence

<220>
<223>  IFG2 SNP polynucleotide sequence

<400>  5
atacacctta cttactgtgt gttggyccag ccagagtgag gaaggagttt g          51


<210>  6
<211>  51
<212>  DNA
<213>  Artificial Sequence

<220>
<223>  IFG2 SNP polynucleotide sequence

<400>  6
atgccatagc agccaccacc gcggcrccta gggctgcggc agggactcgg c          51


<210>  7
<211>  51
<212>  DNA
<213>  Artificial Sequence

<220>
<223>  IFG2 SNP polynucleotide sequence

<400>  7
aaactgccgc aagtctgcag cccggygcca ccatcctgca gcctcctcct g          51


<210>  8
<211>  51
<212>  DNA
<213>  Artificial Sequence

<220>
<223>  IFG2 SNP polynucleotide sequence

<400>  8
ctgaaccagc aaagagaaaa gaaggrcccc agaaatcaca ggtgggcacg t          51


<210>  9
<211>  51
<212>  DNA
<213>  Artificial Sequence

<220>
<223>  IFG2 SNP polynucleotide sequence

<400>  9
cacacacgca cacccccaca aaattrgatg aaaacaataa gcatatctaa g                51

<210>  10
<211>  51
<212>  DNA
<213>  Artificial Sequence

<220>
<223>  IFG2 SNP polynucleotide sequence

<400>  10
ccaatgtttt catggtctga gccccsctcc tgttcccatc tccactgccc c                51

<210>  11
<211>  52
<212>  DNA
<213>  Artificial Sequence

<220>
<223>  IFG2 SNP polynucleotide sequence

<400>  11
acatttcttg gggggtcccc aggagayggg caaagatgat ccctaggtgt gc               52

<210>  12
<211>  51
<212>  DNA
<213>  Artificial Sequence

<220>
<223>  IFG2 SNP polynucleotide sequence

<400>  12
agtcctcggg ggccgtgcac tgatgygggg agtgtgggaa gtctggcggt t                51

<210>  13
<211>  51
<212>  DNA
<213>  Artificial Sequence

<220>
<223>  IFG2 SNP polynucleotide sequence

<400>  13
aggctggccg gaggggaagg ggctarcagg tgtgtaaaca gagggttcca t                51

<210>  14
<211>  51
<212>  DNA
<213>  Artificial Sequence

<220>
<223>  IFG2 SNP polynucleotide sequence

<400>  14
agtcgcagag ggtccctcgg caagcrccct gtgagtgggc cattcggaac a                51

<210>  15
<211>  51
<212>  DNA
<213>  Artificial Sequence

<220>
<223>  IFG2 SNP polynucleotide sequence

<400>  15
gtgttcccgg gggcacttgc cgaccrgccc cttgcgtccc caggtttgca g          51


<210>  16
<211>  51
<212>  DNA
<213>  Artificial Sequence

<220>
<223>  IFG2 SNP polynucleotide sequence

<400>  16
tgcggcccgt gtttgactca actcarctcc tttaacgcta atatttccgg c          51


<210>  17
<211>  5182
<212>  DNA
<213>  Homo sapiens

<400>  17
cgcctgtccc cctcccgagg cccgggctcg cgacggcaga gggctccgtc ggcccaaacc      60

gagctgggcg cccgcggtcc gggtgcagcc tccactccgc cccccagtca ccgcctcccc     120

cggcccctcg acgtggcgcc cttccctccg cttctctgtg ctccccgcgc ccctcttggc     180

gtctggcccc ggcccccgct ctttctcccg caaccttccc ttcgctccct cccgtccccc     240

ccagctccta gcctccgact ccctcccccc ctcacgcccg ccctctcgcc ttcgccgaac     300

caaagtggat taattacacg ctttctgttt ctctccgtgc tgttctctcc cgctgtgcgc     360

ctgcccgcct ctcgctgtcc tctctccccc tcgccctctc ttcggccccc ccctttcacg     420

ttcactctgt ctctcccact atctctgccc ccctctatcc ttgatacaac agctgacctc     480

atttcccgat accttttccc ccccgaaaag tacaacatct ggcccgcccc agcccgaaga     540

cagcccgtcc tccctggaca atcagacgaa ttctcccccc ccccccaaaa aaaagccatc     600

cccccgctct gccccgtcgc acattcggcc cccgcgactc ggccagagcg gcgctggcag     660

aggagtgtcc ggcaggaggg ccaacgcccg ctgttcggtt tgcgacacgc agcagggagg     720

tgggcggcag cgtcgccggc ttccagacac caatgggaat cccaatgggg aagtcgatgc     780

tggtgcttct caccttcttg gccttcgcct cgtgctgcat tgctgcttac cgccccagtg     840

agaccctgtg cggcggggag ctggtggaca ccctccagtt cgtctgtggg gaccgcggct     900

tctacttcag caggcccgca agccgtgtga gccgtcgcag ccgtggcatc gttgaggagt     960

gctgtttccg cagctgtgac ctggccctcc tggagacgta ctgtgctacc cccgccaagt 1020

ccgagaggga cgtgtcgacc cctccgaccg tgcttccgga caacttcccc agataccccg 1080

tgggcaagtt cttccaatat gacacctgga agcagtccac ccagcgcctg cgcaggggcc 1140

tgcctgccct cctgcgtgcc cgccggggtc acgtgctcgc caaggagctc gaggcgttca 1200

gggaggccaa acgtcaccgt cccctgattg ctctacccac ccaagacccc gcccacgggg 1260

gcgccccccc agagatggcc agcaatcgga agtgagcaaa actgccgcaa gtctgcagcc 1320

cggcgccacc atcctgcagc ctcctcctga ccacggacgt ttccatcagg ttccatcccg 1380

aaaatctctc ggttccacgt ccccctgggg cttctcctga cccagtcccc gtgccccgcc 1440

tccccgaaac aggctactct cctcggcccc ctccatcggg ctgaggaagc acagcagcat 1500

cttcaaacat gtacaaaatc gattggcttt aaacaccctt cacataccct ccccccaaat 1560

tatccccaat tatccccaca cataaaaaat caaaacatta aactaacccc cttccccccc 1620

ccccacaaca accctcttaa aactaattgg ctttttagaa acaccccaca aaagctcaga 1680

aattggcttt aaaaaaaaca accaccaaaa aaaatcaatt ggctaaaaaa aaaaagtatt 1740

aaaaacgaat tggctgagaa acaattggca aaataaagga atttggcact ccccaccccc 1800

ctctttctct tctcccttgg actttgagtc aaattggcct ggacttgagt ccctgaacca 1860

gcaaagagaa aagaaggacc ccagaaatca caggtgggca cgtcgctgct accgccatct 1920

cccttctcac gggaattttc agggtaaact ggccatccga aaatagcaac aacccagact 1980

ggctcctcac tcccttttcc atcactaaaa atcacagagc agtcagaggg acccagtaag 2040

accaaaggag gggaggacag agcatgaaaa ccaaaatcca tgcaaatgaa atgtaattgg 2100

cacgaccctc acccccaaat cttacatctc aattcccatc ctaaaaagca ctcatacttt 2160

atgcatcccc gcagctacac acacacaaca cacagcacac gcatgaacac agcacacaca 2220

cgagcacagc acacacacaa acgcacagca cacacagcac acagatgagc acacagcaca 2280

cacacaaacg cacagcacac acacgcacac acatgcacac acagcacaca aacgcacggc 2340

acacacacgc acacacatgc acacacagca cacacacaaa cgcacagcac acacaaacgc 2400

acagcacaca cgcacacaca gcacacacac gagcacacag cacacaaacg cacagcacac 2460

gcacacacat gcacacacag cacacacact agcacacagc acacacacaa agacacagca 2520

cacacatgca cacacagcac acacacgcga acacagcaca cacgaacaca gcacacacag 2580

cacacacaca aacacagcac acacatgcac acagcacacg cacacacagc acacacatga 2640

acacagcaca cagcacacac atgcacacac agcacacacg catgcacagc acacatgaac 2700

acagcacaca cacaaacaca cagcacacac atgcacacac agcacacaca ctcatgcgca 2760

gcacatacat gaacacagct cacagcacac aaacacgcag cacacacgtt gcacacgcaa 2820

gcacccacct gcacacacac atgcgcacac acacgcacac ccccacaaaa ttggatgaaa 2880

acaataagca tatctaagca actacgatat ctgtatggat caggccaaag tcccgctaag 2940

attctccaat gttttcatgg tctgagcccc gctcctgttc ccatctccac tgcccctcgg 3000

ccctgtctgt gccctgcctc tcagaggagg gggctcagat ggtgcggcct gagtgtgcgg 3060

ccggcggcat ttgggataca cccgtagggt gggcggggtg tgtcccaggc ctaattccat 3120

ctttccacca tgacagagat gcccttgtga ggctggcctc cttggcgcct gtccccacgg 3180

cccccgcagc gtgagccacg atgctcccca taccccaccc attcccgata caccttactt 3240

actgtgtgtt ggcccagcca gagtgaggaa ggagtttggc cacattggag atggcggtag 3300

ctgagcagac atgcccccac gagtagcctg actccctggt gtgctcctgg aaggaagatc 3360

ttggggaccc ccccaccgga gcacacctag ggatcatctt tgcccgtctc ctggggaccc 3420

cccaagaaat gtggagtcct cgggggccgt gcactgatgc ggggagtgtg ggaagtctgg 3480

cggttggagg ggtgggtggg gggcagtggg ggctgggcgg ggggagttct ggggtaggaa 3540

gtggtcccgg gagattttgg atggaaaagt caggaggatt gacagcagac ttgcagaatt 3600

acatagagaa attaggaacc cccaaatttc atgtcaattg atctattccc cctctttgtt 3660

tcttggggca tttttccttt tttttttttt tttgtttttt ttttacccct ccttagcttt 3720

atgcgctcag aaaccaaatt aaaccccccc cccatgtaac aggggggcag tgacaaaagc 3780

aagaacgcac gaagccagcc tggagaccac cacgtcctgc cccccgccat ttatcgccct 3840

gattggattt tgtttttcat ctgtccctgt tgcttgggtt gagttgaggg tggagcctcc 3900

tggggggcac tggccactga gcccccttgg agaagtcaga ggggagtgga gaaggccact 3960

gtccggcctg gcttctgggg acagtggctg gtccccagaa gtcctgaggg cggagggggg 4020

ggttgggcag ggtctcctca ggtgtcagga gggtgctcgg aggccacagg agggggctcc 4080

tggctggcct gaggctggcc ggaggggaag gggctagcag gtgtgtaaac agagggttcc 4140

atcaggctgg ggcagggtgg ccgccttccg cacacttgag gaaccctccc ctctccctcg 4200

gtgacatctt gcccgcccct cagcaccctg ccttgtctcc aggaggtccg aagctctgtg 4260

ggacctcttg ggggcaaggt ggggtgaggc cggggagtag ggaggtcagg cgggtctgag 4320

cccacagagc aggagagctg ccaggtctgc ccatcgacca ggttgcttgg gccccggagc 4380

ccacgggtct ggtgatgcca tagcagccac caccgcggcg cctagggctg cggcagggac 4440

tcggcctctg ggaggtttac ctcgccccca cttgtgcccc cagctcagcc cccctgcacg 4500

cagcccgact agcagtctag aggcctgagg cttctgggtc ctggtgacgg ggctggcatg 4560

accccggggg tcgtccatgc cagtccgcct cagtcgcaga gggtccctcg gcaagcgccc 4620

tgtgagtggg ccattcggaa cattggacag aagcccaaag agccaaattg tcacaattgt 4680

ggaacccaca ttggcctgag atccaaaacg cttcgaggca ccccaaatta cctgcccatt 4740

cgtcaggaca cccacccacc cagtgttata ttctgcctcg ccggagtggg tgttcccggg 4800

ggcacttgcc gaccagcccc ttgcgtcccc aggtttgcag ctctcccctg ggccactaac 4860

catcctggcc cgggctgcct gtctgacctc cgtgcctagt cgtggctctc catcttgtct 4920

cctccccgtg tccccaatgt cttcagtggg gggcccctc ttgggtcccc tcctctgcca 4980

tcacctgaag acccccacgc caaacactga atgtcacctg tgcctgccgc ctcggtccac 5040

cttgcggccc gtgtttgact caactcaact cctttaacgc taatatttcc ggcaaaatcc 5100

catgcttggg ttttgtcttt aaccttgtaa cgcttgcaat cccaataaag cattaaaagt 5160

catgaaaaaa aaaaaaaaaa aa 5182

<210> 18
<211> 5139
<212> DNA
<213> Homo sapiens

<400> 18
   ccgctaatgt accatgccct ggtgctggaa agtgcctgag ccagctgccc cagcggcctc 60
   agcactacca agttggcaca aagctcccca aattcggagg ggctcaggga aacgagtgga 120
   ggggatgagg aggtgagggg taaacccatc atttcagttg gcatttgagc aggtgccatg 180
   ctcagcggag atgaggctct cccatctgta ggggccgtat taacatgcac actctaaaag 240
   tgcccttcgt ttctccagcc tcagctttgt ccctctcctc ctccacgtca acctggccag 300
   agggtctgga cgccacagcc agggcacccc ctgctttggt ggtgactgct aatattggcc 360
   aggccggcgg atcatcgtcc aggcagtttc ggcagagagc cttgggcacc agtgactccc 420
   cggtcctctt tatccactgt ccaggagctg cggggactgc gcagggacta gagtacaggg 480
   gccgaagagt caccaccgag cttgtgtggg aggaggtgga ttccagcccc cagccccagg 540
   gctctgaatc gctgccagct cagccccctg cccagcctgc cccacagcct gagccccagc 600
   aggccagaga gcccagtcct gaggtgagct gctgtggcct gtggcccagg cgaccccagc 660
   gctcccagaa ctgaggctgg cagccagccc cagcctcagc cccaactgcg aggcagagag 720
   acaccaatgg gaatcccaat ggggaagtcg atgctggtgc ttctcacctt cttggccttc 780
   gcctcgtgct gcattgctgc ttaccgcccc agtgagaccc tgtgcggcgg ggagctggtg 840
   gacaccctcc agttcgtctg tggggaccgc ggcttctact tcagcaggcc cgcaagccgt 900
   gtgagccgtc gcagccgtgg catcgttgag gagtgctgtt tccgcagctg tgacctggcc 960
   ctcctggaga cgtactgtgc tacccccgcc aagtccgaga gggacgtgtc gacccctccg 1020
   accgtgcttc cggacaactt ccccagatac cccgtgggca agttcttcca atatgacacc 1080
   tggaagcagt ccacccagcg cctgcgcagg ggcctgcctg ccctcctgcg tgcccgccgg 1140
   ggtcacgtgc tcgccaagga gctcgaggcg ttcagggagg ccaaacgtca ccgtcccctg 1200
   attgctctac ccacccaaga ccccgcccac gggggcgccc ccccagagat ggccagcaat 1260
   cggaagtgag caaaactgcc gcaagtctgc agcccggcgc caccatcctg cagcctcctc 1320
   ctgaccacgg acgtttccat caggttccat cccgaaaatc tctcggttcc acgtccccct 1380
   ggggcttctc ctgacccagt ccccgtgccc cgcctccccg aaacaggcta ctctcctcgg 1440
   ccccctccat cgggctgagg aagcacagca gcatcttcaa acatgtacaa aatcgattgg 1500
   ctttaaacac ccttcacata ccctcccccc aaattatccc caattatccc cacacataaa 1560
   aaatcaaaac attaaactaa cccccttccc ccccccccac aacaaccctc ttaaaactaa 1620
   ttggcttttt agaaacaccc cacaaaagct cagaaattgg ctttaaaaaa aacaaccacc 1680
   aaaaaaaatc aattggctaa aaaaaaaaag tattaaaaac gaattggctg agaaacaatt 1740
   ggcaaaataa aggaatttgg cactccccac ccccctcttt ctcttctccc ttggactttg 1800
   agtcaaattg gcctggactt gagtccctga accagcaaag agaaaagaag gaccccagaa 1860
   atcacaggtg ggcacgtcgc tgctaccgcc atctcccttc tcacgggaat tttcagggta 1920
   aactggccat ccgaaaatag caacaaccca gactggctcc tcactccctt ttccatcact 1980
   aaaaatcaca gagcagtcag agggacccag taagaccaaa ggaggggagg acagagcatg 2040
   aaaaccaaaa tccatgcaaa tgaaatgtaa ttggcacgac cctcaccccc aaatcttaca 2100
   tctcaattcc catcctaaaa agcactcata ctttatgcat ccccgcagct acacacacac 2160
   aacacacagc acacgcatga acacagcaca cacacgagca cagcacacac acaaacgcac 2220
   agcacacaca gcacacagat gagcacacag cacacacaca aacgcacagc acacacacgc 2280
   acacacatgc acacacagca cacaaacgca cggcacacac acgcacacac atgcacacac 2340
   agcacacaca caaacgcaca gcacacacaa acgcacagca cacacgcaca cacagcacac 2400
   acacgagcac acagcacaca aacgcacagc acacgcacac acatgcacac acagcacaca 2460
   cactagcaca cagcacacac acaaagacac agcacacaca tgcacacaca gcacacacac 2520
   gcgaacacag cacacacgaa cacagcacac acagcacaca cacaaacaca gcacacacat 2580
   gcacacagca cacgcacaca cagcacacac atgaacacag cacacagcac acacatgcac 2640
   acacagcaca cacgcatgca cagcacacat gaacacagca cacacacaaa cacacagcac 2700
   acacatgcac acacagcaca cacactcatg cgcagcacat acatgaacac agctcacagc 2760
   acacaaacac gcagcacaca cgttgcacac gcaagcaccc acctgcacac acacatgcgc 2820
   acacacacgc acacccccac aaaattggat gaaaacaata agcatatcta agcaactacg 2880
   atatctgtat ggatcaggcc aaagtcccgc taagattctc caatgttttc atggtctgag 2940
   ccccgctcct gttcccatct ccactgcccc tcggccctgt ctgtgccctg cctctcagag 3000
   gagggggctc agatggtgcg gcctgagtgt gcggccggcg gcatttggga tacacccgta 3060
   gggtgggcgg ggtgtgtccc aggcctaatt ccatctttcc accatgacag agatgccctt 3120
   gtgaggctgg cctccttggc gcctgtcccc acggcccccg cagcgtgagc cacgatgctc 3180
   cccatacccc acccattccc gatacacctt acttactgtg tgttggccca gccagagtga 3240
   ggaaggagtt tggccacatt ggagatggcg gtagctgagc agacatgccc ccacgagtag 3300
   cctgactccc tggtgtgctc ctggaaggaa gatcttgggg acccccccac cggagcacac 3360
   ctagggatca tctttgcccg tctcctgggg accccccaag aaatgtggag tcctcggggg 3420
   ccgtgcactg atgcggggag tgtgggaagt ctggcggttg gaggggtggg tggggggcag 3480
   tgggggctgg gcggggggag ttctggggta ggaagtggtc ccgggagatt ttggatggaa 3540
   aagtcaggag gattgacagc agacttgcag aattacatag agaaattagg aacccccaaa 3600
   tttcatgtca attgatctat tccccctctt tgtttcttgg ggcatttttc cttttttttt 3660
   tttttttgtt ttttttttac ccctccttag ctttatgcgc tcagaaacca aattaaaccc 3720
   cccccccatg taacaggggg gcagtgacaa aagcaagaac gcacgaagcc agcctggaga 3780
   ccaccacgtc ctgccccccg ccatttatcg ccctgattgg attttgtttt tcatctgtcc 3840
   ctgttgcttg ggttgagttg agggtggagc ctcctggggg gcactggcca ctgagccccc 3900
   ttggagaagt cagaggggag tggagaaggc cactgtccgg cctggcttct ggggacagtg 3960
   gctggtcccc agaagtcctg agggcggagg ggggggttgg gcagggtctc ctcaggtgtc 4020
   aggagggtgc tcggaggcca caggaggggg ctcctggctg gcctgaggct ggccggaggg 4080
   gaaggggcta gcaggtgtgt aaacagaggg ttccatcagg ctggggcagg gtggccgcct 4140
   tccgcacact tgaggaaccc tcccctctcc ctcggtgaca tcttgcccgc ccctcagcac 4200
   cctgccttgt ctccaggagg tccgaagctc tgtgggacct cttgggggca aggtggggtg 4260
   aggccgggga gtagggaggt caggcgggtc tgagcccaca gagcaggaga gctgccaggt 4320
   ctgcccatcg accaggttgc ttgggccccg gagcccacgg gtctggtgat gccatagcag 4380
   ccaccaccgc ggcgcctagg gctgcggcag ggactcggcc tctgggaggt ttacctcgcc 4440
   cccacttgtg cccccagctc agcccccctg cacgcagccc gactagcagt ctagaggcct 4500
   gaggcttctg ggtcctggtg acggggctgg catgaccccg ggggtcgtcc atgccagtcc 4560
   gcctcagtcg cagagggtcc ctcggcaagc gccctgtgag tgggccattc ggaacattgg 4620
   acagaagccc aaagagccaa attgtcacaa ttgtggaacc cacattggcc tgagatccaa 4680
   aacgcttcga ggcaccccaa attacctgcc cattcgtcag gacacccacc cacccagtgt 4740
   tatattctgc ctcgccggag tgggtgttcc cgggggcact tgccgaccag ccccttgcgt 4800
   ccccaggttt gcagctctcc cctgggccac taaccatcct ggcccgggct gcctgtctga 4860
   cctccgtgcc tagtcgtggc tctccatctt gtctcctccc cgtgtcccca atgtcttcag 4920
   tggggggccc cctcttgggt cccctcctct gccatcacct gaagaccccc acgccaaaca 4980
   ctgaatgtca cctgtgcctg ccgcctcggt ccaccttgcg gcccgtgttt gactcaactc 5040
   aactccttta acgctaatat ttccggcaaa atcccatgct tgggttttgt ctttaacctt 5100
   gtaacgcttg caatcccaat aaagcattaa aagtcatga 5139

<210> 19
<211> 21692
<212> DNA
<213> Homo sapiens

<400> 19
   tctttgcctc ttcctgagtc tttgctagct tggggaagag gttagaaatt tccagtgtga 60
   tttagtccaa tctggtggat gcttctatat gaaccctcac ccagctgctg tcagctttga 120
   gtctgtgctt taattagaaa ggctgcgaac ctgtccaatc gcccaatcca gaaatgcctg 180
   caagtgcccc ctgcagcagt gtcattagag cttagtacca cacagggagt gtctgaggtc 240
   agatcagacg tgaccagtga agtgaaaaac cccccagctg agtaccttgc tcatggaaga 300
   tggaaggaca taactgcaca gtacataact gtaccgggag cacctaaaat ccaatttttc 360
   tgctgattct tgaaccctgc tttgtccccc ttattccccc cccccccgcc attttaccag 420
   tgccacgtcc accaacattc cagggtgtca agtaactgcc aagtgtcact ctaagtaaag 480
   ctacacccac tccccaccac ctccacatag cccccacctc ctagctggca gggagcttct 540
   ggcttatgcc cacgcccaca ggcgcctttc tgccaggtca ggggtgggcc aaacctccac 600
   ccgctaatgt accatgccct ggtgctggaa agtgcctgag ccagctgccc cagcggcctc 660
   agcactacca agttggcaca aagctcccca aattcggagg ggctcaggga aacgagtgga 720
   ggggatgagg aggtgagggg taaacccatc atttcagttg gcatttgagc aggtgccatg 780
   ctcagcggag atgaggctct cccatctgta ggggccgtat taacatgcac actctaaaag 840
   tgcccttcgt ttctccagcc tcagctttgt ccctctcctc ctccacgtca acctggccag 900
   agggtctgga cgccacagcc agggcacccc ctgctttggt ggtgactgct aatattggcc 960
   aggccggcgg atcatcgtcc aggcagtttc ggcagagagc cttgggcacc agtgactccc 1020
   cggtcctctt tatccactgt ccaggagctg cggggactgc gcagggacta gagtacaggt 1080
   aactgggctc ccatcccttg agtgtgagag aaaagctgca ctttaagtgt tcaggcgtgg 1140
   gtgggccggg agcttggggc agctgccagg atcctggttt ctgaaggagg ggaagaactt 1200
   ctgctgctgg agggtgcagg gaagcctcct gagagcagcc tcaacttcag ggatggggtg 1260
   tgcaggaaag gccattgtgg agagggttct cctttagggc tgcacaaagc cactgaggct 1320
   tttgcaagga aaataggttt tccttgacta attcaccaag caaaatggga ggggtagggg 1380
   aggagggcta ggccgctctt cccagcggga acacacagct gtcttcacaa gtgtgaaagg 1440
   aagagtcttt ctgtgtgaaa agtttcctcc cgttgcatcc cccatcccat tcccagagac 1500
   aaacaggaga ctttgcagag gagccagggg cccgagattc tggcgcagag atttatttta 1560
   tacatatata caccatttta caggtaaagc ttccttccct cctgcctccc tatgcctcct 1620
   gaccaccagc aagaaattgg acaggagact gaggagaaat gccgggagag gcaacaaccg 1680
   ccctccatgt ccccctaggg tttagcttct ctcctcctga tggcgcacct ggtccccctt 1740
   gctgctctcc cagcctccct ggcacagaga ggcgccctgg ggccaaggca gtttccctgg 1800
   gaatgctcat tcatgcatga agtttttctc tgttgcaccc tggacccaga ctcctcgatc 1860
   cacccagggt ggtgtctgtg gggagggggt tcatttcccc aggaagcaca gccacgccgt 1920
   ccctcactgg cctcgtcaag cagagctgtg tgtccagtgg cttttgctgg ggccccctcc 1980
   ttatctcctt ccaaggtggg ggtgtttgga ggtggaggag gctttcatat tccgtgccat 2040
   gacccctcaa ggcgggccat tcgtgtgcac cctccacccc cagtgccagg cagaagccca 2100
   tcctcaccca ggaacagggc agcctgtcca acagaagggt ctcggcctct ccatcagcac 2160
   cgggaagccc tttctaggca aacttctcac cacttcttcc ctcccttata ctttgaaaga 2220
   gggagctcta ggcaggggag gggctagagg gggaagccgc tgcccagatc ctgacaaggt 2280
   gacctgaagg aacccgggga gggggatggg acagggctca ggcttggggt gtatggggag 2340
   gggggctttg cttttaaaag aggtcatctc agcaatatct tttgtttttt ccccaggggc 2400
   cgaagagtca ccaccgagct tgtgtgggag gaggtggatt ccagccccca gccccagggc 2460
   tctgaatcgc tgccagctca gccccctgcc cagcctgccc cacagcctga gccccagcag 2520
   gccagagagc ccagtcctga ggtgagctgc tgtggcctgt ggcccaggcg accccagcgc 2580
   tcccagaact gaggctggca gccagcccca gcctcagccc caactgcgag gcagagaggt 2640
   gagtgtctca ggcaccctga ggcctggcag agagggccac aggctctgcg cgggagtctt 2700
   cgaactggga tctccccctt ctgcaagcag ctttggctca gagaggctgg cgtggattca 2760
   gtcacacagc tgggatctgg agttccgtgg ttggctccag gtgcttccgt ctaggggcca 2820
   gagcaggtgt gggcagagca ggttccccgc agtctccacg gcaccgaggt cctggcaggg 2880
   gagctcctgg gagacgaaag agggcaaaga aggggagagg ggcagggaga gagcgggcag 2940
   ccaaagggga gaagatgggg ggcagaaagt gggtagagag ggaaaaaggg aaaatatcat 3000
   tggggaagaa cctaaaaacc caaggaaagc tgggctctgc tgggggctgt gagacccccg 3060
   ggttctcccc gccccaggct gctggccatg gggtcttgca ccaatggcct gacctttctg 3120
   tcggtctgta tttatcaaag tgggtgacag tctcaggcct cctggctgtt cagaattgag 3180
   gtaataacca gaggccttct gagcaaaggg cctaaggggc tccggcgtca ggatcccatt 3240
   gtggtcagga gcctgcgggg cttcccgtgt gcaagagggg tgaaaggtgg ctagaaaggc 3300
   ccagccagtg gcctctgcct cagccagagg gagctctgta gtgggggcag cacccattca 3360
   ctggtcaggc actggggtga caggggaggc tccaggactt ggggagcgtt ggagctggag 3420
   gcacatggat tggagtccct gtacctgccc cacgacaggg cctgcaggga gggatccagc 3480
   aggtgactct tcaggctgat ttgcccatcc cagatagaag ccgggagtgt tctttcaaag 3540
   gtgtctttac cttagacact caataaaatg gtaacacagt ggcgccgcct cagtcctttg 3600
   gagtgtgcac cgtctgaacc cctctcccag ggccctctcc caagcacccc aacctggacc 3660
   catatccccc acgtactttt ggctttgggc agattgagca gccttggggt ggtctgtgct 3720
   gtctggtgtg gagggttgca gttcgggtcc ttagtcctac ttcccaggcc ggccgggctg 3780
   acgccagcga gtgtgtcctt ccccagcgag gggagtgagc gcaaggtcag cgcctcgtct 3840
   gcggcgccct gcagggggtg acggaggggc gctctgagga cccttggaga aaggagctgg 3900
   gtttgtaaaa tgctgggctt ggtcccacgg acggcggagc ggtgagctca gagccagagc 3960
   tggggaggaa atgggaatga gaaaggccca cttcagggct ggtgagcgag gggatgggga 4020
   gcagccacag gccgaggctg gggcatgggc caggctccat ggggtgagtc tgagtccttg 4080
   aggggatgtt catcctctgt ggaatgtggg tttgccagtg gagaggagac cagcgttgcc 4140
   ctggtgaggt gctggttcag ggctgggggg cggacgctgc ttggggctaa agttcctgcc 4200
   ggccaagctc tgggtgggag gagaccctgg ccccctccca acacccttgg actgctggcg 4260
   ggacccttcc tacctccggg ggctggaagt agtgggggag gagccagtct tgaggaagaa 4320
   ccccgatgct ggtcttgact agaggggagc cggtgtgctt ttcgagcctc agggtgaccc 4380
   gcgtctgccc cagcctccag cctgccctgg tcacttctga ctaaataagg agagcactca 4440
   gcaggcagcc ccacgaggga gggggaacat gtgtgcaccc ccactccccc acctgctcct 4500
   ccctccctac agggccacta caccctgctg tgggcacccc aaggtgaccc tcagccttct 4560
   tcctacctta aaaagtccag gcatgcattt tcaagcatga gcggtggccc cctgggggaa 4620
   ggcacctcgg cagggcagaa caaagggaag ggacccccaa acaggtcact ggtgtaattg 4680
   tccccagcac ccccaaagag gaggagaacc cacaactcgg aactggggct cacccccgat 4740
   gcccaacctg tccccagcct gggaagcagg cgtggaggag aaggtggggg gagcctagag 4800
   ctggccctgg gggccctggt tttgtccatg acgggagcct cggcaaccta gtccgctctc 4860
   ccggggacca ggtttgcaga caggcacctt tcaaatgctc ctcaccccca aatttacaag 4920
   tcaccctgca gaggaaaaca tcaacacagc caggggttct ctgctggagg ctcccccttc 4980
   tataggcaca gccggagagg ccagagagct ggggacacgg ggaggctgca gaaggctggt 5040
   gggaaggggg gcagtgatgg gtggggagag atgggccaga tgttcttgga atgggacatg 5100
   ggggtgattg atgcagacag aaatttgaag gggacattcc cacgtgtctt gttctgtggg 5160
   tggaaaatgg gctgtttttc atggtggggg cgggttctcc ctgtcttgcc aagctaatgt 5220
   gaaagagatg cctcatcctg cccagctccc cacacctgtc caaggccatt aacttctgcc 5280
   tccccagtgt caggctttga gatgcccccc ttctagccgg ggtcctccta tggggtgaca 5340
   atggggacaa gcaatgccca ctgtagttgc cccaggatcc cccaccattc tgctggtccc 5400
   cagcggtgcc ccctctctgg cagtaccccc acccacccca caggtcccct tagggccact 5460
   gccccatcgc ccgacattgc ccaacgccaa ggggtgacct tgttcctgcc gacagggccg 5520
   ttgggcgcct gcatgcgggt ttaatatttg cctataagga actgggcttt ccccagccgg 5580
   agtggacaga ctttccctga aaattcgctt ggagagaacg aaaagagacc cctggcaccc 5640
   cagcggcgtg cagccctgca cccccctcct cccgggcccc gtgtttctca ttttcctccc 5700
   cacttcctct gctcttcagt gttacccaaa caaaactggt ttcacccttg tttggtgctg 5760
   gcgaaggccc gaacggcgcg cgcaaagctc cggggcaggc cggaggtggc caccgggggt 5820
   gctccgggcc cccaagccaa gccggggact agcctgcccc cggtggcggc tcggccgcgg 5880
   cttcgcctag gctcgcagcg cggaggcgag tggggcgcag tggcgagggg gagcctgcgg 5940
   acctcccacg cggggaccga gcaggtatct gggagtcccg ggagcgcccg ggaagcagcg 6000
   tcctggtcgc tccctcgcgg cccttgggtt tcttccttac acccggacgc ccgctaagct 6060
   cgggctgccg ccacaaacgc gctctccgtg tggagaaggc aaagaaaaaa aaaaataaaa 6120
   gcaaaaggaa gaaaaacccc aaagaacgaa aagcagaatt tcagccggcc gtgcgcgcca 6180
   gggcgctccg cgctacctgc ccgcgccgcc cgcgctcggg ttcccgggga gggcgccagt 6240
   gctccgcgcg cgccccagcc aaggtgaatc cccggcagcg ccttccttcc gctgcccggg 6300
   aagcttgagc tcaacaatta gcccttgatc ctcgggggat tccaatccac ggaacaactt 6360
   ccctgctttc cccgaactcg gacattttac tttttctggg atcctctaaa tttaagcatt 6420
   gcttcccaag tcttctaaat atactcacca tttcgacggg tcacaataat tttcttggac 6480
   gttaatttcc ggggacgtca aaacacatca gtcccggcgg gcttttccag acttacacta 6540
   tgtggcctgg ggccccagat gtgctttctc ccaggctctg gacagtttta tacaccccct 6600
   ccaggtccca cagatttaca ggccactaac ccggcttccc taattttaaa gacgaagccc 6660
   ttggtccgtg gtggtcgcgc tgaccaattt gcctggctcc ccaggatgtg gacagtgcct 6720
   tttccacatt taggcattgt ttccaaaaca agtggaactt tcccgcataa ttttgaatat 6780
   taactccagg gtctcctaag cttactgttt ccggcacacg tccgccccat tccgcgcccc 6840
   cccaccccac ccccgcgccc cttcccgttc acctcagcat gggacatttg ctgttgggtc 6900
   ccgcaaatct attcacacta acctgggttc cctaaacttt acacgttgaa tcccaagtcc 6960
   ctcatgacac tcagcagggc tggaaggttg gaaccctcag agtatgaaaa ttgcttccca 7020
   taccttcccc caaattcggt cattacaccc agagcatgtt gaatcctttt ctgaatcata 7080
   aacgacctgc cctctgattg cctgagtttc ataaaatgga gggatttccc cagtgattcc 7140
   ccaaagctat tgagaatgtt gtgggtgtgt gagtcacaaa gctttggggc attaacgtct 7200
   atggctctat atgctgcctg gccacgaatc aggtccctta agatgtagac agtgccaccc 7260
   aggtccctgg agcacactga gcagttacca ggaggtgctc aagtgtgacc caggattctc 7320
   caggtccccc caaatcacac agggtctccc gggtccctct gggctacacc aagcacaaaa 7380
   ggaccccttg ggcagagcct acttttattt ctgttatgcc aggtgttgct aacggcccca 7440
   gttcccaaac attccgagca ctttctctgc atcacaacat attgactatt aaacaattct 7500
   ctgggtccca ggagcttcat aacaagaatc ttgcttttct aaaattcagg cattggtctg 7560
   aaaccccaac ggccaggatc acactggacc cttttcctgg tcccccatac ttggatgttc 7620
   tggacgctgc cctccaggcc ctctaggaac attcagcatt gccctcggat cacaggacag 7680
   cactgatttc ttgggctcca aacaacccac tgagtcatct caaagttaag caatatttcc 7740
   ttcaagcaca ctgcacactc cctatcacaa aatctgaaaa ttcctaagtc ctaagaccta 7800
   ggaattctga atcccctttc tttaaaatgt acatatggac ccccaagtcc tccaaggact 7860
   ctgagcaact tccctagatc tttagattca aaaacgattt tcctggagcc cccaaattgc 7920
   ggtattgtct cccagccttc caaagcaaat tgagattttt ttcccttcac aaaacaattg 7980
   aggttttttt ttttttaata ctgatttatg agtctcctga ctttatggtc cctgccctgg 8040
   gtccccctac atttagaaaa tgttccatgg acccccaaag cacactaaaa aatgtccctg 8100
   ggtcccaaga aatcccaggc atggaaaaac ctgcgaccta taagtttcct agctactaac 8160
   taggtttcca gaaatttaga tatcaaatct ccattgggta atttccatgt gtcccaaaaa 8220
   cttgaaatgt gtttcactgg ggctccccca aatgcagacg acatccagga aaatatatag 8280
   tctttttctt atttaccaaa aataagctaa tggaaatcat ttaaaaatta gcatagaaaa 8340
   ataatactga ttttttattt ttttattttt tattttgctt tccccaaatg tactgatcac 8400
   actccaggct cccccaaaat ctagacagtg ctttcttcca tctctgaagg gtgttaaaac 8460
   ctttccctga agccacagta attatgaagg ttattttttc cccggctgct gccagcgtcc 8520
   aggccactaa cttatattct taagatgtga aaattaatct cagcttcccc ctaacacacc 8580
   aagaatgtgt ttggatcccc aaaatgtgtt ccttgctttc atctgccaat tttacgtaat 8640
   atggctctac ggcaaaattc ccaatttcat atggagaatt ttctttaact acccctcctc 8700
   acaaattggt cccccaagct agctggcccc tatttgagac ctctttctct atgttcccaa 8760
   ttgcatggag caacttctct catcccccaa acctgtaatc tatttttctg gagtctcgag 8820
   tttagtcatt aatcacggtt cccacattaa cggagtcccc ggggtcccct cctccaggac 8880
   acccattcgc taagcccgca aggcagaaag aactctgcct tgcgttcccc aaaatttggg 8940
   cattgttccc ggctcgccgg ccacccactg cagcttcccc aaccccgcgc acagcgggca 9000
   ctggtttcgg gcctctctgt ctcctacgaa gtccccagag caactcggat ttgggaaatt 9060
   tctctctagc gttgcccaaa cacacttggg tcggccgcgc gccctcagga cgtggacagg 9120
   gagggcttcc ccgtgtccag gaaagcgacc gggcattgcc cccagtctcc cccaaatttg 9180
   ggcattgtcc ccgggtcttc caacggactg ggcgttgctc ccggacactg aggactggcc 9240
   ccggggtctc gctcaccttc agcagcgtcc accgcctgcc acagagcgtt cgatcgctcg 9300
   ctgcctgagc tcctggtgcg cccgcggacg cagcctccag cttcgcggtg agctccccgc 9360
   cgcgccgatc ccctccgcct ctgcgcccct gaccggctct cggcccgcat ctgctgctgt 9420
   cccgccggtg ctggcgctcg tctccggctg ccgccgggga ggccggcgtg gggcgcggga 9480
   cacggctgcg gacttgcggc tggcggctgc gctcgctcct gctgggcgcc ccgaaatccg 9540
   cgccactttc gtttgctcat tgcaaagatc tcatttgtgg ggaaagcggc tggagggtcc 9600
   caaagtgggg cgggcagggg gctggggcga gggacgcgga ggagaggcgc tcccgccggg 9660
   cggtaaagtg cctctagccc gcgggcctag gactccgccg ggaggcgcgc gcggagcgcg 9720
   ggcgaagtga ttgatggcgg agcgaggggg gcgagggggg ccaggggggc gcgagattcc 9780
   gccggcggcc ccttcccctt ggctaggctt aggcggcggg gggctggcgg ggtgcgggat 9840
   tttgtgcgtg gtttttgact tggtaaaaat cacagtgctt tcttacatcg ttcaaactct 9900
   ccaggagatg gtttccccag acccccaaat tatcgtggtg gcccccgaga ccgaactcgc 9960
   gtctatgcaa gtccaacgca ctgaggacgg ggtaaccatt atccagatat tttgggtggg 10020
   ccgcaaaggc gagctactta gacgcacccc ggtgagctcg gccatgcagg taggatttga 10080
   gctgtgtttc ccgccctgat cctctctcct ctggcggccg gagcctccgt aggctccaag 10140
   cctggcccag attcggcccg gcgcagccgg ccttccgcgc gtcccgcacc tggcgggggc 10200
   tccggggctc cggcgcggca ccggggggcg ctcgggatct ggctgaggct ccaagcgccg 10260
   cgtggccggc tcctcctgct ggggcaggtg gcggctgcgc gccccgcccg agcccagggg 10320
   ccccctcagc cgcaacaacc agcaaggacc ccccgactca gccccaagcc acctgcatct 10380
   gcactcagac ggggcgcacc cgcagtgcag cctcctggtg gggcgctggg agcccgcctg 10440
   cccctgcctg cccggagacc ccagctcacg agcacaggcc gcccgggcac cccagaaacc 10500
   cgggatgggg cccctgaatt ctctagaacg ggcattcagc atggccttgg cgctctgcgg 10560
   ctccctgccc cccacccagc ctcgcccccg cgcacccccc agcccctgcg accgccgccc 10620
   cccccccggg gccccagggc ccccagcccg caccccccgc cccgctcttg gctcgggttg 10680
   cgggggcggg ccgggggcgg ggcgagggct ccgcgggcgc ccattggcgc gggcgcgagg 10740
   ccagcgaggc ccgcgcgggc cctgggccgc gggctggcgc gactataaga gccgggcgtg 10800
   ggcgcccgca gttcgcctgc tctccggcgg agctgcgtga ggcccggccg gccccggccc 10860
   cccccttccg gccgcccccg cctcctggcc cacgcctgcc cgcgctctgc ccaccagcgc 10920
   ctccatcggg caaggcggcc ccgcgtcgac gccgcccgct gcctcgctgc tgactcccgt 10980
   cccgggcgcc gtccgcgggg tcgcgctccg ccgggcctgc ggattccccg ccgcctcctc 11040
   ttcatctacc tcaactcccc ccatccccgc ttcgcccgag gaggcggttc cccccgcagg 11100
   cagtccggct cgcaggccgc cggcgttgtc accccccccc gcgctccccc tccagccctc 11160
   cccccggcgc gcagcctcgg gccgctcccc tttccgcgct gcgtcccgga gcggccccgg 11220
   tgccgccacc gcctgtcccc ctcccgaggc ccgggctcgc gacggcagag ggctccgtcg 11280
   gcccaaaccg agctgggcgc ccgcggtccg ggtgcagcct ccactccgcc ccccagtcac 11340
   cgcctccccc ggcccctcga cgtggcgccc ttccctccgc ttctctgtgc tccccgcgcc 11400
   cctcttggcg tctggccccg gcccccgctc tttctcccgc aaccttccct tcgctccctc 11460
   ccgtcccccc cagctcctag cctccgactc cctccccccc tcacgcccgc cctctcgcct 11520
   tcgccgaacc aaagtggatt aattacacgc tttctgtttc tctccgtgct gttctctccc 11580
   gctgtgcgcc tgcccgcctc tcgctgtcct ctctccccct cgccctctct tcggcccccc 11640
   cctttcacgt tcactctgtc tctcccacta tctctgcccc cctctatcct tgatacaaca 11700
   gctgacctca tttcccgata ccttttcccc cccgaaaagt acaacatctg gcccgcccca 11760
   gcccgaagac agcccgtcct ccctggacaa tcagacgaat tctccccccc cccccaaaaa 11820
   aaagccatcc ccccgctctg ccccgtcgca cattcggccc ccgcgactcg gccagagcgg 11880
   cgctggcaga ggagtgtccg gcaggagggc caacgcccgc tgttcggttt gcgacacgca 11940
   gcagggaggt gggcggcagc gtcgccggct tccaggtaag cggcgtgtgc gggccgggcc 12000
   ggggccgggg ctggggcggc gcgggcttgc gccggacgcc cggcccttcc tccgcccgct 12060
   cccggcccgg ggcctgcggg gctcggcggg gcggctgagc ccggggggga ggaggaggag 12120
   gaggaggagg acggacggct gcgggtcccg ttccctgcgc ggagccccgc gctcaccctg 12180
   gcggcggagc tgggggtggg gtgggggcgt cgggaagggc cgagggaggt gtgaggtgtc 12240
   tgcaggggcg acttcccggt cggtctgtgg gtgcaggggg tgccgcctca catgtgtgat 12300
   tcgtgccttg cgggccctgg cctccggggt gctgggtaac gaggaggggc gcggagccgc 12360
   agaagcccac cctggtatgt tgacgcggtg ccagcgagac cgcgagagga agacgggggt 12420
   gggcggggcc aggatggaga ggggccgagt tggcaggagt catggcagac gccacattcg 12480
   cgacatctcc cccacacccc ctctggctct gtccgcaaca tttccaaaca ggagtcccgg 12540
   gagaggggga gaggggctgc tggtctgagg ctaagaaggg cagagccttc gacccggaga 12600
   gaggccgcgg cccctgccca gtgggcagcg tggaagtttc catacaagga ggtgggaagg 12660
   agaccccccc ccccttcact gccctgtgca gagatgagcc gggggtgcag gatgggagcc 12720
   catggcactt cgctacggga tggtccaggg ctcccggttg ggggtgcagg agagaagaga 12780
   ctggctggga ggagggagag ggcgggagca aaggcgcggg ggagtggtca gcagggagag 12840
   gggtgggggg tagggtggag cccgggctgg gaggagtcgg ctcacacata aaagctgagg 12900
   cactgaccag cctgcaaact ggacattagc ttctcctgtg aaagagactt ccagcttcct 12960
   cctcctcctc ttcctcctcc tcctcctgcc ccagcgagcc ttctgctgag ctgtaggtaa 13020
   ccagggctgt ggagtgagga cccccgctgc catcccactc cagcctgagg cagggcagca 13080
   gggggcacgg cccacgcctg ggcctcgggc cctgcagccg ccagcccgct gcctctcgga 13140
   cagcaccccc ctcccctctt ttcctctgcc cctgccccca cctggtctct gctccctcac 13200
   ctgctccttc cctttctgtt ccttcccttc ggccccctcc ttgcccagct caggactttt 13260
   cctgggccct cacctgctcc gcaccgctgc atgcttcctg tcctgctttc tgccggtccc 13320
   ctgacccgga cctccaagtt cagagtggtg gggcttgttg cggaagcgcg gcgagggcta 13380
   gagtggccag ctggcggagt gtgctcttag aatttggaag ggggtggcag agggggcggt 13440
   gagaggactg gccagggtcc agtcaaggag atgaccaagg aggctttcag atcctcggcg 13500
   cagctgccca ctagtcttta gagagggcat gcaaagttgt gcttctgtcc cactgcctgc 13560
   tcagtcgctc acataattta ttgcatcaaa aactcccctg ggtctgcgga gcgaaggctg 13620
   gggctgcccg cctggagggt tccaccttct gcaggggcag ggccaacttg ctgtggtggc 13680
   tcccggcctc ccacccccga gtgggttaac ccggccctgt ggccctgcag cctgtggagg 13740
   gggtgtgtcc taagactggc ctccccttcc agattgtagt ctggggaacc tggtgtcgga 13800
   cttcccaggt ggcctgagct ggtctctcca gctcccacgg ggagagtttg gtagcgcaaa 13860
   tagggagatg ttctggaggc ccctggcctt actggttcga tttgaggcct ggaaaggagg 13920
   ctctgggcgt gtgtgtgtgt gtttgggggt acccaaggca gactggagtt ggagaactgg 13980
   gtgactggga aaacaaggtt tctagagcat gggtggcgtg gttgtgttaa ccattggagt 14040
   ccttgaccca ggcctggctc agctgcagac tggaaaggtg gaaaagccag gggggagggc 14100
   ggggctggcc cagcaggact ggcctgctgc tttgaggcgg atggtcctcc tggacccccc 14160
   ctgctcagct gggggttgtg ggaggaaggg gactggtcct cctggatgca catgctctgt 14220
   aggggtgggg ctgtctgcca tcttggctgg cgctggaggc ctgagaagtg gcgatgtgac 14280
   gctgggctgg ccctgccccc atggtgtcat aggacggagg ccaggtcggg tgtccagcct 14340
   gggcccctgc agctgtggat gccgctgagc tcctgcaata atgaccgtgg agatggtcac 14400
   ccctcgtgta aaattactag tgcttcttgc aaatggaagg aactgggcct tttctgtgtg 14460
   cttctggacg cttcattctg cacatggccc tgcgccctca cctcggcatt atgacctgtg 14520
   tgttactttt gtaataaaaa taatgtttat aggaaagccg tgctttcaat tttcaactga 14580
   atttgtaggt tggcaaattt ggtttgggag gggcacctct ggcctggggc ttggcctggc 14640
   tgccccgctc acgccacttc tctcccgccc ccagacacca atgggaatcc caatggggaa 14700
   gtcgatgctg gtgcttctca ccttcttggc cttcgcctcg tgctgcattg ctgcttaccg 14760
   ccccagtgag accctgtgcg gcggggagct ggtggacacc ctccagttcg tctgtgggga 14820
   ccgcggcttc tacttcagta agtagctggg aggggcttcc tcagacctgg tcaggcccct 14880
   agagtgaccg gtgaggacgc ccaacctcaa gccaggggag cacactccta ggtcagcagc 14940
   ccagccgctt gctctgagac tttgaccttc ccgccgcgtt tctgagcacg tgcggtgtcc 15000
   cagggcatcc acaccagctg cctttcccat cacacgcctc cttcgaaggg tgggccagag 15060
   gtgcccccta gacgtcaggg gcactcacag gggtctccct gggcatcaga atttctgttg 15120
   ggggccgtga ggctcctgct cctgaggcac cgcacgccta gtgcagggct tcaggctctg 15180
   gaggaagagc ctgcctttct tcctgcacct tttggacatt ctgacaaggg acgtgcgttc 15240
   ggtgaatgat cagaattaaa atcaataaag tgatttatat aattaaaatc aataagacaa 15300
   gtgcagttgg tgggtggcag gggtgagcgg tgcatgcgcc tccttgggcc ccaaggctgc 15360
   cgtggggggt gcccacctgc tgacctcaag gacgcttcag cctttcctca tgtttctctc 15420
   ttggttctcc agcctggggg ctggcaggtg ggtgcatggc ccattgtcct tgagacccca 15480
   cccccagata ggggggctgg gtggatgcag aggcaggcat ggtgcctggg catgcctgat 15540
   ggggcagggg aggggccgct ccttactggc agaggccgca cggcttattc cacctgacac 15600
   tcaccacgtg acatctttac caccactgct tactcacgct gtgaaatggg ctcacaggat 15660
   gcaaatgcac ttcaaagctt ctctctgaaa agttcctgct gcttgactct ggaagcccct 15720
   gcccgccctg gcctctcctg tgccctctct cttgcctgcc ccatttgggg gtaggaagtg 15780
   gcactgcagg gcctggtgcc agccagtcct tgcccaggga gaagcttccc tgcaccaggc 15840
   tttcctgaga ggaggggagg gccaagcccc cacttggggg acccccgtga tggggctcct 15900
   gctccctcct ccggctgatg gcacctgccc tttggcaccc caaggtggag cccccagcga 15960
   ccttcccctt ccagctgagc attgctgtgg gggagagggg gaagacggga ggaaagaagg 16020
   gagtggttcc atcacgcctc ctcactcctc tcctcccgtc ttctcctctc ctgcccttgt 16080
   ctccctgtct cagcagctcc aggggtggtg tgggcccctc cagcctccta ggtggtgcca 16140
   ggccagagtc caagctcagg gacagcagtc cctcctgtgg gggcccctga actgggctca 16200
   catcccacac attttccaaa ccactcccat tgtgagcctt tggtcctggt ggtgtccctc 16260
   tggttgtggg accaagagct tgtgcccatt tttcatctga ggaaggaggc agcagaggcc 16320
   acgggctggt ctgggtccca ctcacctccc ctctcacctc tcttcttcct gggacgcctc 16380
   tgcctgccag ctctcacttc cctcccctga cccgcagggt ggctgcgtcc ttccagggcc 16440
   tggcctgagg gcaggggtgg tttgctcccc cttcagcctc cgggggctgg ggtcagtgcg 16500
   gtgctaacac ggctctctct gtgctgtggg acttccaggc aggcccgcaa gccgtgtgag 16560
   ccgtcgcagc cgtggcatcg ttgaggagtg ctgtttccgc agctgtgacc tggccctcct 16620
   ggagacgtac tgtgctaccc ccgccaagtc cgagagggac gtgtcgaccc ctccgaccgt 16680
   gcttccggtg agggtcctgg gcccctttcc cactctctag agacagagaa atagggcttc 16740
   gggcgcccag cgtttcctgt ggcctctggg acctcttggc cagggacaag gacccgtgac 16800
   ttccttgctt gctgtgtggc ccgggagcag ctcagacgct ggctccttct gtccctctgc 16860
   ccgtggacat tagctcaagt cactgatcag tcacaggggt ggcctgtcag gtcaggcggg 16920
   cggctcaggc ggaagagcgt ggagagcagg cacctgctga ccagcccctt cccctcccag 16980
   gacaacttcc ccagataccc cgtgggcaag ttcttccaat atgacacctg gaagcagtcc 17040
   acccagcgcc tgcgcagggg cctgcctgcc ctcctgcgtg cccgccgggg tcacgtgctc 17100
   gccaaggagc tcgaggcgtt cagggaggcc aaacgtcacc gtcccctgat tgctctaccc 17160
   acccaagacc ccgcccacgg gggcgccccc ccagagatgg ccagcaatcg gaagtgagca 17220
   aaactgccgc aagtctgcag cccggcgcca ccatcctgca gcctcctcct gaccacggac 17280
   gtttccatca ggttccatcc cgaaaatctc tcggttccac gtccccctgg ggcttctcct 17340
   gacccagtcc ccgtgccccg cctccccgaa acaggctact ctcctcggcc ccctccatcg 17400
   ggctgaggaa gcacagcagc atcttcaaac atgtacaaaa tcgattggct ttaaacaccc 17460
   ttcacatacc ctccccccaa attatcccca attatcccca cacataaaaa atcaaaacat 17520
   taaactaacc cccttccccc ccccccacaa caaccctctt aaaactaatt ggctttttag 17580
   aaacacccca caaaagctca gaaattggct ttaaaaaaaa caaccaccaa aaaaaatcaa 17640
   ttggctaaaa aaaaaaagta ttaaaaacga attggctgag aaacaattgg caaaataaag 17700
   gaatttggca ctccccaccc ccctctttct cttctccctt ggactttgag tcaaattggc 17760
   ctggacttga gtccctgaac cagcaaagag aaaagaagga ccccagaaat cacaggtggg 17820
   cacgtcgctg ctaccgccat ctcccttctc acgggaattt tcagggtaaa ctggccatcc 17880
   gaaaatagca acaacccaga ctggctcctc actccctttt ccatcactaa aaatcacaga 17940
   gcagtcagag ggacccagta agaccaaagg aggggaggac agagcatgaa aaccaaaatc 18000
   catgcaaatg aaatgtaatt ggcacgaccc tcacccccaa atcttacatc tcaattccca 18060
   tcctaaaaag cactcatact ttatgcatcc ccgcagctac acacacacaa cacacagcac 18120
   acgcatgaac acagcacaca cacgagcaca gcacacacac aaacgcacag cacacacagc 18180
   acacagatga gcacacagca cacacacaaa cgcacagcac acacacgcac acacatgcac 18240
   acacagcaca caaacgcacg gcacacacac gcacacacat gcacacacag cacacacaca 18300
   aacgcacagc acacacaaac gcacagcaca cacgcacaca cagcacacac acgagcacac 18360
   agcacacaaa cgcacagcac acgcacacac atgcacacac agcacacaca ctagcacaca 18420
   gcacacacac aaagacacag cacacacatg cacacacagc acacacacgc gaacacagca 18480
   cacacgaaca cagcacacac agcacacaca caaacacagc acacacatgc acacagcaca 18540
   cgcacacaca gcacacacat gaacacagca cacagcacac acatgcacac acagcacaca 18600
   cgcatgcaca gcacacatga acacagcaca cacacaaaca cacagcacac acatgcacac 18660
   acagcacaca cactcatgcg cagcacatac atgaacacag ctcacagcac acaaacacgc 18720
   agcacacacg ttgcacacgc aagcacccac ctgcacacac acatgcgcac acacacgcac 18780
   acccccacaa aattggatga aaacaataag catatctaag caactacgat atctgtatgg 18840
   atcaggccaa agtcccgcta agattctcca atgttttcat ggtctgagcc ccgctcctgt 18900
   tcccatctcc actgcccctc ggccctgtct gtgccctgcc tctcagagga gggggctcag 18960
   atggtgcggc ctgagtgtgc ggccggcggc atttgggata cacccgtagg gtgggcgggg 19020
   tgtgtcccag gcctaattcc atctttccac catgacagag atgcccttgt gaggctggcc 19080
   tccttggcgc ctgtccccac ggcccccgca gcgtgagcca cgatgctccc cataccccac 19140
   ccattcccga tacaccttac ttactgtgtg ttggcccagc cagagtgagg aaggagtttg 19200
   gccacattgg agatggcggt agctgagcag acatgccccc acgagtagcc tgactccctg 19260
   gtgtgctcct ggaaggaaga tcttggggac ccccccaccg gagcacacct agggatcatc 19320
   tttgcccgtc tcctggggac cccccaagaa atgtggagtc ctcgggggcc gtgcactgat 19380
   gcggggagtg tgggaagtct ggcggttgga ggggtgggtg gggggcagtg ggggctgggc 19440
   ggggggagtt ctggggtagg aagtggtccc gggagatttt ggatggaaaa gtcaggagga 19500
   ttgacagcag acttgcagaa ttacatagag aaattaggaa cccccaaatt tcatgtcaat 19560
   tgatctattc cccctctttg tttcttgggg catttttcct tttttttttt tttttgtttt 19620
   ttttttaccc ctccttagct ttatgcgctc agaaaccaaa ttaaaccccc cccccatgta 19680
   acaggggggc agtgacaaaa gcaagaacgc acgaagccag cctggagacc accacgtcct 19740
   gccccccgcc atttatcgcc ctgattggat tttgtttttc atctgtccct gttgcttggg 19800
   ttgagttgag ggtggagcct cctggggggc actggccact gagccccctt ggagaagtca 19860
   gaggggagtg gagaaggcca ctgtccggcc tggcttctgg ggacagtggc tggtccccag 19920
   aagtcctgag ggcggagggg ggggttgggc agggtctcct caggtgtcag gagggtgctc 19980
   ggaggccaca ggagggggct cctggctggc ctgaggctgg ccggagggga aggggctagc 20040
   aggtgtgtaa acagagggtt ccatcaggct ggggcagggt ggccgccttc cgcacacttg 20100
   aggaaccctc ccctctccct cggtgacatc ttgcccgccc ctcagcaccc tgccttgtct 20160
   ccaggaggtc cgaagctctg tgggacctct tgggggcaag gtggggtgag gccggggagt 20220
   agggaggtca ggcgggtctg agcccacaga gcaggagagc tgccaggtct gcccatcgac 20280
   caggttgctt gggccccgga gcccacgggt ctggtgatgc catagcagcc accaccgcgg 20340
   cgcctagggc tgcggcaggg actcggcctc tgggaggttt acctcgcccc cacttgtgcc 20400
   cccagctcag cccccctgca cgcagcccga ctagcagtct agaggcctga ggcttctggg 20460
   tcctggtgac ggggctggca tgaccccggg ggtcgtccat gccagtccgc ctcagtcgca 20520
   gagggtccct cggcaagcgc cctgtgagtg ggccattcgg aacattggac agaagcccaa 20580
   agagccaaat tgtcacaatt gtggaaccca cattggcctg agatccaaaa cgcttcgagg 20640
   caccccaaat tacctgccca ttcgtcagga cacccaccca cccagtgtta tattctgcct 20700
   cgccggagtg ggtgttcccg ggggcacttg ccgaccagcc ccttgcgtcc ccaggtttgc 20760
   agctctcccc tgggccacta accatcctgg cccgggctgc ctgtctgacc tccgtgccta 20820
   gtcgtggctc tccatcttgt ctcctccccg tgtccccaat gtcttcagtg gggggccccc 20880
   tcttgggtcc cctcctctgc catcacctga agacccccac gccaaacact gaatgtcacc 20940
   tgtgcctgcc gcctcggtcc accttgcggc ccgtgtttga ctcaactcaa ctcctttaac 21000
   gctaatattt ccggcaaaat cccatgcttg ggttttgtct ttaaccttgt aacgcttgca 21060
   atcccaataa agcattaaaa gtcatgatct tctgagtgtt ccactctctg acttgggtac 21120
   tggactgcca gagggaggga aggggctgag cacctggaag caggcagagg gggatagaag 21180
   agggaagggg aaggaaggcc ttaggggtgt ggacacctct ctccgtcccc tgatcacata 21240
   catggagaaa tgagagagct ggaagccaga ctctcagact cactgtcgtg cacctgaagc 21300
   cagggggtct gggacagtgt caggcaccaa gttctcaaag atgggggtgc cacgaagggt 21360
   aggagcctgg ggggcttttt cagagaaaaa gcaaagtacc atcagtacca actccaggga 21420
   atgcctcccc caccaacagc cttagaggac tggggctggg caacctctaa aaggttctgg 21480
   aaaccgtatc tgtggctgga gatgggggct cagggccact ctcacccagg gactcagccc 21540
   ccttccaagg ttgaggctgc ccagatgtca ttagtaccac tgcctcctgc cagggctcag 21600
   cctgtaccca cctgtcccaa gggtgctggc ctctagcttt caagggtccc tgagcatggg 21660
   aggaggggta ggctgggctg ctgggagcct gg 21692

<210> 20
<211> 4112
<212> DNA
<213> Homo sapiens

<400> 20
gacaacttcc ccagataccc cgtgggcaag ttcttccaat atgacacctg gaagcagtcc 60

acccagcgcc tgcgcagggg cctgcctgcc ctcctgcgtg cccgccgggg tcacgtgctc 120

gccaaggagc tcgaggcgtt cagggaggcc aaacgtcacc gtcccctgat tgctctaccc 180

acccaagacc ccgcccacgg gggcgccccc ccagagatgg ccagcaatcg gaagtgagca 240

aaactgccgc aagtctgcag cccggcgcca ccatcctgca gcctcctcct gaccacggac 300

gtttccatca ggttccatcc cgaaaatctc tcggttccac gtccccctgg ggcttctcct 360

gacccagtcc ccgtgccccg cctccccgaa acaggctact ctcctcggcc ccctccatcg 420
ggctgaggaa gcacagcagc atcttcaaac atgtacaaaa tcgattggct ttaaacaccc 480
ttcacatacc ctccccccaa attatcccca attatcccca cacataaaaa atcaaaacat 540
taaactaacc cccttccccc ccccccacaa caaccctctt aaaactaatt ggctttttag 600
aaacacccca caaaagctca gaaattggct ttaaaaaaaa caaccaccaa aaaaaatcaa 660
ttggctaaaa aaaaaaagta ttaaaaacga attggctgag aaacaattgg caaaataaag 720
gaatttggca ctccccaccc ccctctttct cttctccctt ggactttgag tcaaattggc 780
ctggacttga gtccctgaac cagcaaagag aaaagaagga ccccagaaat cacaggtggg 840
cacgtcgctg ctaccgccat ctcccttctc acgggaattt tcagggtaaa ctggccatcc 900
gaaaatagca acaacccaga ctggctcctc actccctttt ccatcactaa aaatcacaga 960
gcagtcagag ggacccagta agaccaaagg aggggaggac agagcatgaa aaccaaaatc 1020
catgcaaatg aaatgtaatt ggcacgaccc tcacccccaa atcttacatc tcaattccca 1080
tcctaaaaag cactcatact ttatgcatcc ccgcagctac acacacacaa cacacagcac 1140
acgcatgaac acagcacaca cacgagcaca gcacacacac aaacgcacag cacacacagc 1200
acacagatga gcacacagca cacacacaaa cgcacagcac acacacgcac acacatgcac 1260
acacagcaca caaacgcacg gcacacacac gcacacacat gcacacacag cacacacaca 1320
aacgcacagc acacacaaac gcacagcaca cacgcacaca cagcacacac acgagcacac 1380
agcacacaaa cgcacagcac acgcacacac atgcacacac agcacacaca ctagcacaca 1440
gcacacacac aaagacacag cacacacatg cacacacagc acacacacgc gaacacagca 1500
cacacgaaca cagcacacac agcacacaca caaacacagc acacacatgc acacagcaca 1560
cgcacacaca gcacacacat gaacacagca cacagcacac acatgcacac acagcacaca 1620
cgcatgcaca gcacacatga acacagcaca cacacaaaca cacagcacac acatgcacac 1680
acagcacaca cactcatgcg cagcacatac atgaacacag ctcacagcac acaaacacgc 1740
agcacacacg ttgcacacgc aagcacccac ctgcacacac acatgcgcac acacacgcac 1800
acccccacaa aattggatga aaacaataag catatctaag caactacgat atctgtatgg 1860
atcaggccaa agtcccgcta agattctcca atgttttcat ggtctgagcc ccgctcctgt 1920
tcccatctcc actgcccctc ggccctgtct gtgccctgcc tctcagagga gggggctcag 1980
atggtgcggc ctgagtgtgc ggccggcggc atttgggata cacccgtagg gtgggcgggg 2040
tgtgtcccag gcctaattcc atctttccac catgacagag atgcccttgt gaggctggcc 2100
tccttggcgc ctgtccccac ggcccccgca gcgtgagcca cgatgctccc cataccccac 2160
ccattcccga tacaccttac ttactgtgtg ttggcccagc cagagtgagg aaggagtttg 2220
gccacattgg agatggcggt agctgagcag acatgccccc acgagtagcc tgactccctg 2280

gtgtgctcct ggaaggaaga tcttggggac ccccccaccg gagcacacct agggatcatc 2340

tttgcccgtc tcctggggac cccccaagaa atgtggagtc ctcgggggcc gtgcactgat 2400

gcggggagtg tgggaagtct ggcggttgga ggggtgggtg gggggcagtg ggggctgggc 2460

ggggggagtt ctggggtagg aagtggtccc gggagatttt ggatggaaaa gtcaggagga 2520

ttgacagcag acttgcagaa ttacatagag aaattaggaa cccccaaatt tcatgtcaat 2580

tgatctattc cccctctttg tttcttgggg catttttcct tttttttttt tttttgtttt 2640

ttttttaccc ctccttagct ttatgcgctc agaaaccaaa ttaaaccccc cccccatgta 2700

acaggggggc agtgacaaaa gcaagaacgc acgaagccag cctggagacc accacgtcct 2760

gccccccgcc atttatcgcc ctgattggat tttgtttttc atctgtccct gttgcttggg 2820

ttgagttgag ggtggagcct cctggggggc actggccact gagccccctt ggagaagtca 2880

gaggggagtg gagaaggcca ctgtccggcc tggcttctgg ggacagtggc tggtccccag 2940

aagtcctgag ggcggagggg ggggttgggc agggtctcct caggtgtcag gagggtgctc 3000

ggaggccaca ggagggggct cctggctggc ctgaggctgg ccggagggga aggggctagc 3060

aggtgtgtaa acagagggtt ccatcaggct ggggcagggt ggccgccttc cgcacacttg 3120

aggaaccctc ccctctccct cggtgacatc ttgcccgccc ctcagcaccc tgccttgtct 3180

ccaggaggtc cgaagctctg tgggacctct tgggggcaag gtggggtgag gccggggagt 3240

agggaggtca ggcgggtctg agcccacaga gcaggagagc tgccaggtct gcccatcgac 3300

caggttgctt gggccccgga gcccacgggt ctggtgatgc catagcagcc accaccgcgg 3360

cgcctagggc tgcggcaggg actcggcctc tgggaggttt acctcgcccc cacttgtgcc 3420

cccagctcag cccccctgca cgcagcccga ctagcagtct agaggcctga ggcttctggg 3480

tcctggtgac ggggctggca tgaccccggg ggtcgtccat gccagtccgc ctcagtcgca 3540

gagggtccct cggcaagcgc cctgtgagtg ggccattcgg aacattggac agaagcccaa 3600

agagccaaat tgtcacaatt gtggaaccca cattggcctg agatccaaaa cgcttcgagg 3660

caccccaaat tacctgccca ttcgtcagga cacccaccca cccagtgtta tattctgcct 3720

cgccggagtg ggtgttcccg ggggcacttg ccgaccagcc ccttgcgtcc ccaggtttgc 3780

agctctcccc tgggccacta accatcctgg cccgggctgc ctgtctgacc tccgtgccta 3840

gtcgtggctc tccatcttgt ctcctccccg tgtccccaat gtcttcagtg gggggccccc 3900

tcttgggtcc cctcctctgc catcacctga agacccccac gccaaacact gaatgtcacc 3960

tgtgcctgcc gcctcggtcc accttgcggc ccgtgtttga ctcaactcaa ctcctttaac 4020

gctaatattt ccggcaaaat cccatgcttg ggttttgtct ttaaccttgt aacgcttgca 4080

atcccaataa agcattaaaa gtcatgatct tc 4112

<210>  21
<211>  3598
<212>  DNA
<213>  Artificial Sequence

<220>
<223>  Synth1 - synthetic IFG2 nucleotide sequence with partial exon 8
       and 9 sequences

<400>  21
ggcggccgct taatacgact cactataggg agaacccctc cgaccgtgct tccggacaac      60

ttccccagat accccgtggg caagttcttc caatatgaca cctggaagca gtccacccag     120

cgcctgcgca ggggcctgcc tgccctcctg cgtgcccgcc ggggtcacgt gctcgccaag     180

gagctcgagg cgttcaggga ggccaaacgt caccgtcccc tgattgctct acccacccaa     240

gaccccgccc acgggggcgc ccccccagag atggccagca atcggaagtg agcaaaactg     300

ccgcaagtct gcagcccggc gccaccatcc tgcagcctcc tcctgaccac ggacgtttcc     360

atcaggttcc atcccgaaaa tctctcggtt ccacgtcccc ctggggcttc tcctgaccca     420

gtccccgtgc cccgcctccc cgaaacaggc tactctcctc ggccccctcc atcgggctga     480

ggaagcacag cagcatcttc aaacatgtac aaaatcgatt ggctttaaac acccttcaca     540

taccctcccc ccaaattatc cccaattatc cccacacata aaaaatcaaa acattaaact     600

aacccccttc cccccccccc acaacaaccc tcttaaaact aattggcttt ttagaaacac     660

cccacaaaag ctcagaaatt ggctttaaaa aaaacaacca ccaaaaaaaa tcaattggct     720

aaaaaaaaaa agtattaaaa acgaattggc tgagaaacaa ttggcaaaat aaaggaattt     780

ggcactcccc acccccctct ttctcttctc ccttggactt tgagtcaaat tggcctggac     840

ttgagtccct gaaccagcaa agagaaaaga aggaccccag aaatcacagg tgggcacgtc     900

gctgctaccg ccatctccct tctcacggga attttcaggg taaactggcc atccgaaaat     960

agcaacaacc cagactggct cctcactccc ttttccatca ctaaaaatca cagagcagtc    1020

agagggaccc agtaagacca aaggagggga ggacagagca tgaaaaccaa aatccatgca    1080

aatgaaatgt aattggcacg accctcaccc ccaaatctta catctcaatt cccatcctaa    1140

aaagcactca tactttatgc atccccgcct catgcgcagc acatacatga acacagctca    1200

cagcacacaa acacgcagca cacacgttgc acacgcaagc acccacctgc acacacacat    1260

gcgcacacac acgcacaccc ccacaaaatt ggatgaaaac aataagcata tctaagcaac    1320

tacgatatct gtatggatca ggccaaagtc ccgctaagat tctccaatgt tttcatggtc    1380

tgagccccgc tcctgttccc atctccactg cccctcggcc ctgtctgtgc cctgcctctc    1440

agaggagggg gctcagatgg tgcggcctga gtgtgcggcc ggcggcattt gggatacacc    1500

cgtagggtgg gcggggtgtg tcccaggcct aattccatct ttccaccatg acagagatgc    1560

ccttgtgagg ctggcctcct tggcgcctgt ccccacggcc cccgcagcgt gagccacgat 1620
gctccccata ccccacccat tcccgataca ccttacttac tgtgtgttgg cccagccaga 1680
gtgaggaagg agtttggcca cattggagat ggcggtagct gagcagacat gcccccacga 1740
gtagcctgac tccctggtgt gctcctggaa ggaagatctt ggggaccccc ccaccggagc 1800
acacctaggg atcatctttg cccgtctcct ggggaccccc caagaaatgt ggagtcctcg 1860
ggggccgtgc actgatgcgg ggagtgtggg aagtctggcg gttggagggg tgggtggggg 1920
gcagtggggg ctgggcgggg ggagttctgg ggtaggaagt ggtcccggga gattttggat 1980
ggaaaagtca ggaggattga cagcagactt gcagaattac atagagaaat taggaacccc 2040
caaatttcat gtcaattgat ctattccccc tctttgtttc ttggggcatt tttccttttt 2100
tttttttttt tgtttttttt ttacccctcc ttagctttat gcgctcagaa accaaattaa 2160
accccccccc catgtaacag gggggcagtg acaaaagcaa gaacgcacga agccagcctg 2220
gagaccacca cgtcctgccc cccgccattt atcgccctga ttggattttg tttttcatct 2280
gtccctgttg cttgggttga gttgagggtg gagcctcctg gggggcactg gccactgagc 2340
ccccttggag aagtcagagg ggagtggaga aggccactgt ccggcctggc ttctggggac 2400
agtggctggt ccccagaagt cctgagggcg gagggggggg ttgggcaggg tctcctcagg 2460
tgtcaggagg gtgctcggag gccacaggag ggggctcctg gctggcctga ggctggccgg 2520
aggggaaggg gctagcaggt gtgtaaacag agggttccat caggctgggg cagggtggcc 2580
gccttccgca cacttgagga accctcccct ctccctcggt gacatcttgc ccgcccctca 2640
gcaccctgcc ttgtctccag gaggtccgaa gctctgtggg acctcttggg ggcaaggtgg 2700
ggtgaggccg gggagtaggg aggtcaggcg ggtctgagcc cacagagcag gagagctgcc 2760
aggtctgccc atcgaccagg ttgcttgggc cccggagccc acgggtctgg tgatgccata 2820
gcagccacca ccgcggcgcc tagggctgcg gcagggactc ggcctctggg aggtttacct 2880
cgcccccact tgtgccccca gctcagcccc cctgcacgca gcccgactag cagtctagag 2940
gcctgaggct tctgggtcct ggtgacgggg ctggcatgac cccgggggtc gtccatgcca 3000
gtccgcctca gtcgcagagg gtccctcggc aagcgccctg tgagtgggcc attcggaaca 3060
ttggacagaa gcccaaagag ccaaattgtc acaattgtgg aacccacatt ggcctgagat 3120
ccaaaacgct tcgaggcacc ccaaattacc tgcccattcg tcaggacacc cacccaccca 3180
gtgttatatt ctgcctcgcc ggagtgggtg ttcccggggg cacttgccga ccagcccctt 3240
gcgtccccag gtttgcagct ctcccctggg ccactaacca tcctggcccg ggctgcctgt 3300
ctgacctccg tgcctagtcg tggctctcca tcttgtctcc tccccgtgtc cccaatgtct 3360
tcagtggggg gcccctcttt gggtcccctc ctctgccatc acctgaagac ccccacgcca 3420
aacactgaat gtcacctgtg cctgccgcct cggtccacct tgcggcccgt gtttgactca 3480

actcaactcc tttaacgcta atatttccgg caaaatccca tgcttgggtt ttgtctttaa 3540

ccttgtaacg cttgcaatcc caataaagca ttaaaagtca tgatcttcgg cggccgct 3598

<210> 22
<211> 578
<212> DNA
<213> Artificial Sequence

<220>
<223> CA repeat-rich region from SEQ ID NO:20

<400> 22
agctacacac acacaacaca cagcacacgc atgaacacag cacacacacg agcacagcac 60

acacacaaac gcacagcaca cacagcacac agatgagcac acagcacaca cacaaacgca 120

cagcacacac acgcacacac atgcacacac agcacacaaa cgcacggcac acacacgcac 180

acacatgcac acacagcaca cacacaaacg cacagcacac acaaacgcac agcacacacg 240

cacacacagc acacacacga gcacacagca cacaaacgca cagcacacgc acacacatgc 300

acacacagca cacacactag cacacagcac acacacaaag acacagcaca cacatgcaca 360

cacagcacac acacgcgaac acagcacaca cgaacacagc acacacagca cacacacaaa 420

cacagcacac acatgcacac agcacacgca cacacagcac acacatgaac acagcacaca 480

gcacacacat gcacacacag cacacacgca tgcacagcac acatgaacac agcacacaca 540

caaacacaca gcacacacat gcacacacag cacacaca 578

<210> 23
<211> 3598
<212> DNA
<213> Artificial Sequence

<220>
<223> Synth2 - synthetic IFG2 nucleotide sequence with partial exon 8
      and 9 sequences

<400> 23
ggcggccgct taatacgact cactataggg agaacccctc cgaccgtgct tccggacaac 60

ttccccagat accccgtggg caagttcttc caatatgaca cctggaagca gtccacccag 120

cgcctgcgca ggggcctgcc tgccctcctg cgtgcccgcc ggggtcacgt gctcgccaag 180

gagctcgagg cgttcaggga ggccaaacgt caccgtcccc tgattgctct acccacccaa 240

gaccccgccc acgggggcgc ccccccagag atggccagca atcggaagtg agcaaaactg 300

ccgcaagtct gcagcccggt gccaccatcc tgcagcctcc tcctgaccac ggacgtttcc 360

atcaggttcc atcccgaaaa tctctcggtt ccacgtcccc ctggggcttc tcctgaccca 420

gtccccgtgc cccgcctccc cgaaacaggc tactctcctc ggccccctcc atcgggctga 480

ggaagcacag cagcatcttc aaacatgtac aaaatcgatt ggctttaaac acccttcaca 540

taccctcccc ccaaattatc cccaattatc cccacacata aaaaatcaaa acattaaact 600

aacccccttc cccccccccc acaacaaccc tcttaaaact aattggcttt ttagaaacac 660

cccacaaaag ctcagaaatt ggctttaaaa aaaacaacca ccaaaaaaaa tcaattggct 720

aaaaaaaaaa agtattaaaa acgaattggc tgagaaacaa ttggcaaaat aaaggaattt 780

ggcactcccc acccccctct ttctcttctc ccttggactt tgagtcaaat tggcctggac 840

ttgagtccct gaaccagcaa agagaaaaga agggccccag aaatcacagg tgggcatgtc 900

gctgctaccg ccatctccct tctcacggga attttcaggg taaactggcc atccgaaaat 960

agcaacaacc cagactggct cctcactccc ttttccatca ctaaaaatca cagagcagtc 1020

agagggaccc agtaagacca aaggagggga ggacagagca tgaaaaccaa aatccatgca 1080

aatgaaatgt aattggcacg accctcaccc ccaaatctta catctcaatt cccatcctaa 1140

aaagcactca tactttatgc atccccgcct catgcgcagc acatacatga acacagctca 1200

cagcacacaa acacgcagca cacacgttgc acacgcaagc acccacctgc acacacacat 1260

gcgcacacac acgcacaccc ccacacaatt agatgaaaac aataagcata tctaagcaac 1320

tacgatatct gtatggatca ggccaaagtc ccgctaagat tctccaatgt tttcatggtc 1380

tgagcccccc tcctgttccc atctccactg cccctcggcc ctgtctgtgc cctgcctctc 1440

agaggagggg gctcagatgg tgcggcctga gtgtgcggcc ggcggcattt gggatacacc 1500

cgtagggtgg gcggggtgtg tcccaggcct aattccatct ttccaccatg acagagatgc 1560

ccttgtgagg ctggcctcct tggcgcctgt ccccacggcc cccgcagcgt gagccacgat 1620

gctccccata ccccacccat tcccgataca ccttacttac tgcgtgttgg tccagccaga 1680

gtgaggaagg agtttggcca cattggagat ggcggtagct gagcagacat gcccccacga 1740

gtagcctgac tccctggtgt gctcctggaa ggaagatctt ggggaccccc ccaccggagc 1800

acacctaggg atcatctttg cccatctcct ggggaccccc caagaaatgt ggagtcctcg 1860

ggggccgtgc actgatgtgg ggagtgtggg aagtctggcg gttggagggg tgggtggggg 1920

gcagtggggg ctgggcgggg ggagttctgg ggtaggaagt ggtcccggga gattttggat 1980

ggaaaagtca ggaggattga cagcagactt gcagaattac atagagaaat taggaacccc 2040

caaatttcat gtcaattgat ctattccccc tctttgtttc ttggggcagt tttccttttt 2100

tttttttttt tgtttttttt ttacccctcc ttagctttat gcgctcagaa accaaattaa 2160

accccccccc catgtaacag gggggcagtg acaaaagcaa gaacgcacga agccagcctg 2220

gagaccacca cgtcctgccc cccgccattt atcgccctga ttggattttg tttttcatct 2280

gtccctgttg cttgggttga gttgagggtg gagcctcctg gggggcactg gccactgagc 2340

ccccttggag aagtcagagg ggagtggaga aggccactgt ccggcctggc ttctggggac 2400

agtggctggt ccccagaagt cctgagggcg gagggggggg ttgggcaggg tctcctcagg 2460

tgtcaggagg gtgctcggag gccacaggag ggggctcctg gctggcctga ggctggccgg 2520

aggggaaggg gctaacaggt gtgtaaacag agggttccat caggctgggg cagggtggcc 2580

gccttccgca cacttgagga accctcccct ctccctcggt gacatcttgc ccgcccctca 2640

gcaccctgcc ttgtctccag gaggtccgaa gctctgtggg acctcttggg ggcaaggtgg 2700

ggtgaggccg gggagtaggg aggtcaggcg ggtctgagcc cacagagcag gagagctgcc 2760

aggtctgccc atcgaccagg ttgcttgggc cccggagccc acgggtctgg tgatgccata 2820

gcagccacca ccgcggcacc tagggctgcg gcagggactc ggcctctggg aggtttacct 2880

cgcccccact tgtgccccca gctcagcccc cctgcacgca gcccgactag cagtctagag 2940

gcctgaggct tctgggtcct ggtgacgggg ctggcatgac cccgggggtc gtccatgcca 3000

gtccgcctca gtcgcagagg gtccctcggc aagcaccctg tgagtgggcc attcggaaca 3060

ttggacagaa gcccaaagag ccaaattgtc acaattgtgg aacccacatt ggcctgagat 3120

ccaaaacgct tcgaggcacc ccaaattacc tgcccattcg tcaggacacc cacccaccca 3180

gtgttatatt ctgcctcgcc ggagtgggtg ttcccggggg cacttgccga ccggcccctt 3240

gcgtccccag gtttgcagct ctcccctggg ccactaacca tcctggcccg ggctgcctgt 3300

ctgacctccg tgcctagtcg tggctctcca tcttgtctcc tccccgtgtc cccaatgtct 3360

tcagtggggg gccccctctt gggtcccctc ctctgccatc acctgaagac ccccacgcca 3420

aacactgaat gtcacctgtg cctgccgcct cggtccacct tgcggcccgt gtttgactca 3480

actcagctcc tttaacgcta atatttccgg caaaatccca tgcttgggtt ttgtctttaa 3540

ccttgtaacg cttgcaatcc caataaagca ttaaaagtca tgatcttcgg cggccgct 3598

<210> 24
<211> 29
<212> DNA
<213> Artificial Sequence

<220>
<223> Synthetic primer for genotyping SEQ ID NO:8

<400> 24
gtccctgaac cagcaaagag aaaagaagg 29

<210> 25
<211> 82
<212> DNA
<213> Artificial Sequence

<220>
<223> Synthetic amplicon sequence from IGF2 comprising SEQ ID NO:8

<400> 25
tggacttgag tccctgaacc agcaaagaga aaagaaggac cccagaaatc acaggtgggc 60

acgtcgctgc taccgccatc tc                                                82

<210>  26
<211>  30
<212>  DNA
<213>  Artificial Sequence

<220>
<223>  Synthetic primer for genotyping SEQ ID NO:8 with dNTP label

<220>
<221>  modified_base
<222>  (30)..(30)
<223>  3′ A labeled with dR6G

<400>  26
gtccctgaac cagcaaagag aaaagaagga                                        30

<210>  27
<211>  30
<212>  DNA
<213>  Artificial Sequence

<220>
<223>  Synthetic primer for genotyping SEQ ID NO:8 with dNTP label

<220>
<221>  modified_base
<222>  (30)..(30)
<223>  3′ G labeled with dR110

<400>  27
gtccctgaac cagcaaagag aaaagaaggg                                        30